# EUROPÄISCHE PATENTSCHRIFT

(11) **EP 0 986 564 B1**
(45) Veröffentlichungstag und Bekanntmachung des Hinweises auf die Patenterteilung: **15.10.2003**
(21) Anmeldenummer: 98929269.3
(22) Anmeldetag: 29.04.1998
(51) Int. Cl.: C07D 491/14, A61K 31/44

(54) **6-SUBSTITUIERTE 1,2,4A,5A,8A,8B-HEXAHYDRO- UND 1,2,3,4,4A,5A,8A,8B-OKTAHYDRO-6H-PYRROLO[3',4':4,5]FURO[3,2-B]PYRID-8(7H)-ON DERIVATE UND IHRE VERWENDUNG ZUR BEKÄMPFUNG VON ENDOPARASITEN**
6-SUBSTITUTED 1,2,4A,5A,8A,8A HEXAHYDRO- AND 1,2,3,4,4A,5A,8A,8B-OCTAHYDRO-6H-PYRROLO[3',4':4,5]FURO[3,2-b]PYRID-8(7H)-ONE DERIVATIVES AND THEIR USE IN COMBATTING ENDOPARASITES
DERIVES DE 1,2,4A,5A,8A,8B-HEXAHYDRO- ET 1,2,3,4,4A,5A,8A,8B-OCTAHYDRO-6H-PYRROLO[3',4':4,5]FURO[3,2-B]PYRID-8(7H)-ONE 6-SUBSTITUES ET LEUR UTILISATION POUR LUTTER CONTRE DES ENDOPARASITES

(30) Priorität: 12.05.1997 DE 19719839
(43) Veröffentlichungstag der Anmeldung: 22.03.2000
(73) Patentinhaber: Bayer Aktiengesellschaft, 51368 Leverkusen (DE)
(72) Erfinder: JESCHKE, Peter, D-51373 Leverkusen (DE); HARDER, Achim, D-51109 Köln (DE); MENCKE, Norbert, D-51381 Leverkusen (DE); VON SAMSON-HIMMELSTJERNA, Georg, D-42657 Solingen (DE)
(86) Internationale Anmeldenummer: EP9802511
(87) Internationale Veröffentlichungsnummer: WO98051688

(56) Entgegenhaltungen:
- DE-A- 19 538 960

## Beschreibung

Die vorliegende Erfindung betrifft 6-substituierte 1,2,4a,5a,8a,8b-Hexahydro- und 1,2,3,4,4a,5a,8a,8b-Oktahydro-6H-pyrrolo[3',4':4,5]furo[3,2-b]pyrid-8(7H)-on Derivate, ihre Herstellung und ihre Verwendung zur Bekämpfung von Endoparasiten.

4a,5a,8a,8b-Tetrahydro-6H-pyrrolo[3',4':4,5]furo[3,2-b]pyrid-6,8(7H)-dion Derivate, ihre Herstellung und ihre Verwendung zur Bekämpfung von Endoparasiten ist Gegenstand der vorgängigen, veröffentlichten Patentanmeldung DE-OS 19 538 960A1.

Es ist jedoch nichts über die neuen 6-substituierten 1,2,4a,5a,8a,8b-Hexahydro- und 1,2,3,4,4a,5a,8a,8b-Oktahydro-6H-pyrrolo[3',4':4,5]furo[3,2-b]pyrid-8(7H)-on Derivate bekannt geworden.

Die vorliegende Erfindung betrifft:
1. 6-Substituierte 1,2,4a,5a,8a,8b-Hexahydro- und 1,2,3,4,4a,5a,8a,8b-Oktahydro-6H-pyrrolo[3',4':4,5]furo[3,2-b]pyrid-8(7H)-on Derivate der allgemeinen Formel (I) und deren Salze, 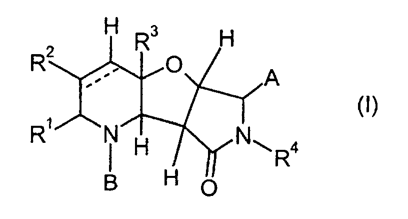 in welcher
   - R¹: für Wasserstoff, geradkettiges oder verzweigtes C₁₋₄-Alkyl oder C₃₋₆-Cycloalkyl steht,
   - R²: für Wasserstoff, geradkettiges oder verzweigtes C₁₋₄-Alkyl, Halogen-C₁₋₄-alkyl, Hydroxy-C₁₋₄-alkyl, C₁₋₄-Alkanoyloxy-C₁₋₄-alkyl, C₁₋₂-Alkoxyalkyl, C₁₋₂-Alkylthioalkyl, C₁₋₂-Alkylsulfinylalkyl, C₁₋₂-Alkylsulfonylalkyl, Amino-C₁₋₂-alkyl, C₁₋₆-Alkylaminoalkyl, C₁₋₆-Dialkylaminoalkyl, C₃₋₆-Cycloalkylaminoalkyl, C₃₋₆-Cycloalkyl, C₁₋₄-Alkoxycarbonyl stehen,
   - R³: für Wasserstoff, geradkettiges oder verzweigtes C₁₋₄-Alkyl steht,
   - R⁴: geradkettiges oder verzweigtes C₁₋₆-Alkyl, Halogen-C₁₋₆-alkyl, C₁₋₄-Alkoxycarbonyl-C₁₋₄-alkyl, C₂₋₆-Alkenyl, C₃₋₆-Cycloalkyl, C₃₋₆-Cycloalkyl-C₁₋₂-alkyl, Aryl, Aryl-C₁₋₂-alkyl, Pyridyl, Thiazolyl, N-Morpholinyl, 2-Chlorpyrid-5-yl-methyl und Chlorthiazol-5-yl-methyl, die gegebenenfalls durch Reste aus der Reihe Halogen, Halogen-C₁₋₄-alkyl, Amino, Hydroxy, Nitro, Cyan, C₁₋₄-Alkoxy, C₁₋₂-Alkylendioxy, Halogen-C₁₋₄-alkoxy, C₁₋₄-Alkylthio, Halogen-C₁₋₄-alkylthio, C₁₋₄-Alkylsulfonyl, C₁₋₄-Alkylamino, C₁₋₄-Dialkylamino, C₁₋₄-Alkylcarbonyl, C₁₋₄-Alkoxycarbonyl substituiert sein können, steht,
   - A: für Hydroxy, C₁₋₆-Alkyl, C₂₋₆-Alkenyl, C₂₋₆-Alkinyl, C₃₋₆-Cycloalkyl, C₃₋₆-Cycloalkyl-C₁₋₂-alkyl, C₁₋₆-Alkoxy, C₂₋₆-Alkenyloxy, C₂₋₆-Alkinyloxy, C₁₋₄-Alkoxycarbonyl, Mercapto, C₁₋₆-Alkylthio, Cyan, Carbamoyl, Thiocarbamoyl, Aryl-C₁₋₂-alkyloxy, Furyl, die gegebenenfalls durch Reste aus der Reihe Halogen, C₁₋₄-Alkyl, Halogen-C₁₋₄-alkyl, Amino, Hydroxy, Nitro, Cyan, C₁₋₄-Alkoxy, C₁₋₂-Alkylendioxy, Halogen-C₁₋₄-alkoxy, C₁₋₄-Alkylthio, Halogen-C₁₋₄-alkylthio, C₁₋₄-Alkylsulfonyl, C₁₋₄-Alkylamino, C₁₋₄-Dialkylamino, substituiert sein können, steht, oder gegebenenfalls für einen Rest aus der Gruppe A¹, A² und A³ 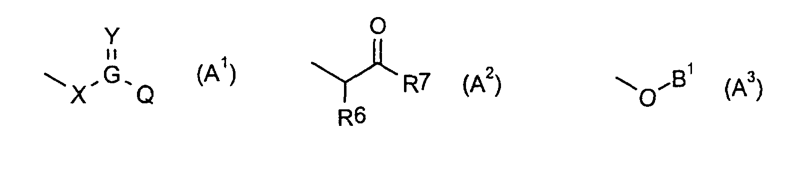 worin
   X für Sauerstoff steht, 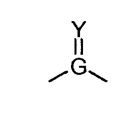 für Carboxy oder Sulfonyl steht,
   Q für geradkettiges oder verzweigtes C₁₋₆-Alkyl, C₂₋₆-Alkenyl, C₂₋₆-Alkinyl, C₃₋₆-Cycloalkyl, C₃₋₆-Cycloalkoxy, C₁₋₆-Alkoxy, C₂₋₆-Alkenyloxy, C₂₋₆-Alkinyloxy, C₁₋₄-Alkylamino, C₁₋₄-Dialkylamino eine über Stickstoff verknüpfte cyclische Aminogruppe bedeuten kann, Aryl, Aryl-C₁₋₂-alkyl, Aryl-C₁₋₂-alkyloxy, die gegebenenfalls substituiert sind, steht,
   R⁶ für Wasserstoff oder Methyl, steht,
   R⁷ für geradkettiges oder verzweigtes C₁₋₆-Alkyl, C₂₋₆-Alkenyl, C₃₋₆-Cycloalkyl, Aryl, die gegebenenfalls substituiert sind, steht,
   R⁶ und R⁷ gemeinsam mit den Atomen, an die sie gebunden sind, für einen 5-, 6- oder 7-gliedrigen carbocyclischen Ring stehen, der gegebenenfalls durch C₁₋₄-Alkyl substituiert sein kann, steht,
   - B: für Wasserstoff, geradkettiges oder verzweigtes C₁₋₆-Alkyl, C₁₋₄-Alkanoyloxyalkyl, C₁₋₂-Alkoxyalkyl, Amino-C₁₋₆-alkyl, C₁₋₆-Alkylamino-C₁₋₆-alkyl, C₁₋₆-Dialkylamino-C₁₋₆-alkyl, C₂₋₆-Alkenyl, C₂₋₆-Alkinyl, Carbamoyl-C₁₋₄-alkyl, Carboxyl-C₁₋₄-alkyl, C₁₋₄-Alkoxydicarbonyl, Aryl, Aryl-C₁₋₂-alkyl, Pyridyl, Pyrimidyl, Pyrrolidyl, Imidazolyl, Thiazolyl, N-Morpholinyl, Pyridylmethyl und Thiazolylmethyl, die gegebenenfalls durch Reste aus der Reihe Halogen, C₁₋₄-Alkyl, Halogen-C₁₋₄-alkyl, Amino, Hydroxy, Nitro, Cyan, C₁₋₄-Alkoxy, C₁₋₂-Alkylendioxy, Halogen-C₁₋₄-alkoxy, C₁₋₄-Alkylthio, Halogen-C₁₋₄-alkylthio, C₁₋₄-Alkylsulfonyl, C₁₋₄-Alkylamino, C₁₋₄-Dialkylamino, C₁₋₄-Alkylcarbonyl, C₁₋₄-Alkoxycarbonyl substituiert sein können, steht, oder gegebenenfalls für einen Rest aus der Gruppe B¹, B², B³ und B⁴ steht 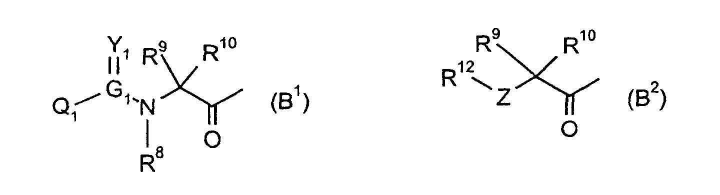 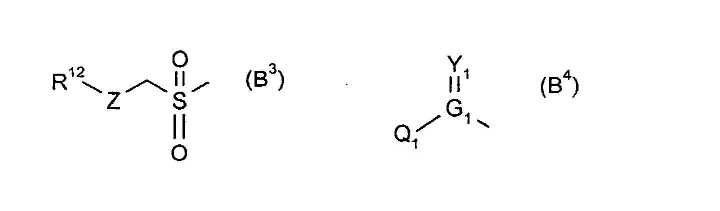 worin
   R⁸ für Wasserstoff, geradkettiges oder verzweigtes C₁₋₆-Alkyl, C₃₋₆-Cycloalkyl steht, oder
   R⁸ und R⁹ gemeinsam mit den Atomen, an die sie gebunden sind, für einen 5- oder 6-gliedrigen Ring stehen, der gegebenenfalls durch Schwefel unterbrochen sein kann und gegebenenfalls durch Hydroxy, tert.-Butoxy, Benzyloxy substituiert ist, stehen,
   R⁹ für Wasserstoff, geradkettiges oder verzweigtes C₁₋₆-Alkyl steht,
   R¹⁰ für Wasserstoff oder Methyl steht, 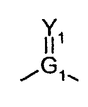 für Carbonyl, -C=CH-NO₂, -C=CH-CN, -C=N-R¹¹, Sulfonyl, steht,
   R¹¹ Halogen-C₁₋₄-alkylcarbonyl, C₁₋₄-Alkylsulfonyl, Nitro oder Cyan steht, und
   Q¹ für einen Rest aus der Gruppe G¹ und G² 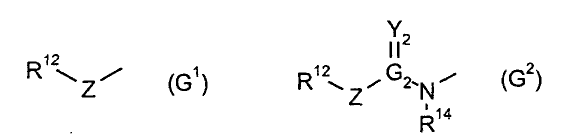 worin 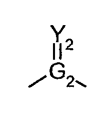 Carbonyl oder Sulfonyl bedeuten kann,
   Z für Sauerstoff oder -NR¹³ steht,
   R¹² für den Fall, dass Z für Stickstoff steht, eine über ein Stickstoffatom verknüpfte cyclische Aminogruppe, die gegebenenfalls durch Reste aus der Reihe Halogen, C₁₋₄-Alkyl, Hydroxy-C₁₋₄-alkyl, Amino-C₁₋₄-alkyl, C₁₋₄-Alkylamino-C₁₋₄-alkyl, C₁₋₄-Dialkylamino-C₁₋₄-alkyl, Amino, Hydroxy, C₁₋₄-Alkoxy, C₁₋₄-Alkylcarbonyl, C₁₋₄-Alkoxycarbonyl substituiert sein können,
   R¹² und R¹³ unabhängig voneinander für Wasserstoff, geradkettiges oder verzweigtes C₁₋₆-Alkyl, C₂₋₄-Alkenyl, C₂₋₄-Alkinyl, C₃₋₆-Cycloalkyl, Pyridylmethyl und Thiazolylmethyl, die gegebenenfalls durch Reste aus der Reihe Halogen, C₁₋₄-Alkyl, Hydroxy-C₁₋₄-alkyl, Amino-C₁₋₄-alkyl, C₁₋₄-Alkylamino-C₁₋₄-alkyl, C₁₋₄-Dialkylamino-C₁₋₄-alkyl, Amino, Hydroxy, C₁₋₄-Alkoxy, C₁₋₄-Alkylcarbonyl, C₁₋₄-Alkoxycarbonyl substituiert sein können, stehen, oder
   R¹² und R¹³ gemeinsam mit dem angrenzenden N-Atom für ein carbocyclisches 5-, 6- oder 7-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O, -N=, -NR¹⁵- oder durch quarternisierten Stickstoff unterbrochen sein kann und gegebenenfalls durch C₁₋₄-Alkyl, Hydroxy-C₁₋₄-alkyl, Amino-C₁₋₄-alkyl, C₁₋₄-Alkylamino-C₁₋₄-alkyl, C₁₋₄-Dialkylamino-C₁₋₄-alkyl, Amino, Hydroxy, C₁₋₄-Alkoxy, C₁₋₄-Alkylcarbonyl, C₁₋₄-Alkoxycarbonyl, Halogen substituiert ist, stehen,
   R¹⁴ für Wasserstoff oder C₁₋₄-Alkyl steht,
   R¹⁵ für Wasserstoff, geradkettiges oder verzweigtes C₁₋₆-Alkyl, C₂₋₆-Alkenyl, C₂₋₆-Alkinyl, C₃₋₆-Cycloalkyl, C₁₋₄-Alkoxycarbonyl, C₁₋₄-Alkylcarbonyl, C₃₋₆-Cycloalkylcarbonyl, Cyan, Aryl, Aryl-C₁₋₂-alkyl, Pyridyl und Thiazolyl, Pyridylmethyl, Thiazolylmethyl, die gegebenenfalls durch Reste aus der Reihe Halogen, C₁₋₄-Alkyl, Halogen-C₁₋₄-alkyl, Amino, Hydroxy, Cyan, C₁₋₄-Alkoxy, C₁₋₂-Alkylendioxy, Halogen-C₁₋₄-alkoxy, C₁₋₄-Alkylthio, Halogen-C₁₋₄-alkylthio, C₁₋₄-Alkylsulfonyl, C₁₋₄-Alkylamino, C₁₋₄-Dialkylamino, C₁₋₄-Alkylcarbonyl, C₁₋₄-Alkoxycarbonyl substituiert sein können, steht,
   sowie deren optische Isomere und Racemate,
   wobei "Aryl" in den Definitionen für einen ein-, zwei- oder mehrwertigen aromatischen Rest, ausgewählt aus Phenyl, Naphthyl, Tetrahydronaphthyl, Indanyl und Fluorenyl steht
   und wobei, soweit nicht anders angegeben, die gegebenenfalls substituierten Reste einen oder mehrere gleiche oder verschiedene Substituenten tragen können, ausgewählt aus: Alkyl mit 1 bis 4 Kohlenstoffatomen, Alkoxy mit 1 bis 4 Kohlenstoffatomen, Alkylthio, Halogenalkyl mit 1 bis 5 Halogenatomen, wobei die Halogenatome gleich odeer verschieden sind, Halogen, Cyan, Nitro, Amino, Monoalkyl- und Dialkylamino mit 1 bis 4 Kohlenstoffatomen je Alkylgruppe, Alkylcarbonylreste, Alkoxycarbonyl mit 2 bis 4 Kohlenstoffatomen, Alkylsulfinyl mit 1 bis 4 Kohlenstoffatomen, Halogenalkylsulfinyl mit 1 bis 4 Kohlenstoffatomen und 1 bis 5 Halogenatomen, Halogenalkylsulfonyl mit 1 bis 4 Kohlenstoffatomen und 1 bis 5 Halogenatomen, Arylsulfonyl mit 6 oder 10 Arylkohlenstoffatomen, Trialkylsilyl mit 1 bis 4 Kohlenstoffatomen, Acyl, Aryl, Aryloxy, die ihrerseits einen der obengenannten Substituenten tragen können sowie dem Formiminorest (-HC=N-O-Alkyl).
   Die Verbindungen der Formel (I) können in Abhängigkeit von der Art der Substituenten als geometrische und/oder optische Isomere oder Isomerengemische unterschiedlicher Zusammensetzung auftreten. Die Erfindung betrifft sowohl die reinen Isomeren als auch die Isomerengemische.
   In der Formel (I) kann anstelle der gestrichelten Linie eine Einfachbindung oder eine Doppelbindung, zwischen dem Kohlenstoffatom, das den Substituenten R² trägt, und dem benachbarten Kohlenstoffatom, stehen.
2. Verfahren zur Herstellung der 6-Hydroxy-1,2,4a,5a,8a,8b-hexahydro-6H-pyrrolo[3',4':4,5]furo[3,2-b]pyrid-8(7H)-on und/oder 6-Hydroxy-1,2,3,4,-4a,5a,8a,8b-oktahydro-6H-pyrrolo[3',4':4,5]furo[3,2-b]pyrid-8(7H)-on Derivate der allgemeinen Formeln (Ia) und (Ib) sowie deren Salze 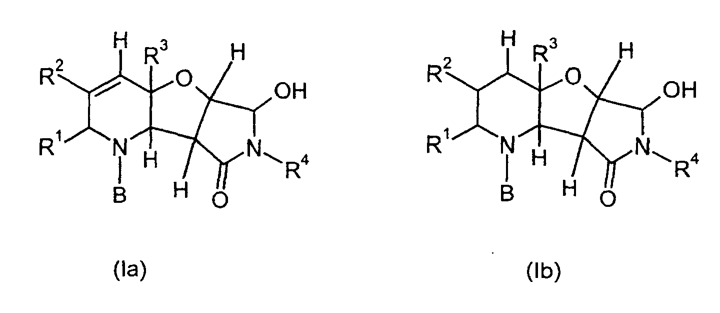 in welchen
   R¹, R², R³, R⁴ und B die in Punkt 1 angegebene Bedeutung besitzen, dadurch gekennzeichnet,
   dass man die 1,2,4a,5a,8a,8b-Hexahydro-6H-pyrrolo[3',4':4,5]furo[3,2-b]pyridin-6,8(7H)-dion
   und/oder1,2,3,4,4a,5a,8a,8b-Oktahydro-6H-pyrrolo[3',4':4,5]furo[3,2-b]pyridin-6,8(7H)-dion Derivate der allgemeinen Formeln (IIa) und (IIb) sowie deren Salze 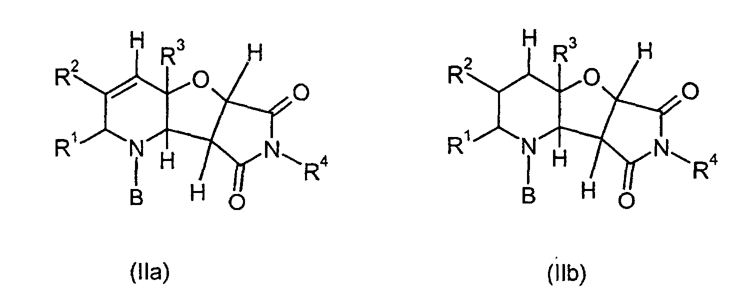 in welchen
   R¹, R², R³, R⁴ und B die in Punkt 1 angegebene Bedeutung besitzen, in Gegenwart eines geeigneten Verdünnungsmittels hydriert,
   oder indem man in einem ersten Reaktionsschritt
   die 1,2,4a,5a,8a,8b-Hexahydro-6H-pyrrolo[3',4':4,5]furo[3,2-b]pyridin-6,8-(7H)-dion und/oder 1,2,3,4,4a,5a,8a,8b-Oktahydro-6H-pyrrolo[3',4':4,5]furo[3,2-b]pyridin-6,8(7H)-dion Derivate der allgemeinen Formeln (IIc) und (IId) sowie deren Salze 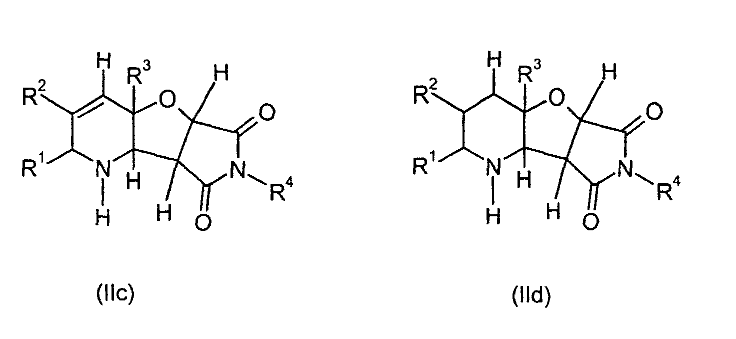 in welchen
   R¹, R², R³ und R⁴ die in Punkt 1 angegebene Bedeutung besitzen,
   in Gegenwart eines geeigneten Verdünnungsmittels zu 6-Hydroxy-1,2,4a,-5a,8a,8b-hexahydro-6H-pyrrolo[3',4':4,5]furo[3,2-b]pyrid-8(7H)-on und/oder 6-Hydroxy-1,2,3,4,4a,5a,8a,8b-oktahydro-6H-pyrrolo[3',4':4,5]furo[3,2-b]pyrid-8(7H)-on Derivaten der allgemeinen Formeln (Ic) und (Id) 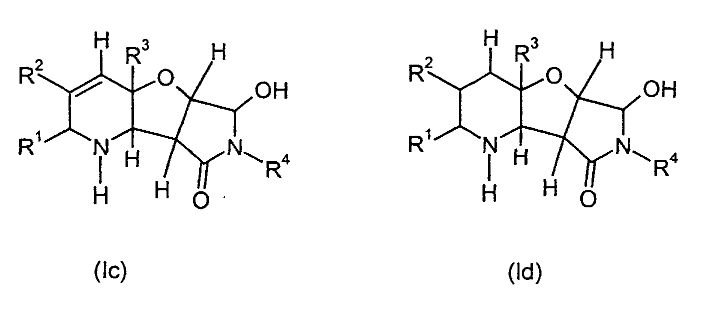 in welchen
   R¹, R², R³ und R⁴ die in Punkt 1 angegebene Bedeutung besitzen,
   hydriert,
   oder indem man zur selektiven Darstellung der Derivate der allgemeinen Formel (IIa) und deren Salze in einem ersten Reaktionsschritt
   die 4a,5a,8a,8b-Tetrahydro-6H-pyrrolo[3',4':4,5]furo[3,2-b]pyridin-6,8(7H)-dion Derivate der allgemeinen Formel (III) und deren Salze, 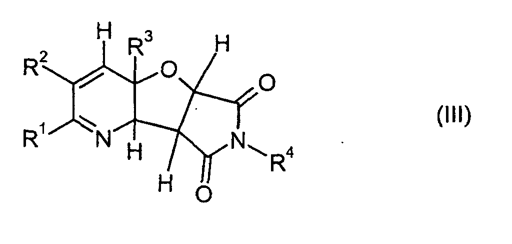 in welcher
   R¹, R², R³ und R⁴ die in Punkt 1 angegebene Bedeutung besitzen,
   in Gegenwart eines geeigneten Verdünnungsmittels zu 6-Hydroxy-1,2,4a,5a,8a,8b-hexahydro-6H-pyrrolo[3',4':4,5]furo[3,2-b]pyrid-8(7H)-on Derivaten der allgemeinen Formel (Ic) 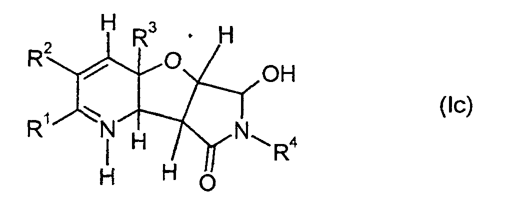 in welcher
   R¹, R², R³ und R⁴ die in Punkt 1 angegebene Bedeutung besitzen,
   hydriert, und nachfolgend in einem zweiten Reaktionsschritt die auf diese Weise erhaltenen Derivate der allgemeinen Formeln (Ic) und (Id) 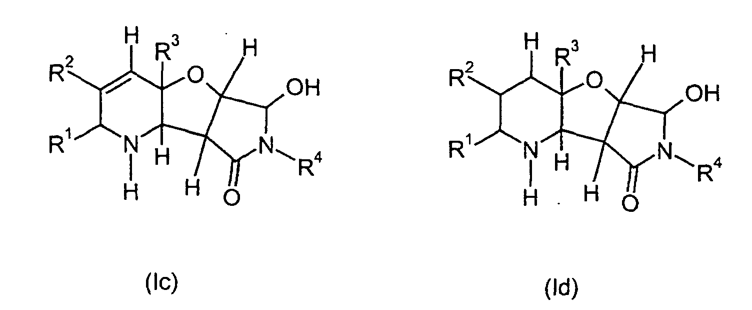 in welchen
   R¹, R², R³ und R⁴ die in Punkt 1 angegebene Bedeutung besitzen,
   a) mit Verbindungen der allgemeinen Formel (IV)

      B - E (IV)

      in welcher
      - B: die weiter oben angegebene Bedeutung hat, und
      - E: für eine elektronenziehende Abgangsgruppe steht,
      gegebenenfalls in Gegenwart von Verdünnungsmitteln und gegebenenfalls in Gegenwart eines basischen Reaktionshilfsmittels umsetzt, oder
   b) mit Verbindungen der allgemeinen Formel (V) 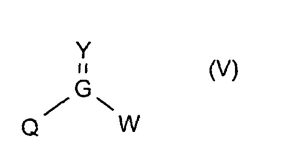 in welcher 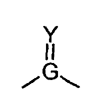 und Q die in Punkt 1 angegebene Bedeutung haben, und
      - W: für eine geeignete Abgangsgruppe, wie beispielsweise Halogen, Alkoxy, Alkylthio oder Aryloxy steht,
      gegebenenfalls in Gegenwart eines Katalysators, gegebenenfalls in Gegenwart eines basischen Reaktionshilfsmittels und gegebenenfalls in Gegenwart von Verdünnungsmitteln umsetzt, oder indem man zur Darstellung der 6-Hydroxy-1,2,4a,5a,8a,8b-hexahydro-6H-pyrrolo[3',4':4,5]furo[3,2-b]pyrid-(7H)-on und/oder 6-Hydroxy-1,2,3,4,-4a,5a,8a,8b-oktahydro-6H-pyrrolo[3',4':4,5]furo[3,2-b]pyrid-7H)-on Derivate der allgemeinen Formeln (Ia) und (Ib) sowie deren Salze,
      in denen die Gruppe 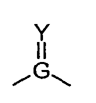 für Carbonyl steht, die Verbindungen der Formel Ic oder Id
   c) mit einem Carbonsäureanhydrid der allgemeinen Formel (VI)

      (Q-C=O)₂O (VI)

      in welcher
      - Q: die unter Punkt 1 angegebene Bedeutung hat,
      gegebenenfalls in Gegenwart eines Katalysators, gegebenenfalls in Gegenwart von Verdünnungsmitteln umsetzt, oder indem man die Verbindungen der Formel (Ic) oder (Id)
   d) mit Aminosäurederivaten der allgemeinen Formel (VII) 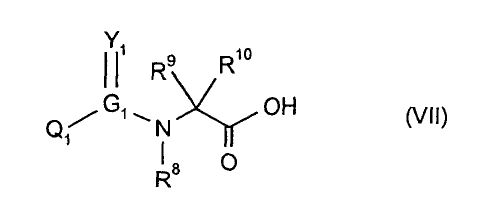 in welcher 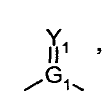 Q¹, R⁸, R⁹ und R¹⁰ die in Punkt 1 angegebene Bedeutung haben,
      gegebenenfalls in Gegenwart von Kupplungsreagenzien und in Gegenwart eines basischen Reaktionshilfsmittels, gegebenenfalls in Gegenwart von Verdünnungsmitteln umsetzt, oder indem man die Verbindungen der Formel (Ic) oder (Id)
   e) mit Verbindungen der allgemeinen Formeln (VIII) oder (IX) 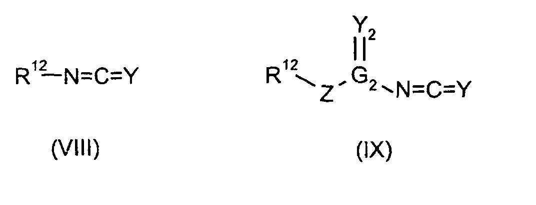 in welchen 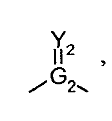 Z und R¹² die in Punkt 1 angegebene Bedeutung haben,
      - Y: für Sauerstoff oder Schwefel steht,
      gegebenenfalls in Gegenwart eines Katalysators, gegebenenfalls in Gegenwart von Verdünnungsmitteln umsetzt.
   Die Erfindung betrifft ferner:
3. Verfahren zur Herstellung von 6-substituierten 1,2,4a,5a,8a,8b-Hexahydro-6H-pyrrolo[3',4':4,5]furo[3,2-b]pyrid-8(7H)-on und/oder 6-substituierte 1,2,-3,4,4a,5a,8a,8b-Oktahydro-6H-pyrrolo[3',4':4,5]furo[3,2-b]pyrid-8(7H)-on Derivate der allgemeinen Formeln (Ie) und (If) sowie deren Salze 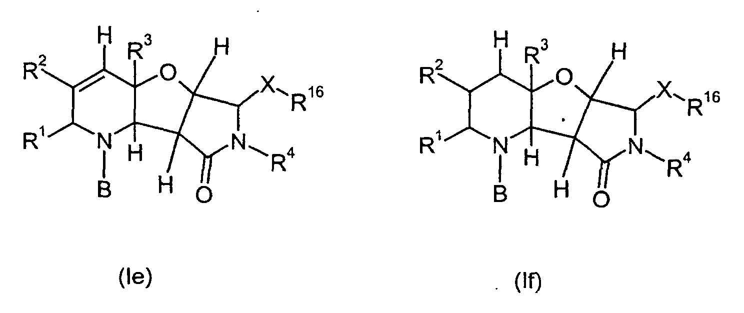 in welchen
   - R¹, R², R³, R⁴, X und B: die in Punkt 1 angegebene Bedeutung besitzen,
   - R ¹⁶: für C₁₋₆-Alkyl, C₂₋₆-Alkenyl, C₂₋₆-Alkinyl, Aryl-C₁₋₂-alkyl, die gegebenenfalls durch Reste aus der Reihe Halogen, C₁₋₄-Alkyl, Halogen-C₁₋₄-alkyl, Amino, Hydroxy, Nitro, Cyan, C₁₋₄-Alkoxy, C₁₋₂-Alkylendioxy, Halogen-C₁₋₄-alkoxy, C₁₋₄-Alkylthio, Halogen-C₁₋₄-alkylthio, C₁₋₄-Alkylsulfonyl, C₁₋₄-Alkylamino, C₁₋₄-Dialkylamino, substituiert sind, oder
   gegebenenfalls für die Gruppe 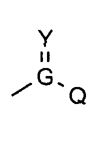 steht,
   in welcher
   G, Y und Q die in Punkt 1 angegebene Bedeutung haben,
   sowie deren optische Isomere und Racemate,
   dadurch gekennzeichnet, dass man
   a) die gemäß Punkt 2 erhältlichen Derivate der allgemeinen Formeln (Ia) und (Ib) sowie deren Salze 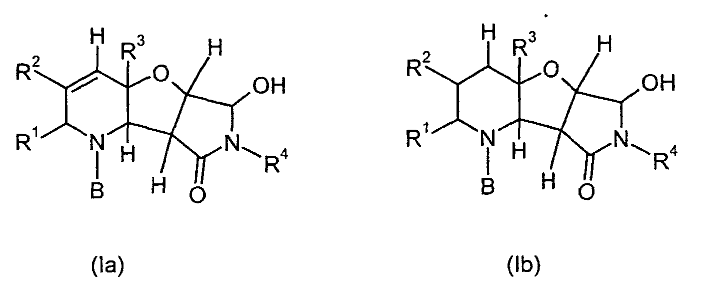 in welchen
      R¹, R², R³, R⁴ und B die in Punkt 1 angegebene Bedeutung besitzen, mit Verbindungen der allgemeinen Formel (X)

      H-X-R¹⁶ (X)

      in welcher
      - R¹⁶ und X: die weiter oben angegebene Bedeutung besitzen,
      gegebenenfalls in Gegenwart einer Säure, gegebenenfalls in Gegenwart von Verdünnungsmitteln umsetzt, oder indem man
   b) zur Darstellung der 6-substituierten 1,2,4a,5a, 8a,8b-Hexahydro-6H-pyrrolo[3',4':4,5]furo[3,2-b]pyrid-8(7H)-on und/oder 6-substituierten 1,2,3,4,4a,5a,8a,8b-Oktahydro-6H-pyrrolo[3',4':4,5]furo[3,2-b]pyrid-8(7H)-on Derivate der allgemeinen Formeln (Ie) und (If) sowie deren Salze,
      in der für den Rest R¹⁶ die Gruppe 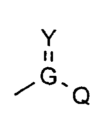 steht,
      in welcher 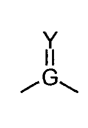 und Q die in Punkt 1 angegebene Bedeutung haben,
      mit Verbindungen der allgemeinen Formel (V) 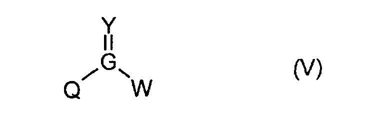 in welcher 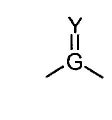 und Q die in Punkt 1 angegebene Bedeutung haben, und
      - W: für eine geeignete Abgangsgruppe steht,
      gegebenenfalls in Gegenwart eines Katalysators, gegebenenfalls in Gegenwart eines basischen Reaktionshilfsmittels und gegebenenfalls in Gegenwart von Verdünnungsmitteln umsetzt, oder indem man
   c) zur Darstellung der Derivate der allgemeinen Formeln (Ie) und (If) sowie deren Salze,
      in denen die Gruppe 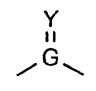 für Carbonyl steht,
      mit einem Carbonsäureanhydrid der allgemeinen Formel (VI)

      (Q-C=O)₂O (VI)

      in welcher
      - Q: die in Punkt 1 angegebene Bedeutung hat, gegebenenfalls in Gegenwart eines Katalysators, gegebenenfalls in Gegenwart von Verdünnungsmitteln umsetzt, oder indem man
   d) mit Verbindungen der allgemeinen Formeln (VIII) oder (IX) 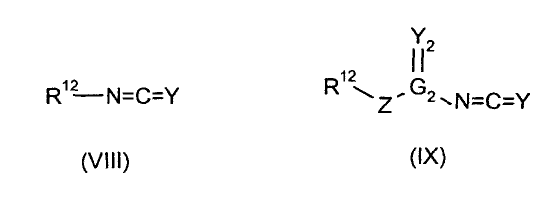 in welchen 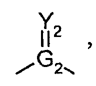 Z, Y und R¹² die unter in Punkt 1 angegebene Bedeutung haben gegebenenfalls in Gegenwart eines Katalysators, gegebenenfalls in Gegenwart von Verdünnungsmitteln umsetzt, oder indem man
   e) zur Darstellung der 6-substituierten 1,2,4a,5a, 8a,8b-Hexahydro-6H-pyrrolo[3',4':4,5]furo[3,2-b]pyrid-8(7H)-on und/oder 6-substituierten 1,2,3,4,4a,5a,8a,8b-Oktahydro-6H-pyrrolo[3',4':4,5]furo[3,2-b]pyrid-8(7H)-on Derivate der allgemeinen Formeln (Ie) und (If) sowie deren Salze, 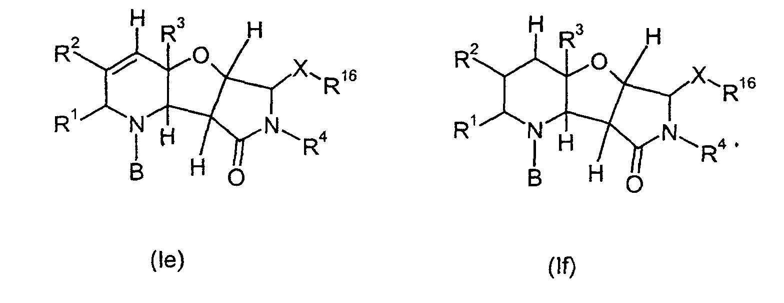 in welchen
      R¹, R², R³, R⁴, X und B die in Punkt 1 angegebene Bedeutung besitzen und für die Reste B und R¹⁶
      die gleiche Gruppe 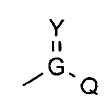 steht,
      worin 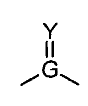 und Q die in Punkt 1 angegebene Bedeutung haben,
      Derivate der allgemeinen Formeln (Ic) und (Id) 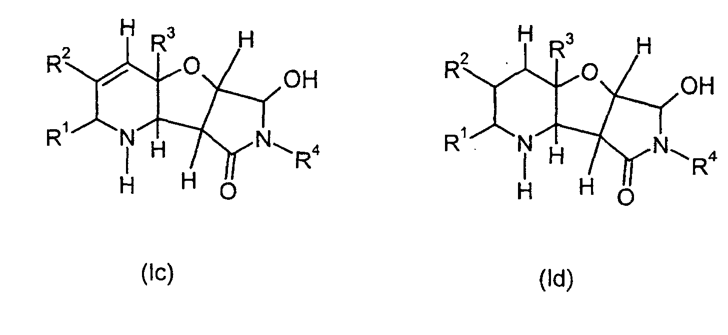 in welchen
      R¹, R², R³, R⁴ die in Punkt 1 angegebene Bedeutung besitzen,
      mit Acylierungsmitteln der Formel (IV)

      E-B (IV)

      bzw.

      E-R¹⁶

      entweder am Rest -NH- in Position 1 oder am Rest -OH in Position 6 oder an beiden Resten umsetzt, wobei E-B bzw. E-R¹⁶ eine der folgenden Verbindungen der allgemeinen Formeln (V), (VI), (VIII) oder (IX)

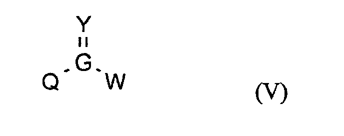

      (Q-C=O)₂O (VI)

      R¹²-N=C=Y (VIII)

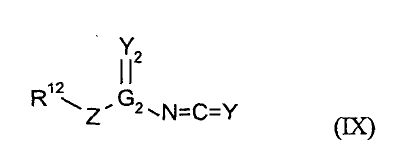

      bedeuten, in welchen 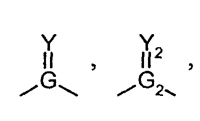 Q, Z, W und R¹² die in Anspruch 1 angegebene Bedeutung haben;
      die Umsetzung erfolgt dabei gegebenenfalls in Gegenwart eines Katalysators, gegebenenfalls in Gegenwart eines basischen Reaktionshilfsmittels und gegebenenfalls in Gegenwart von Verdünnungsmitteln.
   f) zur Darstellung der 6-substituierten 1,2,4a,5a,8a,8b-Hexahydro-6H-pyrrolo[3',4':4,5]furo[3,2-b]pyrid-8(7H)-on und/oder 6-substituierten 1,2,3,4,4a,5a,8a,8b-Oktahydro-6H-pyrrolo[3',4':4,5]furo[3,2-b]-pyrid-8(7H)-on Derivate der allgemeinen Formeln (Ie) und (If) sowie deren Salze, 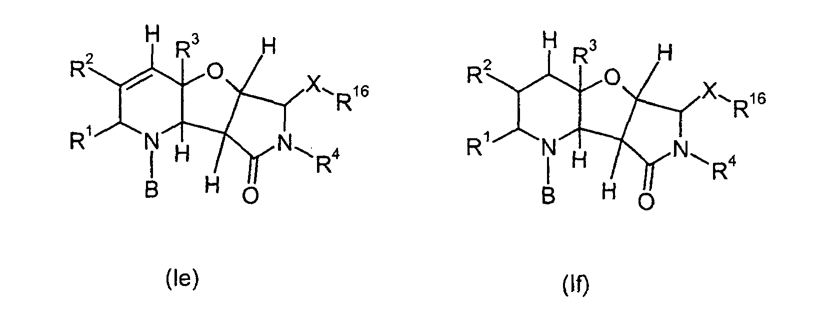 in welchen
      R¹, R², R³, R⁴, X und B die in Punkt 1 angegebene Bedeutung besitzen und für den Rest R¹⁶
      die Gruppe 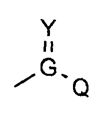 steht,
      worin 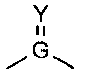 und Q die in Punkt 1 angegebene Bedeutung haben,
      Derivate der allgemeinen Formeln (Ic) und (Id) 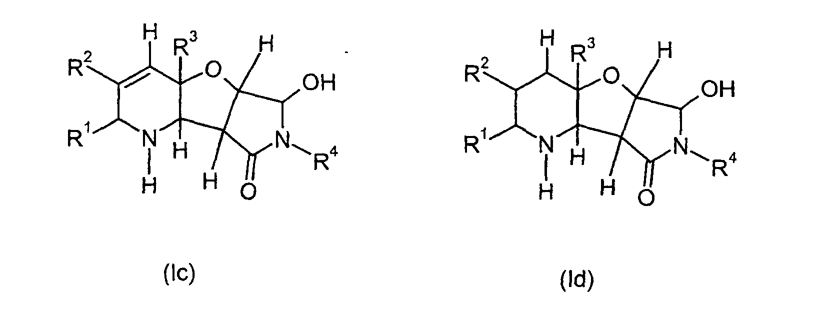 in welchen
      R¹, R², R³, R⁴ die Punkt 1 angegebene Bedeutung besitzen,
      mit einem Acylierungsmittel der Formel (IV)

      E - R¹⁶ (IV)

      am Rest -OH in Position 6 umsetzt, wobei E - R¹⁶ für die Verbindung der allgemeinen Formel (VII) 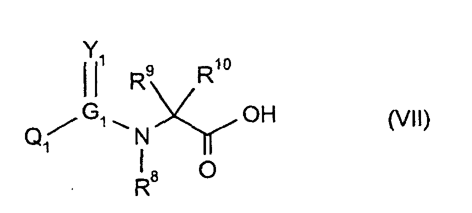 in welcher 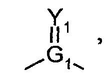 Q¹, R⁸, R⁹ und R¹⁰ die in Punkt 1 angegebene Bedeutung haben; und nachfolgend in einem zweiten Reaktionsschritt die auf diese Weise erhaltenen Derivate der allgemeinen Formeln (Ig) und (Ih) 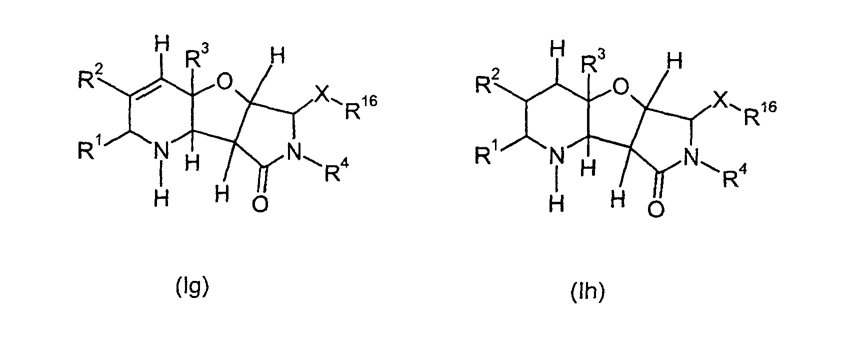 in welchen
      R¹, R², R³, R⁴ und X die in Punkt 1 angegebene Bedeutung besitzen und für
      den Rest R¹⁶ die Gruppe 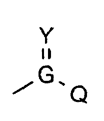 steht,
      worin 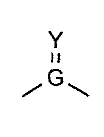 und Q die in Punkt 1 angegebene Bedeutung haben,
      mit einem Acylierungsmittel der Formel (IV)

      E - B (IV)

      am Rest -NH- in Position 1 umsetzt, wobei E-B eine der in Anspruch 3e genannten Verbindungen der allgemeinen Formeln (V), (VI), (VII) oder (IX) bedeutet,
      in welchen 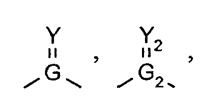 Q, Z, W, und R¹² die in Punkt 1 und 2 angegebene
      Bedeutung haben, umsetzt;
      die Umsetzung erfolgt dabei gegebenenfalls in Gegenwart eines Katalysators, gegebenenfalls in Gegenwart eines basischen Reaktionsmittels und gegebenenfalls in Gegenwart von Verdünnungsmitteln.
4. Verfahren zur Herstellung der 6-substituierten 1,2,4a,5a,8a,8b-Hexahydro-6H-pyrrolo[3',4':4,5]furo[3,2-b]pyrid-8(7H)-on und/oder 6-substituierte 1,2,-3,4,4a,5a,8a,8b-Oktahydro-6H-pyrrolo[3',4':4,5]furo[3,2-b]pyrid-8(7H)-on Derivate der allgemeinen Formeln (Ii) und (Ij) sowie deren Salze 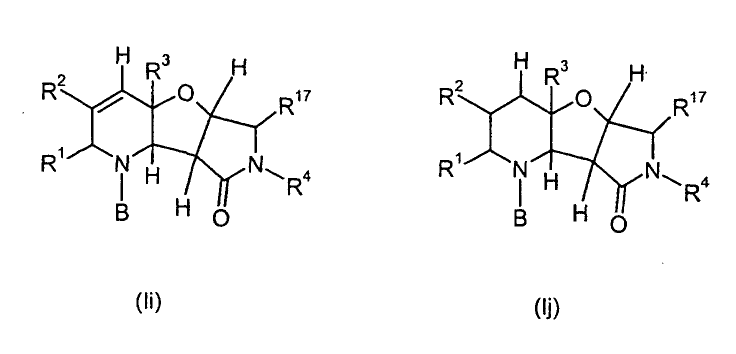 in welchen
   - R¹, R², R³, R⁴ und B: die in Punkt 1 angegebene Bedeutung besitzen,
   - R¹⁷: für C₁₋₆-Alkyl, C₂₋₆-Alkenyl, C₂₋₆-Alkinyl, Furyl, Cyan, C₁₋₄-Alkoxycarbonyl, Carbamoyl, Thiocarbamoyl, die gegebenenfalls durch Reste aus der Reihe Halogen, C₁₋₄-Alkyl, Halogen-C₁₋₄-alkyl, Amino, Hydroxy, Nitro, Cyan, C₁₋₄-Alkoxy, C₁₋₂-Alkylendioxy, Halogen-C₁₋₄-alkoxy, C₁₋₄-Alkylthio, Halogen-C₁₋₄-alkylthio, C₁₋₄-Alkylsulfonyl, C₁₋₄-Alkylamino, C₁₋₄-Dialkylamino, substituiert sind, oder gegebenenfalls für einen Rest aus der Gruppe A² steht, 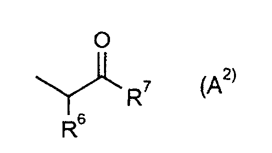 worin
   R⁶ und R⁷ die weiter oben in Punkt 1 angegebene Bedeutung besitzen,
   sowie deren optische Isomere und Racemate,
   dadurch gekennzeichnet, dass man
   entweder die z.B. gemäß Punkt 2 erhältlichen Derivate der allgemeinen Formeln (Ia) und (Ib) 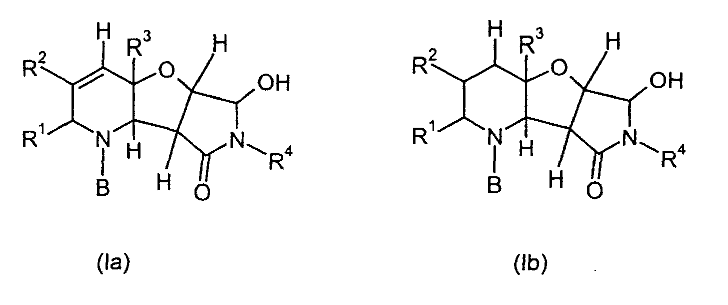 in welchen
   R¹, R², R³, R⁴ und B die in Punkt 1 angegebene Bedeutung besitzen, oder besonders bevorzugt
   die z. B. gemäß Punkt 3 erhältlichen Derivate der allgemeinen Formeln (Ie) und (If) 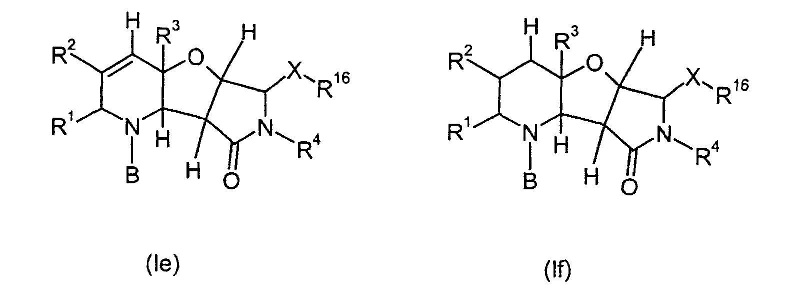 in welcher
   - R¹, R², R³, R⁴ und B: die in Punkt 3 angegebene Bedeutung besitzen, und
   - R¹⁶: für C₁₋₆-Alkyl, Aryl-C₁₋₂-alkyl, die gegebenenfalls substituiert sind, steht,
   - X: für Sauerstoff, Schwefel oder Sulfonyl steht,

   a) mit metallorganischen Verbindungen der allgemeinen Formel (XI)

      (R¹⁸)₃M-R¹⁷ (XI)

      in welcher
      - R¹⁸: für C₁₋₄-Alkyl, steht
      - M: für ein Metallatom, insbesondere Silizium oder Zinn, steht
      - R¹⁷: für C₁₋₆-Alkyl, C₂₋₆-Alkenyl, C₂₋₆-Alkinyl, die gegebenenfalls durch Reste aus der Reihe Halogen, C₁₋₄-Alkyl, Halogen-C₁₋₄-alkyl, Amino, Hydroxy, Nitro, Cyan, C₁₋₄-Alkoxy, C₁₋₂-Alkylendioxy, Halogen-C₁₋₄-alkoxy, C₁₋₄-Alkylthio, Halogen-C₁₋₄-alkylthio, C₁₋₄-Alkylsulfonyl, C₁₋₄-Alkylamino, C₁₋₄-Dialkylamino, substituiert sind, oder für Cyan steht,
      gegebenenfalls in Gegenwart von Verdünnungsmitteln und gegebenenfalls in Gegenwart eines Katalysators umsetzt, oder
   b) mit Verbindungen der allgemeinen Formel (XII) 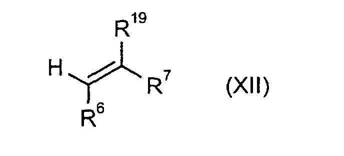 in welcher
      - R⁶ und R⁷: für die weiter oben angegebene Bedeutung haben,
      - R¹⁹: für Wasserstoff, -O-Acyl, -O-Sn-O-SO₂-CF₃, -O-B(CH₂-CH₃)₂, oder für die Reste
      - O-M(R¹⁸)₃ und 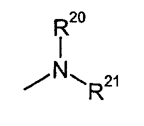 steht,
      worin
      - M und R¹⁸: die angegebene Bedeutung besitzen, und
      - R²⁰ und R²¹: unabhängig voneinander für Wasserstoff, geradkettiges oder verzweigtes Alkyl, Alkenyl, Alkinyl, Cycloalkyl, Cycloalkylalkyl, Arylalkyl stehen, die gegebenenfalls substituiert sind, oder
      - R²⁰ und R²¹: gemeinsam mit dem angrenzenden N-Atom für ein carbocyclisches 5-, 6- oder 7-gliedriges Ringsystem, das gegebenenfalls auch durch Sauerstoff, Schwefel oder Stickstoff unterbrochen sein kann und gegebenenfalls substituiert ist, stehen,
      gegebenenfalls in Gegenwart eines Katalysators und gegebenenfalls in Gegenwart von Verdünnungsmitteln umsetzt, oder
   c) mit Aromaten oder Heteroaromaten der allgemeinen Formel (XIII)

      H-R¹⁷ (XIII)

      worin
      - R¹⁷: für den Furylrest steht, der gegebenenfalls substituiert ist durch Reste aus der Reihe Halogen, C₁₋₄-Alkyl, Halogen-C₁₋₄-alkyl, Amino, Hydroxy, Nitro, Cyan, C₁₋₄-Alkoxy, C₁₋₂-Alkylendioxy, Halogen-C₁₋₄-alkoxy, C₁₋₄-Alkylthio, Halogen-C₁₋₄-alkylthio, C₁₋₄-Alkylsulfonyl, C₁₋₄-Alkylamino, C₁₋₄-Dialkylamino, steht
      gegebenenfalls in Gegenwart eines Katalysators und gegebenenfalls in Gegenwart von Verdünnungsmitteln umsetzt.

Die erfindungsgemäßen 6-substituierten 1,2,4a,5a,8a,8b-Hexahydro- und 1,2,3,4, 4a,5a,8a,8b-Oktahydro-6H-pyrrolo[3',4':4,5]furo[3,2-b]pyrid-8(7H)-on Derivate und deren Salze sind durch die allgemeine Formel (I) allgemein definiert.

Die erfindungsgemäßen 6-substituierten 1,2,4a,5a,8a,8b-Hexahydro- und 1,2,3,4, 4a,5a,8a,8b-Oktahydro-6H-pyrrolo[3',4':4,5]furo[3,2-b]pyrid-8(7H)-on Derivate und deren Säureadditionssalze und Metallsalzkomplexe besitzen gute endoparasitizide, insbesondere anthelmintische Wirkung und können bevorzugt im Bereich der Veterinärmedizin eingesetzt werden.

Gegebenenfalls substituiertes Alkyl allein oder als Bestandteil eines Restes in den allgemeinen Formeln bedeutet geradkettiges oder verzweigtes Alkyl mit vorzugsweise 1 bis 6, insbesondere 1 bis 4 Kohlenstoffatomen. Beispielhaft seien gegebenenfalls substituiertes Methyl, Ethyl, n-Propyl, Isopropyl, n-Butyl, Isobutyl, sec.-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1,2,2-Trimethylpropyl, 1-Ethylbutyl und Ethylbutyl genannt.

Vorzugsweise seien Methyl, Ethyl, n-Propyl, Isopropyl, n-Butyl, Isobutyl, sec-Butyl und tert-Butyl genannt.

Gegebenenfalls substituiertes Alkenyl allein oder als Bestandteil eines Restes in den allgemeinen Formeln bedeutet geradkettiges oder verzweigtes Alkenyl mit vorzugsweise 2 bis 6, insbesondere 2 bis 4 Kohlenstoffatomen. Beispielhaft seien gegebenenfalls substituiertes Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-pro-penyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Me-thyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl genannt.

Vorzugsweise seien gegebenenfalls substituiertes Ethenyl, 2-Propenyl, 2-Butenyl oder 1-Methyl-2-propenyl genannt.

Gegebenenfalls substituiertes Alkinyl allein oder als Bestandteil eines Restes in den allgemeinen Formeln bedeutet geradkettiges oder verzweigtes Alkinyl mit vorzugsweise 2 bis 6, insbesondere 2 bis 4 Kohlenstoffatomen. Beispielhaft seien gegebenenfalls substituiertes Ethinyl, 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 11-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Me-thyl-3-pentinyl, 2-Methyl-4-pentinyl, 3-Methyl-4-pentinyl, 4-Methyl-2-pentinyl, 1,1-Dime-thyl-3-butinyl, 1,2-Dimethyl-3-butinyl, 2,2-Dimethyl-3-butinyl, 1-Ethyl-3-butinyl, 2-Ethyl-3-butinyl und 1-Ethyl-1-methyl-2-propinyl genannt.

Vorzugsweise seien gegebenenfalls substituiertes Ethinyl, 2-Propinyl oder 2-Butinyl genannt.

Gegebenenfalls substituiertes Cycloalkyl allein oder als Bestandteil eines Restes in den allgemeinen Formeln bedeutet mono-, bi- und tricyclisches Cycloalkyl, vorzugsweise mit 3 bis 10, insbesondere 3, 5 oder 7 Kohlenstoffatomen. Beispielhaft seien gegebenenfalls substituiertes Cyclopropyl, Cyclobutyl, Cyclopentyl, Cyclohexyl, Cycloheptyl, Cyclooktyl, Bicyclo[2.2.1]heptyl, Bicyclo[2.2.2]oktyl und Adamantyl genannt.

Halogenalkyl allein oder als Bestandteil eines Restes in den allgemeinen Formeln enthält 1 bis 4, insbesondere 1 oder 2 Kohlenstoffatome mit vorzugsweise 1 bis 9, insbesondere 1 bis 5 gleiche oder verschiedene Halogenatome vorzugsweise Fluor, Chlor oder Brom, insbesondere Fluor und Chlor. Beispielhaft seien Trifluormethyl, Trichlormethyl, Chlordifluormethyl, Dichlorfluormethyl, Chlormethyl, Brommethyl, 1-Fluorethyl, 2-Fluorethyl, 2,2-Difluorethyl, 2,2,2-Trifluorethyl, 2,2,2-Trichlorethyl, 2-Chlor-2,2-difluorethyl, Pentafluorethyl und Pentafluor-tert-butyl genannt.

Gegebenenfalls substituiertes Alkoxy allein oder als Bestandteil eines Restes in den allgemeinen Formeln bedeutet geradkettiges oder verzweigtes Alkoxy mit vorzugsweise 1 bis 6, insbesondere 1 bis 4 Kohlenstoffatomen. Beispielhaft seien gegebenenfalls substituiertes Methoxy, Ethoxy, n-Propoxy, Isopropoxy, n-Butoxy, Isobutoxy, sec-Butoxy und tert-Butoxy genannt.

Gegebenenfalls substituiertes Halogenalkoxy allein oder als Bestandteil eines Restes in den allgemeinen Formeln bedeutet geradkettiges oder verzweigtes Halogenalkoxy mit vorzugsweise 1 bis 6, insbesondere 1 bis 4 Kohlenstoffatomen. Beispielhaft seien gegebenenfalls substituiertes Difluormethoxy, Trifluormethoxy, Trichlormethoxy, Chlordifluormethoxy, 1-Fluorethoxy, 2-Fluorethoxy, 2,2-Difluorethoxy, 1,1,2,2-Tetrafluorethoxy, 2,2,2-Trifluorethoxy und 2-Chlor-1,1,2-trifluorethoxy genannt.

Gegebenenfalls substituiertes Alkylthio allein oder als Bestandteil eines Restes in den allgemeinen Formeln bedeutet geradkettiges oder verzweigtes Alkylthio mit vorzugsweise 1 bis 6, insbesondere 1 bis 4 Kohlenstoffatomen. Beispielhaft seien gegebenenfalls substituiertes Methylthio, Ethylthio, n-Propylthio, Isopropylthio, n-Butylthio, Isobutylthio, sec-Butylthio und tert-Butylthio genannt.

Gegebenenfalls substituiertes Halogenalkylthio allein oder als Bestandteil eines Restes in den allgemeinen Formeln bedeutet geradkettiges oder verzweigtes Halogenalkylthio mit vorzugsweise 1 bis 6, insbesondere 1 bis 4 Kohlenstoffatomen. Beispielhaft seien gegebenenfalls substituiertes Difluormethylthio, Trifluormethylthio, Trichlormethylthio, Chlordifluormethylthio, 1-Fluorethylthio, 2-Fluorethylthio, 2,2-Difluorethylthio, 1,1,2,2-Tetrafluorethylthio, 2,2,2-Trifluorethylthio und 2-Chlor-1,1,2-trifluorethylthio genannt.

Gegebenenfalls substituiertes Alkylcarbonyl allein oder als Bestandteil eines Restes in den allgemeinen Formeln bedeutet geradkettiges oder verzweigtes Alkylcarbonyl mit vorzugsweise 1 bis 6, insbesondere 1 bis 4 Kohlenstoffatomen. Beispielhaft seien gegebenenfalls substituiertes Methylcarbonyl, Ethylcarbonyl, n-Propylcarbonyl, Isopropylcarbonyl, sec-Butylcarbonyl und tert-Butylcarbonyl genannt.

Gegebenenfalls substituiertes Cycloalkylcarbonyl allein oder als Bestandteil eines Restes in den allgemeinen Formeln bedeutet mono-, bi- und tricyclisches Cycloalkylcarbonyl, vorzugsweise mit 3 bis 10, insbesondere 3, 5 oder 7 Kohlenstoffatomen. Beispielhaft seien gegebenenfalls substituiertes Cyclopropylcarbonyl, Cyclobutylcarbonyl, Cyclopentylcarbonyl, Cyclohexylcarbonyl, Cycloheptylcarbonyl, Cyclooktylcarbonyl, Bicyclo[2.2.1]heptylcarbonyl, Bicyclo[2.2.2]oktylcarbonyl und Adamantylcarbonyl genannt.

Gegebenenfalls substituiertes Alkoxycarbonyl allein oder als Bestandteil eines Restes in den allgemeinen Formeln bedeutet geradkettiges oder verzweigtes Alkoxy mit vorzugsweise 1 bis 6, insbesondere 1 bis 4 Kohlenstoffatomen. Beispielhaft seien gegebenenfalls substituiertes Methoxycarbonyl, Ethoxycarbonyl, n-Propoxycarbonyl, Isopropoxycarbonyl, n-Butoxycarbonyl, Isobutoxycarbonyl, sec-Butoxycarbonyl und tert-Butoxycarbonyl genannt.

Aryl ist beispielsweise ein ein-, zwei- oder mehrkerniger aromatischer Rest wie Phenyl, Naphthyl, Tetrahydronaphthyl, Indanyl, Fluorenyl und ähnliches, vorzugsweise aber Phenyl oder Naphthyl.

Gegebenenfalls substituiertes Aryl in den allgemeinen Formeln bedeutet vorzugsweise gegebenenfalls substituiertes Phenyl oder Naphthyl, insbesondere Phenyl.

Gegebenenfalls substituiertes Arylalkyl in den allgemeinen Formeln bedeutet vorzugsweise gegebenenfalls im Arylteil und/oder Alkyl substituiertes Arylalkyl mit vor-zugsweise 6 oder 10, insbesondere 8 Kohlenstoffatomen im Arylteil (vorzugsweise Phenyl oder Naphthyl, insbesondere Phenyl) und vorzugsweise 1 bis 4, insbesondere 1 oder 2 Kohlenstoffatomen im Alkylteil, wobei der Alkylteil geradkettig oder verzweigt sein kann. Beispielhaft und vorzugsweise seien gegebenenfalls substituiertes Benzyl und Phenylethyl genannt.

Die gegebenenfalls substituierten Reste der allgemeinen Formeln können einen oder mehrere, vorzugsweise 1 bis 3, insbesondere 1 bis 2 gleiche oder verschiedene Substituenten tragen. Als Substituenten seien beispielhaft und vorzugsweise aufgeführt: Alkyl mit vorzugsweise 1 bis 4, insbesondere 1 bis 2 Kohlenstoffatomen, wie Methyl, Ethyl, n-Propyl, Isopropyl, n-Butyl, Isobutyl, sec-Butyl und tert-Butyl; Alkoxy mit vorzugsweise 1 bis 4, insbesondere 1 bis 2 Kohlenstoffatomen, wie Methoxy, Ethoxy, n-Propoxy, Isopropoxy, n-Butoxy, Isobutoxy, sec-Butoxy und tert-Butoxy; Alkylthio wie Methylthio, Ethylthio, n-Propylthio, Isopropylthio, n-Butylthio, Isobutylthio, sec-Butylthio; Halogenalkyl mit vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor oder Chlor stehen, wie Difluormethyl, Trifluormethyl, Trichlormethyl; Hydroxy; Halogen, vorzugsweise Fluor, Chlor, Brom und Iod, insbesondere Fluor und Chlor; Cyan; Nitro; Amino; Monoalkyl- und Dialkylamino mit vorzugsweise 1 bis 4, insbesondere 1 oder 2 Kohlenstoffatomen je Alkylgruppe, wie Methylamino, Methylethylamino, Dimethylamino, n-Propylamino, Isopropylamino, Methyln-butylamino; Alkylcarbonylreste wie Methylcarbonyl; Alkoxycarbonyl mit vorzugsweise 2 bis 4, insbesondere 2 bis 3 Kohlenstoffatomen wie Methoxycarbonyl und Ethoxycarbonyl; Alkylsulfinyl mit 1 bis 4, insbesondere 1 bis 2 Kohlenstoffatomen; Halogenalkylsulfinyl mit 1 bis 4, insbesondere 1 bis 2 Kohlenstoffatomen und 1 bis 5 Halogenatomen wie Trifluormethylsulfinyl; Halogenalkylsulfonyl mit 1 bis 4, insbesondere 1 bis 2 Kohlenstoffatomen und 1 bis 5 Halogenatomen wie Trifluormethylsulfonyl, Perfluor-n-butylsulfonyl, Perfluorisobutylsulfonyl; Arylsulfonyl mit vorzugsweise 6 oder 10 Arylkohlenstoffatomen wie Phenylsulfonyl; Trialkylsilyl mit 1 bis 4, insbesondere 1 bis 2 Kohlenstoffatomen wie Trimethylsilyl oder Triethylsilyl, Acyl, Aryl, Aryloxy, die ihrerseits einen der obengenannten Substituenten tragen können sowie den Formiminorest (-HC=N-O-Alkyl).

Als geeignete cyclische Aminogruppen kommen heteroaromatische oder aliphatische Ringsysteme mit einem oder mehreren Stickstoffatomen als Heteroatom in Frage, bei denen die Heterocyclen gesättigt oder ungesättigt, ein Ringsystem oder mehrere kondensierte Ringsysteme sein können, und gegebenenfalls weitere Heteroatome wie Stickstoff, Sauerstoff und Schwefel usw. enthalten. Außerdem können cyclische Aminogruppen auch ein Spiroring oder verbrücktes Ringsystem bedeuten. Die Anzahl der Atome, die cyclische Aminogruppen bilden, ist nicht beschränkt, beispielsweise bestehen sie im Falle eines Einringsystems aus 3 bis 8 Atomen und im Falle eines Dreiringsystems aus 7 bis 11 Atomen.

Beispielhaft für cyclische Aminogruppen mit gesättigten und ungesättigten monocyclischen Gruppen mit einem Stickstoffatom als Heteroatom seien 1-Azetidinyl, Pyrrolidino, 2-Pyrrolin-1-yl, 1-Pyrrolyl, Piperidino, 1,4-Dihydropyridin-1-yl, 1,2,5,6-Tetrahydropyridin-1-yl, Homopiperidino genannt; beispielhaft für cyclische Aminogruppen mit gesättigten und ungesättigten monocyclischen Gruppen mit zwei oder mehreren Stickstoffatomen als Heteroatome seien 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydropyridazin-1-yl, 1,2-dihydropyrimidin-1-yl, Perhydropyrimidin-1-yl, 1,4-Diaza-cycloheptan-1-yl genannt; beispielhaft für cyclische Aminogruppen mit gesättigten und ungesättigten monocyclischen Gruppen mit einem bis drei Stickstoffatomen und einem bis zwei Schwefelatomen als Heteroatome seien Thiazolidin-3-yl, Isothiazolin-2-yl, Thiomorpholino, oder Dioxothiomorpholino genannt; beispielhaft für cyclische Aminogruppen mit gesättigten und ungesättigten kondensierten cyclischen Gruppen seien Indol-1-yl, 1,2-Dihydrobenzimidazol-1-yl, Perhydropyrrolo[1,2-a]pyrazin-2-yl genannt; beispielhaft für cyclische Aminogruppen mit spirocyclischen Gruppen sei das 2-Azaspiro[4,5]decan-2-yl genannt; beispielhaft für cyclische Aminogruppen mit verbrückten heterocyclischen Gruppen sei das 2-Azabicyclo[2,2,1]heptan-7-yl ge-nannt.

Bevorzugt sind Verbindungen der allgemeinen Formel (I) und deren Salze 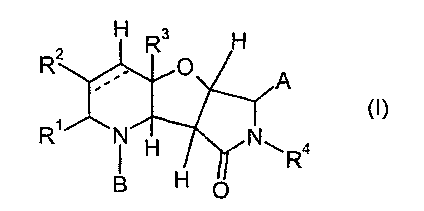 in welcher
- R¹: für Wasserstoff, geradkettiges oder verzweigtes C₁₋₄-Alkyl, insbesondere Methyl, Ethyl, Propyl, Isopropyl, Isobutyl, sec-Butyl, C₃₋₆-Cycloalkyl, insbesondere Cyclopropyl, steht,
- R²: für Wasserstoff, geradkettiges oder verzweigtes C₁₋₄-Alkyl, insbesondere Methyl, Ethyl, Propyl, Isopropyl, Isobutyl, sec-Butyl, tert-Butyl, Halogen-C₁₋₄-alkyl, insbesondere Brommethyl, Chlormethyl, Hydroxy-C₁₋₄-alkyl, insbesondere Hydroxymethyl, C₁₋₄-Alkanoyloxy-C₁₋₄-alkyl, insbesondere Acetoxymethyl, C₁₋₂-Alkoxyalkyl, insbesondere Methoxymethyl, C₁₋₂-Alkylthioalkyl, insbesondere Methylthiomethyl, C₁₋₂-Alkylsulfinylalkyl, insbesondere Methylsulfmylmethyl, C₁₋₂-Alkyl-sulfonylalkyl, insbesondere Methylsulfonylmethyl, Amino-C₁₋₂-alkyl, insbesondere Aminomethyl, C₁₋₆-Alkylaminoalkyl, insbesondere N-Methylaminomethyl, C₁₋₆-Dialkylaminoalkyl, insbesondere N,N-Dimethylaminomethyl, C₃₋₆-Cycloalkylaminoalkyl, insbesondere Morpholinomethyl, Thiomorpholinomethyl, C₃₋₆-Cycloalkyl, insbesondere Cyclopropyl, C₁₋₄-Alkoxycarbonyl, insbesondere Methoxycarbonyl, Ethoxycarbonyl, stehen,
- R³: für Wasserstoff, geradkettiges oder verzweigtes C₁₋₄-Alkyl, insbesondere Methyl, Ethyl, Propyl, Isopropyl, Isobutyl, sec-Butyl, tert-Butyl, steht,
- R⁴: geradkettiges oder verzweigtes C₁₋₆-Alkyl, insbesondere Methyl, Ethyl, Propyl, Isopropyl, Isobutyl, sec-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethyl-propyl, Hexyl, 1-Methylpentyl, Halogen-C₁₋₆-alkyl, insbesondere Fluormethyl, Difluormethyl, Difluorchlormethyl, 1-Fluorethyl, C₁₋₄-Alkoxycarbonyl-C₁₋₄-alkyl, insbesondere Methoxycarbonylmethyl, C₂₋₆-Alkenyl, insbesondere 2-Propenyl, C₃₋₆-Cycloalkyl, insbesondere Cyclopropyl, Cyclobutyl, Cyclopentyl, Cyclohexyl, C₃₋₆-Cycloalkyl-C₁₋₂-alkyl, insbesondere Cyclopropylmethyl, Cyclobutylmethyl, Aryl, insbesondere Phenyl, Aryl-C₁₋₂-alkyl, insbesondere Benzyl, 1-Phenylethyl, 2-Phenylethyl, Hetaryl, insbesondere Pyridyl, Thiazolyl, N-Morpholinyl, Hetaryl-C₁₋₂-alkyl, insbesondere 2-Chlorpyrid-5-yl-methyl und Chlorthiazol-5-yl-methyl, die gegebenenfalls durch Reste aus der Reihe Halogen, insbesondere Fluor, Chlor, Brom oder Iod, C₁₋₄-Alkyl, insbesondere Methyl, Halogen-C₁₋₄-alkyl, insbesondere Trifluormethyl, Trichlormethyl, Amino, Hydroxy, Nitro, Cyan, C₁₋₄-Alkoxy, insbesondere Methoxy, C₁₋₂-Alkylendioxy, insbesondere Methylendioxy oder Ethylendioxy, Halogen-C₁₋₄-alkoxy, insbesondere Trifluormethoxy, Difluormethoxy, C₁₋₄-Alkylthio, insbesondere Methylthio, Halogen-C₁₋₄-alkylthio, insbesondere Trifluormethylthio, C₁₋₄-Alkylsulfonyl, insbesondere Methylsulfonyl, C₁₋₄-Alkylamino, insbesondere N-Methylamino, C₁₋₄-Dialkylamino, insbesondere N,N-Dimethylamino, C₁₋₄-Alkylcarbonyl, insbesondere Methylcarbonyl, C₁₋₄-Alkoxycarbonyl, insbesondere Methoxycarbonyl, substituiert sein können, steht,
A für Hydroxy, C₁₋₆-Alkyl, insbesondere Methyl, Ethyl, Propyl, Isopropyl, Isobutyl, sec-Butyl, tert-Butyl, C₂₋₆-Alkenyl, insbesondere Vinyl, 2-Propenyl, 2-Butenyl, 3-Butenyl, 1-Methyl-2-propenyl, C₂₋₆-Alkinyl, insbesondere Ethinyl, 2-Propinyl, 2-Butinyl, 3-Butinyl, C₃₋₆-Cycloalkyl, insbesondere Cyclopropyl, Cyclobutyl, Cyclopentyl, Cyclohexyl, C₃₋₆-Cycloalkyl-C₁₋₂-alkyl, insbesondere Cyclopropylmethyl, C₁₋₆-Alkoxy, insbesondere Methoxy, Ethoxy, Propoxy, Isopropoxy, Isobutoxy, sec-Butoxy, C₂₋₆-Alkenyloxy, insbesondere 2-Propenyloxy, 2-Butenyloxy, 3-Butenyloxy, C₂₋₆-Alkinyloxy, insbesondere 2-Propinyloxy, 2-Butinyloxy, 3-Butinyloxy, C₁₋₄-Alkoxycarbonyl, insbesondere Methoxycarbonyl, Mercapto, C₁₋₆-Alkylthio, insbesondere Methylthio, Ethylthio, Propylthio, Isopropylthio, Cyan, Carbamoyl, Thiocarbamoyl, Aryl-C₁₋₂-alkyloxy, insbesondere Benzyloxy, Hetaryl, insbesondere Furyl, die gegebenenfalls durch Reste aus der Reihe Halogen, insbesondere Fluor, Chlor, Brom oder Iod, C₁₋₄-Alkyl, insbesondere Methyl, Halogen-C₁₋₄-alkyl, insbesondere Trifluormethyl, Amino, Hydroxy, Nitro, Cyan, C₁₋₄-Alkoxy, insbesondere Methoxy, C₁₋₂-Alkylendioxy, insbesondere Methylendioxy, Halogen-C₁₋₄-alkoxy, insbesondere Trifluormethoxy, C₁₋₄-Alkylthio, insbesondere Methylthio, Halogen-C₁₋₄-alkylthio, insbesondere Trifluormethylthio, C₁₋₄-Alkylsulfonyl, insbesondere Methylsulfonyl, C₁₋₄-Alkylamino, insbesondere N-Methylamino, C₁₋₄-Dialkyl- amino, insbesondere N,N-Dimethylamino, substituiert sein können, steht, oder gegebenenfalls für einen Rest aus der Gruppe A¹, A² und A³ 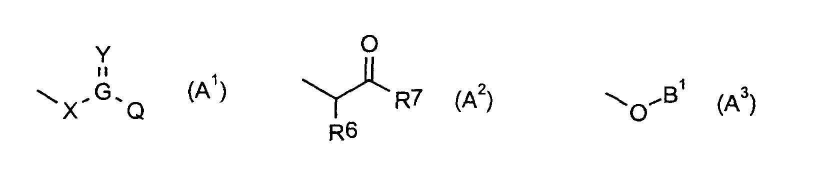 worin
X für Sauerstoff steht, 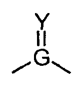 für Carbonyl oder Sulfonyl steht,
Q für geradkettiges oder verzweigtes C₁₋₆-Alkyl, insbesondere Methyl, Ethyl, Propyl, Isopropyl, Isobutyl, sec-Butyl, tert-Butyl, C₂₋₆-Alkenyl, insbesondere Vinyl, 2-Propenyl, 2-Butenyl, 3-Butenyl, 1-Methyl-2-propenyl, 2-Methyl-2-propenyl, C₂₋₆-Alkinyl, insbesondere 2-Propinyl, 2-Butinyl, 3-Butinyl, C₃₋₆-Cycloalkyl, insbesondere Cyclopropyl, Cyclobutyl, Cyclopentyl, Cyclohexyl, C₃₋₆-Cycloalkoxy, insbesondere Cyclopropyloxy, C₁₋₆-Alkoxy, insbesondere Methoxy, Ethoxy, Propoxy, Isopropoxy, Isobutoxy, sec-Butoxy, tert-Butoxy, C₂₋₆-Alkenyloxy, insbesondere Vinyloxy, 2-Propenyloxy, 2-Butenyloxy, 3-Butenyloxy, 1-Methyl-2-propenyloxy, 2-Methyl-2-propenyloxy, C₂₋₆-Alkinyloxy, insbesondere 2-Propinyloxy, 2-Butinyloxy, C₁₋₄-Alkylamino, insbesondere N-Methylamino, N-Ethylamino, C₁₋₄-Dialkylamino, insbesondere N,N-Dimethylamino, N,N-Diethylamino, eine über Stickstoff verknüpfte cyclische Aminogruppe bedeuten kann, insbesondere Morpholino, Thiomorpholino, Piperazino, Piperidino, Pyrrolidino, Aryl, insbesondere Phenyl, Aryl-C₁₋₂-alkyl, insbesondere Benzyl, Aryl-C₁₋₂-alkyloxy, insbesondere Benzyloxy, die gegebenenfalls substituiert sind, steht,
R⁶ für Wasserstoff oder Methyl, steht,
R⁷ für geradkettiges oder verzweigtes C₁₋₆-Alkyl, insbesondere Methyl, Ethyl, Propyl, Isopropyl, Isobutyl, sec-Butyl, tert-Butyl, C₂₋₆-Alkenyl, insbesondere Vinyl, 2-Propenyl, 2-Butenyl, 3-Butenyl, C₃₋₆-Cycloalkyl, insbesondere Cyclopropyl, Cyclobutyl, Cyclopentyl, Aryl insbesondere Phenyl, oder Hetaryl, die gegebenenfalls substituiert sind, steht,
R⁶ und R⁷ gemeinsam mit den Atomen, an die sie gebunden sind, für einen 5-, 6- oder 7-gliedrigen carbocyclischen Ring stehen, der gegebenenfalls durch C₁₋₄-Alkyl, insbesondere Methyl, substituiert sein kann, steht,
- B: für Wasserstoff, geradkettiges oder verzweigtes C₁₋₆-Alkyl, insbesondere Methyl, Ethyl, Propyl, Isopropyl, Isobutyl, sec-Butyl, C₁₋₄-Alkanoyloxyalkyl, insbesondere Acetoxymethyl, Acetoxyethyl, 2-Acetoxypropyl, C₁₋₂-Alkoxyalkyl, insbesondere Methoxymethyl, Amino-C₁₋₆-alkyl, insbesondere Aminomethyl, Aminoethyl, C₁₋₆-Alkylamino-C₁₋₆-alkyl, insbesondere N-Methylaminomethyl, C₁₋₆-Dialkylamino-C₁₋₆-alkyl, insbesondere N,N-Dimethylaminomethyl, C₂₋₆-Alkenyl, insbesondere Vinyl, 2-Propenyl, 2-Butenyl, C₂₋₆-Alkinyl, insbesondere 2-Propinyl, Carbamoyl-C₁₋₄-alkyl, insbesondere Carbamoylmethyl, Carboxyl-C₁₋₄-alkyl, insbesondere Carboxylmethyl, C₁₋₄-Alkoxydicarbonyl, insbesondere Methoxydicarbonyl, Aryl, insbesondere Phenyl, Aryl-C₁₋₂-alkyl, insbesondere Benzyl, 1-Phenylethyl, 2-Phenylethyl, Hetaryl, insbesondere Pyridyl, Pyrimidyl, Pyrrolidyl, Imidazolyl, Thiazolyl, N-Morpholinyl, Hetaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, C₁₋₄-Alkyl, insbesondere Methyl, Halogen-C₁₋₄-alkyl, insbesondere Trifluormethyl, Amino, Hydroxy, Nitro, Cyan, C₁₋₄-Alkoxy, insbesondere Methoxy, C₁₋₂-Alkylendioxy, insbesondere Methylendioxy oder Ethylendioxy, Halogen-C₁₋₄-alkoxy, insbesondere Trifluormethoxy, Difluormethoxy, C₁₋₄-Alkylthio, insbesondere Methylthio, Halogen-C₁₋₄-alkylthio, insbesondere Trifluormethylthio, C₁₋₄-Alkylsulfoyl, insbesondere Methylsulfonyl, C₁₋₄-Alkylamino, insbesondere N-Methylamino, C₁₋₄-Dialkylamino, insbesondere N,N-Dimethylamino, C₁₋₄-Alkylcarbonyl, insbesondere Methylcarbonyl, C₁₋₄-Alkoxycarbonyl, insbesondere Methoxycarbonyl, substituiert sein können, steht, oder gegebenenfalls für einen Rest aus der Gruppe B¹, B², B³ und B⁴ steht 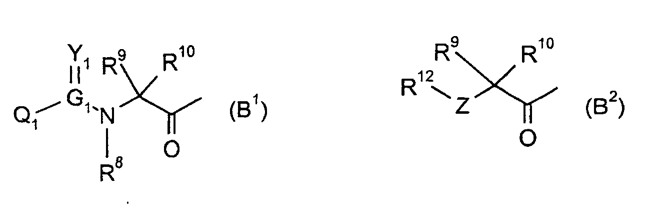 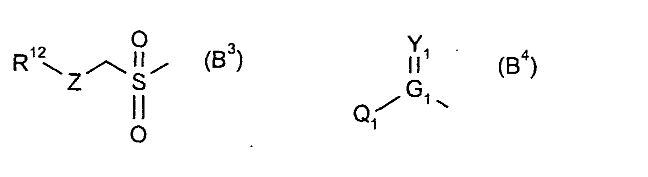 worin
R⁸ für Wasserstoff, geradkettiges oder verzweigtes C₁₋₆-Alkyl, insbesondere Methyl, Ethyl, C₃₋₆-Cycloalkyl, insbesondere Cyclopropyl, steht,
R⁸ und R⁹ gemeinsam mit den Atomen, an die sie gebunden sind, für einen 5- oder 6-gliedrigen Ring stehen, der gegebenenfalls Schwefel unterbrochen sein kann und gegebenenfalls durch Hydroxy, tert.-Butoxy, Benzyloxy substituiert ist, stehen,
R⁹ für Wasserstoff, geradkettiges oder verzweigtes C₁₋₆-Alkyl, insbesondere Methyl, Ethyl, Propyl, Isopropyl, sec-Butyl, steht,
R¹⁰ für Wasserstoff oder Methyl steht, 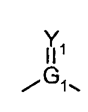 für Carbonyl, -C=CH-NO₂, -C=CH-CN, -C=N-R¹¹, Sulfonyl, steht,
R¹¹ Halogen-C₁₋₄-alkylcarbonyl, insbesondere Trifluormethylcarbonyl, Trichlormethylcarbonyl, C₁₋₄-Alkylsulfonyl, insbesondere Methylsulfonyl, Ethylsulfonyl, Nitro oder Cyan steht, und
Q¹ für einen Rest aus der Gruppe G¹ und G² 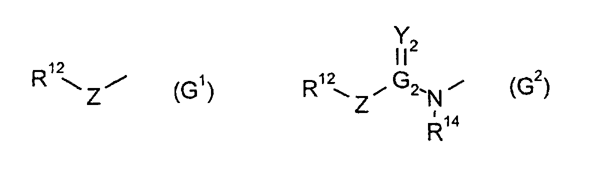 worin 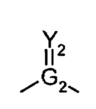 Carbonyl oder Sulfonyl bedeuten kann,
Z für Sauerstoff oder -NR¹³ steht,
R¹² für den Fall, dass Z für Stickstoff steht, eine über ein Stickstoffatom verknüpfte cyclische Aminogruppe, insbesondere Pyrrolidino, 2-Pyrrolin-2-yl, 1-Pyrrolyl, Piperidino, 1,4-Dihydropyridin-1-yl, 1-Homopiperazinyl, Morpholino, Thiomorpholino, Dioxothiomorpholino, die gegebenenfalls durch Reste aus der Reihe Halogen, insbesondere Fluor, Chlor, Brom oder Iod, C₁₋₄-Alkyl, insbesondere Methyl, Hydroxy-C₁₋₄-alkyl, insbesondere Hydroxymethyl, Amino-C₁₋₄-alkyl, insbesondere Aminomethyl, Aminoethyl, C₁₋₄-Alkylamino-C₁₋₄-alkyl, insbesondere N-Methylaminomethyl, N-Methylaminoethyl, C₁₋₄-Dialkylamino-C₁₋₄-alkyl, insbesondere N,N-Dimethylaminomethyl, N,N-Dimethylaminoethyl, Amino, Hydroxy, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, substituiert sein können,
R¹² und R¹³ unabhängig voneinander für Wasserstoff, geradkettiges oder verzweigtes C₁₋₆-Alkyl, insbesondere Methyl, Ethyl, Propyl, Isopropyl, sec-Butyl, C₂₋₄-Alkenyl, insbesondere Vinyl, 2-Propenyl, 2-Butenyl, 1-Methyl-2-propenyl, C₂₋₄-Alkinyl, insbesondere Ethinyl, 2-Propinyl, 2-Butinyl, C₃₋₆-Cycloalkyl, insbesondere Cyclopropyl, Hetaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, C₁₋₄-Alkyl, insbesondere Methyl, Hydroxy-C₁₋₄-alkyl, insbesondere Hydroxymethyl, Amino-C₁₋₄-alkyl, insbesondere Aminomethyl, Aminoethyl, C₁₋₄-Alkylamino-C₁₋₄-alkyl, insbesondere N-Methylaminomethyl, N- Methylaminoethyl, C₁₋₄-Dialkylamino-C₁₋₄-alkyl, insbesondere N,N-Dimethylaminomethyl, N,N-Dimethylaminoethyl, Amino, Hydroxy, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, substituiert sein können, stehen, oder
R¹² und R¹³ gemeinsam mit dem angrenzenden N-Atom für ein carbocyclisches 5-, 6- oder 7-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O, -N=, -NR¹⁵- oder durch quarternisierten Stickstoff unterbrochen sein kann und gegebenenfalls durch C₁₋₄-Alkyl, insbesondere Methyl, Hydroxy-C₁₋₄-alkyl, insbesondere Hydroxymethyl, Amino-C₁₋₄-alkyl, insbesondere Aminomethyl, Aminoethyl, C₁₋₄-Alkylamino-C₁₋₄-alkyl, insbesondere N-Methylaminomethyl, N-Methylaminoethyl, C₁₋₄-Dialkylamino-C₁₋₄-alkyl, insbesondere N,N-Dimethylaminomethyl, N,N-Dimethylaminoethyl, Amino, Hydroxy, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, Halogen, insbesondere Fluor, Chlor, Brom oder Iod, substituiert ist, stehen,
R¹⁴ für Wasserstoff oder C₁₋₄-Alkyl, insbesondere Methyl, Ethyl, steht,
R¹⁵ für Wasserstoff, geradkettiges oder verzweigtes C₁₋₆-Alkyl, insbesondere Methyl, Ethyl, C₂₋₆-Alkenyl, insbesondere Vinyl, 2-Propenyl, C₂₋₆-Alkinyl, insbesondere 2-Propinyl, C₃₋₆-Cycloalkyl, insbesondere Cyclopropyl, C₁₋₄-Alkoxycarbonyl, insbesondere Methoxycarbonyl, Ethoxycarbonyl, C₁₋₄-Alkylcarbonyl, Methylcarbonyl, C₃₋₆-Cycloalkylcarbonyl, insbesondere Cyclopropylcarbonyl, Cyan, Aryl, insbesondere Phenyl, Aryl-C₁₋₂-alkyl, insbesondere Benzyl, Hetaryl, insbesondere Pyridyl und Thiazolyl, Hetaryl-C₁₋₂-alkyl, insbesondere Pyridylmethyl uns Thiazolylmethyl, die gegebenenfalls durch Reste aus der Reihe Halogen, insbesondere Fluor, Chlor, Brom oder Iod, C₁₋₄-Alkyl, insbesondere Methyl, Halogen-C₁₋₄-alkyl, insbesondere Trifluormethyl, Trichlormethyl, Amino, Hydroxy, Cyan, C₁₋₄-Alkoxy, insbesondere Methoxy, C₁₋₂-Alkylendioxy, insbesondere Methylendioxy oder Ethylendioxy, Halogen-C₁₋₄-alkoxy, insbesondere Trifluormethoxy, C₁₋₄-Alkylthio, insbesondere Methylthio, Halogen-C₁₋₄-alkylthio, insbesondere Trifluormethylthio, C₁₋₄-Alkylsulfonyl, insbesondere Methylsulfonyl, C₁₋₄-Alkylamino, insbesondere N-Methylamino, C₁₋₄-Dialkylamino, insbesondere N,N-Dimethylamino, C₁₋₄-Alkylcarbonyl, insbesondere Methylcarbonyl, C₁₋₄-Alkoxycarbonyl, insbesondere Methoxycarbonyl, substituiert sein können, steht,
sowie deren optische Isomere und Racemate.

Ganz besonders bevorzugt sind Verbindungen der allgemeinen Formel (I) und deren Salze 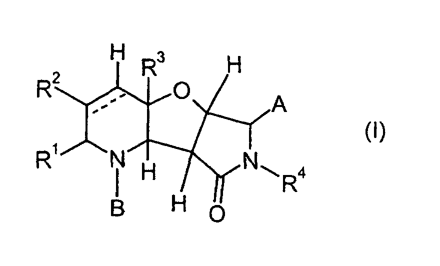 in welcher
- R¹: für Wasserstoff steht,
- R²: für geradkettiges oder verzweigtes C₁₋₄-Alkyl, insbesondere Methyl oder Ethyl steht,
- R³: für geradkettiges oder verzweigtes C₁₋₄-Alkyl, insbesondere Methyl oder Ethyl steht,
- R⁴: geradkettiges oder verzweigtes C₁₋₆-Alkyl, insbesondere Methyl, Ethyl, Propyl, Isopropyl, Isobutyl, sec-Butyl, n-Butyl, tert-Butyl, C₂₋₆-Alkenyl, insbesondere 2-Propenyl, C₃₋₆-Cycloalkyl, insbesondere Cyclopropyl, Cyclobutyl, Cyclopentyl, Cyclohexyl, C₃₋₆-Cycloalkyl-C₁₋₂-alkyl, insbesondere Cyclopropylmethyl, Aryl, insbesondere Phenyl, Aryl-C₁₋₂-alkyl, insbesondere Benzyl, 1-Phenylethyl, 2-Phenylethyl, Hetaryl-C₁₋₂-alkyl, insbesondere 2-Chlorpyrid-5-yl-methyl und Chlor-thiazol-5-yl-methyl, die gegebenenfalls durch Reste aus der Reihe Halogen, insbesondere Fluor, Chlor, Brom oder Iod, C₁₋₄-Alkyl, insbesondere Methyl, Halogen-C₁₋₄-alkyl, insbesondere Trifluormethyl, Trichlormethyl, Amino, Hydroxy, Nitro, Cyan, C₁₋₄-Alkoxy, insbesondere Methoxy, Halogen-C₁₋₄-alkoxy, insbesondere Trifluormethoxy, Difluormethoxy, C₁₋₄-Alkylamino, insbesondere N-Methylamino, C₁₋₄-Dialkylamino, insbesondere N,N-Dimethylamino, substituiert sein können, steht,

- A: für Hydroxy, C₁₋₆-Alkyl, insbesondere Propyl, sec-Butyl, C₂₋₆-Alkenyl, insbesondere Vinyl, 2-Propenyl, 2-Butenyl, 3-Butenyl, C₂₋₆-Alkinyl, insbesondere 2-Propinyl, 2-Butinyl, 3-Butinyl, C₃₋₆-Cycloalkyl, insbesondere Cyclopropyl, C₃₋₆-Cycloalkyl-C₁₋₂-alkyl, insbesondere Cyclopropylmethyl, C₁₋₆-Alkoxy, insbesondere Methoxy, Ethoxy, Propoxy, Isopropoxy, sec-Butoxy, C₂₋₆-Alkenyloxy, insbesondere 2-Propenyloxy, C₂₋₆-Alkinyloxy, insbesondere 2-Propinyloxy, C₁₋₄-Alkoxycarbonyl, insbesondere Methoxycarbonyl, Cyan, Aryl-C₁₋₂-alkyloxy, insbesondere Benzyloxy, Hetaryl, insbesondere Furyl, die gegebenenfalls durch Reste aus der Reihe Halogen, insbesondere Fluor, Chlor, Brom oder Iod, C₁₋₄-Alkyl, insbesondere Methyl, Amino, Hydroxy, Nitro, Cyan, C₁₋₄-Alkoxy, insbesondere Methoxy, Halogen-C₁₋₄-alkoxy, insbesondere Trifluormethoxy, C₁₋₄-Alkylamino, insbesondere N-Methylamino, C₁₋₄-Dialkylamino, insbesondere N,N-Dimethylamino, substituiert sein können, steht, oder gegebenenfalls für einen Rest aus der Gruppe A¹, A² und A³ 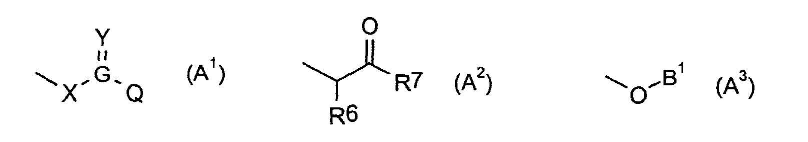 worin
X für Sauerstoff steht, 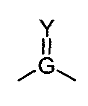 für Carbonyl oder Sulfonyl steht,
Q für geradkettiges oder verzweigtes C₁₋₆-Alkyl, insbesondere Methyl, Ethyl, Propyl, Isopropyl, Isobutyl, sec-Butyl, C₂₋₆-Alkenyl, insbesondere Vinyl, 2-Propenyl, C₃₋₆-Cycloalkyl, insbesondere Cyclopropyl, C₃₋₆-Cycloalkoxy, insbesondere Cyclopropyloxy, C₁₋₆-Alkoxy, insbesondere Methoxy, Ethoxy, Propoxy, Isopropoxy, Isobutoxy, sec-Butoxy, C₂₋₆-Alkenyloxy, insbesondere 2-Propenyloxy, C₁₋₄-Alkylamino, insbesondere N-Methylamino, N-Ethylamino, C₁₋₄-Dialkylamino, insbesondere N,N-Dimethylamino, N,N-Diethylamino, eine über Stickstoff verknüpfte cyclische Aminogruppe bedeuten kann, insbesondere Morpholino, Piperazino, Piperidino, Pyrrolidino, Aryl, insbesondere Phenyl, Aryl-C₁₋₂-alkyl, insbesondere Benzyl, Aryl- C₁₋₂-alkyloxy, insbesondere Benzyloxy, die gegebenenfalls substituiert sind, steht,
R⁶ für Wasserstoff steht,
R⁷ für geradkettiges oder verzweigtes C₁₋₆-Alkyl, insbesondere Methyl, Ethyl, Propyl, Isopropyl, sec-Butyl, C₂₋₆-Alkenyl, insbesondere Vinyl, 2-Propenyl, 2-Butenyl, C₃₋₆-Cycloalkyl, insbesondere Cyclopropyl, Aryl insbesondere Phenyl, oder Hetaryl, die gegebenenfalls substituiert sind, steht,
R⁶ und R⁷ gemeinsam mit den Atomen, an die sie gebunden sind, für einen 5-, 6- oder 7-gliedrigen carbocyclischen Ring stehen, der gegebenenfalls durch C₁₋₄-Alkyl, insbesondere Methyl, substituiert sein kann, steht,
B für Wasserstoff, geradkettiges oder verzweigtes C₁₋₆-Alkyl, insbesondere Methyl, Ethyl, C₂₋₆-Alkenyl, insbesondere 2-Propenyl, 2-Butenyl, Carbamoyl-C₁₋₄-alkyl, insbesondere Carbamoylmethyl, Carboxyl-C₁₋₄-alkyl, insbesondere Carboxylmethyl, Aryl, insbesondere Phenyl, Aryl-C₁₋₂-alkyl, insbesondere Benzyl, Hetaryl, insbesondere Pyridyl, Pyrimidyl, Pyrrolidyl, Imidazolyl, Thiazolyl, N-Morpholinyl, Hetaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, C₁₋₄-Alkyl, insbesondere Methyl, Halogen-C₁₋₄-alkyl, insbesondere Trifluormethyl, Amino, Hydroxy, Nitro, Cyan, C₁₋₄-Alkoxy, insbesondere Methoxy, Halogen-C₁₋₄-alkoxy, insbesondere Trifluormethoxy, Difluormethoxy, C₁₋₄-Alkyl-amino, insbesondere N-Methylamino, C₁₋₄-Dialkylamino, insbesondere N,N-Dimethylamino, substituiert sein können, steht, oder gegebenenfalls für einen Rest aus der Gruppe B¹, B², B³ und B⁴ steht 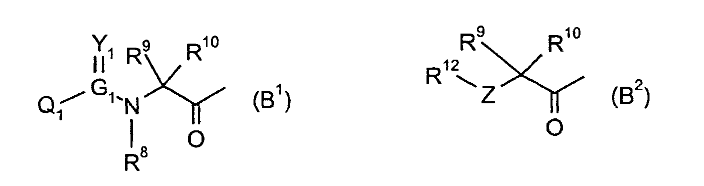 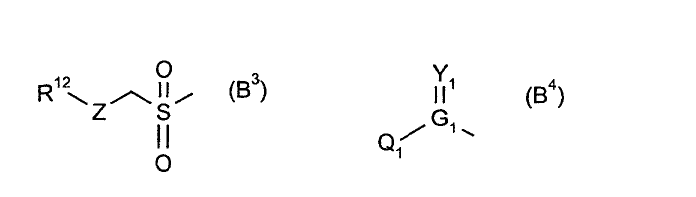 worin
R⁸ für Wasserstoff oder Methyl stehen,
R⁸ und R⁹ gemeinsam mit den Atomen, an die sie gebunden sind, für einen 5- oder 6-gliedrigen Ring stehen, der gegebenenfalls Schwefel unterbrochen sein kann und gegebenenfalls durch Hydroxy, tert.-Butoxy, Benzyloxy substituiert ist, stehen,
R⁹ für Wasserstoff, geradkettiges oder verzweigtes C₁₋₆-Alkyl, insbesondere Methyl, Ethyl, Propyl, Isopropyl, sec-Butyl, steht,
R¹⁰ für Wasserstoff steht, 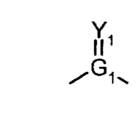 für Carbonyl oder Sulfonyl steht,
Q¹ für einen Rest aus der Gruppe G¹ und G² 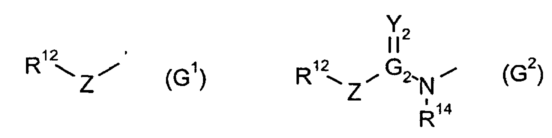 worin 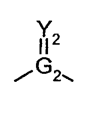 Carbonyl oder Sulfonyl bedeuten kann,
Z für Sauerstoff oder -NR¹³ steht,
R¹² für den Fall, dass Z für Stickstoff steht, eine über ein Stickstoffatom verknüpfte cyclische Aminogruppe, insbesondere Pyrrolidino, 1-Pyrrolyl, Piperidino, Morpholino, Thiomorpholino oder Dioxothiomorpholino bedeuten,
R¹² und R¹³ unabhängig voneinander für Wasserstoff, geradkettiges oder verzweigtes C₁₋₆-Alkyl, insbesondere Methyl, Ethyl, Propyl, Isopropyl, sec-Butyl, C₂₋₄-Alkenyl, insbesondere Vinyl, 2-Propenyl, 1-Methyl-2-propenyl, C₂₋₄-Alkinyl, insbesondere Ethinyl, 2-Propinyl, C₃₋₆-Cycloalkyl, insbesondere Cyclopropyl, Hetaryl-C₁₋₂-alkyl, insbesondere 5-Chlor-pyridylmethyl und Chlor-thiazol-5-yl-methyl, stehen, oder
R¹² und R¹³ gemeinsam mit dem angrenzenden N-Atom für ein carbocyclisches 5-, 6- oder 7-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O, -N=, -NR¹⁵- oder durch quarternisierten Stickstoff unterbrochen sein kann und gegebenenfalls durch C₁₋₄-Alkyl, insbesondere Methyl, Hydroxy-C₁₋₄-alkyl, insbesondere Hydroxymethyl, C₁₋₄-Dialkylamino-C₁₋₄-alkyl, insbesondere N,N-Dimethylaminomethyl, N,N-Dimethylaminoethyl, C₁₋₄-Alkoxycarbonyl, insbesondere Methoxycarbonyl, substituiert ist, stehen,
R¹⁴ für C₁₋₄-Alkyl, insbesondere Methyl, steht,
R¹⁵ für geradkettiges oder verzweigtes C₁₋₆-Alkyl, insbesondere Methyl, C₂₋₆-Alkenyl, insbesondere Vinyl, 2-Propenyl, C₂₋₆-Alkinyl, insbesondere 2-Propinyl, C₃₋₆-Cycloalkyl, insbesondere Cyclopropyl, C₁₋₄-Alkoxycarbonyl, insbesondere Methoxycarbonyl, C₁₋₄-Alkylcarbonyl, Methylcarbonyl, C₃₋₆-Cycloalkylcarbonyl, insbesondere Cyclopropylcarbonyl, steht,
sowie deren optische Isomere und Racemate.

Die erfindungsgemäßen Verbindungen der allgemeinen Formel (I) und deren Salze enthalten außerdem ein oder mehrere Chiralitätszentren und können somit in reinen Stereoisomeren oder in Form verschiedener Enantiomeren- und Diastereoisomerengemische vorliegen, die erforderlichenfalls in an sich bekannter Weise getrennt werden können. Die Erfindung betrifft daher sowohl die reinen Enantiomeren und Diastereomeren, als auch deren Gemische. Sie werden zur Bekämpfung von Endoparasiten, besonders auf dem Gebiet der Medizin und Veterinärmedizin verwendet.

Die vorliegende Erfindung betrifft auch die Verbindungen der allgemeinen Formel (I) in Form eines Säureadditionssalzes. Säuren, die zur Salzbildung herangezogen werden können, sind anorganische Säuren, wie Salzsäure, Bromwasserstoffsäure, Salpetersäure, Schwefelsäure, Phosphorsäure oder organische Säuren wie Ameisensäure, Essigsäure, Propionsäure, Malonsäure, Oxalsäure, Fumarsäure, Adipinsäure, Stearinsäure, Weinsäure, Ölsäure, Methansulfonsäure, Benzolsulfonsäure oder Toluolsulfonsäure.

Als geeignete Salze der Verbindungen der allgemeinen Formel (I) können übliche nicht toxische Salze, d.h. Salze mit verschiedenen Basen und Salze mit zugesetzten Säuren, genannt werden. Vorzugsweise sind Salze mit anorganischen Basen wie Alkalimetallsalze, beispielsweise Natrium-, Kalium- oder Cäsiumsalze, Erdalkalimetallsalze, beispielsweise Kalzium- oder Magnesiumsalze, Ammoniumsalze, Salze mit organischen Basen sowie mit organischen Aminen, beispielsweise Triethylammonium-, Pyridinium-, Picolinium-, Ethanolammonium-, Triethanolammonium-, Dicyclohexylammonium- oder N,N'-Dibenzylethylendiammoniumsalze, Salze mit anorganischen Säuren, beispielsweise Hydrochloride, Hydrobromide, Dihydrosulfate oder Trihydrophosphate, Salze mit organischen Carbonsäuren oder organischen Sulfosäuren, beispielsweise Formiate, Acetate, Trifluoracetate, Maleate, Tartrate, Methansulfonate, Benzolsulfonate oder para-Toluolsulfonate, Salze mit basischen Aminosäuren oder sauren Aminosäuren, beispielsweise Arginate, Aspartate oder Glutamate, zu nennen.

Beispiele für die erfindungsgemäßen Verbindungen sind in den Tabellen 1 bis 26 aufgeführt.

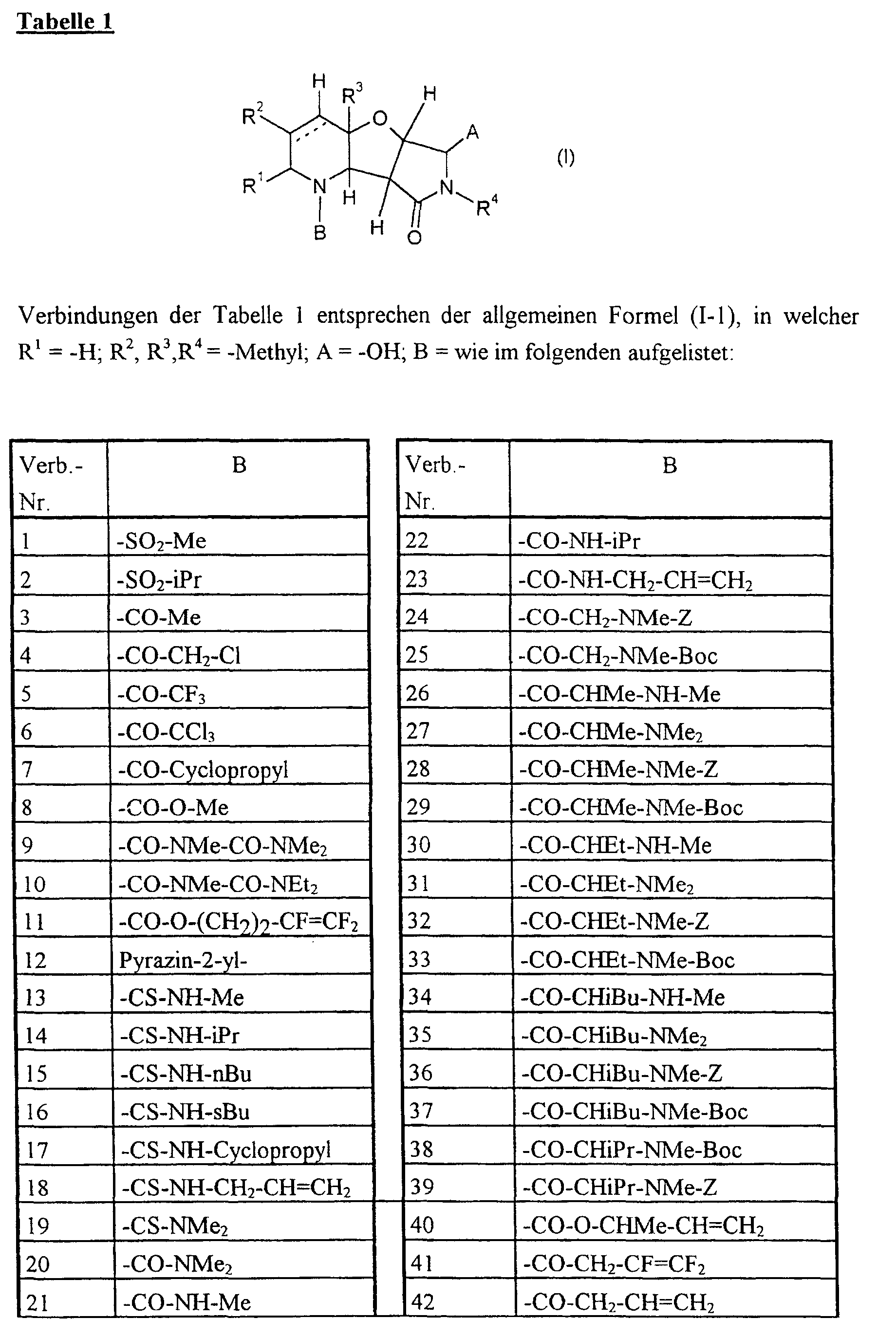

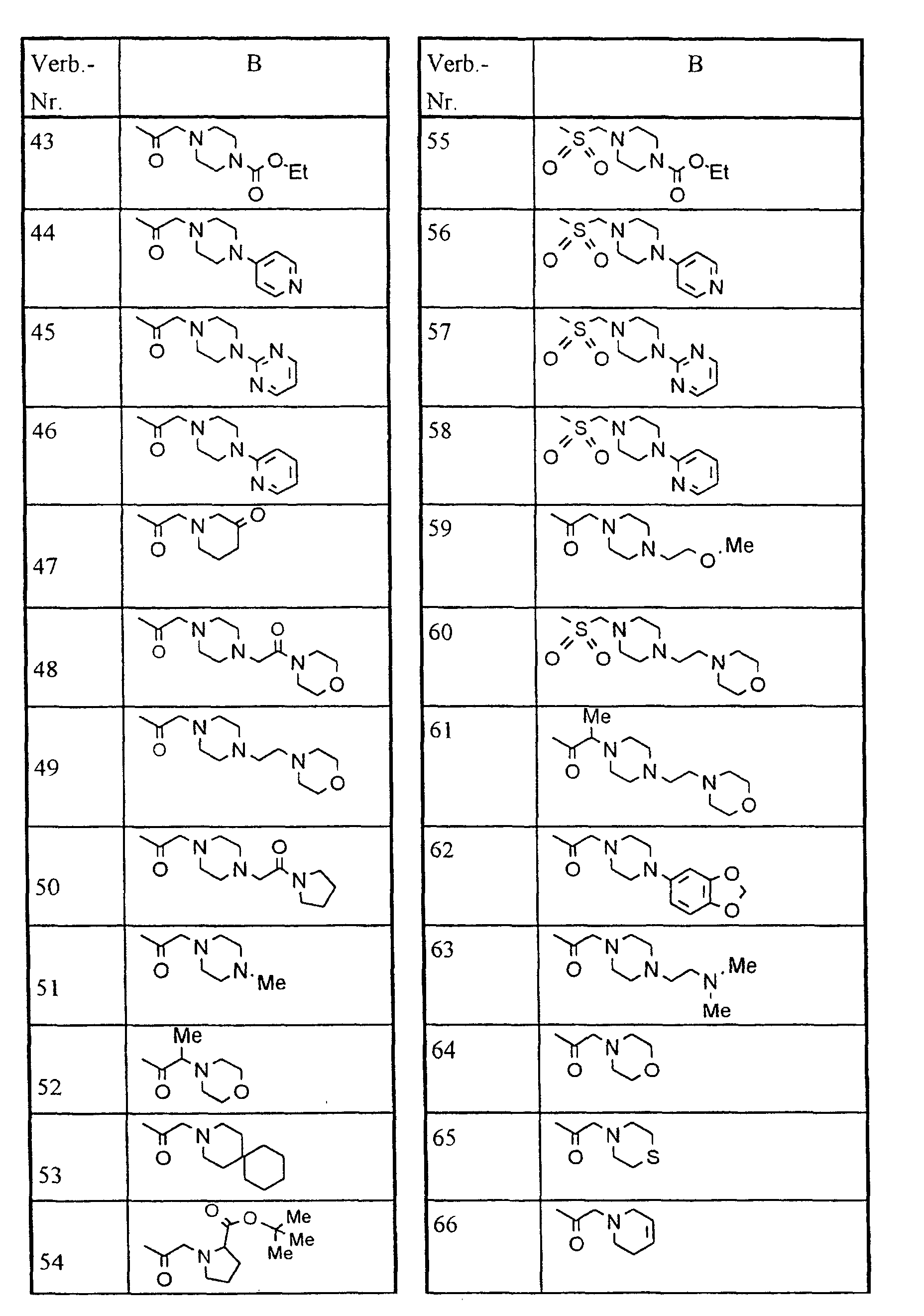

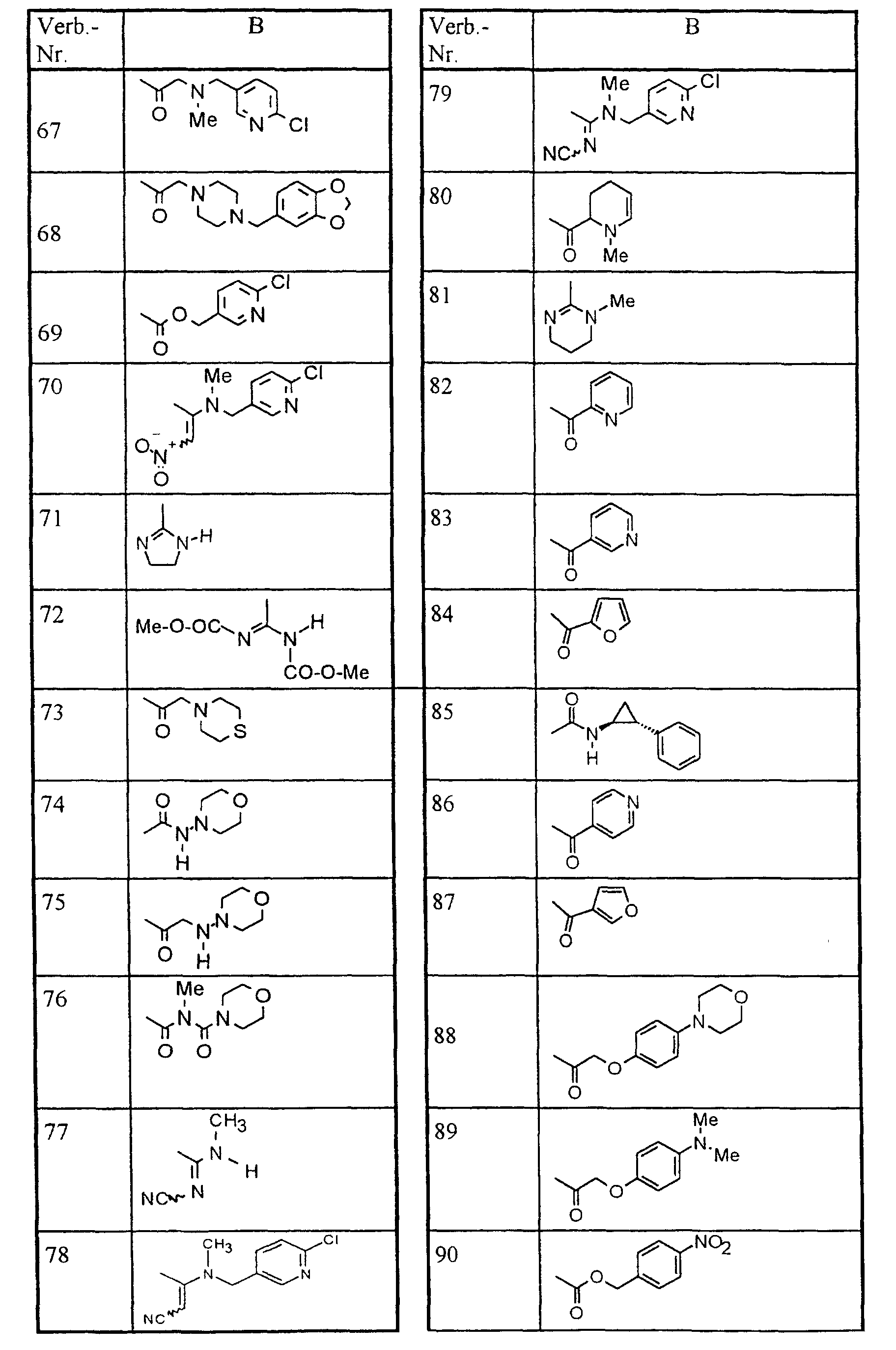

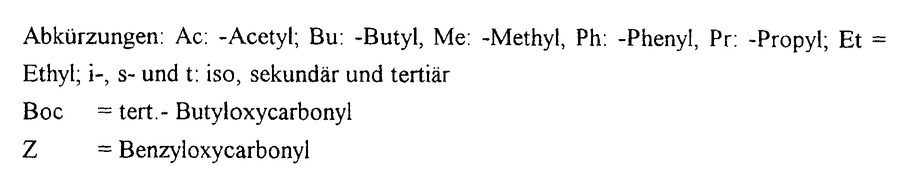

### Tabelle 2

Tabelle 2 enthält die Verbindungen der allgemeinen Formel (I-1), in welcher R¹ = -H; R², R³ = -Methyl; R⁴ = -Ethyl; A = -OH; B die in Tabelle 1 aufgelisteten Bedeutungen besitzt.

### Tabelle 3

Tabelle 3 enthält die Verbindungen der allgemeinen Formel (I-1), in welcher R¹ = -H; R², R³ = -Methyl; R⁴ = -nPropyl; A = -OH; B die in Tabelle 1 aufgelisteten Bedeutungen besitzt.

### Tabelle 4

Tabelle 4 enthält die Verbindungen der allgemeinen Formel (I-1), in welcher R¹ = -H; R², R³ = -Methyl; R⁴ = -Isopropyl; A = -OH; B die in Tabelle 1 aufgelisteten Bedeutungen besitzt.

### Tabelle 5

Tabelle 5 enthält die Verbindungen der allgemeinen Formel (I-1), in welcher R¹ = -H; R², R³ = -Methyl; R⁴ = -Cyclopropyl; A = -OH; B die in Tabelle 1 aufgelisteten Bedeutungen besitzt.

### Tabelle 6

Tabelle 6 enthält die Verbindungen der allgemeinen Formel (I-1), in welcher R¹ = -H; R², R³ = -Methyl; R⁴ = -nButyl; A = -OH; B die in Tabelle 1 aufgelisteten Bedeutungen besitzt.

### Tabelle 7

Tabelle 7 enthält die Verbindungen der allgemeinen Formel (I-1), in welcher R¹ = -H; R², R³ = -Methyl; R⁴ = -sec-Butyl; A = -OH; B die in Tabelle 1 aufgelisteten Bedeutungen besitzt.

### Tabelle 8

Tabelle 8 enthält die Verbindungen der allgemeinen Formel (I-1), in welcher R¹ = -H; R², R³ = -Methyl; R⁴ = 2-Phenylethyl; A = -OH; B die in Tabelle 1 aufgelisteten Bedeutungen besitzt.

### Tabelle 9

Tabelle 9 enthält die Verbindungen der allgemeinen Formel (I-1), in welcher R¹ = -H; R², R³, R⁴ = -Methyl; A = -O-Me; B die in Tabelle 1 aufgelisteten Bedeutungen besitzt.

### Tabelle 10

Tabelle 10 enthält die Verbindungen der allgemeinen Formel (I-1), in welcher R¹ = -H; R², R³ = -Methyl; R⁴ = -Ethyl; A = -O-Me; B die in Tabelle 1 aufgelisteten Bedeutungen besitzt.

### Tabelle 11

Tabelle 11 enthält die Verbindungen der allgemeinen Formel (I-1), in welcher R¹ = -H; R², R³, R⁴ = -Methyl; A = -O-Isopropyl; B die in Tabelle 1 aufgelisteten Bedeutungen besitzt.

### Tabelle 12

Tabelle 12 enthält die Verbindungen der allgemeinen Formel (I-1), in welcher R¹ = -H; R², R³ = -Methyl; R⁴ = -Ethyl; A = -O-Isopropyl; B die in Tabelle 1 aufgelisteten Bedeutungen besitzt.

### Tabelle 13

Tabelle 13 enthält die Verbindungen der allgemeinen Formel (I-1), in welcher R¹ = -H; R², R³, R⁴ = -Methyl; A = -O-Acetyl; B die in Tabelle 1 aufgelisteten Bedeutungen besitzt.

### Tabelle 14

Tabelle 14 enthält die Verbindungen der allgemeinen Formel (I-1), in welcher R¹ = -H; R², R³ = -Methyl; R⁴ = -Ethyl; A = -O-Acetyl; B die in Tabelle 1 aufgelisteten Bedeutungen besitzt.

### Tabelle 15

Tabelle 15 enthält die Verbindungen der allgemeinen Formel (I-1), in welcher R¹ = -H; R², R³ = -Methyl; R⁴ = -Cyclopropyl; A = -O-Acetyl; B die in Tabelle 1 aufgelisteten Bedeutungen besitzt.

### Tabelle 16

Tabelle 16 enthält die Verbindungen der allgemeinen Formel (I-1), in welcher R¹ = -H; R², R³, R⁴ = -Methyl; A = -CN; B die in Tabelle 1 aufgelisteten Bedeutungen besitzt.

### Tabelle 17

Tabelle 17 enthält die Verbindungen der allgemeinen Formel (I-1), in welcher R¹ = -H; R², R³ = -Methyl R⁴ = -Ethyl; A = -CN; B die in Tabelle 1 aufgelisteten Bedeutungen besitzt.

### Tabelle 18

Tabelle 18 enthält die Verbindungen der allgemeinen Formel (I-1), in welcher R¹ = -H; R², R³, R⁴ = -Methyl; A = -CH₂-CH=CH₂; B die in Tabelle 1 aufgelisteten Bedeutungen besitzt.

### Tabelle 19

Tabelle 19 enthält die Verbindungen der allgemeinen Formel (I-1), in welcher R¹ = -H; R², R³ = -Methyl R⁴ = -Ethyl; A = -CH₂-CH=CH₂; B die in Tabelle 1 aufgelisteten Bedeutungen besitzt.

### Tabelle 20

Tabelle 20 enthält die Verbindungen der allgemeinen Formel (I-1), in welcher R¹ = -H; R², R³, R⁴ = -Methyl; A = Fur-2-yl; B die in Tabelle 1 aufgelisteten Bedeutungen besitzt.

### Tabelle 21

Tabelle 21 enthält die Verbindungen der allgemeinen Formel (I-1), in welcher R¹ = -H; R², R³ = -Methyl R⁴ = -Ethyl; A = Fur-2-yl; B die in Tabelle 1 aufgelisteten Bedeutungen besitzt.

### Tabelle 22

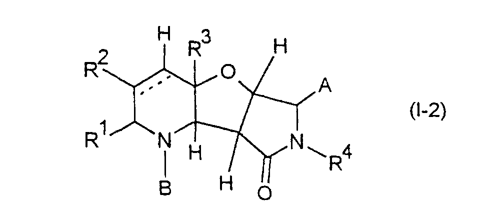

Tabelle 22 enthält Verbindungen der allgemeinen Formel (I-2), in welcher R¹ = -H; R², R³,R⁴ = -Methyl; A = -OH; B die in Tabelle 1 aufgelisteten Bedeutungen besitzt.

### Tabelle 23

Tabelle 23 enthält Verbindungen der allgemeinen Formel (1-2), in welcher R¹ = -H; R², R³ = -Methyl, R⁴ = -Ethyl; A = -OH; B die in Tabelle 1 aufgelisteten Bedeutungen besitzt.

### Tabelle 24

Tabelle 24 enthält Verbindungen der allgemeinen Formel (I-2), in welcher R¹ = -H; R², R³ = -Methyl, R⁴ = -Cyclopropyl; A = -OH; B die in Tabelle 1 aufgelisteten Bedeutungen besitzt.

### Tabelle 25

Tabelle 25 enthält Verbindungen der allgemeinen Formel (1-2), in welcher R¹ = -H, R², R³,R⁴ = -Methyl; A = -O-Acetyl; B die in Tabelle 1 aufgelisteten Bedeutungen besitzt.

### Tabelle 26

Tabelle 26 enthält Verbindungen der allgemeinen Formel (I-2), in welcher R¹ = -H; R², R³ = -Methyl, R⁴ = -Ethyl; A = -O-Acetyl; B die in Tabelle 1 aufgelisteten Bedeutungen besitzt.

Die Verbindungen der allgemeinen Formel (I) sind neu, sie lassen sich beispielsweise nach den oben unter Punkt 3, 5 und 7 angegebenen Verfahren herstellen.

Überraschender Weise und erfindungsgemäß können die neuen 6-Hydroxy-1,2,4a, 5a,8a,8b-hexahydro-6H-pyrrolo[3',4':4,5]furo[3,2-b]pyrid-8(7H)-on- und 6-Hydroxy-1,2,3,4,4a,5a,8a,8b-oktahydro-6H-pyrrolo[3',4':4,5]furo[3,2-b]pyrid-8(7H)-on Derivate der allgemeinen Formeln (Ia-d) nach Verfahren 2 via regioselektiver Hydrierung einer Imidcarbonylfunktion im Pyrrolidinstrukturteil aus entsprechenden 1,2,4a,5a,-8a,8b-Hexahydro- oder 1,2,3,4,4a,5a,8a,8b-Oktahydro-6H-pyrrolo[3',4':4,5]furo[3,2-b]pyridin-6,8(7H)-dion Derivaten der allgemeinen Formeln (IIa-d) entstehen und für Folgereaktionen gemäß den Verfahren 2, 3 und 4 genutzt werden.

Erwartungsgemäß können die Verbindungen der allgemeinen Formeln (Ia-d) in Abhängigkeit der Substituenten in Form eines Isomerengemisches, bestehend aus einem 6α-Hydroxy-Isomer und einem 6β-Hydroxy-Isomer, vorliegen.

Nachfolgend werden die erfindungsgemäßen Verfahren 2, 3 und 4 anhand ausgewählter Beispiele erläutert (vgl. auch Herstellungsbeispiele).

Setzt man beispielsweise bei Verfahren 2 zur Hydrierung das 7-(p-Tolyl)-4aα, 5aα,8aα,8bα-(±)-tetrahydro-3,4a-dimethyl-6H-pyrrolo[3',4':4,5]furo[3,2-b]pyridin-6,8 (7H)-dion als Verbindung der allgemeinen Formel (III) ein, so entstehen die zwei 6α-Hydroxy- und 6β-Hydroxy-7-(p-tolyl)-1,2,4aα,5aα,8aα,8bα-(±)-hexahydro-3,4adimethyl-6H-pyrrolo[3',4':4,5]furo[3,2-b]pyrid-8(7H)-one als Isomerengemisch (vgl. Schema I ).

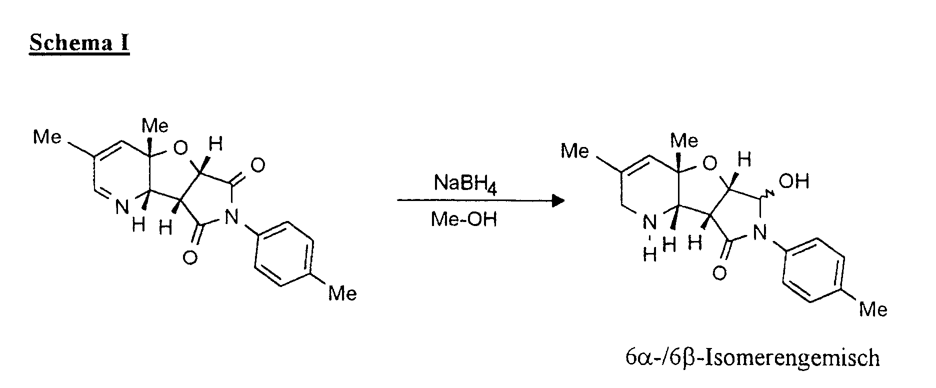

Als Beispiele zur regio- und stereoselektiven Herstellung der erfindungsgemäßen 6β-Isomere seien die Hydrierungen des 1-Allyloxycarbonyl-7-ethyl-1,2,4aα,5aα,8aα, 8bα-(±)-hexahydro-3,4a-dimethyl- und 1-Allyloxycarbonyl-1,2,3,4,4aα,5aα,8aα,8bα-(±)-oktahydro-3β,4a,7-trimethyl-6H-pyrrolo[3',4':4,5]furo[3,2-b]pyridin-6,8(7H)-dions als Verbindungen der allgemeinen Formeln (IIa) und (IIb) genannt, die nach dem genannten Verfahren regio- und stereoselektiv zum 1-Allyloxycarbonyl-7-ethyl-6β-hydroxy-1,2,4aα,5aα,8aα,8bα-(±)-hexahydro-3,4a-dimethyl- und 1-Allyloxycarbonyl-6β-hydroxy-1,2,3,4,4aα,5aα,8aα,8bα-(±)-oktahydro-3β,4a,7-trimethyl-6Hpyrrolo[3',4':4,5]furo[3,2-b]pyrid-8(7H)-on als 6β-Hydroxy-Isomere führen (vgl. Schema II ):

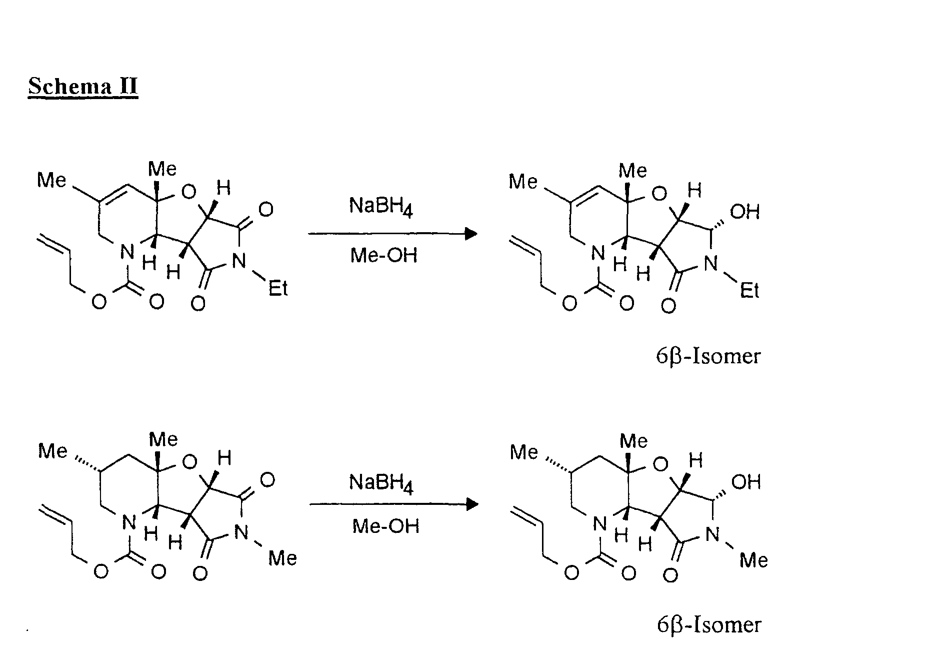

Die neuen 6-Hydroxy-1,2,4aα,5aα,8aα,8bα-(±)-hexahydro-6H-pyrrolo[3',4':4,5]furo [3,2-b]pyrid-8(7H)-on Derivate der allgemeinen Formeln (Ia) lassen sich aber auch alternativ nach Verfahren 3 via gleichzeitiger selektiver Hydrierung der C=N-Doppelbindung im Dihydropyridinteil sowie durch regio- und stereoselektive Hydrierung einer Imidcarbonylfunktion im Pyrrolidinstrukturteil aus entsprechenden 4aα,5aα, 8aα,8bα-(±)-Tetrahydro-6H-pyrrolo[3',4':4,5]furo[3,2-b]pyridin-6,8(7H)-dion Derivaten der allgemeinen Formel (III) erhalten.

Als weiteres Beispiel des erfindungsgemäßen Verfahrens 2 zur regio- und stereoselektiven Herstellung des erfindungsgemäßen 6β-Isomers vom 6-Hydroxy-1,2,4aα, 5aα,8aα,8bα-(±)-hexahydro-3,4a,7-trimethyl-6H-pyrrolo[3',4':4,5]furo[3,2-b]pyrid-8 (7H)-on der allgemeinen Formel (Ia) sei deshalb die Hydrierung des 4aα,5aα,8aα, 8bα-(±)-Tetrahydrohexahydro-3,4a,7-trimethyl-6H-pyrrolo[3',4':4,5]furo[3,2-b]pyridin-6,8(7H)-dions gezeigt (vgl. Schema III).

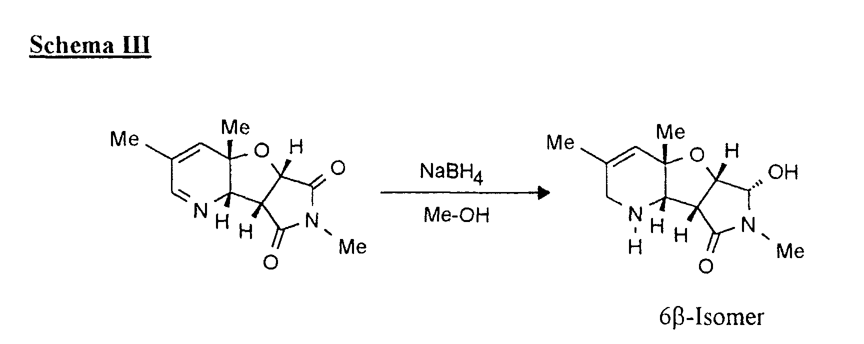

Die zur Durchführung des erfindungsgemäßen Verfahrens 2 als Ausgangsstoffe benötigten Verbindungen sind durch die Formeln (II) und (III) allgemein definiert. In den Formeln stehen R¹, R², R³, R⁴, und B vorzugsweise für diejenigen Reste, die bereits im Zusammenhang mit der Beschreibung der erfindungsgemäßen Stoffe der Formel (I) als bevorzugt für diese Substituenten genannt wurden.

Die als Ausgangsstoffe verwendeten 4aα,5aα,8aα,8bα-(±)-Tetrahydro-6H-pyrrolo [3',4':4,5]furo[3,2-b]pyridin-6,8(7H)-dion Derivate sind teilweise bekannt und können z. T. nach literaturbekannten Methoden erhalten werden (vgl. z. B.: T. Hisano et al. Chem. Pharm. Bull. 35 (3), (1987), S. 1049-1057; Heterocycles 29 (6), (1989), S. 1029-1032; Chem. Pharm. Bull. 38 (3), (1990), S. 605-611; Chem. Pharm. Bull. 39 (1), (1991), S. 10-17 und DE-OS 19 538 960-A1).

Für die Hydrierung der 4a,5a,8a,8b-Tetrahydro-6H-pyrrolo[3',4':4,5]furo[3,2-b]pyridin-6,8(7H)-dion Derivate und deren Salze kommen die verschiedensten Hydrierungsreagenzien, wie beispielsweise Alkalimetallhydride, insbesondere Natriumborhydrid (NaBH₄), Lithiumaluminiumhydrid (LiAlH₄), Lithiumtriethylborhydrid (Li[Et₃ BH]), Lithium-tri-*sec*-borhydrid (Li[*sec*-Bu₃BH]), Natrium-bis(2-methoxyethoxy)aluminiumhydrid, Alkylaluminiumhydride, insbesondere Diisobutylaluminiumhydrid (DI-BAL-H), oder Tetramethylammoniumtriacetoxyborhydrid u. a., in Frage (vgl. H. de Koning, W. N. Speckamp, Houben Weyl E 21, S. 1953 sowie dort zitierte Literatur).

Selbstverständlich kann auch ein "Borhydrid-Harz" beispielsweise "Borohydride on Amberlite® IRA-406", zur Hydrierung verwendet werden (vergl. Sande A.R. et al. Tetrahedron LeH. 1984, 25, S. 3501).

Zur Durchführung der Hydrierung werden bevorzugt Alkalimetallhydride, insbesondere Natriumborhydrid (NaBH₄) oder Lithiumaluminiumhydrid (LiAlH₄), eingesetzt.

Im allgemeinen ist es vorteilhaft das erfindungsgemäße Verfahren 2 in Gegenwart von Verdünnungsmitteln durchzuführen. Verdünnungsmittel werden vorteilhaft in einer solchen Menge eingesetzt, daß das Reaktionsgemisch während des ganzen Verfahrens gut rührbar bleibt. Als Verdünnungsmittel zur Durchführung des erfindungsgemäßen Verfahrens 2 kommen alle inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether wie Ethylpropylether, Methyl-tert-butylether, n-Butylether, Anisol, Phenetol, Cyclohexylmethylether, Dimethylether, Diethylether, Dipropylether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids: Amine wie Trimethyl-, Triethyl-, Tripropyl-, Tributylamin, N-Methyl-morpholin, Pyridin und Tetramethylendiamin, Nitrokohlenwasserstoffe wie Niromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, Hexan, Heptan, Oktan, Nonan und technische Kohlenwasserstoffe, beispielsweise sogenannte White Spirits mit Komponenten mit Siedepunkten im Bereich beispielsweise von 40°C bis 250°C, Cymol, Benzinfraktionen innerhalb eines Siedepunktintervalles von 70°C bis 190°C, Cyclohexan, Methylcyclohexan, Petrolether, Ligroin, Octan, Benzol, Toluol, Chlorbenzol, Brombenzol, Nitrobenzol, Xylol; Ester wie Methyl-, Ethyl-, Butyl-, Isobutylacetat, sowie Dimethyl-, Dibutyl-, Ethylencarbonat; Amide wie Hexamethylenphosphortriamid, Formamid, N-Methyl-formamid, N,N-Dimethylformamid, N,N-Dipropylformamid, N,N-Dibutylformamid, N-Methyl-pyrrolidin, N-Methyl-caprolactam, 1,3-Dimethyl-3,4,5,6-tetrahydro-2(1H)-pyri-midin, Octylpyrrolidon, Octylcaprolactam, 1,3-Dimethyl-2-imidazolindion, N-Formyl-piperidin, N,N'-1,4-Diformylpiperazin; Ketone wie Aceton, Acetophenon, Methyl-ethylketon, Methylbutylketon.

Selbstverständlich kann man in das erfindungsgemäße Verfahren auch Gemische der genannten Lösungs- und Verdünnungsmittel einsetzen.

Bevorzugte Verdünnungsmittel zur Hydrierung sind inerte organische Lösungsmit-tel, wie z. B. Alkohole, insbesondere Methanol oder Ethanol, Ether, insbesondere Tetrahydrofuran und Dioxan.

Die Hydrierung nach Verfahren 2 wird durchgeführt, indem man die 4a,5a,8a,8b-Tetrahydro-, 1,2,4a,5a,8a,8b-Hexahydro- oder 1,2,3,4,4a,5a,8a,8b-Oktahydro-, 6H-pyrrolo[3',4':4,5]furo[3,2-b]pyridin-6,8(7H)-dion Derivate der allgemeinen Formeln (II) und (III) in Gegenwart eines geeigneten Hydrierungsmittels, beispielsweise Natriumborhydrid, in einem der angegebenen Verdünnungsmittel umsetzt.

Die Reaktionsdauer beträgt 10 Minuten bis 48 Stunden. Die Umsetzung erfolgt bei Temperaturen zwischen -60°C und +100°C, bevorzugt zwischen -30°C und +80°C, besonders bevorzugt bei -10°C bis Raumtemperatur. Es wird unter Normaldruck und Schutzgasatmosphäre (Stickstoff oder Helium) gearbeitet.

Zur Durchführung des erfindungsgemäßen Verfahrens 2 setzt man pro Mol an Verbindungen der allgemeinen Formeln (II) und (III) im allgemeinen 1,0 bis 3,0 Mol, vorzugsweise einen geringen Überschuß an Hydrierungsmittel ein.

Nach vollendeter Hydrierung wird der gesamte Reaktionsansatz neutralisiert, im Vakuum eingeengt, der verbleibende Rückstand in einem Verdünnungsmittel aufgenommen und mehrmals gewaschen. Die anfallenden Produkte lassen sich in üblicher Weise durch Umkristallisieren, Vakuumdestillation oder Säulenchromatographie reinigen (vgl. auch die Herstellungsbeispiele).

Als Beispiel zur Herstellung geeigneter Salze von Verbindungen der allgemeinen Formel (Ic), wie beispielsweise das Hydrogensulfat vom 7-Ethyl-6β-hydroxy-1,2, 4aα,5aα,8aα,8bα-(±)-hexahydro-3,4a-dimethyl-6H-pyrrolo[3',4':4,5]furo[3,2-b]pyrid-8(7H)-on gezeigt (vgl. Schema IV):

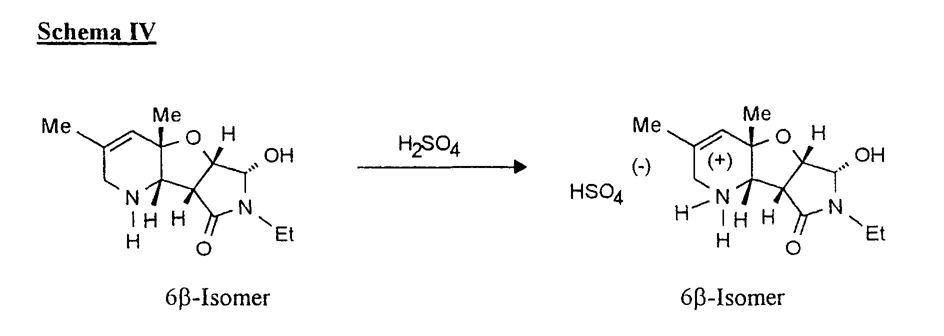

Die Salzbildung wird durchgeführt, indem Verbindungen der allgemeinen Formel (Ic), beispielsweise in Gegenwart von anorganischen Säuren, wie Schwefelsäure, in einem der bei Verfahren 2 angegebenen Verdünnungsmittel umsetzt.

Die Reaktionsdauer beträgt 10 Minuten bis 24 Stunden. Die Umsetzung erfolgt bei Temperaturen zwischen -60°C und +150°C, bevorzugt zwischen -10°C und +80°C, besonders bevorzugt bei 0°C bis Raumtemperatur. Es wird unter Normaldruck gearbeitet. Zur Salzbildung setzt man pro Mol an Verbindung der allgemeinen Formel (Ic) im allgemeinen einen Überschuß an Säure ein.

Nach vollendeter Umsetzung wird das zumeist ausgefallene Salz abgetrennt, gewaschen und im Vakuum getrocknet (vgl. auch Herstellungsbeispiele).

Setzt man beispielsweise bei Verfahren 2b zur Herstellung der neuen 6-Hydroxy-1,2,4a,5a,8a,8b-(+/-)-hexahydro-6H-pyrrolo[3',4':4,5]furo[3,2-b]pyrid-8(7H)-one der allgemeinen Formeln (Ia) als Verbindungen der allgemeinen Formel (Ic) das Hydrogensulfat vom 7-Ethyl-6β-hydroxy-1,2,4aα,5aα,8aα,8bα-(±)-hexahydro-3,4adimethyl-6H-pyrrolo[3',4':4,5]furo[3,2-b]pyrid-8(7H)-on und als Verbindungen der allgemeinen Formel (V) Chlorameisensäureallylester ein, so läßt sich das Verfahren durch das folgende Reaktionsschema V wiedergeben:

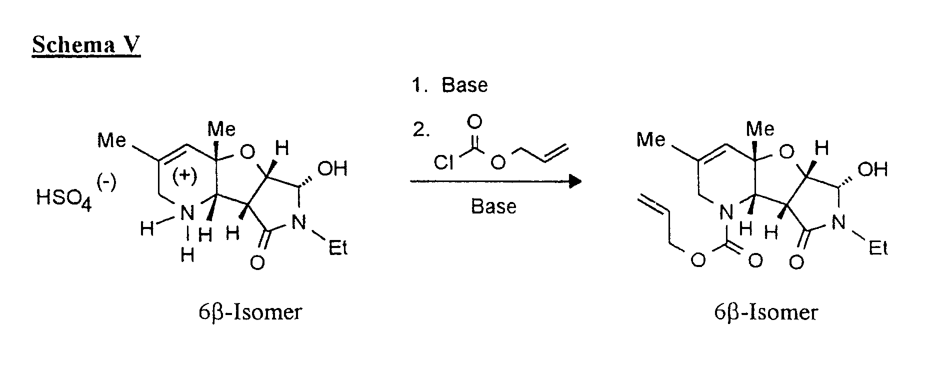

Die zur Durchführung des erfindungsgemäßen Verfahrens 2b als Ausgangsstoffe benötigten 6-Hydroxy-1,2,4a,5a,8a,8b-hexahydro-6H-pyrrolo[3',4':4,5]furo[3,2-b]pyrid-8(7H)-on Derivate und deren Salze sind durch die Formeln (Ic) allgemein definiert. In diesen Formeln (Ic) stehen R¹, R², R³, R⁴ vorzugsweise für diejenigen Reste, die bereits im Zusammenhang mit der Beschreibung der erfindungsgemäßen Stoffe der allgemeinen Formel (I) als bevorzugt für diese Substituenten genannt werden.

Die als Ausgangsmaterialien verwendeten 6-Hydroxy-1,2,4a,5a,8a,8b-Hexahydro-6Hpyrrolo[3',4':4,5]furo[3,2-b]pyrid-8(7H)-on Derivate und deren Salze der allgemeinen Formeln (Ic) sind neu und können nach dem weiter oben beschriebenen Hydrierungsverfahren aus den Derivaten der allgemeinen Formeln (III) erhalten werden.

In den Formeln (V) haben 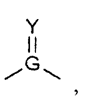 Q und W die Bedeutung die bereits im Zusammenhang mit der Beschreibung der erfindungsgemäßen Stoffe der allgemeinen Formel (I) als bevorzugt für diese Substituenten genannt wurde.

Die Verbindungen der Formel (V) sind allgemein bekannte Verbindungen der organischen Chemie bzw. können z.T. kommerziell oder nach literaturbekannten Methoden erhalten werden (z.B.: persubstituierte Allophansäurehalogenide: DE-OS 2 008 116; Carbamoylchloride: Liebigs Ann. 229, S. 85; Carbamate: Houben Weyl, Methoden der organischen Chemie, Band E 4).

Die Freisetzung der Verbindungen (Ic) aus deren Salze und die darauf folgende Umsetzung mit den Verbindungen der allgemeinen Formeln (V) führt man vorzugsweise in Gegenwart eines basischen Reaktionshilfsmittels unter Verwendung von Verdünnungsmitteln durch.

Als Verdünnungsmittel zur Durchführung des erfindungsgemäßen Verfahrens 2b finden inerte, aprotische Lösungsmittel wie z. B. Dioxan, Acetonitril oder Tetrahydrofuran aber auch Halogenkohlenwasserstoffe, insbesondere Chlorkohlenwasserstoffe, wie Methylenchlorid oder Chloroform, Verwendung.

Als basische Reaktionshilfsmittel zur Durchführung des erfindungsgemäßen Verfahrens 2b können alle geeigneten Säurebindemittel eingesetzt werden wie Amine, insbesondere tertiäre Amine sowie Alkali- und Erdalkaliverbindungen.

Beispielhaft seien dafür erwähnt die Hydride, Hydroxide, Oxide und Carbonate des Lithiums, Natriums, Kaliums, Magnesiums, Calciums und Bariums, ferner weitere basische Verbindungen wie Amidinbasen oder Guanidinbasen wie 7-Methyl-1,5,7-triazabicyclo(4.4.0)dec-5-en (MTBD); Diazabicyclo(4.3.0)nonen (DBN), Diazabicyclo (2.2.2.)octan (DABCO), 1,8-Diazabicyclo(5.4.0)undecen (DBU), Cyclohexyltetra-butylguanidin (CyTBG), Cyclohexyltetramethylguanidin (CyTMG), N,N,N,N-Tetramethyl-1,8-naphthalindiamin, Pentamethylpiperidin, tertiäre Amine wie Triethylamin, Trimethylamin, Tribenzylamin, Triisopropylamin, Tributylamin, Tribenzylamin, Tricyclohexylamin, Triamylamin, Trihexylamin, N,N-Dimethylanilin, N,N-Dimethyltoluidin, N,N-Dimethyl-p-aminopyridin, N-Methyl-pyrrolidin, N-Methylpiperidin, N-Methylimidazol, N-Methyl-pyrrol, N-Methyl-morpholin, N-Methyl-hexamethylenimin, Pyridin, 4-Pyrrolidino-pyridin, 4-Dimethylamino-pyridin, Chinolin, α-Picolin, β-Picolin, Isochinolin, Pyrimidin, Acridin, N,N,N',N'-Tetramethylendiamin, N,N',N'-Tetraethylendiamin, Chinoxalin, N-Propyldiisopropylamin, N-Ethyldiisopropylamin, N,N'-Dime-thylcyclohexylamin, 2,6-Lutidin, 2,4-Lutidin oder Triethylendiamin.

Vorzugsweise finden tertiäre Amine, insbesondere Trialkylamine wie Triethylamin, N,N-Diisopropylethylamin, n-Propyl-diisopropylamin, N,N'-Dimethyl-cyclohexylamin oder N-Methylmorpholin sowie Pyridinderivate, insbesondere 4-Pyrrolidino-pyridin oder 4-Dimethylaminopyridin Verwendung.

Das Verfahren 2b wird durchgeführt, indem man zunächst aus gegebenenfalls vorliegenden Salzen der Verbindungen der allgemeinen Formel (Ic) in Gegenwart eines basischen Reaktionshilfsmittels die Verbindungen der allgemeinen Formel (Ic) freisetzt und in einem zweiten Reaktionsschritt mit Verbindungen der allgemeinen Formeln (V) in einem der angegebenen Verdünnungsmittel umsetzt.

Die Reaktionsdauer beträgt 4 bis 72 Stunden. Die Umsetzung erfolgt bei Temperaturen zwischen -10°C und +150°C, bevorzugt zwischen -5°C und +80°C, besonders bevorzugt bei 0°C bis Raumtemperatur. Es wird unter Normaldruck gearbeitet. Zur Durchführung des erfindungsgemäßen Verfahrens 2 setzt man pro Mol an Verbindung der Formel (Ic) im allgemeinen 1,0 bis 3,0 Mol, vorzugsweise 1,0 bis 1,5 Mol Acylierungsagenz, ein.

Nach vollendeter Umsetzung wird die Reaktionslösung gewaschen, die organische Phase abgetrennt, getrocknet und im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Alternativ kann die Herstellung der Carbamate natürlich auch aus Verbindungen der allgemeinen Formel (Ic) und (Id), Kohlendioxid und einem Alkylierungsmittel der allgeminen Formel (IV) in Gegenwart von basischen Alkalimetall-, Erdalkalimetalloder Ammoniumsalzen erfolgen (vgl. dazu: EP-OS 511 948, EP-OS 628 542 und dort zitierte Literatur, DE-A 195 38960.3).

Setzt man beispielsweise bei Verfahren 2d und 2e zur Herstellung der neuen 6-Hydroxy-1,2,4a,5a,8a,8b-(±)-hexahydro-6H-pyrrolo[3',4':4,5]furo[3,2-b]pyrid-8(7H)-one der allgemeinen Formeln (Ia) als Verbindungen der allgemeinen Formel (Ic) 7-Ethyl-6β-hydroxy-1,2,4aα,5aα,8aα,8bα-(±)-hexahydro-3,4a-dimethyl-6H-pyrrolo[3',4':4,5]furo[3,2-b]pyrid-8(7H)-on und als Verbindungen der allgemeinen Formeln (VII) und (VIII) N-Benzyloxycarbonylsarkosin (Z-Sar-OH) bzw. Methylisocyanat ein, so lassen sich die Verfahren durch das folgende Reaktionsschema VI wiedergeben:

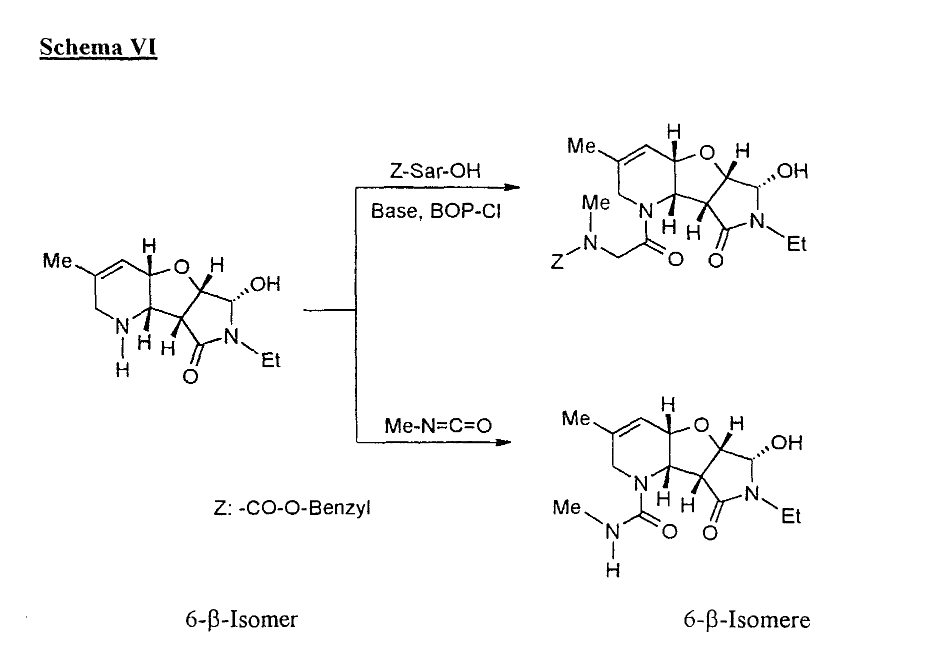

Die zur Durchführung des erfindungsgemäßen Verfahrens 2d und 2e als Ausgangsstoffe benötigten 6-Hydroxy-1,2,4a,5a,8a,8b-hexahydro-6H-pyrrolo[3',4':4,5]furo[3,2-b]pyrid-8(7H)-on Derivate sind durch die Formeln (Ic) allgemein definiert. In diesen Formeln (Ic) stehen R¹, R², R³, R⁴ vorzugsweise für diejenigen Reste, die bereits im Zusammenhang mit der Beschreibung der erfindungsgemäßen Stoffe der allgemeinen Formel (I) als bevorzugt für diese Substituenten genannt werden.

Die insbesondere für die Durchführung des erfindungsgemäßen Verfahrens 2d als Ausgangsstoffe zu verwendenden Verbindungen sind allgemein durch die Formel (VII) definiert.

In der Formel (VII) haben R⁸, R⁹, R¹⁰, 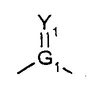 und Q¹ die Bedeutung die bereits im Zusammenhang mit der Beschreibung der erfindungsgemäßen Stoffe der allgemeinen Formel (I) als bevorzugt für diese Substituenten genannt wurde.

Die als Ausgangsstoffe verwendeten natürlichen oder synthetischen Aminosäuren können, falls chiral, in der (S)- oder (R)-Form (bzw. L- oder D-Form) vorliegen.

### Beispielsweise seien genannt:

Aad, Abu, γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, (Cys)₂, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, HyI, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp,Trta, Tyr, Val, Nal, Tbg, Npg, Chg, Thia, (vgl. z.B. Houben-Weyl, Methoden der Organischen Chemie, Band XV/1 und 2, Stuttgart, 1974).

Die Verbindungen der allgemeinen Formel (VII) können z.T. kommerziell oder nach literaturbekannten Methoden erhalten werden (vgl. z. B.: N-Methylaminosäuren: R. Bowmann et al. J. Chem. Soc. (1950) S. 1346; J. R. McDermott et al. Can. J. Chem. 51 (1973) S. 1915; H. Wurziger et al. Kontakte (Merck, Darmstadt) 3 (1987) S. 8).

Die Umsetzung der 6-Hydroxy-1,2,4a,5a,8a,8b-hexahydro-6H-pyrrolo[3',4':4,5]furo [3,2-b]pyrid-8(7H)-on Derivate der allgemeinen Formel (Ic) mit Aminosäurederivaten der Formel (VII) führt man vorzugsweise in Gegenwart von Kupplungsreagenzien und in Gegenwart eines basischen Reaktionshilfsmittels unter Verwendung von Verdünnungsmitteln durch.

Als Kupplungsreagenzien zur Durchführung des Verfahrens 2d finden alle, die zur Herstellung einer Amidbindung geeignet sind (vgl. z. B. Houben-Weyl, Methoden der Organischen Chemie, Band 15/2; Bodansky et al., Peptide Synthesis 2nd ed. (Wiley & Sons, New York 1976) oder Gross, Meienhofer, The Peptides: Analysis Synthesis, Biology (Academic Press, New York 1979), Verwendung. Vorzugsweise werden folgende Methoden herangezogen: Aktivestermethode mit Pentachlor- (Pcp) und Pentafluorphenol (Pfp), N-Hydroxysuccinimid (HOSu), N-Hydroxy-5-norbornen-2,3-dicarbox-amid (HONB), 1-Hydroxy-benzotriazol (HOBt) oder 3-Hydroxy-4-oxo-3,4-dihydro-1,2,3-benzotriazin als Alkoholkomponente, Kupplung mit Carbodiimiden wie Dicyclohexylcarbodiimid (DCCI), nach dem DCC-Additiv-Verfahren, oder mit n-Propanphosphonsäureanhydrid (PPA) und Gemischt-Anhydrid-Methode mit Pivaloylchlorid, Ethyl- (EEDQ) und Isobutylchlorformiat (IIDQ) oder Kupplung mit Phosphoniumreagenzien, wie Benzotriazol-1-yl-oxy-tris(dimethylaminophosphonium)-hexafluorophosphat (BOP), Bis(2-oxo-3-oxa-zolidinyl)-phosphoniumsäurechlorid (BOP-Cl), Benzotriazol-1-yl-tris-pyrrolidino-phosphonium-hexafluorophosphat (PyBOP®), Bromo-tris-pyrrolidino-phosphonium-hexafluorophosphat (PyBroP®), oder mit Phosphonsäureesterreagenzien, wie Cyanphosphonsäurediethylester (DEPC) und Diphenylphosphorylazid (DPPA), Uroniumreagenzien, wie 2-(1H-Benzotriazol-1-yl)-1,1,3, 3-tetramethyluronium-tetrafluoroborat (TBTU), 2-(5-Norbornen-2,3-dicarboxamido)-1,1,3,3-tetramethyluronium-tetrafluoroborat (TNTU), 2-(2-Oxo-1(2H)-pyridyl)-1,1,3,3-bispentamethylen-tetramethyluronium-tetrafluoroborat (TOPPipU), O-(N-Succin-imidyl-1,1,3,3-tetramethyluronium-tetrafluoroborat (TSTU) oder wie 2-(1H-Benzo-triazol-1-yl)-1,1,3,3-tetramethyluronium-hexafluorophosphat (HBTU).

Bevorzugt ist die Kupplung mit Phosphoniumreagenzien wie Benzotriazol-1-yl-oxytris(dimethylaminophosphonium)-hexafluorophosphat (BOP), Bis(2-oxo-3-oxazolidinyl)-phosphoniumsäurechlorid (BOP-Cl), Benzotriazol-1-yl-tris-pyrrolidino-phosphonium-hexafluorophosphat (PyBOP®), Bromo-tris-pyrrolidinophosphonium-hexafluorophosphat (PyBroP®), und Phosphonsäurereagenzien, wie Cyanphosphonsäurediethylester (DEPC) oder Diphenylphosphorylazid (DPPA).

Als basische Reaktionshilfsmittel zur Durchführung des erfindungsgemäßen Verfahrens 2d können ebenso alle für das Verfahren 2b geeigneten Säurebindemittel eingesetzt werden.

Vorzugsweise kommen tertiäre Amine, insbesondere Trialkylamine wie Triethylamin, N,N-Diisopropylethylamin, N-Propyldiisopropylamin, N,N'-Dimethylcyclohexylamin oder N-Methylmorpholin in Frage.

Als Verdünnungsmittel zur Durchführung des erfindungsgemäßen Verfahrens 2d finden die bei Verfahren 2b genannten Lösungsmittel wie z. B. Halogenkohlenwasserstoffe, insbesondere Chlorkohlenwasserstoffe, wie Methylenchlorid, Chloroform oder 1,2-Dichlorethan und Gemische von diesen mit anderen genannten Verdünnungsmittel, Verwendung.

Das Verfahren 2d wird im allgemeinen so durchgeführt, indem man Verbindungen der allgemeinen Formel (Ic) in Gegenwart eines der angegebenen Kupplungsreagenzien und in Gegenwart eines der angegebenen basischen Reaktionshilfsmittels mit Verbindungen der allgemeinen Formeln (VII) und in einem der angegebenen Verdünnungsmittel umsetzt.

Die Reaktionsdauer beträgt 4 bis 72 Stunden. Die Umsetzung erfolgt bei Temperaturen zwischen -10°C und +120°C, bevorzugt zwischen -5°C und +50°C, besonders bevorzugt bei 0°C bis Raumtemperatur. Es wird unter Normaldruck gearbeitet.

Zur Durchführung des erfindungsgemäßen Verfahrens 2d setzt man pro Mol an Verbindung der Formel (Ic) im allgemeinen 1,0 bis 3,0 Mol, vorzugsweise 1,0 bis 1,5 Mol Kupplungsreagenz, ein.

Die insbesondere für die Durchführung des erfindungsgemäßen Verfahrens 2e als Ausgangsstoffe zu verwendenden Verbindungen sind allgemein durch die Formel (VIII ) oder (IX) definiert.

In diesen Formeln (VII) oder (IX) haben R¹², Y, 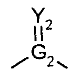 und Z die Bedeutung die bereits im Zusammenhang mit der Beschreibung der erfindungsgemäßen Stoffe der allgemeinen Formel (I) als bevorzugt für diese Substituenten genannt wurde.

Die Verbindungen der allgemeinen Formeln (VII) oder (IX) können z. T. kommerziell oder nach literaturbekannten Methoden erhalten werden (vgl. z.B.: Houben-Weyl, Methoden der organischen Chemie, Band E 4).

Die Umsetzung der 6-Hydroxy-1,2,4a,5a,8a,8b-hexahydro-6H-pyrrolo[3',4':4,5]furo [3,2-b]pyrid-8(7H)-on Derivate der allgemeinen Formel (Ic) mit Verbindungen der allgemeinen Formeln (VII) oder (IX) führt man vorzugsweise in Gegenwart von Verdünnungsmitteln, gegebenenfalls in Gegenwart eines basischen Reaktionshilfsmittels, durch.

Als Verdünnungsmittel zur Durchführung des erfindungsgemäßen Verfahrens 2e finden die bei Verfahren 2b genannten Lösungsmittel wie z.B. Halogenkohlenwasserstoffe, insbesondere Chlorkohlenwasserstoffe, wie Methylenchlorid, Chloroform oder 1,2-Dichlorethan, Nitrile wie Acetonitril, Propionitril, Butyronitril, insbesondere Acetonitril, Ether wie Ethylpropylether, n-Butylether, Diethylether, Dipropylether, Diisopropylether, Tetrahydrofuran oder Dioxan, insbesondere Tetrahydrofuran oder Dioxan, aliphatische oder aromatische Kohlenwasserstoffe wie n-Hexan, n-Heptan, Benzol, Toluol oder Xylene und Gemische von diesen mit anderen genannten Verdünnungsmittel, Verwendung.

Das Verfahren 2e kann auch in Gegenwart von basischen Reaktionshilfsmitteln durchgeführt werden. Als solche basischen Reaktionshilfsmittel zur Durchführung des erfindungsgemäßen Verfahrens 2e können alle weiter oben genannten Säurebindemittel, vorzugsweise jedoch tertiäre Amine, insbesondere Trialkylamine wie Triethylamin, N,N-Diisopropylethylamin oder N-Methylmorpholin, und Amidinbasen oder Guanidinbasen wie Diazabicyclo(4.3.0)nonen (DBN), Diazabicyclo (2.2.2.)octan (DABCO), 1,8-Diazabicyclo(5.4.0)undecen (DBU), insbesondere 1,8-Diazabicyclo(5.4.0)undecen (DBU), Verwendung.

Das Verfahren 2e wird im allgemeinen so durchgeführt, indem man Verbindungen der allgemeinen Formel (Ic) mit Verbindungen der allgemeinen Formeln (VII) oder (IX), gegebenenfalls in Gegenwart eines der angegebenen basischen Reaktionshilfsmittel mit Verbindungen der allgemeinen Formeln (VII) in einem der angegebenen Verdünnungsmittel umsetzt.

Die Reaktionsdauer beträgt 4 bis 72 Stunden. Die Umsetzung erfolgt bei Temperaturen zwischen -10°C und +180°C, bevorzugt zwischen -5°C und +120°C, besonders bevorzugt bei 0°C bis Siedetemperatur des verwendeten Vedünnungsmittels.

Es kann grundsätzlich unter Normaldruck gearbeitet, aber auch bei erhöhtem oder erniedrigtem Druck gearbeitet werden. Vorzugsweise arbeitet man bei Normaldruck oder bei Drucken bis zu 15 bar.

Zur Durchführung des erfindungsgemäßen Verfahrens 2e setzt man pro Mol an Verbindung der Formel (Ic) im allgemeinen 1,0 bis 3,0 Mol, vorzugsweise 1,0 bis 1,5 Mol an Verbindung der allgemeinen Formeln (VII) oder (IX), ein.

Nach vollendeter Umsetzung wird die Reaktionslösung gewaschen, die organische Phase abgetrennt, getrocknet und im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Setzt man beispielsweise bei Verfahren 3a zur Herstellung der neuen 6-substituierten 1,2,4a,5a,8a,8b-Hexahydro-/1,2,3,4,4a,5a,8a,8b-Oktahydro-6H-pyrrolo[3',4':4,5]furo[3,2-b]pyrid-8(7H)-on Derivate der allgemeinen Formeln (I) als Verbindungen der allgemeinen Formel (Ia) und (Ib) 1-Allyloxycarbonyl-6β-hydroxy-1,2,4aα,5aα,8aα,-8bα-(±)-hexahydro-3,4a,7-trimethyl- und 1-Allyloxycarbonyl-6β-hydroxy-1,2, 4aα,5aα,8aα,8bα-(±)-oktahdro-3,4a,7-trimethyl-6H-pyrrolo[3',4':4,5]furo[3,2-b]pyrid-8(7H)-on und als Verbindungen der allgemeinen Formel (X) Ethanol (R¹⁶: - Ethyl; X: -O-) oder Natronlauge (R¹⁶: -H; X: -O-) ein, so läßt sich das Verfahren durch das folgende Reaktionsschema VII wiedergeben:

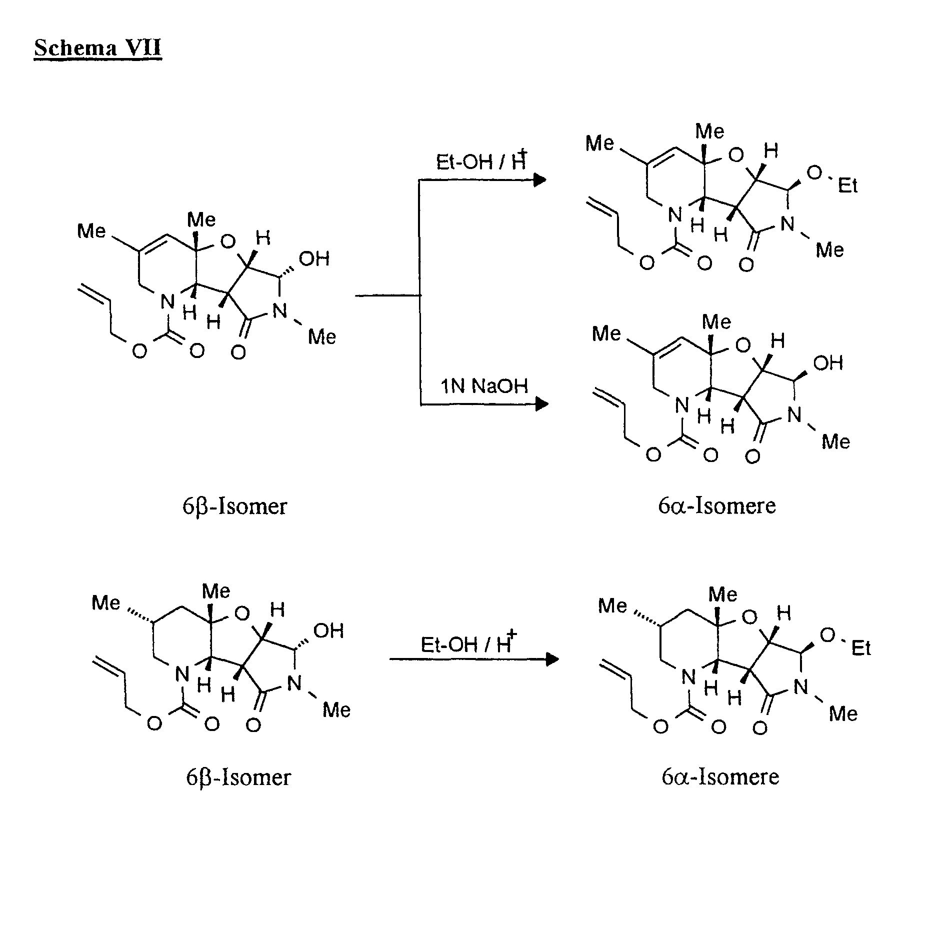

Bei Verwendung der 6β-Isomere von Verbindungen der allgemeinen Formeln (Ia) und (Ib) kann bei dem erfindungsgemäßen Verfahren 3 eine Inversion in 6-Position unter Ausbildung entsprechender 6α-Isomere erfolgen.

Die zur Durchführung des erfindungsgemäßen Verfahrens 3a als Ausgangsstoffe benötigten 6-Hydroxy-1,2,4a,5a,8a,8b-hexahydro-/ 1,2,3,4,4a,5a,8a,8b-oktahydro-6Hpyrrolo[3',4':4,5]furo[3,2-b]pyrid-8(7H)-on Derivate sind durch die Formeln (Ia) und (Ib) allgemein definiert. In diesen Formeln (Ia) und (Ib) stehen R¹, R², R³, R⁴ und B vorzugsweise für diejenigen Reste, die bereits im Zusammenhang mit der Beschreibung der erfindungsgemäßen Stoffe der allgemeinen Formel (I) als bevorzugt für diese Substituenten genannt werden.

Die als Ausgangsmaterialien verwendeten 6-Hydroxy-1,2,4a,5a,8a,8b-hexahydro-/1, 2,3,4,4a,5a,8a,8b-Oktahydro-6H-pyrrolo[3',4':4,5]furo[3,2-b]pyrid-8(7H)-on Derivate der allgemeinen Formeln (Ia) und (Ib) sind neu und können nach dem weiter oben beschriebenen Hydrierungsverfahren aus den 1,2,4a,5a,8a,8b-Hexahydro-/1,2,3,4,4a,5a,8a,8b-Oktahydro-6H-pyrrolo[3',4':4,5]furo[3,2-b]pyrid-6,8(7H)-dion Derivaten der allgemeinen Formeln (II) und (III) erhalten werden.

Die außerdem für die Durchführung des erfindungsgemäßen Verfahrens 3a als Ausgangsstoffe zu verwendenden Verbindungen sind allgemein durch die Formel (X) definiert.

In der Formel (X) haben R¹⁶ und X die Bedeutung die bereits im Zusammenhang mit der Beschreibung der erfindungsgemäßen Stoffe der allgemeinen Formel (I) als bevorzugt für diese Substituenten genannt wurde.

Im allgemeinen ist es vorteilhaft das erfindungsgemäße Verfahren 3a in Gegenwart von Verdünnungsmitteln und gegebenenfalls in Gegenwart eines sauren oder basischen Reaktionshilfsmittels durchzuführen.

Als Verdünnungsmittel zur Durchführung des erfindungsgemäßen Verfahrens 3a kommen alle in Verfahren 2 genannten inerten Lösungsmittel in Frage.

Bevorzugte Verdünnungsmittel sind inerte organische Lösungsmittel, wie z.B. Alkohole, insbesondere Methanol, Ethanol, Propanol und Butanol.

Als saure Reaktionshilfsmittels zur Durchführung des erfindungsgemäßen Verfahrens 3a können alle geeigneten Mineralsäuren eingesetzt werden. Als solche seien praktisch alle Mineralsäuren genannt. Zu den Mineralsäuren gehören vorzugsweise Halogenwasserstoffsäuren wie Fluorwasserstoffsäure, Bromwasserstoffsäure, Chlorwasserstoffsäure oder Iodwasserstoffsäure sowie Schwefelsäure Schweflige Säure, Phosphorsäure, Phosphorige Säure und Salpetersäure.

Bevorzugt zur Durchführung des erfindungsgemäßen Verfahrens 3a werden Schwefelsäure und Chlorwasserstoffsäure eingesetzt.

Das Verfahren 3a wird durchgeführt, indem man Verbindungen der allgemeinen Formeln (Ia) und (Ib) in Gegenwart eines sauren Reaktionshilfsmittels mit Verbindun-gen der allgemeinen Formel (X) in einem der angegebenen Verdünnungsmittel umsetzt.

Die Reaktionsdauer beträgt 30 Minuten bis 48 Stunden. Die Umsetzung erfolgt bei Temperaturen zwischen -60°C und +100°C, bevorzugt zwischen -30°C und +80°C, besonders bevorzugt bei -10°C bis Raumtemperatur. Es wird unter Normaldruck gearbeitet.

Zur Durchführung des erfindungsgemäßen Verfahrens 3a setzt man pro Mol an Verbindungen der allgemeinen Formeln (Ia) und (Ib) im allgemeinen einen Überschuß an Verbindungen der allgemeinen Formel (X) und eine katalytische Menge an Reaktionshilfsmittel ein.

Nach vollendeter Umsetzung wird die gesamte Reaktionslösung neutralisiert, im Vakuum eingeengt, der verbleibende Rückstand in einem der genannten Verdünnungsmittel aufgenommen und gewaschen. Nach Abtrennen der organischen Phase wird erneut eingeengt und die anfallenden Produkte lassen sich in üblicher Weise durch Umkristallisieren, Vakuumdestillation oder Säulenchromatographie reinigen (vgl. auch die Herstellungsbeispiele).

Alternativ können die 6-Alkoxy-Derivate auch in einer Zweistufenreaktion via O-Mesylierung in Gegenwart einer Base und anschließender Reaktion mit dem entsprechenden Alkohol erhalten werden (vgl. W. N. Speckamp, H. Hiemstra, Tetrahedron, Vol. 41, No. 20 (1985), S. 4367-4416).

Setzt man bei den Verfahren 3b und 3c zur Herstellung der neuen 6-substituierten 1,2,4a,5a,8a,8b-Hexahydro-6H-pyrrolo[3',4':4,5]furo[3,2-b]pyrid-8(7H)-on Derivate der allgemeinen Formeln (Ie) als Verbindungen der allgemeinen Formel (Ia) 1-Allyloxycarbonyl-6β-hydroxy-1,2,4aα,5aα,8aα,8bα-(±)-hexahydro-3,4a,7-trimethyl-6H-pyrrolo[3',4':4,5]furo[3,2-b]pyrid-8(7H)-on und als Verbindungen der allgemeinen Formel (V) Cyclopropancarbonsäurechlorid und als Verbindungen der allgemeinen Formel (VI) Essigsäureanhydrid ein, so lassen sich die Verfahren durch folgendes Reaktionsschema VIII wiedergeben:

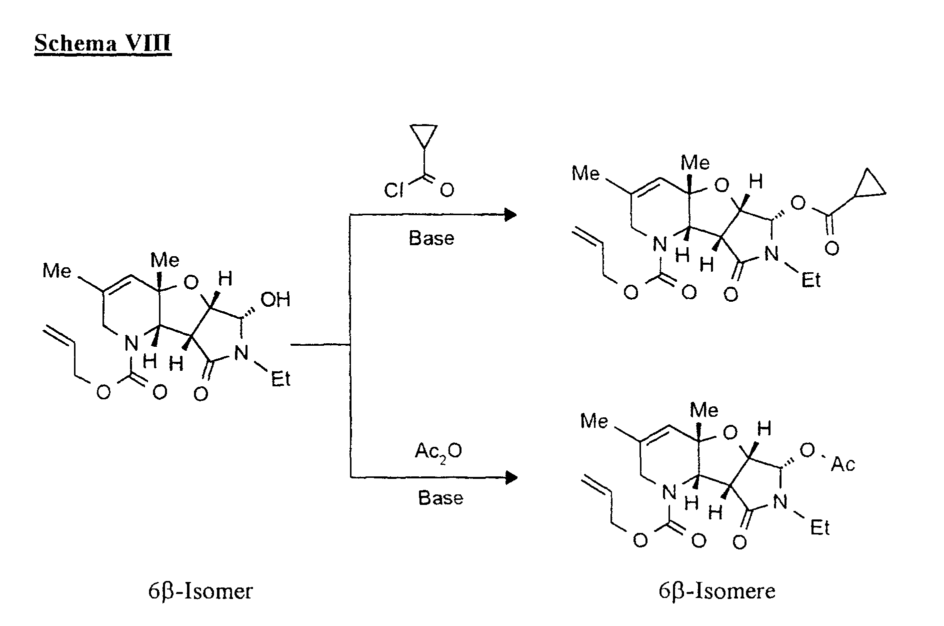

Im Falle der Verwendung von Verbindungen der allgemeinen Formel (Ie) als 6β-Isomere, erfolgt zumeist bei den erfindungsgemäßen Acylierungen keine Inversion in der 6-Position bzw. liegt das entstandene 6β-Isomere im Übeschuß vor.

Die zur Durchführung des erfindungsgemäßen Verfahrens 3 als Ausgangsstoffe benötigten 6-Hydroxy-1,2,4a,5a,8a,8b-hexahydro-/ 1,2,3,4,4a,5a,8a,8b-oktahydro-6H-pyrrolo[3',4':4,5]furo[3,2-b]pyrid-8(7H)-on Derivate sind durch die Formel (Ia) allgemein definiert. In dieser Formel (Ia) stehen R¹,R², R³, R⁴ und B vorzugsweise für diejenigen Reste, die bereits im Zusammenhang mit der Beschreibung der erfindungsgemäßen Stoffe der allgemeinen Formel (I) als bevorzugt für diese Substituenten genannt werden.

Die als Ausgangsmaterialien verwendeten 6-Hydroxy-1,2,4a,5a,8a,8b-hexahydro-6Hpyrrolo[3',4':4,5]furo[3,2-b]pyrid-8(7H)-on Derivate der allgemeinen Formel (Ia) sind neu und können nach dem weiter oben beschriebenen Hydrierungsverfahren aus den entsprechenden 1,2,4a,5a,8a,8b-Hexahydro-6H-pyrrolo[3',4':4,5]furo[3,2-b]pyrid-6,8(7H)-dion Derivaten der allgemeinen Formel (III) erhalten werden.

Setzt man hingegen bei den Verfahren 3e und 3f zur Herstellung der neuen 6-substituierten 1,2,4a,5a,8a,8b-Hexahydro-6H-pyrroio[3',4':4,5]furo[3,2-b]pyrid-8(7H)-on Derivate der allgemeinen Formeln (Ie) und (Ig) als Verbindungen der allgemeinen Formel (Ic) 7-Alkyl-6β-hydroxy-1,2,4aα,5aα,8aα,8bα-(±)-hexahydro-3,4adimethyl-6H-pyrrolo[3',4':4,5]furo[3,2-b]pyrid-8(7H)-on und als Verbindungen der allgemeinen Formel (V) Chlorameisensäureallylester und als Verbindungen der allgemeinen Formel (VII) N-Benzyloxycarbonyl-N-methyl-leucin (Z-MeLeu-OH) ein, so lassen sich die Verfahren durch das folgende Reaktionsschema IX wiedergeben:

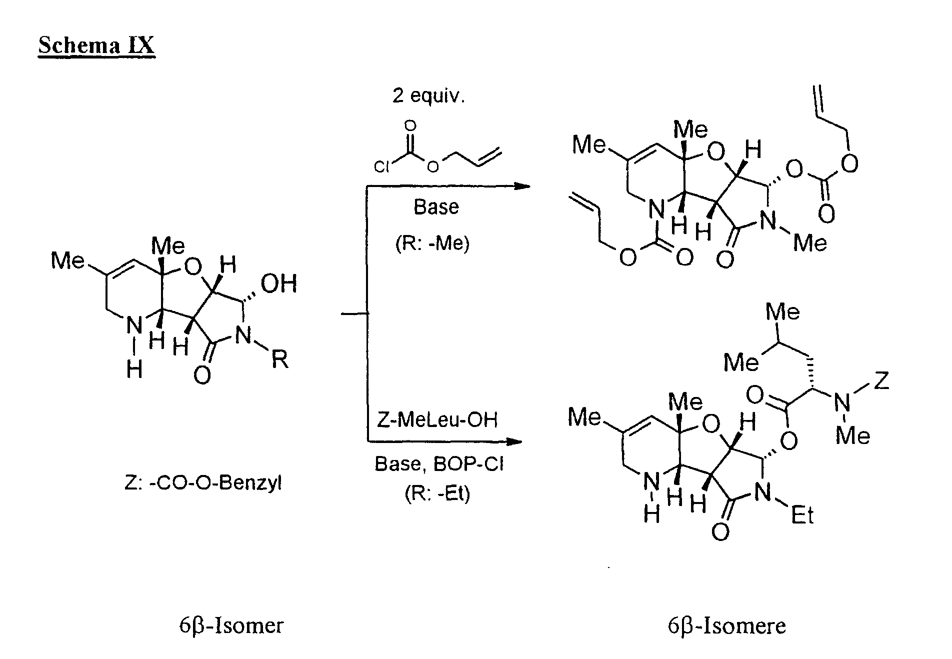

Die außerdem für die Durchführung der erfindungsgemäßen Verfahren 3e und 3f als Ausgangsstoffe zu verwendenden Verbindungen sind allgemein durch die Formeln (V) und (VII) definiert.

In den Formeln (V) und (VII) haben 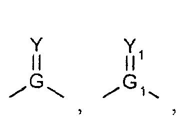 Q und W, R⁸, R⁹ und R¹⁰ die Bedeutung, die bereits im Zusammenhang mit der Beschreibung der erfindungsgemäßen Stoffe der allgemeinen Formel (I) als bevorzugt für diese Substituenten genannt werden.

Die Verbindungen der Formel (V) sind allgemein bekannte Verbindungen der organischen Chemie bzw. können z. T. kommerziell oder nach literaturbekannten Methoden erhalten werden (z. B.: persubstituierte Allophansäurehalogenide: DE-OS 2 008 116; Carbamoylchloride: Liebigs Ann. 229, S. 85; Carbamate: Houben Weyl, Methoden der organischen Chemie, Band E 4).

Die verwendeten natürlichen oder synthetischen Aminosäuren der Formel (VII) sind ebenso allgemein bekannte Verbindungen der organischen Chemie bzw. können z. T. kommerziell oder nach literaturbekannten Methoden erhalten werden (vgl. Verfahren 2d).

Die Umsetzung der Verbindungen (Ic) mit den Verbindungen der allgemeinen Formeln (V) führt man vorzugsweise in Gegenwart von basischen Reaktionshilfs-mitteln unter Verwendung von Verdünnungsmitteln durch.

Als basische Reaktionshilfsmittel zur Durchführung des erfindungsgemäßen Verfahrens 3e können alle bei Verfahren 2 genannten Säurebindemittel, wie Amine, insbesondere tertiäre Amine sowie Alkali- und Erdalkaliverbindungen Verwendung finden.

Vorzugsweise finden bei Verfahren 3e die tertiären Amine, insbesondere Trialkylamine wie Triethylamin, N,N-Diisopropylethylamin, n-Propyldiisopropylamin, N,N'-Dimethylcyclohexylamin oder N-Methylmorpholin sowie Pyridinderivate, insbesondere Pyridin, 4-Pyrrolidino-pyridin oder 4-Dimethylaminopyridin Verwendung.

Selbstverständlich kann man in das erfindungsgemäße Verfahren 3e auch Gemische der genannten Säurebindemittel einsetzen.

Als Verdünnungsmittel zur Durchführung des erfindungsgemäßen Verfahrens 3e finden die bei Verfahren 2 genannten inerten, aprotischen Lösungsmittel wie z.B. Dioxan, Acetonitril oder Tetrahydrofuran aber auch Halogenkohlenwasserstoffe, insbesondere Chlorkohlenwasserstoffe, wie Methylenchlorid oder Chloroform, Verwendung.

Das Verfahren 3e wird durchgeführt, indem man Verbindungen der allgemeinen Formel (Ia) in Gegenwart von basischen Reaktionshilfsmitteln mit Verbindungen der allgemeinen Formeln (V) und (VI) in einem der angegebenen Verdünnungsmittel umsetzt.

Die Reaktionsdauer beträgt 4 bis 72 Stunden. Die Umsetzung erfolgt bei Temperaturen zwischen -10°C und +150°C, bevorzugt zwischen -5°C und +80°C, besonders bevorzugt bei 0°C bis Raumtemperatur. Es wird unter Normaldruck gearbeitet. Zur Durchführung des erfindungsgemäßen Verfahrens 3e setzt man pro Mol an Verbindung der Formel (Ia) im allgemeinen 2,0 bis 6,0 Mol, vorzugsweise 2,0 bis 3,0 Mol Acylierungsagenz, ein.

Die Umsetzung der 7-Alkyl-6-hydroxy-1,2,4a,5a,8a,8b-hexahydro-3,4a-dimethyl-6H-pyrrolo[3',4':4,5]furo[3,2-b]pyrid-8(7H)-on Derivate der allgemeinen Formel (Ic) mit Aminosäurederivaten der Formel (VII) führt man vorzugsweise in Gegenwart von Kupplungsreagenzien und in Gegenwart eines basischen Reaktionshilfsmittels unter Verwendung von Verdünnungsmitteln durch.

Als Kupplungsreagenzien zur Durchführung des Verfahrens 3f können ebenso alle für das Verfahren 2d geeigneten Kupplungsreagenzien eingesetzt werden.

Bevorzugt ist die Kupplung mit Phosphoniumreagenzien wie Benzotriazol-1-yl-oxytris(dimethylaminophosphonium)-hexafluorophosphat (BOP), Bis(2-oxo-3-oxazolidinyl)-phosphoniumsäurechlorid (BOP-Cl), Benzotriazol-1-yl-tris-pyrrolidino-phosphonium-hexafluorophosphat (PyBOP®), Bromo-tris-pyrrolidinophosphonium-hexafluorophosphat (PyBroP®), und Phosphonsäurereagenzien, wie Cyanphosphonsäurediethylester (DEPC) oder Diphenylphosphorylazid (DPPA).

Als basische Reaktionshilfsmittel zur Durchführung des erfindungsgemäßen Verfahrens 3f können ebenso alle für das Verfahren 2d geeigneten Säurebindemittel eingesetzt werden.

Vorzugsweise kommen tertiäre Amine, insbesondere Trialkylamine wie Triethylamin, N,N-Diisopropylethylamin, N-Propyldiisopropylamin, N,N'-Dimethylcyclohexylamin oder N-Methylmorpholin in Frage.

Als Verdünnungsmittel zur Durchführung des erfindungsgemäßen Verfahrens 3f finden die bei Verfahren 2d genannten Lösungsmittel wie z. B. Halogenkohlenwasserstoffe, insbesondere Chlorkohlenwasserstoffe, wie Methylenchlorid, Chloroform oder 1,2-Dichlorethan und Gemische von diesen mit anderen genannten Verdünnungsmittel, Verwendung.

Das Verfahren 3f wird im allgemeinen so durchgeführt, indem man Verbindungen der allgemeinen Formel (Ic) in Gegenwart eines der angegebenen Kupplungsrea-genzien und in Gegenwart eines der angegebenen basischen Reaktionshilfsmittels mit Verbindungen der allgemeinen Formeln (VII) und in einem der angegebenen Verdünnungsmittel umsetzt.

Die Reaktionsdauer beträgt 4 bis 72 Stunden. Die Umsetzung erfolgt bei Temperaturen zwischen -10°C und +120°C, bevorzugt zwischen -5°C und +50°C, besonders bevorzugt bei 0°C bis Raumtemperatur. Es wird unter Normaldruck gearbeitet.

Zur Durchführung des erfindungsgemäßen Verfahrens 3f setzt man pro Mol an Verbindung der Formel (Ic) im allgemeinen 1,0 bis 3,0 Mol, vorzugsweise 1,0 bis 1,5 Mol Kupplungsreagenz, ein.

Nach vollendeter Umsetzung wird die Reaktionslösung gewaschen, die organische Phase abgetrennt, getrocknet und im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Setzt man bei dem Verfahren 4a zur Herstellung der neuen 6-substituierten 1,2, 4a,5a,8a,8b-(±)-Hexahydro- und 6-substituierten 1,2,3,4,4a,5a,8a,8b-(±)-Oktahydro-6H-pyrrolo[3',4':4,5]furo[3,2-b]pyrid-8(7H)-on Derivate der allgemeinen Formeln (Ig,h ) als Verbindung der allgemeinen Formel (Ia) 1-Allyloxycarbonyl-6β-hydroxy-1,2,4aα,5aα,8aα,8bα-(±)-hexahydro-3,4a,7-trimethyl-6H-pyrrolo[3',4':4,5]furo[3,2-b]pyrid-8(7H)-on oder als Verbindungen der allgemeinen Formeln (Ie,f )) 1-Allyloxycarbonyl-6α-ethoxy-7-ethyl-1,2,4aα,5aα,8aα,8bα-(±)-hexahydro-3,4a-dimethyl- und 1-Allyloxycarbonyl-6α-ethoxy-1,2,3,4,4aα,5aα,8aα,8bα-(±)-oktahydro-3β,4a,7-trimethyl-6H-pyrrolo[3',4':4,5]furo[3,2-b]pyrid-8(7H)-on und als Verbindungen der allgemeinen Formel (X) Allyltrimethylsilan ein, so läßt sich das Verfahren durch das folgende Reaktionsschema X wiedergeben:

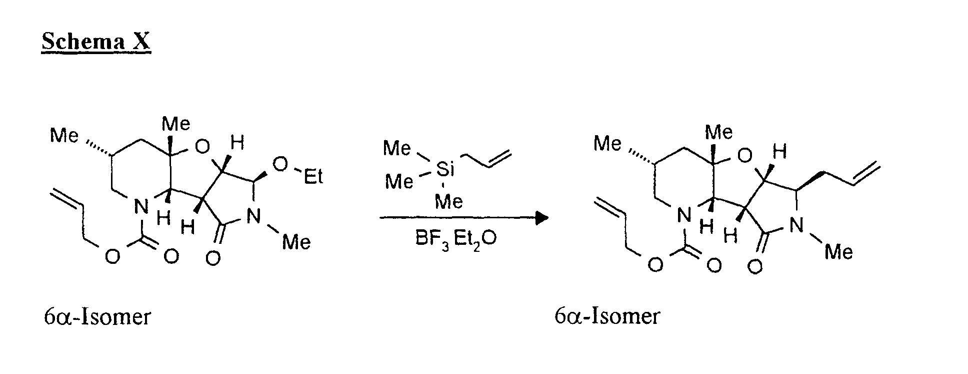

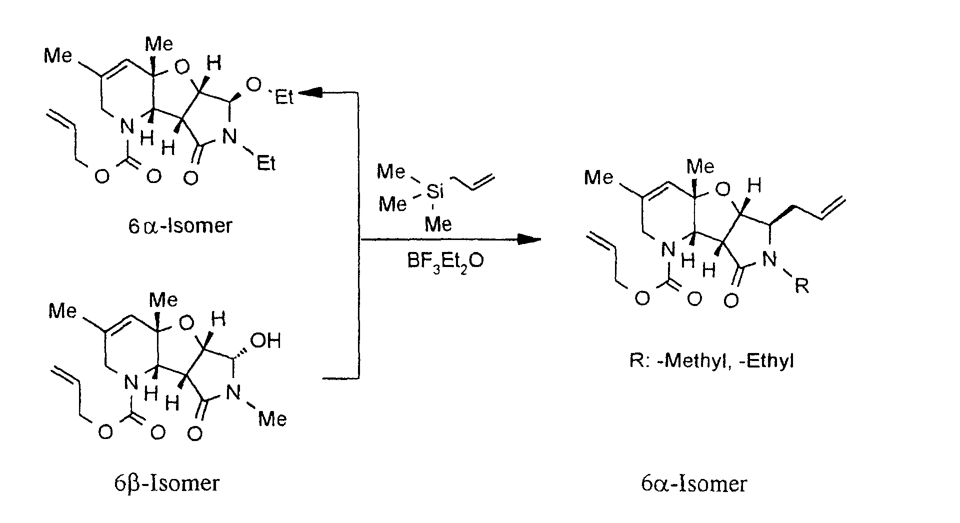

Die zur Durchführung des erfindungsgemäßen Verfahrens 4a als Ausgangsstoffe benötigten 6-Hydroxy-1,2,4a,5a,8a,8b-hexahydro- und 6-Hydroxy-1,2,3,4,4a,5a,8a, 8b-oktahydro-6H-pyrrolo[3',4':4,5]furo[3,2-b]pyrid-8(7H)-on Derivate sind durch die Formeln (Ia,b) allgemein definiert.

Die weiterhin zur Durchführung des erfindungsgemäßen Verfahrens 4a als Ausgangsstoffe benötigten 6-Ethoxy-1,2,4a,5a,8a,8b-hexahydro- und 6-Ethoxy-1,2,3,4, 4a,5a,8a,8b-oktahydro-6H-pyrrolo[3',4':4,5]furo[3,2-b]pyrid-8(7H)-on Derivate sind durch die Formeln (Ie,f) allgemein definiert.

In dieser Formeln (Ia,b) und (Ie,f) stehen R¹, R², R³, R⁴, R¹⁶, X und B vorzugswei-se für diejenigen Reste, die bereits im Zusammenhang mit der Beschreibung der erfindungsgemäßen Stoffe der allgemeinen Formel (I) als bevorzugt für diese Substituenten genannt werden.

Die als Ausgangsmaterialien verwendeten 6-Hydroxy-1,2,4a,5a,8a,8b-hexahydro- und 6-Hydroxy-1,2,3,4,4a,5a,8a,8b-oktahydro-6H-pyrrolo[3',4';4,5]furo[3,2-b]pyrid-8 (7H)-on Derivate der allgemeinen Formeln (Ia,b) sowie 6-Alkoxy-1,2,4a,5a,8a,8bhexahydro- und 6-Alkoxy-1,2,3,4,4a,5a,8a,8b-oktahydro-6H-pyrrolo[3',4':4,5]furo[3,2-b]pyrid-8(7H)-on Derivate der allgemeinen Formeln (Ie,f) sind neu und können nach den weiter oben beschriebenen Verfahren erhalten werden.

Die außerdem für die Durchführung des erfindungsgemäßen Verfahrens 4a als Ausgangsstoffe zu verwendenden Verbindungen sind allgemein durch die Formel (X) definiert.

In der Formel (X) haben R¹⁷, R¹⁸ und M die Bedeutung die bereits im Zusammenhang mit der Beschreibung der erfindungsgemäßen Stoffe der allgemeinen Formel (I) als bevorzugt für diese Substituenten genannt wurde.

Die metallorganischen Verbindungen der Formel (XI) sind allgemein bekannte Verbindungen der organischen Chemie bzw. können z.T. kommerziell oder nach literaturbekannten Methoden erhalten werden (vgl. Houben Weyl, Methoden der organischen Chemie, Band 13/5 und 13/6, 1980).

Die Umsetzung der Verbindungen (Ia,b) und (Ie,f) mit den Verbindungen der allgemeinen Formel (X) führt man vorzugsweise in Gegenwart eines Katalysators unter Verwendung von Verdünnungsmitteln durch (vgl. auch Houben-Weyl, Methoden der organischen Chemie, Band E 21, S. 1968).

Als Katalysatoren zur Durchführung des erfindungsgemäßen Verfahrens 4a können alle geeigneten Lewis-Säuren in Frage kommen, wie Aluminiumchlorid, Bortrifluorid oder sein Etherat, Titan(IV)-diisopropyloxydichlorid, Titan(IV)-chlorid, Zinn(IV)-chlorid, Zinn(II)-triflat, Zink(II)-chlorid, Zink(II)-bromid, Magnesium(II)-bromid, Ethyl-aluminiumdichlorid oder Trimethylsilyltriflat,

Bevorzugte Lewis-Säuren sind Bortrifluorid oder sein Etherat und Titan(IV)-chlorid.

Als Verdünnungsmittel zur Durchführung des erfindungsgemäßen Verfahrens 4a finden die bei Verfahren 2 genannten inerten, aprotischen Lösungsmittel wie z.B. Ether, Dioxan, Acetonitril oder Tetrahydrofuran, Halogenkohlenwasserstoffe, insbesondere Chlorkohlenwasserstoffe, wie Methylenchlorid oder Chloroform aber auch aromatische Kohlenwasserstoffe wie Benzol oder Toluol, Verwendung.

Das Verfahren 4a wird durchgeführt, indem man Verbindungen der allgemeinen Formeln (Ia,b) oder (Ie,f) in Gegenwart eines Katalysators mit Verbindungen der allgemeinen Formeln (XI) in einem der angegebenen Verdünnungsmittel umsetzt.

Die Reaktionsdauer beträgt 1 bis 48 Stunden. Die Umsetzung erfolgt bei Temperaturen zwischen -150°C und +100°C, bevorzugt zwischen -100°C und +50°C, besonders bevorzugt bei -85°C bis Raumtemperatur. Es wird unter Normaldruck und Schutzgasatmosphäre gearbeitet. Zur Durchführung des erfindungsgemäßen Verfahrens 4a setzt man pro Mol an Verbindung der Formeln (Ia,b) oder (Ie,f) im allgemeinen 1,0 bis 5,0 Mol, vorzugsweise 1,5 bis 3,0 Mol metalloranische Verbindung der Formel (XI), ein.

Nach vollendeter Umsetzung wird die Reaktionslösung gewaschen, die organische Phase abgetrennt, getrocknet und im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Setzt man bei dem Verfahren 4b zur Herstellung der neuen 6-substituierten 1,2, 4a,5a,8a,8b-(±)-Hexahydro-6H-pyrrolo[3',4':4,5]furo[3,2-b]pyrid-8(7H)-on Derivate der allgemeinen Formeln (Ii) als Verbindung der allgemeinen Formel (Ie) 1-Allyloxycarbonyl-6α-ethoxy-7-ethyl-1,2,4aα,5aα,8aα,8bα-(±)-hexahydro-3,4a-dimethyl-6H-pyrrolo[3',4':4,5]furo[3,2-b]pyrid-8(7H)-on und als Verbindung der allgemeinen Formel (XI) 1-Phenyltrimethylsiloxyethylen ein, so läßt sich das Verfahren durch das nachfolgende Reaktionsschema XI beschreiben:

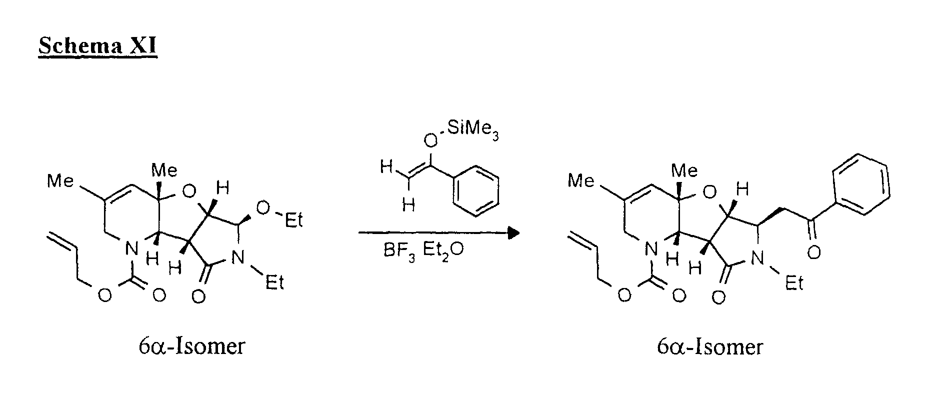

Die zur Durchführung des erfindungsgemäßen Verfahrens 4b als Ausgangsstoffe benötigten 6-Alkoxy-1,2,4a,5a,8a,8b-hexahydro-6H-pyrrolo[3',4':4,5]furo[3,2-b]pyrid-8(7H)-on Derivate sind durch die Formeln (Ie) allgemein definiert.

In dieser Formel (Ie) stehen R¹, R², R³, R⁴, R¹⁶, X und B vorzugsweise für diejeni-gen Reste, die bereits im Zusammenhang mit der Beschreibung der erfindungs-gemäßen Stoffe der allgemeinen Formel (I) als bevorzugt für diese Substituenten genannt werden.

Die als Ausgangsmaterialien verwendeten 6-Alkoxy-1,2,4a,5a,8a,8b-hexahydro-6Hpyrrolo[3',4':4,5]furo[3,2-b]pyrid-8(7H)-on Derivate der allgemeinen Formeln (Ie) sind neu und können nach den weiter oben beschriebenen Verfahren erhalten werden.

Die außerdem für die Durchführung des erfindungsgemäßen Verfahrens 4b als Ausgangsstoffe zu verwendenden Verbindungen sind allgemein durch die Formel (XII) definiert.

In der Formel (XII) haben R⁶, R⁷ und R¹⁹ die Bedeutung die bereits im Zusammenhang mit der Beschreibung der erfindungsgemäßen Stoffe der allgemeinen Formel (I) als bevorzugt für diese Substituenten genannt werden.

Die Verbindungen der Formel (XII) sind allgemein bekannte Verbindungen der organischen Chemie bzw. können z.T. kommerziell oder nach literaturbekannten Methoden erhalten werden (vgl. Houben Weyl, Methoden der organischen Chemie, Band 13/5 und 13/6, 1980).

Die Umsetzung der Verbindungen (Ie) mit den Verbindungen der allgeimenen Formel (XI) führt man vorzugsweise in Gegenwart eines Katalysators unter Verwendung von Verdünnungsmitteln durch.

Als Katalysatoren zur Durchführung des erfindungsgemäßen Verfahrens 4b können ebenso alle für das Verfahren 4a geeigneten Lewis-Säuren in Frage kommen.

Bevorzugte Lewis-Säuren für das Verfahren 4b sind Bortrifluorid oder sein Etherat Titan(IV)-chlorid oder Zinn(IV)-chlorid.

Als Verdünnungsmittel zur Durchführung des erfindungsgemäßen Verfahrens 4b finden die bei Verfahren 2 genannten inerten, aprotischen Lösungsmittel wie z.B. Ether, Dioxan, Acetonitril oder Tetrahydrofuran, Halogenkohlenwasserstoffe, insbesondere Chlorkohlenwasserstoffe, wie Methylenchlorid oder Chloroform aber auch aromatische Kohlenwasserstoffe wie Benzol oder Toluol, Verwendung.

Das Verfahren 4b wird durchgeführt, indem man Verbindungen der allgemeinen Formeln (Ie) in Gegenwart eines Katalysators mit Verbindungen der allgemeinen Formeln (XII) in einem der angegebenen Verdünnungsmittel umsetzt.

Die Reaktionsdauer beträgt 1 bis 48 Stunden. Die Umsetzung erfolgt bei Temperaturen zwischen -150°C und +100°C, bevorzugt zwischen -100°C und +50°C, besonders bevorzugt bei -85°C bis Raumtemperatur. Es wird unter Normaldruck und Schutzgasatmosphäre gearbeitet. Zur Durchführung des erfindungsgemäßen Verfahrens 4b setzt man pro Mol an Verbindung der Formeln (Ie) im allgemeinen 1,0 bis 5,0 Mol, vorzugsweise 1,5 bis 3,0 Mol Verbindung der Formel (XII), ein.

Nach vollendeter Umsetzung wird die Reaktionslösung gewaschen, die organische Phase abgetrennt, getrocknet und im Vakuum eingeengt. die anfallenden Produkte lassen sich in üblicher Weise durch Umkristallisieren, Vakuumdestillation oder Säulenchromatographie reinigen (vgl. auch die Herstellungsbeispiele).

Setzt man bei dem Verfahren 4c zur Herstellung der neuen 6-substituierten-1,2, 4a,5a,8a,8b-(±)-Hexahydro- und 6-substituierten-1,2,4a,5a,8a,8b-(±)-Oktahydro-6Hpyrrolo[3',4':4,5]furo[3,2-b]pyrid-8(7H)-on Derivate der allgemeinen Formeln (Ig,h) als Verbindung der allgemeinen Formel (Ie) 1-Allyloxycarbonyl-6α-ethoxy-7-ethyl-1,2,4aα,5aα,8aα,8bα-(±)-hexahydro-3,4a-dimethyl-6H-pyrrolo[3',4':4,5]furo[3,2-b]pyrid-8(7H)-on und als Verbindung der allgemeinen Formel (XIII) Furan ein, so läßt sich das Verfahren durch das Reaktionsschema XII wiedergeben:

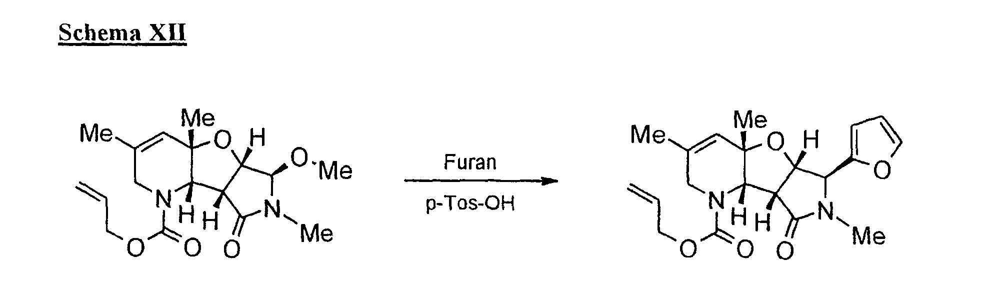

Die zur Durchführung des erfindungsgemäßen Verfahrens 4c als Ausgangsstoffe benötigten 6-Alkoxy-1,2,4a,5a,8a,8b-hexahydro-6H-pyrrolo[3',4':4,5]furo[3,2-b]pyrid-8 (7H)-on Derivate sind durch die Formeln (Ie) allgemein definiert.

In dieser Formel (Ie) stehen R¹, R², R³, R⁴, R¹⁶, X und B vorzugsweise für diejenigen Reste, die bereits im Zusammenhang mit der Beschreibung der erfindungsgemäßen Stoffe der allgemeinen Formel (I) als bevorzugt für diese Substituenten genannt werden.

Die als Ausgangsmaterialien verwendeten 6-Alkoxy-1,2,4a,5a,8a,8b-hexahydro-6Hpyrrolo[3',4':4,5]furo[3,2-b]pyrid-8(7H)-on Derivate der allgemeinen Formeln (Ie) sind neu und können nach den weiter oben beschriebenen Verfahren erhalten werden.

Die außerdem für die Durchführung des erfindungsgemäßen Verfahrens 4c als Ausgangsstoffe zu verwendenden Verbindungen sind allgemein durch die Formel (XIII) definiert.

In der Formel (XIII) hat R¹⁷ die Bedeutung die bereits im Zusammenhang mit der Beschreibung der erfindungsgemäßen Stoffe der allgemeinen Formel (I) als bevorzugt für diese Substituenten genannt werden.

Die Verbindungen der Formel (XIII) sind allgemein bekannte Verbindungen der organischen Chemie bzw. können z.T. kommerziell oder nach literaturbekannten Methoden erhalten werden (vgl. Furane: P. Bosshard, C. H. Engster in: Advances Heterocycl. Chem.; Hrsg.: A. R. Katritzky, A. J. Boulton, Vol. 7, New York, Academic Press 1966, S. 377).

Die Umsetzung der Verbindungen (Ie) mit den Verbindungen der allgeimenen For-mel (XIII) führt man vorzugsweise in Gegenwart eines Katalysators unter Verwen-dung von Verdünnungsmitteln durch.

Als Katalysatoren zur Durchführung des erfindungsgemäßen Verfahrens 4c können ebenso alle für das Verfahren 3 geeigneten Säuren in Frage kommen.

Bevorzugte Säuren für das Verfahren 4c sind Mineralsäuren, insbesondere Schwefelsäure oder organische Säuren, insbesondere Essigsäure oder Sulfonsäuren wie para-Toluolsulfonsäure oder β-Naphthalinsulfonsäure.

Als Verdünnungsmittel zur Durchführung des erfindungsgemäßen Verfahrens 4c können die bei Verfahren 3 genannten inerten, aprotischen Lösungsmittel wie z. B. Ether, Dioxan, Acetonitril oder Tetrahydrofuran, Halogenkohlenwasserstoffe, insbesondere Chlorkohlenwasserstoffe, wie Methylenchlorid oder Chloroform aber auch aromatische Kohlenwasserstoffe wie Benzol oder Toluol, Verwendung finden. Es ist aber auch vorteilhaft, nur die Verbindungen der allgemeinen Formel (XIII) äquimolar oder im Überschuß einzusetzen.

Das Verfahren 4c wird durchgeführt, indem man Verbindungen der allgemeinen Formeln (Ie) in Gegenwart eines Katalysators mit Verbindungen der allgemeinen Formeln (XIII), gegebenenfalls in einem der angegebenen Verdünnungsmittel umsetzt.

Die Reaktionsdauer beträgt 1 bis 48 Stunden. Die Umsetzung erfolgt bei Temperaturen zwischen -100°C und +100°C, bevorzugt zwischen -50°C und +50°C, besonders bevorzugt bei -10°C bis Raumtemperatur. Es wird unter Normaldruck gearbeitet. Zur Durchführung des erfindungsgemäßen Verfahrens 7c setzt man pro Mol an Verbindung der Formeln (Ie) im allgemeinen 1,0 bis 5,0 Mol, vorzugsweise 1,5 bis 3,0 Mol Verbindung der Formel (XII), ein.

Nach vollendeter Umsetzung wird die Reaktionslösung gewaschen, die organische Phase abgetrennt, getrocknet und im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Mit den erfindungsgemäßen Verfahren 2, 3 und 4 sind, aus den einzelnen Bausteinen mit sowohl (S)- als auch (R)-Konfiguration (bzw. L- und D-Konfiguration), erfindungsgemäße Verbindungen unter Beibehaltung der ursprünglichen Konfiguration der Ausgangsstoffe erhältslich. In Abhängigkeit der eingesetzten Ausgangsstoffe ist jedoch auch eine gezielte Inversion in Position 6 der erfindungsgemäßen Verbindungen denkbar.

Mit den in den vorstehenden Verfahrensvarianten 2, 3 und 4 bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen, insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., 'Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonismus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp. , Bunostomum spp. , Globocephalus spp. , Syngamus spp. , Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen. Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

### Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspensionen zur oralen oder dermalen Anwendung sowie zur Injektion; halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

### Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl /Caprinsäure-biglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter ebentuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

### Als hydrophile Phase seien genannt:

Wasser, Alkohole wie z.B. Propylenglycol, Glycerin, Sorbitol und ihre Gemische.
Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethynolaminsalz.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen, die vor Anwendung verdünnt werden, enthalten den Wirkstoff in Konzentrationen von 0,5 - 90 Gew.-%, bevorzugt von 5 - 50 Gew.-%.

### Beispiel A

### In vivo Nematodentest

### Haemonchus contortus / Schaf

Experimentell mit *Haemonchus contortus* infizierte Schafe wurden nach Ablauf der Präpatenzzeit des Parasiten behandelt. Die Wirkstoffe wurden als reiner Wirkstoff oral und/oder intravenös appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel-Nr. | Dosis effectiva in [mg/kg] |
|---|---|
| 7 | 1,0 |
| 13 | 1,0 |
| 17 | 1,0 |
| 40 | 1,0 |
| 52 | 1,0 |
| 88 | 1,0 |

### Beispiel B

### In vivo Nematodentest

### Trichostrongylus colubriformis / Schaf

Experimentell mit *Trichostrongylus colubriformis* infizierte Schafe wurden nach Ablauf der Präpatenzzeit des Parasiten behandelt. Die Wirkstoffe wurden als reiner Wirkstoff oral und/oder intravenös appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel-Nr. | Dosis effectiva in [mg/kg] |
|---|---|
| 13 | 1,0 |
| 17 | 1,0 |
| 40 | 1,0 |

### Herstellungsbeispiele

### 6α- und 6β-Hydroxy-7-(p-tolyl)-1,2,4aα,5aα,8aα,8bα-(±)-hexahydro-3,4a-dimethyl-6H-pyrrolo[3',4': 4,5]furo[3,2-b]pyrid-8(7H)-one

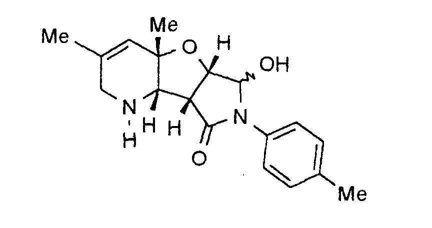

1,6 g (4,9 mMol) 7-(p-Tolyl)-4aα,5aα,8aα,8bα-(±)-tetrahydro-3,4a-dimethyl-6Hpyrrolo[3',4':4,5]furo[3,2-b]pyridin-6,8(7H)-dion werden in 50 ml Methanol suspendiert und bei -5°C portionsweise mit 0,24 g (6,37 mMol) NaBH₄ versetzt. Nach einstündigem Rühren bei 0°C wird ca. 18 Stunden bei Raumtemperatur gerührt. Anschließend wird mit 1N HCl angesäuert (pH 7-8) und die gesamte Reaktionslösung im Vakuum eingeengt. Danach wird der verbleibende Rückstand in Chloroform aufgenommen und die organische Phase mehrmals gegen Wasser sowie gesättigte NaCl-Lösung geschüttelt. Die organische Phase wird abgetrennt, über Magnesiumsulfat getrocknet und im Vakuum eingeengt. Man erhält ein 6α-/6β-Hydroxy-Isomerengemisch, das sich über eine Kieselgelsäule (Kieselgel 60 - Merck, Korngröße: 0,04 bis 0,063 mm) mit dem Fließmittel Cyclohexan : Aceton (2 : 1) auftrennen läßt.

### Beispiel 1

### 6α-Hydroxy-7-(p-tolyl)-1,2,4aα,5aα,8aα,8bα-(±)-hexahydro-3,4a-dimethyl-6H-pyrrolo[3',4':4,5]furo[3,2-b]pyrid-8(7H)-on

Fp.: 171°C

¹H-NMR (400 MHz, CDCl₃, δ): 1,25, 1,73, 2,33 (s, 9H, -CH₃); 3,19-3,26 (m, 3H, -N-CH₂-, -N-CH-); 3,63 (dd, 1H, -CH-CO-); 4,08, 4,93 (2d, 2H, -CH₂-Aryl; J = 14,6 Hz); 4,68 (dd, 1H, -O-CH-); 4,78 (d, 1H, -CH-OH); 5,53 (s, 1H, =CH-); 6,1 (br.s, 1H, -OH); 7,12, 7,14 (2d, 4H, Aryl-H, J = 8,1 Hz) ppm.

### Beispiel 2

### 6β-Hydroxy-7-(p-tolyl)-1,2,4aα,5aα,8aα,8bα-(±)-hexahydro-3,4a-dimethyl-6H-pyrrolo[3',4':4,5]furo[3,2-b]pyrid-8(7H)-on

Fp.: 143-144°C

¹H-NMR (400 MHz, CDCl₃, δ): 1,17, 1,41, 2,31 (s, 9H, -CH₃); 3,18 (br, 2H, -N-OCH₂-); 3,25 (d, 1H, -N-CH-); 3,64 (dd, 1H, -CH-CO-); 4,04, 4,65 (2d, 2H, -CH₂-Aryl; J = 14,6 Hz); 4,37 (dd, 1H, -O-CH-); 5,05 (d, 1H, -CH-OH); 5,43 (s, 1H, =CH-); 7,10, 7,23 (2d, 4H, Aryl-H, J = 8,1 Hz) ppm.

EI-MS m/z (%): 328 (M⁺, 2); 310 (28); 108 (100).

### Beispiel 3

### 6β-Hydroxy-1,2,4aα,5aα,8aα,8bα-(±)-hexahydro-3,4a,7-trimethyl-6H-pyrrolo[3',4':4, 5]furo[3,2-b]pyrid-8(7H)-on

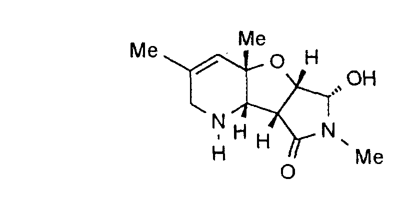

Die Darstellung des 6β-Hydroxy-Isomers erfolgt analog dem Beispiel 1 aus:

| | |
|---|---|
| 5,00 g (0,020 Mol) | 4aα,5aα,8aα,8bα-(±)-Tetrahydro-3,4a,7-trimethyl-6H-pyrrolo[3',4':4,5]furo[3,2-b]pyridin-6,8(7H)-dion |
| 0,98 g (0,026 Mol) | NaBH₄ |
| 100 ml | Methanol |

Man erhält 3,2 g (66,9 % der Theorie) 6β-Hydroxy-1,2,4aα,5aα,8aα,8bα-(±)-hexahydro-3,4a,7-trimethyl-6H-pyrrolo[3',4':4,5]furo[3,2-b]pyrid-8(7H)-on.

Fp.: 158-160°C

¹³C-NMR (150 MHz, CDCl₃, δ): 20,5 25,2 (2 x -CH₃); 51,2 (-CO-CH-); 27,2 -N-CH₃); 75,4, 84,9 (1 x -O-CH-); 83,9 (HO-CH-N-); 170,8 (1 x -N-C=O); 47,7 (1 x -NH-CH₂-); 59,0 (-NH-CH-); 122,8 (=CH-); 135,0 (=C-Me) ppm.

EI-MS m/z (%): 338 (M⁺, 3); 108 (100).

### Beispiel 4

### 7-Ethyl-6β-hydroxy-1,2,4aα,5aα,8aα,8bα-(±)-hexahydro-3,4a-dimethyl-6H-pyrrolo[3',4':4,5]furo[3,2-b]pyrid-8(7H)-on

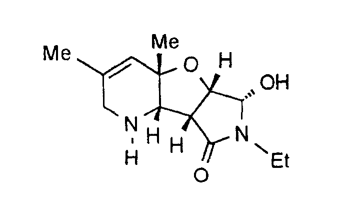

Die Darstellung des 6β-Hydroxy-Isomers erfolgt analog dem Beispiel 3 aus:

| | |
|---|---|
| 8,00 g (0,034 Mol) | 7-Ethyl-4aα,5aα,8aα,8bα-(±)-Tetrahydro-3,4a-dimethyl-6H-pyrrolo[3',4':4,5]furo[3,2-b]pyridin-6,8(7H)-dion |
| 1,67 g (0,044 Mol) | NaBH₄ |
| 160 ml | Methanol |

Man erhält 6,7 g (78,5 % der Theorie) 7-Ethyl-6β-hydroxy-1,2,4aα,5aα,8aα,8bα-(±)-hexahydro-3,4a-dimethyl-6H-pyrrolo [3',4':4,5]furo[3,2-b]pyrid-8(7H)-on.

Fp.: 114°C

### Beispiel 5

### Hydrogensulfat des 7-Ethyl-6β-hydroxy-1,2,4aα,5aα,8aα,8bα-(±)-hexahydro-3,4adimethyl-6H-pyrrolo[3',4':4,5]furo[3,2-b]pyrid-8(7H)-ons

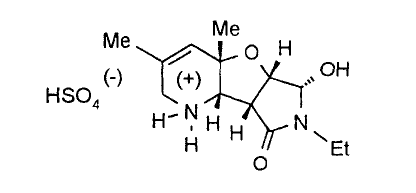

10,0 g (0,04 Mol) 7-Ethyl-6β-hydroxy-1,2,4aα,5aα,8aα,8bα-(±)-hexahydro-3,4adimethyl-6H-pyrrolo[3',4':4,5]furo[3,2-b]pyrid-8(7H)-on werden in 50 ml Ethanol vorgelegt, mit 21 ml 2M H₂SO₄ in Ethanol versetzt (pH 2) und 72 Stunden bei Raumtemperatur gerührt. Der ausgefallenen Feststoff wird abgetrennt und mit Ethanol gewaschen. Man erhält 8,4 g (60,4 % der Theorie) 7-Ethyl-6β-hydroxy-1,2,4aα,5aα, 8aα,8bα-(±)-hexahydro-3,4a-dimethyl-6H-pyrrolo[3',4':4,5]furo[3,2-b]pyrid-8 (7H)-on H₂SO₄.

Fp.: 168 - 170°C (Zers.)

### Beispiel 6

### 7-Ethyl-6α-ethoxy-1,2,4aα,5aα,8aα,8bα-(±)-hexahydro-3,4a-dimethyl-6H-pyrrolo [3',4':4, 5]furo[3,2-b]pyrid-8(7H)-on

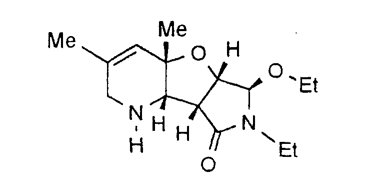

Nach Einengen der Mutterlauge aus Beispiel 5 wird das zurückbleibende Rohprodukt über eine Kieselgelsäule (Kieselgel 60 - Merck, Korngröße: 0,04 bis 0,063 mm) mit dem Fließmittel Cyclohexan : Aceton (2 : 1) chromatographiert. Man erhält 0,5 g (4,5 % der Theorie) 7-Ethyl-6α-ethoxy-1,2,4aα,5aα,8aα,8bα-(±)-hexahydro-3,4adimethyl-6H-pyrrolo[3',4':4,5]furo[3,2-b]pyrid-8(7H)-on.

¹H-NMR (400 MHz, CDCl₃, δ): 1,19, 1,25 (2t, 6H, -CH₃); 1,71, 1,88 (2s, 6H, -CH₃); 3,17-3,70 (m, 7H; 2 x -N-CH₂-, -O-CH₂, -N-CH-, -O-CH-, -CH-); 4,36 (d, 1H, -O-CH-); 4,85 (s, 1H, -CH-OH); 5,62 (s, 1H, =CH-) ppm.

EI-MS m/z (%): 280 (M+., 16); 251 (30); 108 (100).

### Beispiel 7

### 6β-Hydroxy-1,2,3,4,4aα,5aα,8aα,8bα-(±)-oktahydro-3β,4a,7-trimethyl-6H-pyrrolo[3', 4':4,5]furo[3,2-b]pyrid-8(7H)-on

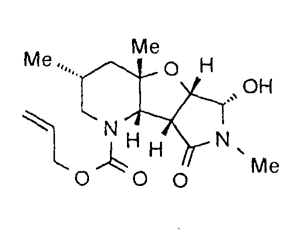

Die Darstellung des 6β-Hydroxy-Isomeren erfolgt analog dem Beispiel 1 aus:

| | |
|---|---|
| 6,40 g (0,020 Mol) | 1,2,3,4,4aα,5aα,8aα,8bα-(±)-Oktahydro-3β,4a,7-trimethyl-6H-pyrrolo[3',4': 4,5]furo[3,2-b]pyridin-6,8(7H)-dion |
| 0,98 g (0,026 Mol) | NaBH₄ |
| 100 ml | Methanol |

Man erhält 6,3 g (96,6 % der Theorie) 6β-Hydroxy-1,2,3,4,4aα,5aα,8aα,8bα-(±)-oktahydro-3β,4a,7-trimethyl-6H-pyrrolo[3',4':4,5]furo[3,2-b]pyrid-8(7H)-on.

Fp.: 88-89°C

¹³C-NMR (150 MHz, CDCl₃, δ): 20,0, 24,5 (2 x -CH₃); 23,4 (-CH-); 26,5 (-N-CH₃); 73,2, 82,2, 82,9 (3 x -O-CH-); 156,3, 169,5 (2 x -N-C=O); 40,5, 48,1 (2 x -CH₂-); 48,8 (-CH-); 62,4 (-N-CH-); 66,1 (-O-CH₂-); 117,0 (=CH₂); 133,0 (-CH=) ppm.

EI-MS m/z (%): 324 (M^{+.}, 8); 239 (12); 221 (100).

### Beispiel 8

### 1-Allyloxycarbonyl-7-ethyl-6β-hydroxy-1,2,4aα,5aα,8aα,8bα-(±)-hexahydro-3,4a-dimethyl-6H-pyrrolo[3',4':4,5]furo[3,2-b]pyrid-8(7H)-on

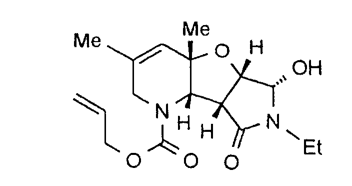

5,0 g (14,2 mMol) 7-Ethyl-6β-hydroxy-1,2,4aα,5aα,8aα,8bα-(±)-hexahydro-3,4a-dimethyl-6H-pyrrolo[3',4':4,5]furo[3,2-b]pyrid-8(7H)-on H₂SO₄ werden in 100 ml Methylenchlorid suspendiert, mit 30 ml Wasser, 5,0 g (39,0 mMol) N,N-Diisopropylethylamin ("Hünig's Base") versetzt und 30 Minuten bei Raumtemperatur gerührt. Nach Abtrennen der Wasserphase wird die organische Pase im Vakuum eingeengt. Anschließend wird eine Lösung aus 2,8 g (11,0 mMol) des freigesetzten 7-Ethyl-6β-hydroxy-1,2,4aα,5aα,8aα,8bα-(±)-hexahydro-3,4a-dimethyl-6H-pyrrolo[3',4':4,5]furo[3,2-b]pyrid-8(7H)-ons, 2,7 g (20,8 mMol) N,N-Diisopropylethylamin ("Hünig's Base"), 1,4 g (12,0 mMol) Allylchlorformiat in 50 ml Methylenchlorid zunächst eine Stunde bei 0°C und weitere 18 Stunden bei Raumtemperatur gerührt. Danach wird die gesamte Reaktionslösung mehrmals mit Wasser gewaschen. Nach Abtrennen der organischen Phase und Trocknen über Magnesiumsulfat wird im Vakuum eingeengt und das zurückbleibende Rohprodukt über eine Kieselgelsäule (Kieselgel 60 - Merck, Korngröße: 0,04 bis 0,063 mm) mit dem Fließmittel Cyclohexan : Aceton (2 : 1) chromatographiert. Man erhält 2,6 g (70,2 % der Theorie) 1-Allyloxycarbonyl-7-ethyl-6β-hydroxy-1,2,4aα,5aα,8aα,8bα-(±)-hexahydro-3,4a-dimethyl-6H-pyrrolo[3', 4':4,5]furo[3,2-b]pyrid-8(7H)-on.

¹H-NMR (300 MHz, CDCl₃, δ): 4,73 (dd, 1H, 5aα-H; J¹ = 7,4 Hz; J² = 1,0 Hz); 5,54 (s, 1H, =CH-); 6,11 (d, 1H, NH, J = 12,1 Hz) ppm.

EI-MS m/z (%): 336 (M⁺, 18); 318 (20); 251(45); 233 (100).

APCI-MS-LOOP sauer m/z (%): 337 (MH⁺, 21), 319 (100).

### Beispiel 9

### 1-(N-Benzyloxycarbonyl-N-methyl-glycyl)-7-ethyl-6β-hydroxy-1,2,4aα,5aα,8aα,8bα-(±)-hexahydro-3,4a-dimethyl-6H-pyrrolo[3',4':4,5]furo[3,2-b]pyrid-8(7H)-on

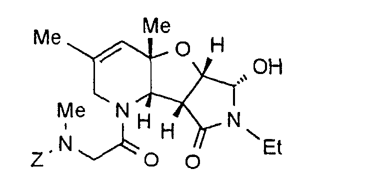

Zu einer Lösung von 2,2 g (8,7 mMol) 7-Ethyl-6β-hydroxy-1,2,4aα,5aα,8aα,8bα-(±)-hexahydro-3,4a-dimethyl-6H-pyrrolo[3',4':4,5]furo[3,2-b]pyrid-8(7H)-on und 1,6 g (7,25 mMol) N-Benzyloxycarbonyl-sarkosin (Z-Sar-OH) in 80 ml Methylenchlorid werden bei 0°C 2,2 g (17,2 mMol) N,N-Diisopropylethylamin (Hünig's Base) sowie 2,2 g (8,7 mMol) Bis(2-oxo-3-oxazolidinyl)-phosphoniumsäurechlorid (BOP-Cl) zugegeben und 30 Minuten bei 0°C, anschließend 18 Stunden bei Raumtemperatur gerührt. Die Reaktionslösung wird zweimal mit Wasser geschüttelt, die organische Phase abgetrennt und nach dem Trocknen über Natriumsulfat im Vakuum eingeengt. Das zurückbleibende Rohprodukt wird über eine Kieselgelsäule (Kieselgel 60-Merck, Korngröße: 0,04 bis 0,063 mm) mit dem Fließmittel Cyclohexan : Aceton (2:1) chromatographiert. Man erhält 2,3 g (57,8 % der Theorie) 1-(N-Benzyloxycarbonyl-N-methyl-glycyl)-7-ethyl-6β-hydroxy-1,2,4aα,5aα,8aα,8bα-(±)-hexahydro-3,4a-dimethyl-6H-pyrrolo[3',4':4,5]furo[3,2-b]pyrid-8(7H)-on.

EI-MS m/z (%): 457 (M⁺, 19), 251 (80), 233 (85), 91 (100).

### Beispiel 10

### 1-(N-Methylaminocarbonyl)-7-ethyl-6β-hydroxy-1,2,4aα,5aα,8aα,8bα-(±)-hexahydro-3,4a-dimethyl-6H-pyrrolo[3',4':4,5]furo[3,2-b]pyrid-8(7H)-on

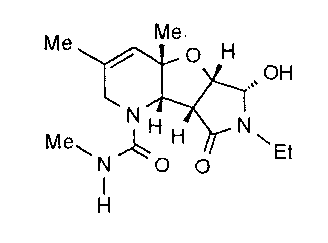

2,2 g (8,7 mMol) 7-Ethyl-6β-hydroxy-1,2,4aα,5aα,8aα,8bα-(±)-hexahydro-3,4a-dimethyl-6H-pyrrolo[3',4':4,5]furo[3,2-b]pyrid-8(7H)-on werden mit 0,6 g (8,7 mMol) Methylisocyanat in 20 ml Toluol eine Stunde unter Rückflußtemperatur gerührt. Nach Abkühlen wird der ausgefallene Feststoff abgetrennt und mit Essigsäureethylester verrührt. Man erhält 2,0 g (74,3 % der Theorie) 1-(N-Methylaminocarbonyl)-7-ethyl-6β-hydroxy-1,2,4aα,5aα,8aα,8bα-(±)-hexahydro-3,4a-dimethyl-6H-pyrrolo[3',4':4,5]furo[3,2-b]pyrid-8(7H)-on.

¹H-NMR (300 MHz, CDCl₃, δ): 4,61 (dd, 1H, 5aα-H; J¹ = 7,4 Hz; J² = 1,0 Hz); 5,06 (d, 1H, 6β-H, J = 7,4 Hz); 5,32 (s, 1H, =CH-) ppm.

¹³C-NMR (150 MHz, CDCl₃, δ): 13,1, 20,0, 26,2, 27,6 (4 x -CH₃); 35,1 (1 x -N-CH₂-); 158,3 (1 x -NH-C=O); 170,3 (1 x -N-C=O); 46,3 (1 x -CH-); 73,9 (1 x -O-CH-); 81,7 (1 x HO-CH-N-); 78,2 (1 x C-Me); 58,8 (1 x -N-CH-); 45,6 (1 x -N-CH₂-); 129,8 (1 x =C-Me); 125,6 (1 x -CH=) ppm.

EI-MS m/z (%): 309 (M⁺, 8), 234 (22), 108 (100).

### Beispiel 11

### 1-Allyloxycarbonyl-6α-ethoxy-1,2,4aα,5aα,8aα,8bα-(±)-hexahydro-3,4a,7-trimethyl-6H-pyrrolo[3',4':4,5]furo[3,2-b]pyrid-8(7H)-on

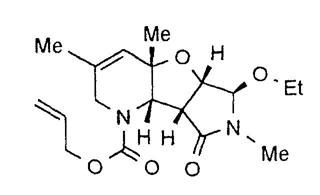

1,6 g (5,0 mMol) 1-Allyloxycarbonyl-6β-hydroxy-1,2,4aα,5aα,8aα,8bα-(±)-hexahydro-3,4a,7-trimethyl-6H-pyrrolo[3',4':4,5]furo[3,2-b]pyrid-8(7H)-on werden in 50 ml Ethanol gelöst, mit 2 ml 2M H₂SO₄ versetzt und 48 Stunden bei Raumtemperatur gerührt. Anschließend wird mit gesättigter NaHCO₃-Lösung auf pH-Wert 7 eingestellt und die gesamte Reaktionslösung im Vakuum eingeengt. Danach wird der verbleibende Rückstand in Methylenchlorid aufgenommen und die organische Phase mehrmals gegen Wasser geschüttelt. Nach Abtrennen der organischen Phase und Trocknen über Magnesiumsulfat wird im Vakuum eingeengt und das zurückbleibende Rohprodukt über eine Kieselgelsäule (Kieselgel 60 - Merck, Korngröße: 0,04 bis 0,063 mm) mit dem Fließmittel Cyclohexan : Aceton (4 : 1) chromatographiert. Man erhält 0,8 g (45,7 % der Theorie) 1-Allyloxycarbonyl-6α-ethoxy-1,2,4aα,5aα,8aα,-8bα-(±)-hexahydro-3,4a,7-trimethyl-6H-pyrrolo[3',4':4,5]furo[3,2-b] pyrid-8(7H)-on.

Fp.: 68-69°C

¹H-NMR (300 MHz, CDCl₃, δ): 4,43 (d, 1H, 5aβ-H) ppm.

### Beispiel 12

### 1-Allyloxycarbonyl-6α-ethoxy-1,2,3,4,4aα,5aα,8aα,8bα-(±)-oktahydro-3β,4a,7-trimethyl-6H-pyrrolo[3',4':4,5]furo[3,2-b]pyrid-8(7H)-on

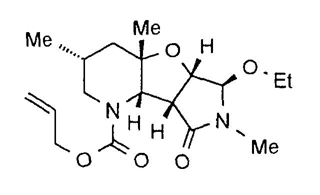

Die Darstellung des 6α-Ethoxy-Isomeren erfolgt analog dem Beispiel 7 aus:

| | |
|---|---|
| 2,0 g (6,0 mMol) | 1-Allyloxycarbonyl-6β-hydroxy-1,2,3,4,4aα,5aα,8aα,8bα-(±)-oktahydro-3β,4a,7-trimethyl-6H-pyrrolo[3',4':4,5]furo[3,2-b]-pyrid-8(7H)-on |
| 0,5 ml | 2M H₂SO₄ |
| 30 ml | Ethanol |

Man erhält 1,0 g (47,3 % der Theorie) 1-Allyloxycarbonyl-6α-ethoxy-1,2,3,4,4aα,5aα,8aα,8bα-(±)-oktahydro-3β,4a,7-trimethyl-6H-pyrrolo[3',4':4,5]furo[3,2-b]pyrid-8(7H)
on.

Fp.: Oel

¹³C-NMR (150 MHz, CHCl₃, δ): 48,1, 39,2, 40,4 (3 x -CH₂-); 62,7, 62,9, 66,0, 66,1 (4 x -O-CH₂-); 15,0; 15,8, 20,2, 26,7, 27,1 (5 x -CH₃); 23,3, 47,3, 47,4 (3 x -CH-); 61,3 (1 x -N-CH-); 77,7, 78,0, 92,6, 92,8 (4 x -O-CH-); 27,5 (1 x -N-CH₃); 156,1, 156,6, 171,3, 171,4 (4 x -N-C=O); 133,0, 133,3 (2 x -CH=); 116,9, 117,0 (2 x =CH₂) ppm.
(E/Z-Isomerengemisch)

EI-MS m/z (%): 352 (M⁺, 8); 267 (100); 108 (40); 41 (46).

### Beispiel 13

### 1-Allyloxycarbonyl-6α-hydroxy-1,2,4aα,5aα,8aα,8bα-(±)-hexahydro-3,4a,7-trimethyl-6H-pyrrolo[3',4':4,5]furo[3,2-b]pyrid-8(7H)-on

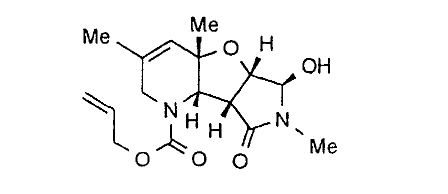

1,1g (3,0 mMol) 1-Allyloxycarbonyl-6β-hydroxy-1,2,4aα,5aα,8aα,8bα-(±)-hexahydro-3,4a,7-trimethyl-6H-pyrrolo[3',4':4,5]furo[3,2-b]pyrid-8(7H)-on werden in 75 ml Ethanol gelöst, mit 6,5 ml 4N NaOH versetzt und 18 Stunden bei Raumtemperatur gerührt. Anschließend wird mit 10 %iger HCl auf pH-Wert 7 eingestellt und die gesamte Reaktionslösung im Vakuum eingeengt. Danach wird der verbleibende Rückstand in Methylenchlorid aufgenommen und die organische Phase mehrmals gegen Wasser geschüttelt. Nach Abtrennen der organischen Phase und Trocknen über Magnesiumsulfat wird im Vakuum eingeengt und das zurückbleibende Rohprodukt über eine Kieselgelsäule (Kieselgel 60 - Merck, Korngröße: 0,04 bis 0,063 mm) mit dem Fließmittel Cyclohexan : Aceton (2 : 1) chromatographiert. Man erhält 0,65 g (67,4 % der Theorie) 1-Allyloxycarbonyl-6α-hydroxy-1,2,4aα,5aα, 8aα,8bα-(±)-hexahydro-3,4a,7-trimethyl-6H-pyrrolo[3',4':4,5]furo[3,2-b]pyrid-8(7H)-on.

Fp.: 102°C

¹H-NMR (300 MHz, CDCl₃, δ): 5,30 (s, 1H, 6β-H) ppm.

¹³C-NMR (150 MHz, CHCl₃, δ): 45,8, 46,0 (2 x -CH₂-), 66,4, 66,5 (2 x -O-CH₂-); 20,0, 20,1, 25,9, 26,0 (4 x -CH₃); 45,3, 45,9 (2 x -CH-); 60,6, 60,9 (2 x -N-CH-); 77,7, 77,9, 82,0, 82,1, 90,2, 90,3 (6 x -O-CH-); 26,9 (1 x -N-CH₃); 131,1, 130,4 (2 x Me-C=); 124,2, 124,7 (2 x =CH-), 172,0, 172,3, 155,6, 156,2 (4 x -N-C=O); 132,6, 132,8 (2 x -CH=); 117,5 (1x =CH₂) ppm (E/Z-Isomerengemisch).

### Beispiel 14

### 6β-Acetoxy-1-allyloxycarbonyl-7-ethyl-1,2,4aα,5aα,8aα,8bα-(±)-hexahydro-3,4a-dimethyl-6H-pyrrolo[3',4':4,5]furo[3,2-b]pyrid-8(7H)-on

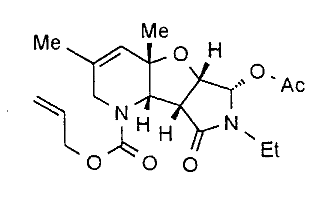

4,4 g (13,0 mMol) 1-Allyloxycarbonyl-6β-hydroxy-7-ethyl-1,2,4aα,5aα,8aα,8bα-(±)-hexahydro-3,4a-dimethyl-6H-pyrrolo[3',4':4,5]furo[3,2-b]pyrid-8(7H)-on werden in 50 ml Methylenchlorid gelöst und bei 0°C mit 1,4 g (13,5 mMol) Triethylamin sowie 0,16 g (1,3 mMol) 4-(N,N-Dimethylamino)-pyridin versetzt. Anschließend werden 2,0 g (19,5 mMol) Acetanhydrid zugetropft, eine Stunden bei 0-5°C und weitere 20 Stunden bei Raumtemperatur gerrührt. Anschließend wird mit 10 %iger HCl, gesättigter NaHCO₃-Lösung und Wasser gewaschen. Nach Abtrennen der organischen Phase und Trocknen über Magnesiumsulfat wird im Vakuum eingeengt und das zurückbleibende Rohprodukt über eine Kieselgelsäule (Kieselgel 60 - Merck, Korngröße: 0,04 bis 0,063 mm) mit dem Fließmittel Cyclohexan : Aceton (4 : 1) chromatographiert. Man erhält 3,4 g (69,9 % der Theorie) 6β-Acetoxy-1-allyloxycarbonyl-7-ethyl-1,2,4aα,5aα,8aα,8bα-(±)-hexahydro-3,4a-dimethyl-6H-pyrrolo[3',4':4,5]furo[3,2 -b]pyrid-8(7H)-on.

¹H-NMR (300 MHz, CDCl₃, δ): 5,85 (d, 1H, 6α-H) ppm.

EI-MS m/z (%): 378 (M+., 12), 318 (25); 293 (19), 233 (100), 148 (60).

### Beispiel 15

### 1-Allyloxycarbonyl-6β-cyclopropylcarbonyloxy-7-ethyl-1,2,4aα,5aα,8aα,8bα-(±)-hexahydro-3,4a-dimethyl-6H-pyrrolo[3',4':4,5]furo[3,2-b]pyrid-8(7H)-on

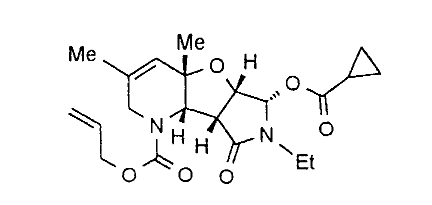

Die Darstellung des 6β-Cyclopropylcarbonyloxy-Isomeren erfolgt analog dem Beispiel 14 aus:

| | |
|---|---|
| 1,20 g (3,6 mMol) | 1-Allyloxycarbonyl-6β-hydroxy-7-ethyl-1,2,4aα,5aα,8aα,8bα-(±)-hexahydro-3,4a,7-trimethyl-6H-pyrrolo[3',4':4,5]furo[3,2-b]pyrid-8(7H)-on |
| 0,37 g (3,7 mMol) | Triethylamin |
| 0,56 g (5,4 mMol) | Cyclopropancarbonsäurechlorid |
| ca. 0,04 g | 4-(N,N-Dimethylamino)-pyridin |
| 20 ml | Methylenchlorid |

Man erhält 0,25 g (17,1 % der Theorie) 1-Allyloxycarbonyl-6β-cyclopropylcarbonyloxy-7-ethyl-1,2,4aα,5aα,8aα,8bα-(±)-hexahydro-3,4a-dimethyl-6H-pyrrolo[3',4':4,5] furo[3,2-b]pyrid-8(7H)-on.

EI-MS m/z (%): 404 (M⁺, 7), 319 (30), 108 (82).

### Beispiel 16

### 6β-(N-Benzyloxycarbonyl-N-methyl-leucinyloxy)-7-ethyl-1,2,4aα,5aα,8aα,8bα-(±)-hexahydro-3,4a-dimethyl-6H-pyrrolo[3',4':4,5]furo[3,2-b]pyrid-8(7H)-on

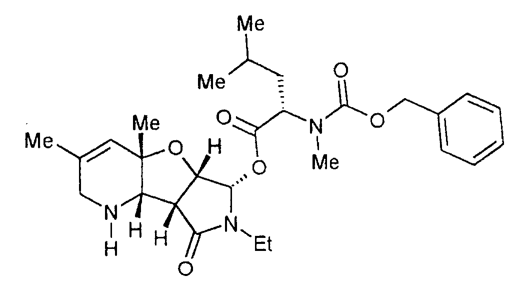

Die Darstellung des 6β-O-Acylderivates erfolgt analog dem Beispiel 9 aus:

| | |
|---|---|
| 2,2 g (8,70 mMol) | 7-Ethyl-6β-hydroxy-1,2,4aα,5aα,8aα,8bα-(±)-hexahydro-3,4a-dimethyl-6H-pyrrolo[3',4':4,5]furo[3,2-b]pyrid-8(7H)-on |
| 1,9 g (7, 25 mMol) | N-Benzyloxycarbonyl-N-methyl-leucin (Z-MeLeu-OH) |
| 2,2 g (17,2 mMol) | N,N-Diisopropylethylamin (Hünig's Base) |
| 2,2 g (8,70 mMol) | Bis(2-oxo-3-oxazolidinyl)-phosphoniumsäurechlorid (BOP-Cl) |
| 80 ml | Methylenchlorid |

Nach Aufarbeitung wird das zurückbleibende Rohprodukt über eine Kieselgelsäule (Kieselgel 60 - Merck, Korngröße: 0,04 bis 0,063 mm) mit dem Fließmittel Cyclohexan : Aceton (2 : 1 ) chromatographiert. Man erhält 150 mg (3,4 % der Theorie) 6β-(N-Benzyloxycarbonyl-N-methyl-leucinyloxy)-7-ethyl-1,2,4aα,5aα,8aα,8bα-(±)-hexahydro-3,4a-dimethyl-6H-pyrrolo[3',4':4,5]furo[3,2-b]pyrid-8(7H)-on als Diastereomerengemisch.

F.: Öl

¹³C-NMR (150 MHz, CDCl₃, δ): 12,8, 12,9, 21,3, 23,3, 24,4, 24,5, (4 x -CH₃); 36,0, 36,7 (1 x -N-CH₂-); 30,1 (1 x -N-CH₃); 24,2, 24,4 (1 x -CH-); 36,2, 37,5 (1 x -CH₂-); 49,0 (1 x -NH-CH₂-); 50,1, 50,3 (1 x -CO-CH-); 56,7 (1 x -CO-CH-); 60,7, 60,8 (1 x -NH-CH-); 67,4, 67,5 (1 x -O-CH₂-); 72,8, 73,0 (1 x -O-CH-); 83,6, 84,1 (1 x CO-O-CH-); 83,5, 83,6 (1 x-C-Me); 122,8, 123,3 (1 x =C*-*H); 136,3, 136,5 (1 x =C-Me); 127,7, 17,9, 128,4, 139,1, 140,1 (4 x Aryl -CH=) 156,0, 156,7, 170,9 (1 x -N-C=O); ppm.

EI-MS m/z (%): 513 (M⁺, 1), 234 (18), 108 (100).

### Beispiel 17

### 1-Allyloxycarbonyl-6β-allyloxycarbonyloxy-1,2,4aα,5aα,8aα,8bα-(±)-hexahydro-3,4a,7-trimethyl-6H-pyrrolo[3',4':4,5]furo[3,2-b]pyrid-8(7H)-on

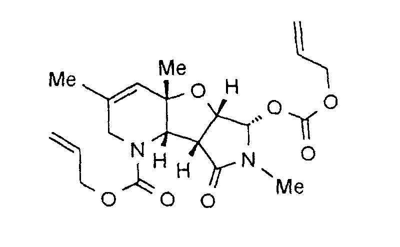

Zu einer Lösung aus 5,0 g (0,02 Mol) 6β-Hydroxy-1,2,4aα,5aα,8aα,8bα-(±)-hexahydro-3,4a,7-trimethyl-6H-pyrralo[3',4':4,5]furo[3,2-b]pyrid-8(7H)-on in 150 ml Methylenchlorid werden bei 0°C 12,9 g (0,10 Mol) N,N-Diisopropylethylamin ("Hünig's Base") sowie 5,7 g (0,048 Mol) Allylchlorformiat getropft und 18 Stunden bei Raumtemperatur gerührt. Anschließend wird zweimal mit Wasser gewaschen. Nach Abtrennen der organischen Phase und Trocknen über Magnesiumsulfat wird im Vakuum eingeengt und das zurückbleibende Rohprodukt über eine Kieselgelsäule (Kieselgel 60 - Merck, Korngröße: 0,04 bis 0,063 mm) mit dem Fließmittel Cyclohexan : Aceton (2 : 1) chromatographiert. Man erhält 1,4 g (17,2 % der Theorie) 1-Allyloxycarbonyl-6β-allyloxycarbonyloxy-1,2,4aα,5aα,8aα,8bα-(±)-hexahydro-3,4a,7-trimethyl-6H-pyrrolo[3',4':4,5]furo[3,2-b]pyrid-8(7H)-on.

¹H-NMR (300 MHz, CDCl₃, δ): 5,67 (d, 1H, 6α-H; J = 5,6 Hz) ppm.

EI-MS m/z (%): 322 (M⁺, 38), 237 (88), 219 (100).

### Beispiel 18

### 1-Acetyl-6β-acetoxy-7-ethyl-1,2,4aα,5aα,8aα,8bα-(±)-hexahydro-3,4a-dimethyl-6Hpyrrolo[3',4':4,5]furo[3,2-b]pyrid-8(7H)-on

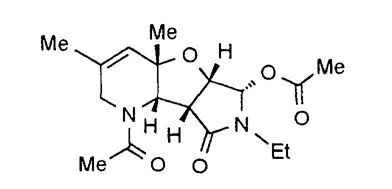

Die Darstellung des 6β-Acetoxy-Isomeren erfolgt analog dem Beispiel 14 aus:

| | |
|---|---|
| 1,20 g (3,6 mMol) | 6β-Hydroxy-7-ethyl-1,2,4aα,5aα,8aα,8bα-(±)-hexahydro-3,4a-dimethyl-6H-pyrrolo[3',4':4,5]furo[3,2-b]pyrid-8(7H)-on |
| 1,80 g (17,9 mMol) | Triethylamin |
| 2,04 g (20,0 mMol) | Essigsäureanhydrid |
| ca. 0,22 g | 4-(N,N-Dimethylamino)-pyridin |
| 30 ml | Methylenchlorid |

Man 0,5 g (38,8 % der Theorie) erhält 1-Acetyl-6β-acetoxy-7-ethyl-1,2,4aα,5aα, 8aα,8bα-(±)-hexahydro-3,4a-dimethyl-6H-pyrrolo[3',4':4,5]furo[3,2-b]pyrid-8(7H)-on.

¹H-NMR (300 MHz, CDCl₃, δ): 5,85 (d, 1H, 6α-H; J = 5,6 Hz) ppm.

EI-MS m/z (%): 336 (M⁺, 21), 233 (42), 108 (100).

### Beispiel 19

### 6α-Allyl-1-allyloxycarbonyl-1,2,4aα,5aα,8aα,8bα-(±)-hexahydro-3,4a,7-trimethyl-6H-pyrrolo[3',4':4,5]furo[3,2-b]pyrid-8(7H)-on

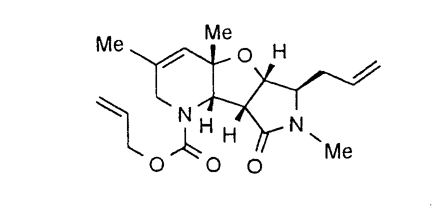

1,2 g (3,0 mMol) 1-Allyloxycarbonyl-6α-ethoxy-1,2,4aα,5aα,8aα,8bα-(±)-hexahydro-3,4a,7-trimethyl-6H-pyrrolo[3',4':4,5]furo[3,2-b]pyrid-8(7H)-on werden in 30 ml Methylenchlorid gelöst und bei -70°C nacheinander mit 0,68 g (6,0 mMol) Allyltrimethylsilan sowie 0,85 g (6,0 mMol) BF₃ Et₂O versetzt (Argon-Atmosphäre). Anschließend rührt man noch 5 Minuten bei -70°C und weitere 1,5 bis 2 Stunden Raumtemperatur. Danach wird mit gesättigter NaHCO₃-Lösung und Wasser gewaschen. Nach Abtrennen der organischen Phase und Trocknen über Magnesiumsulfat wird im Vakuum eingeengt und das zurückbleibende Rohprodukt über eine Kieselgelsäule (Kieselgel 60 - Merck, Korngröße: 0,04 bis 0,063 mm) mit dem Fließmittel Cyclohexan : Aceton (4 : 1) chromatographiert. Man erhält 0,42 g (40,4 % der Theorie) 6α-Allyl-1-allyloxycarbonyl-1,2,4aα,5aα,8aα,8bα-(±)-hexahydro-3,4a,7-trimethyl-6H-pyrrolo[3',4':4,5]furo[3,2-b]pyrid-8(7H)-on.

¹H-NMR (300 MHz, CDCl₃, δ): 4,42 (dd, 1H, 5aα-H; J¹ = 7,4 Hz; J² = 1,0 Hz) ppm.

EI-MS m/z (%): 346 (M⁺, 42), 261 (100).

### Beispiel 20

### 6α-Allyl-1-allyloxycarbonyl-7-ethyl-1,2,4aα,5aα,8aα,8bα-(±)-hexahydro-3,4a-dimethyl-6H-pyrrolo[3',4':4,5]furo[3,2-b]pyrid-8(7H)-on

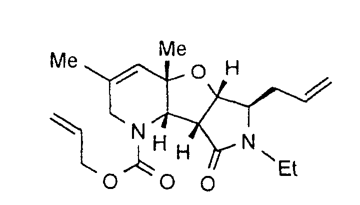

Die Darstellung des 6α-Allyl-Isomeren erfolgt analog dem Beispiel 19 aus:

| | |
|---|---|
| 1,90 g (5,00 mMol) | 1-Allyloxycarbonyl-6β-hydroxy-7-ethyl-1,2,4aα,5aα,8aα,8bα-(±)-hexahydro-3β,4a,7-trimethyl-6H-pyrrolo[3',4':4,5]furo [3,2-b]pyrid-8(7H)-on |
| 1,10 g (10,0 mMol) | Allyltrimethylsilan |
| 1,42 g (10,0 mMol) | BF₃·Et₂O |
| 50 ml | Methylenchlorid |

Man erhält 0,75 g (41,6 % der Theorie) 6α-Allyl-1-allyloxycarbonyl-7-ethyl-1,2,4aα, 5aα,8aα,8bα-(±)-hexahydro-3,4a-dimethyl-6H-pyrrolo[3',4':4,5]furo[3,2-b]pyrid-8 (7H)-on.

¹H-NMR (300 MHz, CDCl₃, δ): 4,42 (dd, 1H, 5aα-H; J¹ = 7,4 Hz; J² = 1,0 Hz) ppm.

EI-MS m/z (%): 360 (M⁺, 38); 275 (100); 148 (62); 41 (54).

### Beispiel 21

### 6α-Allyl-1-allyloxycarbonyl-1,2,3,4,4aα,5aα,8aα,8bα-(±)-oktahydro-3β,4a,7-trimethyl-6H-pyrrolo[3',4':4,5]furo[3,2-b]pyrid-8(7H)-on

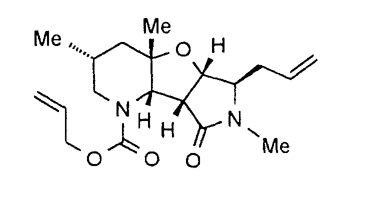

Die Darstellung des 6α-Allyl-Isomeren erfolgt analog dem Beispiel 19 aus:

| | |
|---|---|
| 0,80 g (2,3 mMol) | 1-Allyloxycarbonyl-6α-ethoxy-1,2,3,4,4aα,5aα,8aα,8bα-(±)-oktahydro-3β,4a,7-trimethyl-6H-pyrrolo[3',4':4,5]furo[3,2-b]pyrid-8(7H)-on |
| 1,07 g (4,6 mMol) | Allyltrimethylsilan |
| 0,66 g (4,6 mMol) | BF₃ · Et₂O |
| 20 ml | Methylenchlorid |

Man erhält 0,3 g (33,7 % der Theorie) 6α-Allyl-1-allyloxycarbonyl-1,2,3,4,4aα,5aα, 8aα,8bα-(±)-oktahydro-3β,4a,7-trimethyl-6H-pyrrolo[3',4':4,5]furo[3,2-b]pyrid-8(7H)-on.

¹H-NMR (400 MHz, CHCl₃, δ): 4,28 (dd, 1H, 5aα-H; J = 5,5 Hz).

EI-MS m/z (%): 348 (M⁺, 9); 263 (100); 138 (12); 108 (62); 41 (54).

### Beispiel 22

### 6α-Benzoylmethyl-1-allyloxycarbonyl-7-ethyl-1,2,4aα,5aα,8aα,8bα-(±)-hexahydro-3,4a-dimethyl-6H-pyrrolo[3',4':4,5]furo[3,2-b]pyrid-8(7H)-on

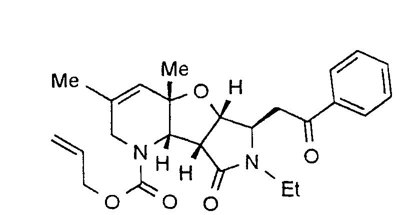

Die Darstellung des 6α-Benzoylmethyl-Isomeren erfolgt analog dem Beispiel 19 aus:

| | |
|---|---|
| 2,00 g (5,50 mMol) | 1-Allyloxycarbonyl-6α-ethoxy-7-ethyl-1,2,4aα,5aα,8aα,8bα-(±)-hexahydro-3,4a,7-dtmethyl-6H-pyrrolo[3',4':4,5]furo[3,2-b]pyrid-8(7H)-on |
| 2,10 g (11,0 mMol) | 1-Phenyltrimethylsiloxyethylen |
| 1,60 g (11,0 mMol) | BF₃, Et₂O |
| 50 ml | Methylenchlorid |

Man erhält 0,5 g (21,4 % der Theorie) 6α-Benzoylmethyl-1-allyloxycarbonyl-7-ethyl-1,2,4aα,5aα,8aα,8bα-(±)-hexahydro-3,4a-dimethyl-6H-pyrrolo[3',4':4,5]furo[3,2-b] pyrid-8(7H)-on.

EI-MS m/z (%): 338 (M⁺, 38); 353 (75); 105 (100).

### Beispiel 23

### 1-Allyloxycarbonyl-6α-cyan-7-ethyl-1,2,4aα,5aα,8aα,8bα-(±)-hexahydro-3,4a-dimethyl-6H-pyrrolo[3',4':4,5]furo[3,2-b]pyrid-8(7H)-on

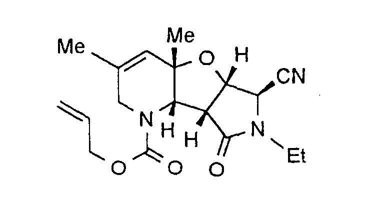

Die Darstellung des 6α-Cyan-Isomeren erfolgt analog dem Beispiel 19 aus:

| | |
|---|---|
| 1,20 g (3,0 mMol) | 1-Allyloxycarbonyl-6α-ethoxy-7-ethyl-1,2,4aα,5aα,8aα,8bα-(±)-hexahydro-3,4a,dimethyl-6H-pyrrolo[3',4':4,5]furo[3,2-b]pyrid-8(7H)-on |
| 0,59 g (6,0 mMol) | Trimethylsilylcyanid |
| 0,85 g (6,0 mMol) | BF₃ Et₂O |
| 30 ml | Methylenchlorid |

Man erhält 0,3 g (31,2 % der Theorie) 1-Allyloxycarbonyl-6α-cyan-7-ethyl-1,2,4aα, 5aα,8aα,8bα-(±)-hexahydro-3,4a-dimethyl-6H-pyrrolo[3',4':4,5]furo[3,2-b]pyrid-8 (7H)-on.

¹H-NMR (300 MHz, CDCl₃, δ): 4,27 (s, 1H, 6β-H); 4,90 (d, 1H, 5aα-H, J = 7,4 Hz) ppm.

¹³C-NMR (150 MHz, CDCl₃, δ): 11,6, 11,7 20,2, 20,3, 26,0, 26,1 (6 x -CH₃); 36,2 (1 x -CO-N-CH₂-); 155,3, 156,0, 170,3, 170,6 (4 x -N-C=O); 45,3, 46,0 (2 x -CH-CO-); 45,8, 46,0 (2 x -N-CH₂-), 55,5, 60,3, 60,6 (2 x -N-CH-); 66,4, 66,5 (2 x -O-CH₂-); 76,8, 78,6, 78,8 (3 x -O-CH-); 116,0 (1 x -CN); 117,5; 117,7 (2 x =CH₂); 123,7, 124,2 (2 x -CH=); 131,4, 131,9 (2 x Me-C=); 132,6; 132,7 (2 x =CH-CH₂-) ppm (E/Z-Isomerengemisch).

EI-MS m/z (%): 345 (M⁺, 12); 260 (100); 124 (18); 41 (15).

### Beispiel 24

### 1-Allyloxycarbonyl-6α-(fur-2-yl)-1,2,4aα,5aα,8aα,8bα-(±)-hexahydro-3,4a,7-trimethyl-6H-pyrrolo[3',4':4,5]furo[3,2-b]pyrid-8(7H)-on

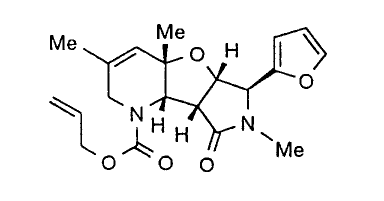

1,3 (3,9 mMol) 1-Allyloxycarbonyl-6α-cyan-1,2,4aα,5aα,8aα,8bα-(±)-hexahydro-3, 4a,7-trimethyl-6H-pyrrolo[3',4':4,5]furo[3,2-b]pyrid-8(7H)-on werden in Gegenwart von 0,74 g (3,9 mMol) *para*-Toluolsulfonsäure mit 5 ml Furan 24 Stunden unter Rückflußtemperatur gerührt. Nach Abkühlen wird mit NatriumhydrogencarbonatLösung versetzt, die organische Phase abgetrennt und im Vakuum eingeengt. Das zurückbleibende Rohprodukt wird über eine Kieselgelsäule (Kieselgel 60 - Merck, Korngröße: 0,04 bis 0,063 mm) mit dem Fließmittel Cyclohexan : Aceton (6 : 1) chromatographiert. Man erhält 0,7 g (48,3 % der Theorie) 1-Allyloxycarbonyl-6α-(fur-2-yl)-1,2,4aα,5aα,8aα,8bα-(±)-hexahydro-3,4a,7-trimethyl-6H-pyrrolo[3',4':4,5] furo[3,2-b]pyrid-8(7H)-on.

¹H-NMR (300 MHz, CDCl₃, δ): 4,42 (s, 1H, 6β-H); 4,64 (d, 1H, 5aα-H, J = 7,4 Hz); 6,23, 6,32, 7,36 (3m, 3H, Fyryl-H) ppm.

EI-MS m/z (%): 345 (M⁺, 12); 260 (100); 124 (18); 41 (15).

Analog zu den Verfahren können die in den nachstehenden Tabellen 27 und 28 aufgeführten Verbindungen der allgemeinen Formel (I-1) und (I-2) hergestellt werden.

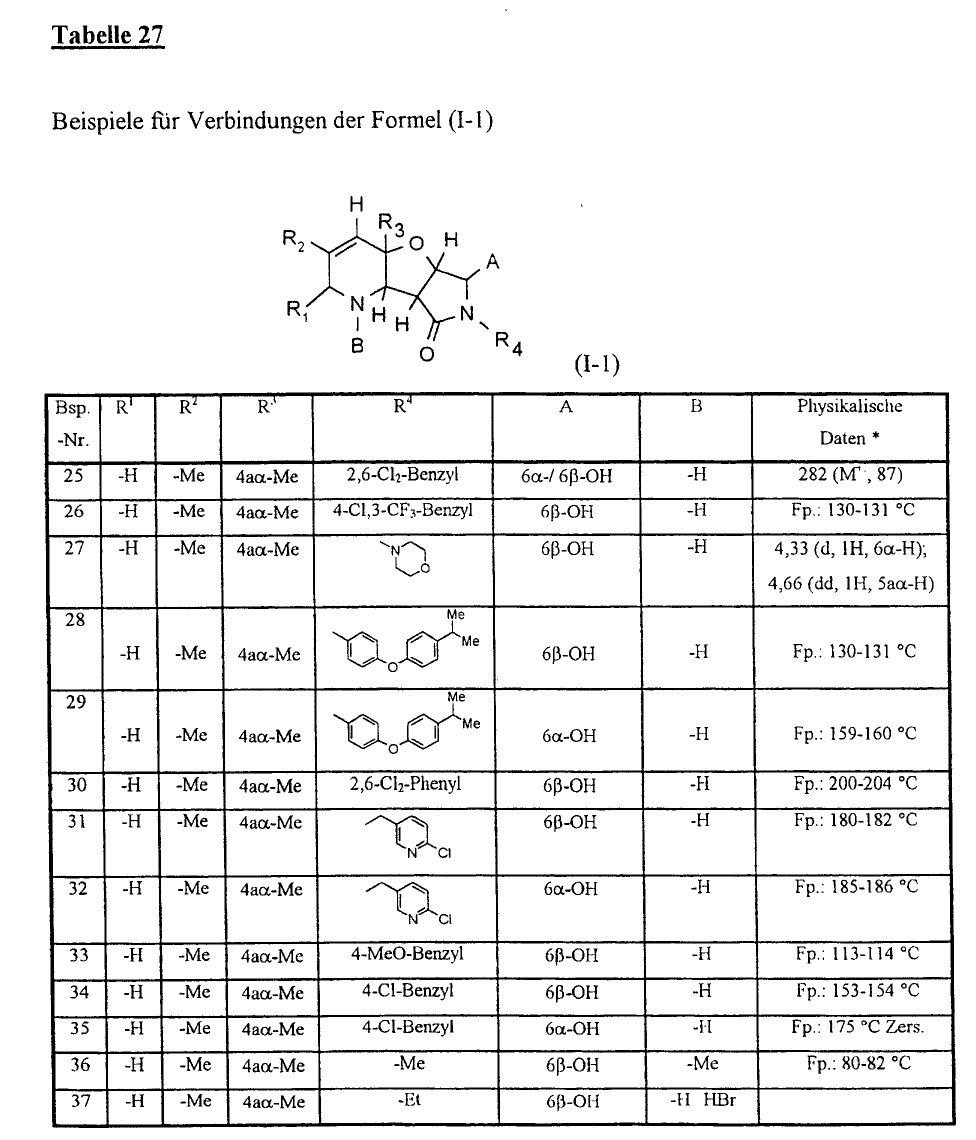

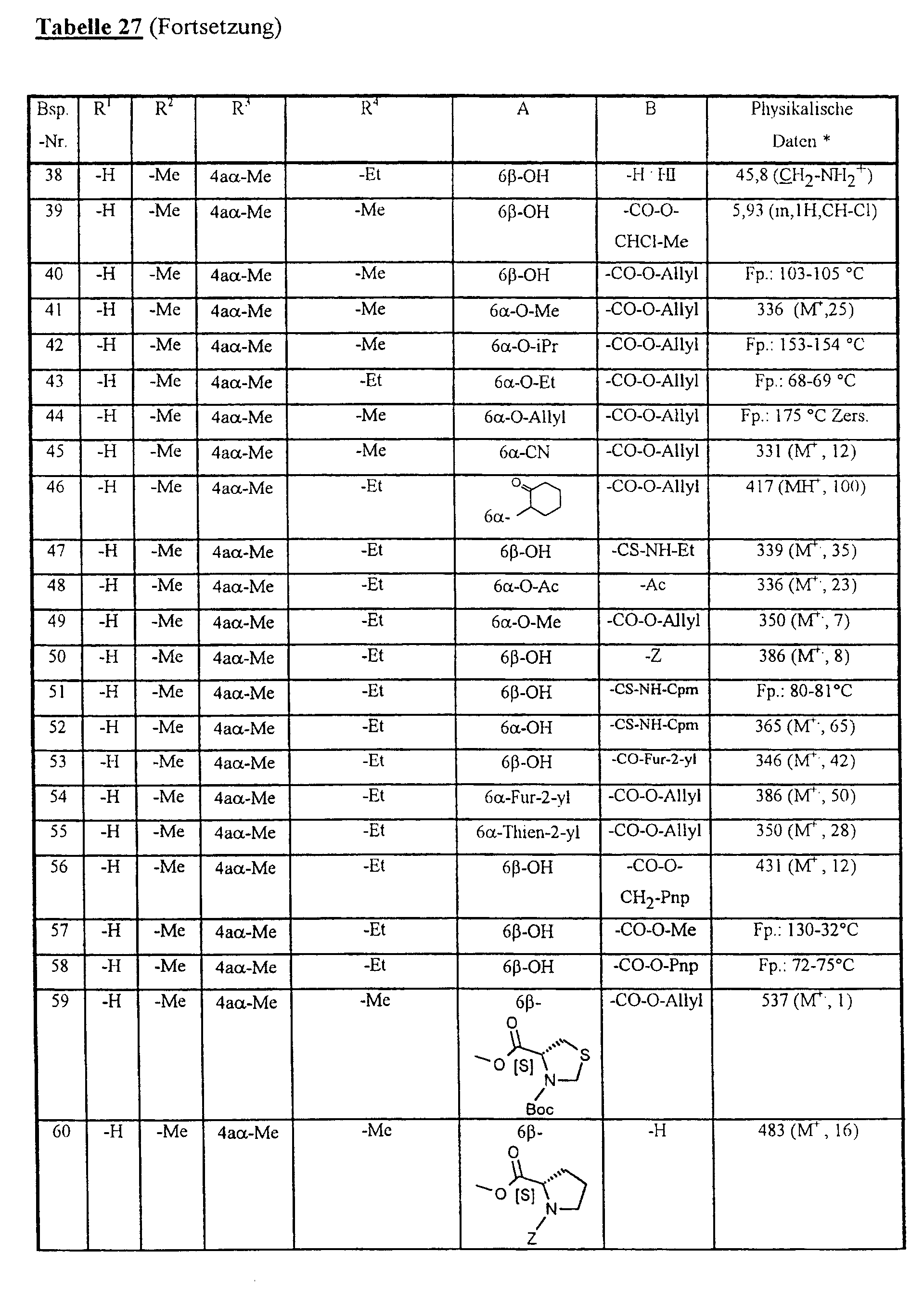

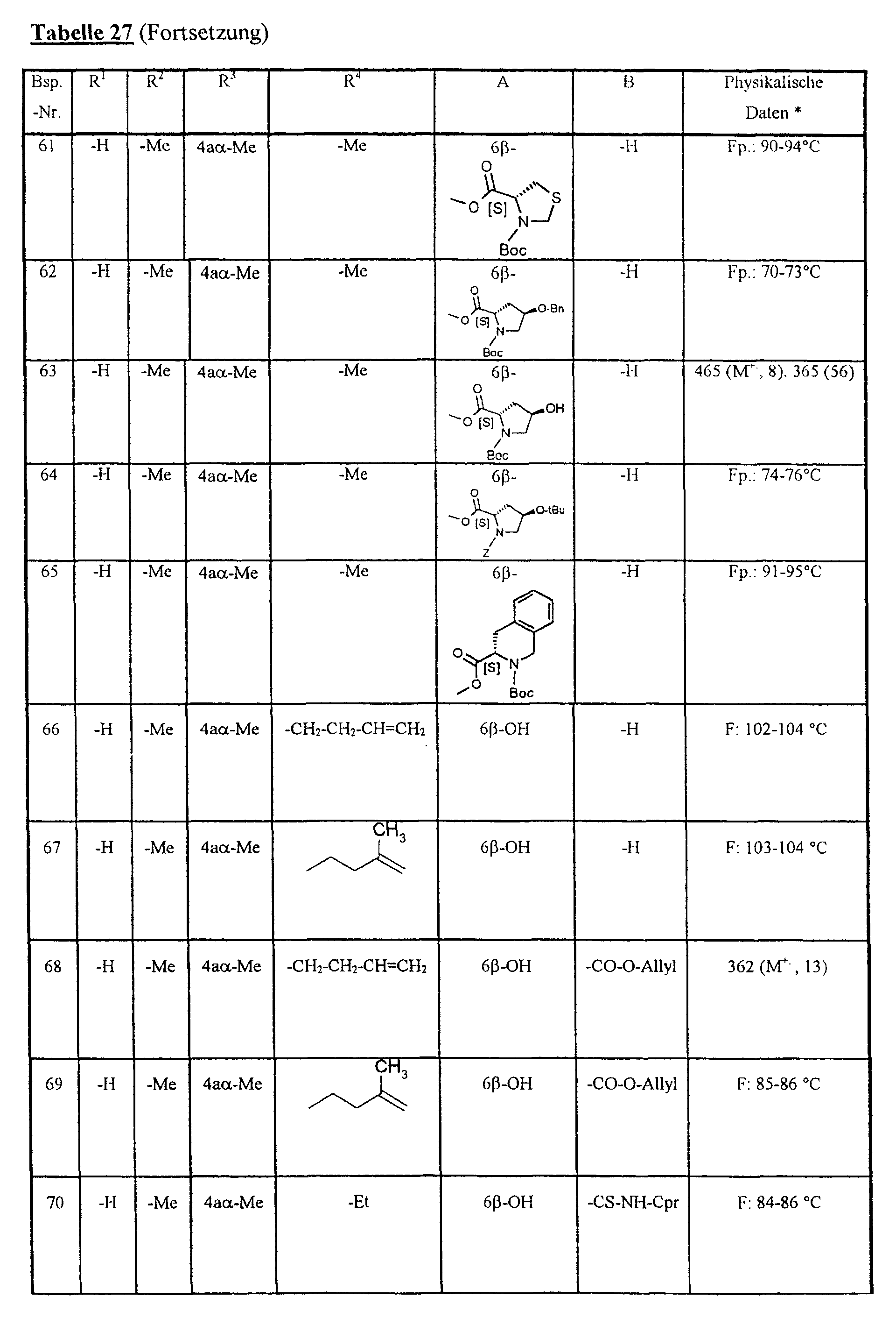

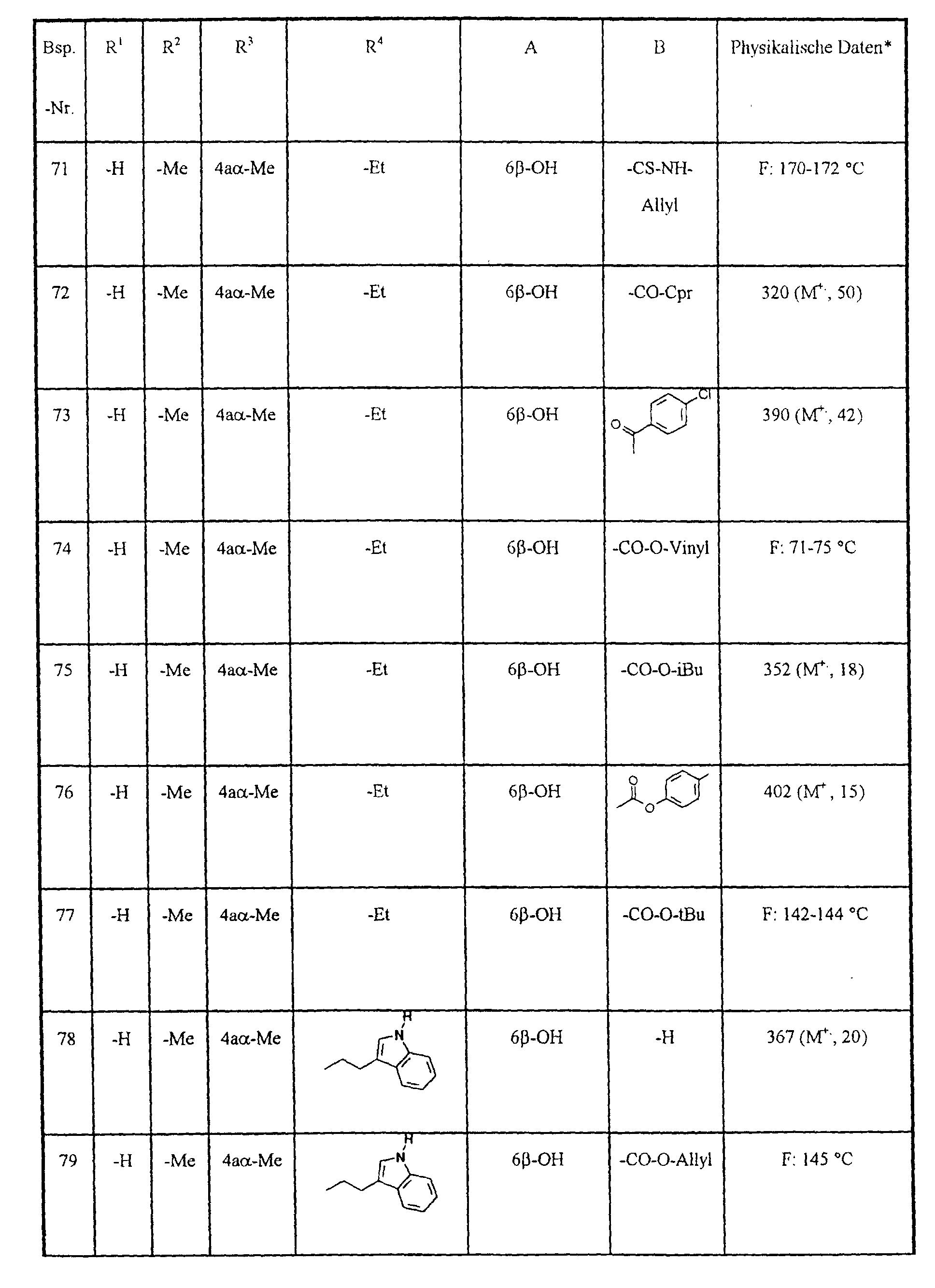

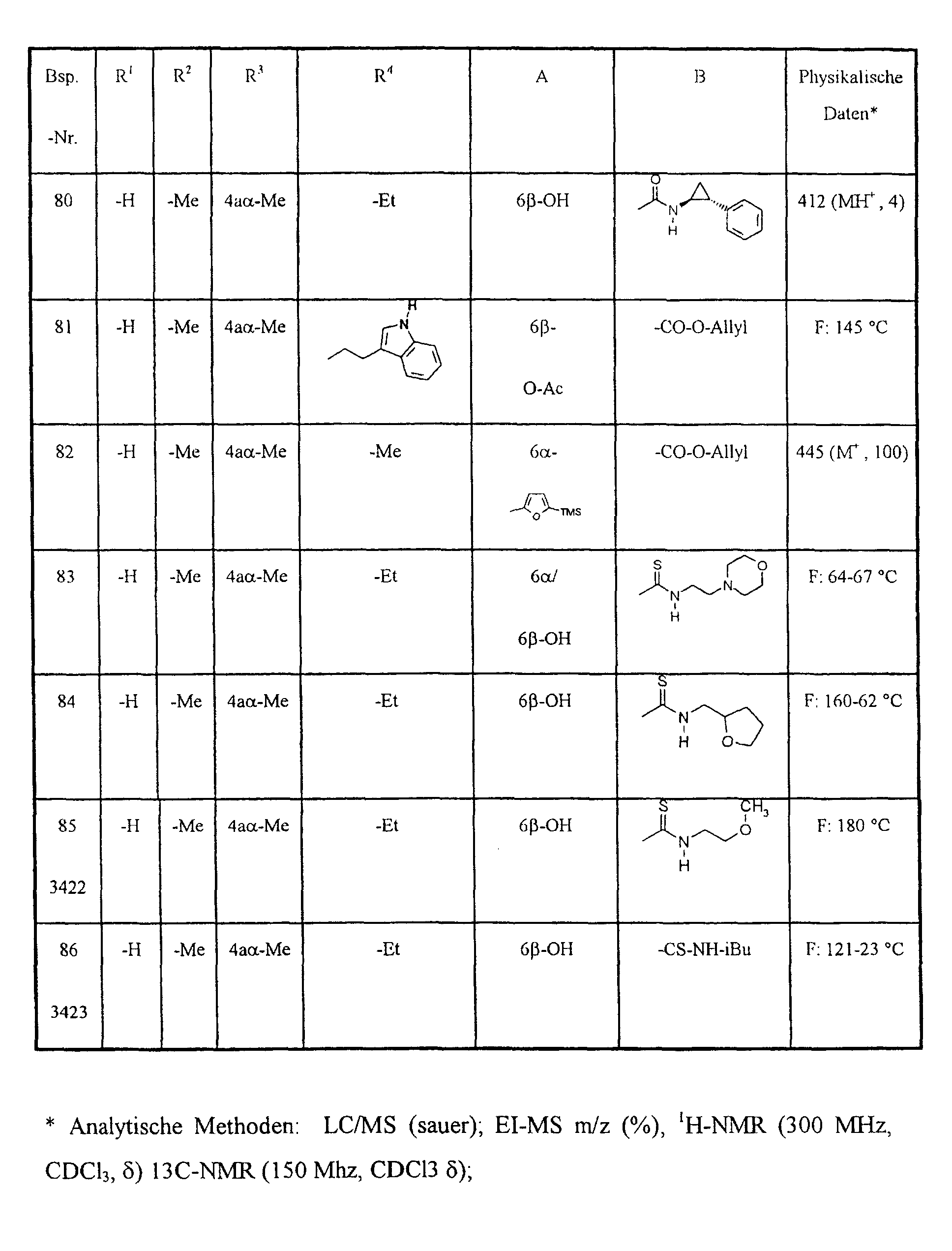

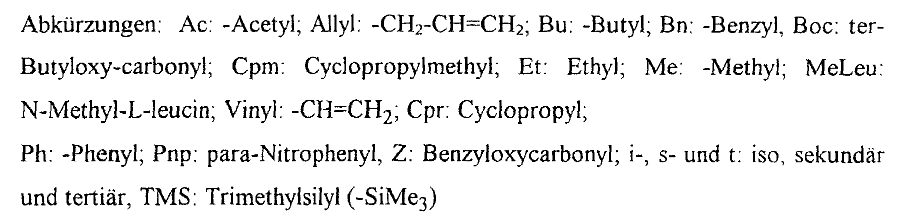

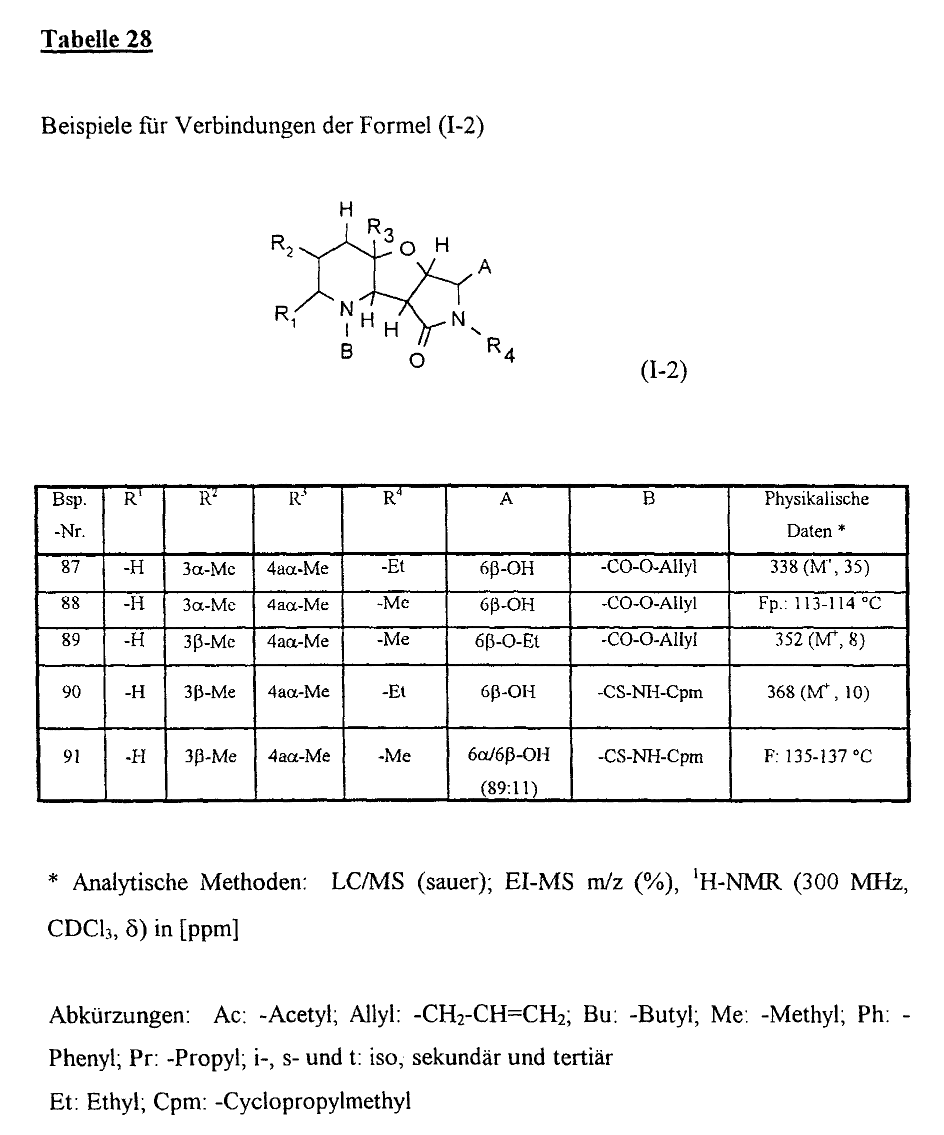

## Patentansprüche

1. 6-Substituierte 1,2,4a,5a,8a,8b-Hexahydro- und 1,2,3,4,4a,5a,8a,8b-Oktahydro-6H-pyrrolo[3',4':4,5]furo[3,2-b]pyrid-8(7H)-on Derivate der allgemeinen Formel (I) und deren Salze, 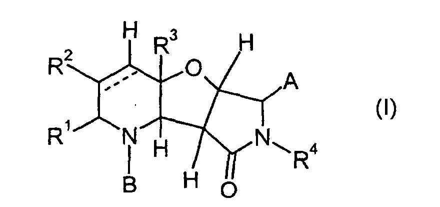 in welcher
R¹ für Wasserstoff, geradkettiges oder verzweigtes C₁₋₄-Alkyl oder C₃₋₆-Cycloalkyl steht,
R² für Wasserstoff, geradkettiges oder verzweigtes C₁₋₄-Alkyl, Halogen-C₁₋₄-alkyl, Hydroxy-C₁₋₄-alkyl, C₁₋₄-Alkanoyloxy-C₁₋₄-alkyl, C₁₋₂-Alkoxyalkyl, C₁₋₂-Alkylthioalkyl, C₁₋₂-Alkylsulfinylalkyl, C₁₋₂-Alkylsulfonylalkyl, Amino-C₁₋₂-alkyl, C₁₋₆-Alkylaminoalkyl, C₁₋₆-Dialkylaminoalkyl, C₃₋₆-Cycloalkylaminoalkyl, C₃₋₆-Cycloalkyl, C₁₋₄-Alkoxycarbonyl stehen,
R³ für Wasserstoff, geradkettiges oder verzweigtes C₁₋₄-Alkyl steht,
R⁴ geradkettiges oder verzweigtes C₁₋₆-Alkyl, Halogen-C₁₋₆-alkyl, C₁₋₄-Alkoxycarbonyl-C₁₋₄-alkyl, C₂₋₆-Alkenyl, C₃₋₆-Cycloalkyl, C₃₋₆-Cycloalkyl-C₁₋₂-alkyl, Aryl, Aryl-C₁₋₂-alkyl, Pyridyl, Thiazolyl, N-Morpholinyl, 2-Chlorpyrid-5-yl-methyl und Chlorthiazol-5-yl-methyl, die gegebenenfalls durch Reste aus der Reihe Halogen, Halogen-C₁₋₄-alkyl, Amino, Hydroxy, Nitro, Cyan, C₁₋₄-Alkoxy, C₁₋₂-Alkylendioxy, Halogen-C₁₋₄-alkoxy, C₁₋₄-Alkylthio, Halogen-C₁₋₄-alkylthio, C₁₋₄-Alkylsulfonyl, C₁₋₄-Alkylamino, C₁₋₄-Dialkylamino, C₁₋₄-Alkylcarbonyl, C₁₋₄-Alkoxycarbonyl substituiert sein können, steht,
A für Hydroxy, C₁₋₆-Alkyl, C₂-₆-Alkenyl, C₂₋₆-Alkinyl, C₃₋₆-Cycloalkyl, C₃₋₆-Cycloalkyl-C₁₋₂-alkyl, C₁₋₆-Alkoxy, C₂₋₆-Alkenyloxy, C₂₋₆-Alkinyloxy, C₁₋₄-Alkoxycarbonyl, Mercapto, C₁₋₆-Alkylthio, Cyan, Carbamoyl, Thiocarbamoyl, Aryl-C₁₋₂-alkyloxy, Furyl, die gegebenenfalls durch Reste aus der Reihe Halogen, C₁₋₄-Alkyl, Halogen-C₁₋₄-alkyl, Amino, Hydroxy, Nitro, Cyan, C₁₋₄-Alkoxy, C₁₋₂-Alkylendioxy, Halogen-C₁₋₄-alkoxy, C₁₋₄-Alkylthio, Halogen-C₁₋₄-alkylthio, C₁₋₄-Alkylsulfonyl, C₁₋₄-Alkylamino, C₁₋₄-Dialkylamino, substituiert sein können, steht, oder gegebenenfalls für einen Rest aus der Gruppe A¹, A² und A³ 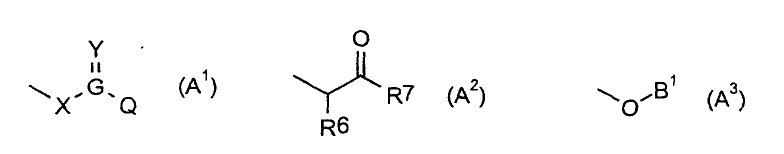 worin
X für Sauerstoff steht, 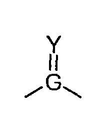
für Carboxy oder Sulfonyl steht,
Q für geradkettiges oder verzweigtes C₁₋₆-Alkyl, C₂₋₆-Alkenyl, C₂₋₆-Alkinyl, C₃₋₆-Cycloalkyl, C₃₋₆-Cycloalkoxy, C₁₋₆-Alkoxy, C₂₋₆-Alkenyloxy, C₂₋₆-Alkinyloxy, C₁₋₄-Alkylamino, C1-4-Dialkylamino eine über Stickstoff verknüpfte cyclische Aminogruppe bedeuten kann, Aryl, Aryl-C₁₋₂-alkyl, Aryl-C₁₋₂-alkyloxy, die gegebenenfalls substituiert sind, steht,
R⁶ für Wasserstoff oder Methyl, steht,
R⁷ für geradkettiges oder verzweigtes C₁₋₆-Alkyl, C₂₋₆-Alkenyl, C₃₋₆-Cycloalkyl, Aryl, die gegebenenfalls substituiert sind, steht,
R⁶ und R⁷ gemeinsam mit den Atomen, an die sie gebunden sind, für einen 5-, 6- oder 7-gliedrigen carbocyclischen Ring stehen, der gegebenenfalls durch C₁₋₄-Alkyl substituiert sein kann, steht,
B für Wasserstoff, geradkettiges oder verzweigtes C₁₋₆-Alkyl, C₁₋₄-Alkanoyloxyalkyl, C₁₋₂-Alkoxyalkyl, Amino-C₁₋₆-alkyl, C₁₋₆-Alkyl-amino-C₁₋₆-alkyl, C₁₋₆-Dialkylamino-C₁₋₆-alkyl, C₂₋₆-Alkenyl, C₂₋₆-Alkinyl, Carbamoyl-C₁₋₄-alkyl, Carboxyl-C₁₋₄-alkyl, C₁₋₄-Alkoxydicarbonyl, Aryl, Aryl-C₁₋₂-alkyl, Pyridyl, Pyrimidyl, Pyrrolidyl, Imidazolyl, Thiazolyl, N-Morpholinyl, Pyridylmethyl und Thiazolylmethyl, die gegebenenfalls durch Reste aus der Reihe Halogen, C₁₋₄-Alkyl, Halogen-C₁₋₄-alkyl, Amino, Hydroxy, Nitro, Cyan, C₁₋₄-Alkoxy, C₁₋₂-Alkylendioxy, Halogen-C₁₋₄-alkoxy, C₁₋₄-Alkylthio, Halogen-C₁₋₄-alkylthio, C₁₋₄-Alkylsulfonyl, C₁₋₄-Alkylamino, C₁₋₄-Dialkylamino, C₁₋₄-Alkylcarbonyl, C₁₋₄-Alkoxycarbonyl substituiert sein können, steht, oder gegebenenfalls für einen Rest aus der Gruppe B¹, B², B³ und B⁴ steht 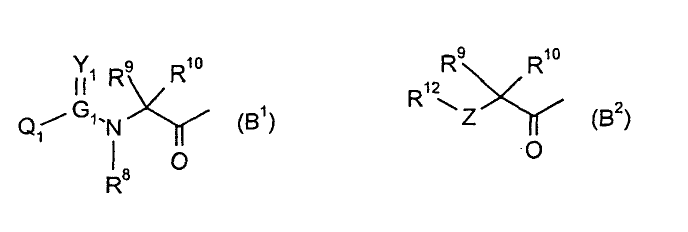 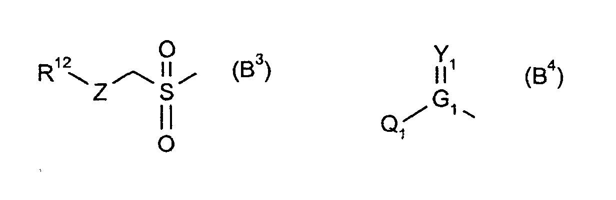 worin
R⁸ für Wasserstoff, geradkettiges oder verzweigtes C₁₋₆-Alkyl, C₃₋₆-Cycloalkyl steht, oder
R⁸ und R⁹ gemeinsam mit den Atomen, an die sie gebunden sind, für einen 5- oder 6-gliedrigen Ring stehen, der gegebenenfalls durch Schwefel unterbrochen sein kann und gegebenenfalls durch Hydroxy, tert.-Butoxy, Benzyloxy substituiert ist, stehen,
R⁹ für Wasserstoff, geradkettiges oder verzweigtes C₁₋₆-Alkyl steht,
R¹⁰ für Wasserstoff oder Methyl steht, 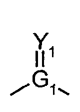
für Carbonyl, -C=CH-NO₂, -C=CH-CN, -C=N-R¹¹, Sulfonyl, steht,
R¹¹ Halogen-C₁₋₄-alkylcarbonyl, C₁₋₄-Alkylsulfonyl, Nitro oder Cyan steht, und
Q¹ für einen Rest aus der Gruppe G¹ und G² 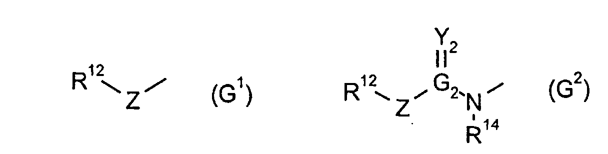
worin 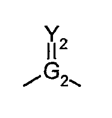 Carbonyl oder Sulfonyl bedeuten kann,
Z für Sauerstoff oder -NR¹³ steht,
R¹² für den Fall, dass Z für Stickstoff steht, eine über ein Stickstoffatom verknüpfte cyclische Aminogruppe, die gegebenenfalls durch Reste aus der Reihe Halogen, C₁₋₄-Alkyl, Hydroxy-C₁₋₄-alkyl, Amino-C₁₋₄-alkyl, C₁₋₄-Alkylamino-C₁₋₄-alkyl, C₁₋₄-Dialkylamino-C₁₋₄-alkyl, Amino, Hydroxy, C₁₋₄-Alkoxy, C₁₋₄-Alkylcarbonyl, C₁₋₄-Alkoxycarbonyl substituiert sein können,
R¹² und R¹³ unabhängig voneinander für Wasserstoff, geradkettiges oder verzweigtes C₁₋₆-Alkyl, C₂₋₄-Alkenyl, C₂₋₄-Alkinyl, C₃₋₆-Cycloalkyl, Pyridylmethyl und Thiazolylmethyl, die gegebenenfalls durch Reste aus der Reihe Halogen, C₁₋₄-Alkyl, Hydroxy-C₁₋₄-alkyl, Amino-C₁₋₄-alkyl, C₁₋₄-Alkylamino-C₁₋₄-alkyl, C₁₋₄-Dialkylamino-C₁₋₄-alkyl, Amino, Hydroxy, C₁₋₄-Alkoxy, C₁₋₄-Alkylcarbonyl, C₁₋₄-Alkoxycarbonyl substituiert sein können, stehen, oder
R¹² und R¹³ gemeinsam mit dem angrenzenden N-Atom für ein carbocyclisches 5-, 6- oder 7-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O, -N=, -NR¹⁵- oder durch quarternisierten Stickstoff unterbrochen sein kann und gegebenenfalls durch C₁₋₄-Alkyl, Hydroxy-C₁₋₄-alkyl, Amino-C₁₋₄-alkyl, C₁₋₄-Alkylamino-C₁₋₄-alkyl, C₁₋₄-Dialkylamino-C₁₋₄-alkyl, Amino, Hydroxy, C₁₋₄-Alkoxy, C₁₋₄-Alkylcarbonyl, C₁₋₄-Alkoxycarbonyl, Halogen substituiert ist, stehen,
R¹⁴ für Wasserstoff oder C₁₋₄-Alkyl steht,
R¹⁵ für Wasserstoff, geradkettiges oder verzweigtes C₁₋₆-Alkyl, C₂₋₆-Alkenyl, C₂₋₆-Alkinyl, C₃₋₆-Cycloalkyl, C₁₋₄-Alkoxycarbonyl, C₁₋₄-Alkylcarbonyl, C₃₋₆-Cycloalkylcarbonyl, Cyan, Aryl, Aryl-C₁₋₂-alkyl, Pyridyl und Thiazolyl, Pyridylmethyl, Thiazolylmethyl, die gegebenenfalls durch Reste aus der Reihe Halogen, C₁₋₄-Alkyl, Halogen-C₁₋₄-alkyl, Amino, Hydroxy, Cyan, C₁₋₄-Alkoxy, C₁₋₂-Alkylendioxy, Halogen-C₁₋₄-alkoxy, C₁₋₄-Alkylthio, Halogen-C₁₋₄-alkylthio, C₁₋₄-Alkylsulfonyl, C₁₋₄-Alkylamino, C₁₋₄-Dialkylamino, C₁₋₄-Alkylcarbonyl, C₁₋₄-Alkoxycarbonyl substituiert sein können, steht,
sowie deren optische Isomere und Racemate,
wobei "Aryl" in den Definitionen für einen ein-, zwei- oder mehrwertigen aromatischen Rest, ausgewählt aus Phenyl, Naphthyl, Tetrahydronaphthyl, Indanyl und Fluorenyl steht
und wobei, soweit nicht anders angegeben, die gegebenenfalls substituierten Reste einen oder mehrere gleiche oder verschiedene Substituenten tragen können, ausgewählt aus: Alkyl mit 1 bis 4 Kohlenstoffatomen, Alkoxy mit 1 bis 4 Kohlenstoffatomen, Alkylthio, Halogenalkyl mit 1 bis 5 Halogenatomen, wobei die Halogenatome gleich oder verschieden sind, Halogen, Cyan, Nitro, Amino, Monoalkyl- und Dialkylamino mit 1 bis 4 Kohlenstoffatomen je Alkylgruppe, Alkylcarbonylreste, Alkoxycarbonyl mit 2 bis 4 Kohlenstoffatomen, Alkylsulfinyl mit 1 bis 4 Kohlenstoffatomen, Halogenalkylsulfinyl mit 1 bis 4 Kohlenstoffatomen und 1 bis 5 Halogenatomen, Halogenalkylsulfonyl mit 1 bis 4 Kohlenstoffatomen und 1 bis 5 Halogenatomen, Arylsulfonyl mit 6 oder 10 Arylkohlenstoffatomen, Trialkylsilyl mit 1 bis 4 Kohlenstoffatomen, Acyl, Aryl, Aryloxy, die ihrerseits einen der obengenannten Substituenten tragen können sowie dem Formiminorest (-HC=N-O-Alkyl).

2. Verfahren zur Herstellung der 6-Hydroxy-1,2,4a,5a,8a,8b-hexahydro-6H-pyrrolo[3',4':4,5]furo[3,2-b]pyrid-8(7H)-on und/oder 6-Hydroxy-1,2,3,4,-4a,5a,8a,8b-oktahydro-6H-pyrrolo[3',4':4,5]furo[3,2-b]pyrid-8(7H)-on Derivate der allgemeinen Formeln (Ia) und (Ib) sowie deren Salze gemäß Anspruch 1 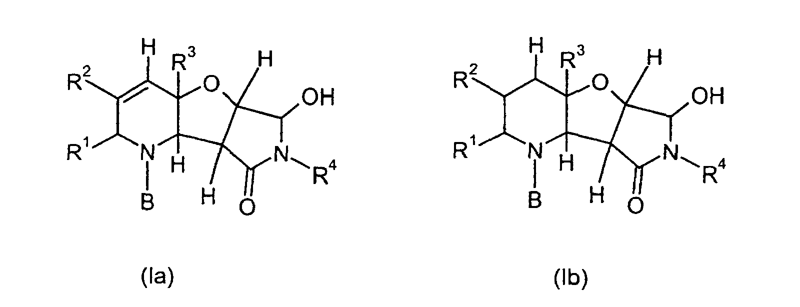 in welchen
R¹, R², R³, R⁴ und B die in Anspruch 1 angegebene Bedeutung besitzen, **dadurch gekennzeichnet,**
**dass** man die 1,2,4a,5a,8a,8b-Hexahydro-6H-pyrrolo[3',4':4,5]furo[3,2-b]pyridin-6,8(7H)-dion
und/oder1,2,3,4,4a,5a,8a,8b-Oktahydro-6H-pyrrolo[3',4':4,5]furo[3,2-b]pyridin-6,8(7H)-dion Derivate der allgemeinen Formeln (IIa) und (IIb) sowie deren Salze 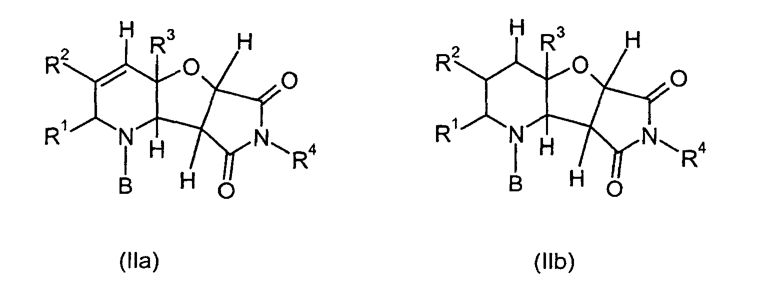 in welchen
R¹, R², R³, R⁴ und B die In Anspruch 1 angegebene Bedeutung besitzen, in Gegenwart eines geeigneten Verdünnungsmittels hydriert,
oder indem man in einem ersten Reaktionsschritt
die 1,2,4a,5a,8a,8b-Hexahydro-6H-pyrrolo[3',4':4,5]furo[3,2-b]pyridin-6,8-(7H)-dion und/oder 1,2,3,4,4a,5a,8a,8b-Oktahydro-6H-pyrrolo[3',4':4,5]furo[3,2-b]pyridin-6,8(7H)-dion Derivate der allgemeinen Formeln (IIc) und (IId) sowie deren Salze 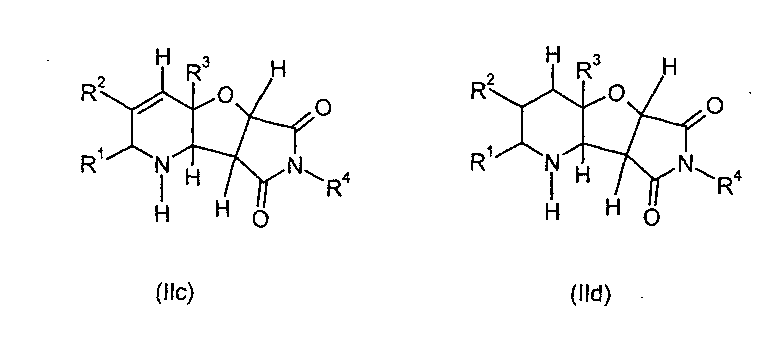 in welchen
R¹, R², R³ und R⁴ die in Anspruch 1 angegebene Bedeutung besitzen,
in Gegenwart eines geeigneten Verdünnungsmittels zu 6-Hydroxy-1,2,4a,-5a,8a,8b-hexahydro-6H-pyrrolo[3',4':4,5]furo[3,2-b]pyrid-8(7H)-on und/oder 6-Hydroxy-1,2,3,4,4a,5a,8a,8b-oktahydro-6H-pyrrolo[3',4':4,5]furo[3,2-b]pyrid-8(7H)-on Derivaten der allgemeinen Formeln (Ic) und (Id) 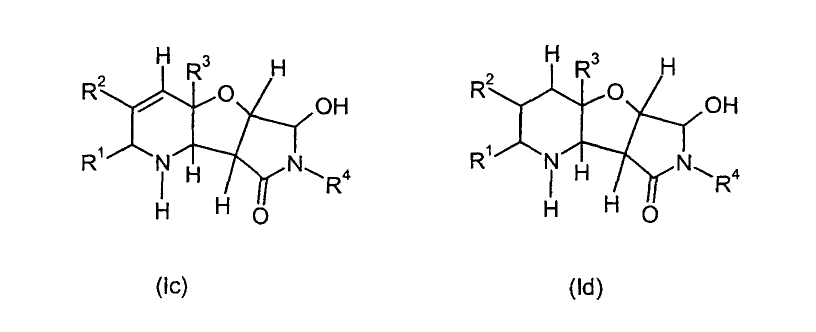 in welchen
R¹, R², R³ und R⁴ die in Anspruch 1 angegebene Bedeutung besitzen,
hydriert,
oder indem man zur selektiven Darstellung der neuen Derivate der allgemeinen Formel (IIa) und deren Salze in einem ersten Reaktionsschritt
die 4a,5a,8a,8b-Tetrahydro-6H-pyrrolo[3',4':4,5]furo[3,2-b]pyridin-6,8(7H)-dion Derivate der allgemeinen Formel (III) und deren Salze, 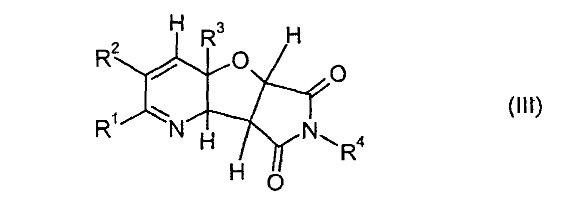 in welcher
R¹, R², R³ und R⁴ die in Anspruch 1 angegebene Bedeutung besitzen,
in Gegenwart eines geeigneten Verdünnungsmittels zu 6-Hydroxy-1,2,4a,5a,8a,8b-hexahydro-6H-pyrrolo[3',4':4,5]furo[3,2-b]pyrid-8(7H)-on Derivaten der allgemeinen Formel (Ic) 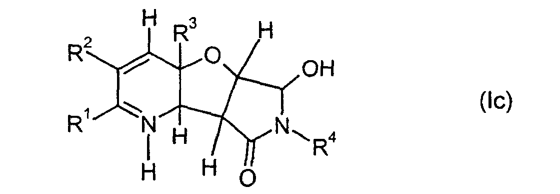 in welcher
R¹, R², R³ und R⁴ die in Anspruch 1 angegebene Bedeutung besitzen,
hydriert, und nachfolgend in einem zweiten Reaktionsschritt die auf diese Weise erhaltenen Derivate der allgemeinen Formeln (Ic) und (Id) 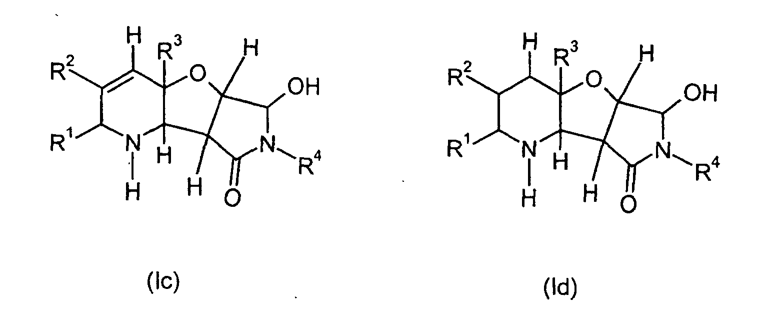 in welchen
R¹, R², R³ und R⁴ die in Anspruch 1 angegebene Bedeutung besitzen,
a) mit Verbindungen der allgemeinen Formel (IV)
B - E (IV)
in welcher
B die weiter oben angegebene Bedeutung hat, un
E für eine elektronenziehende Abgangsgruppe steht,
gegebenenfalls in Gegenwart von Verdünnungsmitteln und gegebenenfalls in Gegenwart eines basischen Reaktionshilfsmittels umsetzt, oder
b) mit Verbindungen der allgemeinen Formel (V) 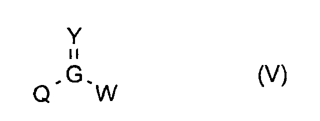 in welcher 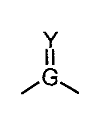 und Q die in Anspruch 1 angegebene Bedeutung haben, und
W für eine geeignete Abgangsgruppe, wie beispielsweise Halogen, Alkoxy, Alkylthio oder Aryloxy steht,
gegebenenfalls in Gegenwart eines Katalysators, gegebenenfalls in Gegenwart eines basischen Reaktionshilfsmittels und gegebenenfalls in Gegenwart von Verdünnungsmitteln umsetzt, oder indem man zur Darstellung der neuen 6-Hydroxy-1,2,4a,5a,8a,8b-hexahydro-6H-pyrrolo[3',4':4,5]furo[3,2-b]pyrid-(7H)-on und/oder 6-Hydroxy-1,2,3,4,-4a,5a,8a,8b-oktahydro-6H-pyrrolo[3',4':4,5]furo[3,2-b]pyrid-7H)-on Derivate der allgemeinen Formeln (Ia) und (Ib) sowie deren Salze, in denen die Gruppe 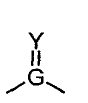 für Carbonyl steht, die Verbindungen der Formel Ic oder Id
c) mit einem Carbonsäureanhydrid der allgemeinen Formel (VI)
(Q-C=O)₂O (VI)
in welcher
Q die unter Anspruch 1 angegebene Bedeutung hat,
gegebenenfalls in Gegenwart eines Katalysators, gegebenenfalls in Gegenwart von Verdünnungsmitteln umsetzt, oder indem man die Verbindungen der Formel (Ic) oder (Id)
d) mit Aminosäurederivaten der allgemeinen Formel (VII) 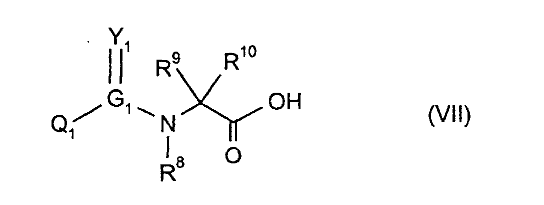 in welcher 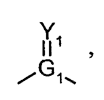 Q¹, R⁸, R⁹ und R¹⁰ die in Anspruch 1 angegebene Bedeutung haben,
gegebenenfalls in Gegenwart von Kupplungsreagenzien und in Gegenwart eines basischen Reaktionshilfsmittels, gegebenenfalls in Gegenwart von Verdünnungsmitteln umsetzt, oder indem man die Verbindungen der Formel (Ic) oder (Id)
e) mit Verbindungen der allgemeinen Formeln (VIII) oder (IX) 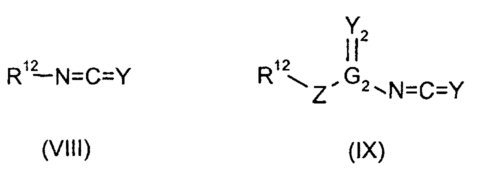 in welchen 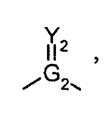 Z und R¹² die in Anspruch 1 angegebene Bedeutung haben,
Y für Sauerstoff oder Schwefel steht,
gegebenenfalls in Gegenwart eines Katalysators, gegebenenfalls in Gegenwart von Verdünnungsmitteln umsetzt.

3. Verfahren zur Herstellung von 6-substituierten 1,2,4a,5a,8a,8b-Hexahydro-6H-pyrrolo[3',4':4,5]furo[3,2-b]pyrid-8(7H)-on und/oder 6-substituierte 1,2,-3,4,4a,5a,8a,8b-Oktahydro-6H-pyrrolo[3',4':4,5]furo[3,2-b]pyrid-8(7H)-on Derivate der allgemeinen Formeln (Ie) und (If) sowie deren Salze gemäß Anspruch 1 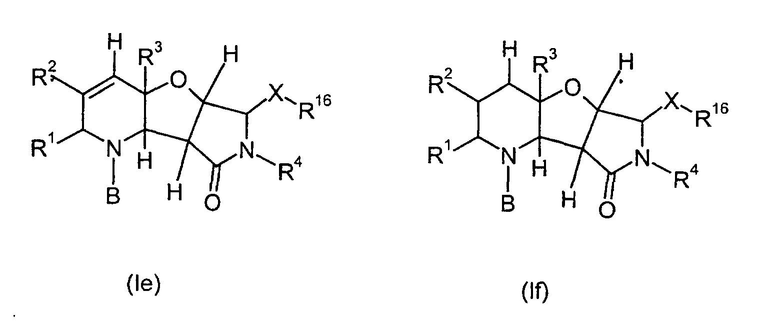 in welchen
R¹, R², R³, R⁴, X und B die in Anspruch 1 angegebene Bedeutung besitzen,
R¹⁶ für C₁₋₆-Alkyl, C₂₋₆-Alkenyl, C₂₋₆-Alkinyl, Aryl-C₁₋₂-alkyl, die gegebenenfalls durch Reste aus der Reihe Halogen, C₁₋₄-Alkyl, Halogen-C₁₋₄-alkyl, Amino, Hydroxy, Nitro, Cyan, C₁₋₄-Alkoxy, C₁₋₂-Alkylendioxy, Halogen-C₁₋₄-alkoxy, C₁₋₄-Alkylthio, Halogen-C₁₋₄-alkylthio, C₁₋₄-Alkylsulfonyl, C₁₋₄-Alkylamino, C₁₋₄-Dialkylamino, substituiert sind, oder
gegebenenfalls für die Gruppe 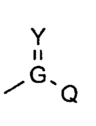 steht,
in welcher
G, Y und Q die in Anspruch 1 angegebene Bedeutung haben,
sowie deren optische Isomere und Racemate,
**dadurch gekennzeichnet, dass** man
a) die gemäß Anspruch 2 erhältlichen Derivate der allgemeinen Formeln (Ia) und (Ib) sowie deren Salze 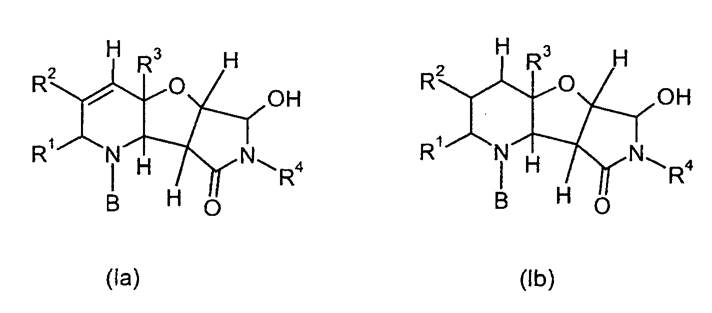 in welchen
R¹, R², R³, R⁴ und B die in Anspruch 1 angegebene Bedeutung besitzen, mit Verbindungen der allgemeinen Formel (X)
H-X-R¹⁶ (X)
in welcher
R¹⁶ und X die weiter oben angegebene Bedeutung besitzen,
gegebenenfalls in Gegenwart einer Säure, gegebenenfalls in Gegenwart von Verdünnungsmitteln umsetzt, oder indem man
b) zur Darstellung der 6-substituierten 1,2,4a,5a, 8a,8b-Hexahydro-6H-pyrrolo[3',4':4,5]furo[3,2-b]pyrid-8(7H)-on und/oder 6-substituierten 1,2,3,4,4a,5a,8a,8b-Oktahydro-6H-pyrrolo[3',4':4,5]-furo[3,2-b]pyrid-8(7H)-on Derivate der allgemeinen Formeln (Ie) und (If) sowie deren Salze,
in der für den Rest R¹⁶ die Gruppe 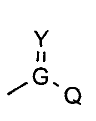 steht,
in welcher 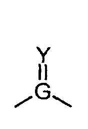 und Q die in Anspruch 1 angegebene Bedeutung haben,
mit Verbindungen der allgemeinen Formel (V) 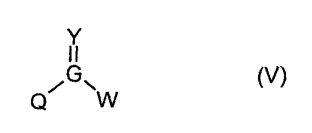 in welcher 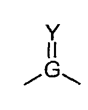 und Q die in Anspruch 1 angegebene Bedeutung haben, und
W für eine geeignete Abgangsgruppe steht,
gegebenenfalls in Gegenwart eines Katalysators, gegebenenfalls in Gegenwart eines basischen Reaktionshilfsmittels und gegebenenfalls in Gegenwart von Verdünnungsmitteln umsetzt, oder indem man
c) zur Darstellung der Derivate der allgemeinen Formeln (Ie) und (If) sowie deren Salze,
in denen die Gruppe 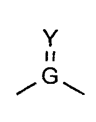 für Carbonyl steht,
mit einem Carbonsäureanhydrid der allgemeinen Formel (VI)
(Q-C=O)₂O (VI)
in welcher
Q die in Anspruch 1 angegebene Bedeutung hat, gegebenenfalls in Gegenwart eines Katalysators, gegebenenfalls in Gegenwart von Verdünnungsmitteln umsetzt, oder indem man
d) mit Verbindungen der allgemeinen Formeln (VIII) oder (IX)
R¹²―N=C=Y (VIII) 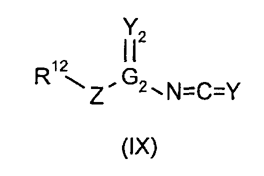
in welchen 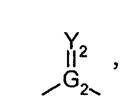 Z, Y und R¹² die unter in Anspruch 1 angegebene Bedeutung haben gegebenenfalls in Gegenwart eines Katalysators, gegebenenfalls in Gegenwart von Verdünnungsmitteln umsetzt, oder indem man
e) zur Darstellung der 6-substituierten 1,2,4a,5a, 8a,8b-Hexahydro-6H-pyrrolo[3',4':4,5]furo[3,2-b]pyrid-8(7H)-on und/oder 6-substituierten 1,2,3,4,4a,5a,8a,8b-Oktahydro-6H-pyrrolo[3',4':4,5]furo[3,2-b]pyrid-8(7H)-on Derivate der allgemeinen Formeln (Ie) und (If) sowie deren Salze, 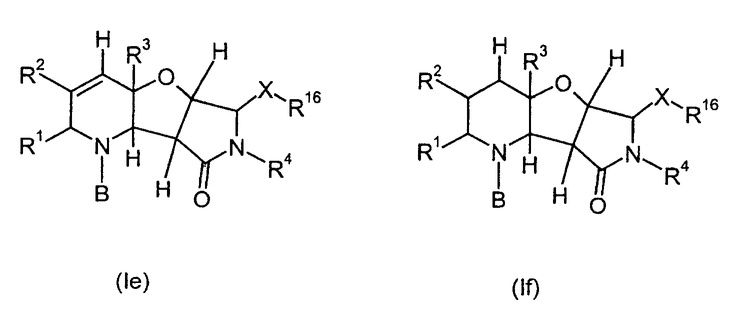 in welchen
R¹, R², R³, R⁴, X und B die in Anspruch 1 angegebene Bedeutung besitzen und für die Reste B und R¹⁶
die gleiche Gruppe 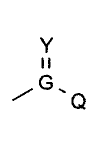 steht,
worin 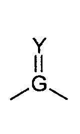 und Q die in Anspruch 1 angegebene Bedeutung haben,
Derivate der allgemeinen Formeln (Ic) und (Id) 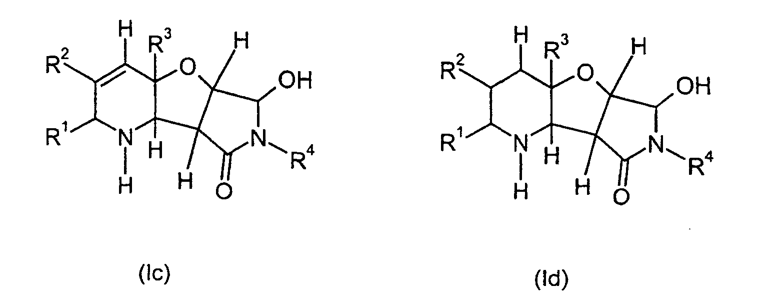 in welchen
R¹, R², R³, R⁴ die in Anspruch 1 angegebene Bedeutung besitzen,
mit Acylierungsmitteln der Formel (IV)
E-B (IV)
bzw.
E-R16
entweder am Rest -NH- in Position 1 oder am Rest -OH in Position 6 oder an beiden Resten umsetzt, wobei E-B bzw. E-R¹⁶ eine der folgenden Verbindungen der allgemeinen Formeln (V), (VI), (VIII) oder (IX) 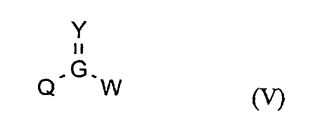
(Q-C=O)₂O (VI)
R¹²-N=C=Y (VIII) 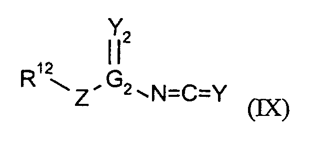
bedeuten, in welchen 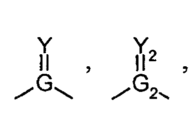 Q, Z, W und R¹² die in Anspruch 1 angegebene Bedeutung haben;
die Umsetzung erfolgt dabei gegebenenfalls in Gegenwart eines Katalysators, gegebenenfalls in Gegenwart eines basischen Reaktionshilfsmittels und gegebenenfalls in Gegenwart von Verdünnungsmitteln.
f) zur Darstellung der 6-substituierten 1,2,4a,5a,8a,8b-Hexahydro-6H-pyrrolo[3',4':4,5]furo[3,2-b]pyrid-8(7H)-on und/oder 6-substituierten 1,2,3,4,4a,5a,8a,8b-Oktahydro-6H-pyrrolo[3',4':4,5]furo[3,2-b]-pyrid-8(7H)-on Derivate der allgemeinen Formeln (Ie) und (If) sowie deren Salze, 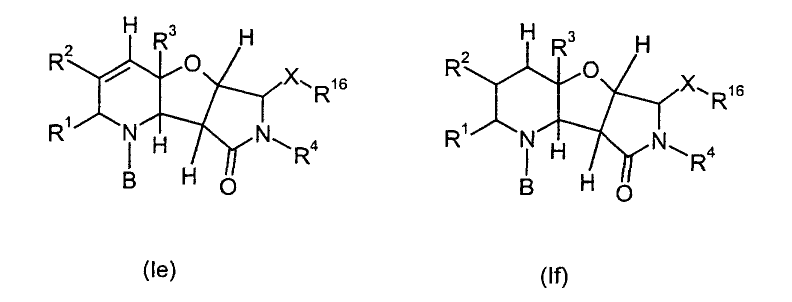 in welchen
R¹, R², R³, R⁴, X und B die in Anspruch 1 angegebene Bedeutung besitzen und für den Rest R¹⁶
die Gruppe 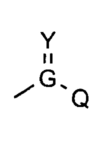 steht,
worin 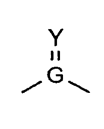 und Q die in Anspruch 1 angegebene Bedeutung haben,
Derivate der allgemeinen Formeln (Ic) und (Id) 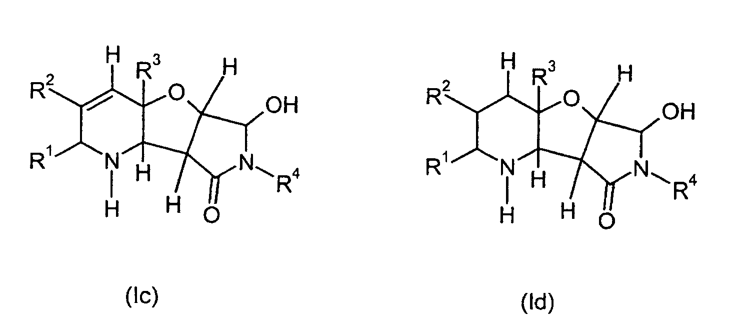 in welchen
R¹, R², R³, R⁴ die Anspruch 1 angegebene Bedeutung besitzen,
mit einem Acylierungsmittel der Formel (IV)
E - R¹⁶ (IV)
am Rest -OH in Position 6 umsetzt, wobei E - R¹⁶ für die Verbindung der allgemeinen Formel (VII) 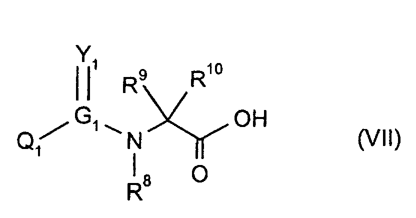 in welcher 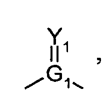 Q¹, R⁸, R⁹ und R¹⁰ die Anspruch 1 angegebene Bedeutung haben; und nachfolgend in einem zweiten Reaktionsschritt die auf diese Weise erhaltenen Derivate der allgemeinen Formeln (Ig) und (Ih) 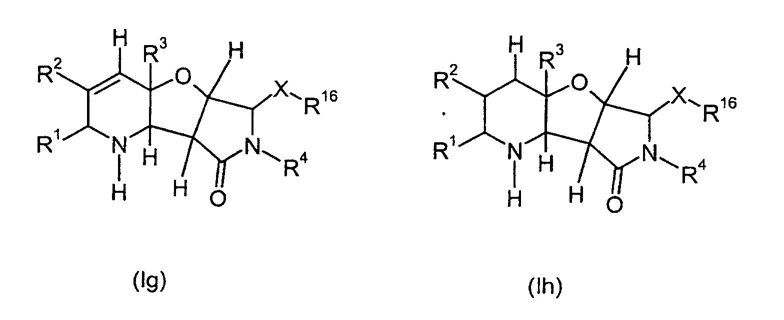 in welchen
R¹, R², R³, R⁴ und X die in Anspruch 1 angegebene Bedeutung besitzen und für
den Rest R¹⁶ die Gruppe 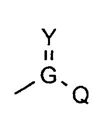 steht,
worin 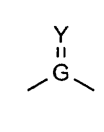 und Q die Anspruch 1 angegebene Bedeutung haben,
mit einem Acylierungsmittel der Formel (IV)
E - B (IV)
am Rest -NH- in Position 1 umsetzt, wobei E-B eine der in Anspruch 3e genannten Verbindungen der allgemeinen Formeln (V), (VT), (VII) oder (IX) bedeutet,
in welchen 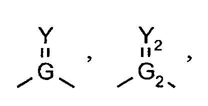 Q, Z, W, und R¹² die in Anspruch 1 und 2 angegebene Bedeutung haben, umsetzt;
die Umsetzung erfolgt dabei gegebenenfalls in Gegenwart eines Katalysators, gegebenenfalls in Gegenwart eines basischen Reaktionsmittels und gegebenenfalls in Gegenwart von Verdünnungsmitteln.

4. Verfahren zur Herstellung der 6-substituierten 1,2,4a,5a,8a,8b-Hexahydro-6H-pyrrolo[3',4':4,5]furo[3,2-b]pyrid-8(7H)-on und/oder 6-substituierte 1,2,-3,4,4a,5a,8a,8b-Oktahydro-6H-pyrrolo[3',4':4,5]furo[3,2-b]pyrid-8(7H)-on Derivate der allgemeinen Formeln (Ii) und (Ij) sowie deren Salze gemäß Anspruch 1 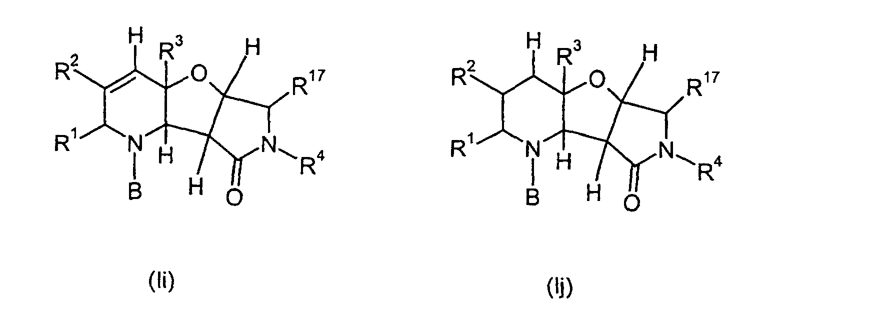 in welchen
R¹, R², R³, R⁴ und B die in Anspruch 1 angegebene Bedeutung besitzen,
R¹⁷ für C₁₋₆-Alkyl, C₂₋₆-Alkenyl, C₂₋₆-Alkinyl, Furyl, Cyan, C₁₋₄-Alkoxycarbonyl, Carbamoyl, Thiocarbamoyl, die gegebenenfalls durch Reste aus der Reihe Halogen, C₁₋₄-Alkyl, Halogen-C₁₋₄-alkyl, Amino, Hydroxy, Nitro, Cyan, C₁₋₄-Alkoxy, C₁₋₂-Alkylendioxy, Halogen-C₁₋₄-alkoxy, C₁₋₄-Alkylthio, Halogen-C₁₋₄-alkylthio, C₁₋₄-Alkylsulfonyl, C₁₋₄-Alkylamino, C₁₋₄-Dialkylamino, substituiert sind, oder gegebenenfalls für einen Rest aus der Gruppe A² steht, 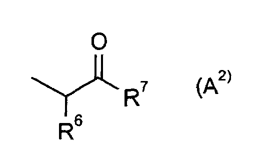 worin
R⁶ und R⁷ die weiter oben in Anspruch 1 angegebene Bedeutung besitzen,
sowie deren optische Isomere und Racemate,
**dadurch gekennzeichnet, dass** man
entweder die z.B. gemäß Anspruch 2 erhältlichen Derivate der allgemeinen Formeln (Ia) und (Ib) 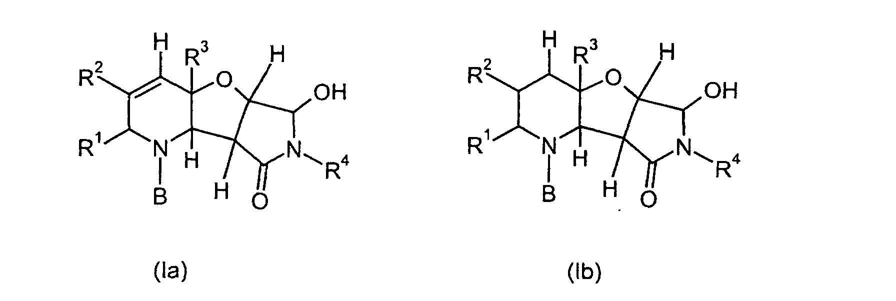 in welchen
R¹, R², R³, R⁴ und B die in Anspruch 1 angegebene Bedeutung besitzen, oder besonders bevorzugt
die z. B. gemäß Anspruch 3 erhältlichen Derivate der allgemeinen Formeln (Ie) und (If) 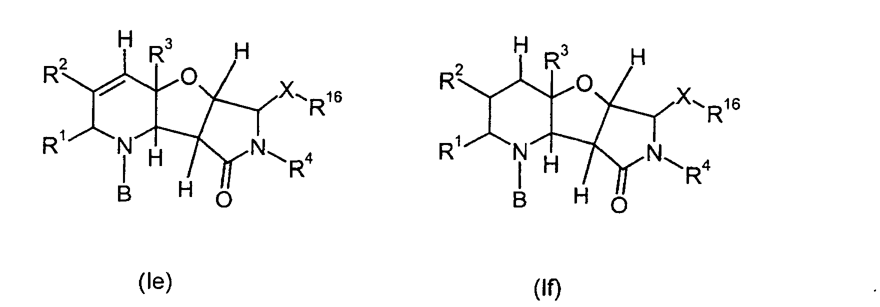 in welcher
R¹, R², R³, R⁴ und B die in Anspruch 3 angegebene Bedeutung besitzen, und
R¹⁶ für C₁₋₆-Alkyl, Aryl-C₁₋₂-alkyl, die gegebenenfalls substituiert sind, steht,
X für Sauerstoff, Schwefel oder Sulfonyl steht,
a) mit metallorganischen Verbindungen der allgemeinen Formel (XI)
(R¹⁸)₃M-R¹⁷ (XI)
in welcher
R¹⁸ für C₁₋₄-Alkyl, steht
M für ein Metallatom, insbesondere Silizium oder Zinn, steht
R¹⁷ für C₁₋₆-Alkyl, C₂₋₆-Alkenyl, C₂₋₆-Alkinyl, die gegebenenfalls durch Reste aus der Reihe Halogen, C₁₋₄-Alkyl, Halogen-C₁₋₄-alkyl, Amino, Hydroxy, Nitro, Cyan, C₁₋₄-Alkoxy, C₁₋₂-Alkylendioxy, Halogen-C₁₋₄-alkoxy, C₁₋₄-Alkylthio, Halogen-C₁₋₄-alkylthio, C₁₋₄-Alkylsulfonyl, C₁₋₄-Alkylamino, C₁₋₄-Dialkylamino, substituiert sind, oder für Cyan steht,
gegebenenfalls in Gegenwart von Verdünnungsmitteln und gegebenenfalls in Gegenwart eines Katalysators umsetzt, oder
b) mit Verbindungen der allgemeinen Formel (XII) 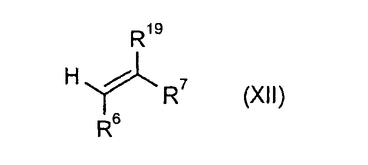 in welcher
R⁶ und R⁷ für die weiter oben angegebene Bedeutung haben,
R¹⁹ für Wasserstoff, -O-Acyl, -O-Sn-O-SO₂-CF₃, -O-B(CH₂-CH₃)₂, oder für die Reste
- O-M(R¹⁸)₃
und 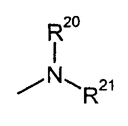 steht,
worin
M und R¹⁸ die angegebene Bedeutung besitzen, und
R²⁰ und R²¹ unabhängig voneinander für Wasserstoff, geradkettiges oder verzweigtes Alkyl, Alkenyl, Alkinyl, Cycloalkyl, Cycloalkylalkyl, Arylalkyl stehen, die gegebenenfalls substituiert sind, oder
R²⁰ und R²¹ gemeinsam mit dem angrenzenden N-Atom für ein carbocyclisches 5-, 6- oder 7-gliedriges Ringsystem, das gegebenenfalls auch durch Sauerstoff, Schwefel oder Stickstoff unterbrochen sein kann und gegebenenfalls substituiert ist, stehen,
gegebenenfalls in Gegenwart eines Katalysators und gegebenenfalls in Gegenwart von Verdünnungsmitteln umsetzt, oder
c) mit Aromaten oder Heteroaromaten der allgemeinen Formel (XIII)
H-R¹⁷ (XIII)
worin
R¹⁷ für den Furylrest steht, der gegebenenfalls substituiert ist durch Reste aus der Reihe Halogen, C₁₋₄-Alkyl, Halogen-C₁₋₄-alkyl, Amino, Hydroxy, Nitro, Cyan, C₁₋₄-Alkoxy, C₁₋₂-Alkylendioxy, Halogen-C₁₋₄-alkoxy, C₁₋₄-Alkylthio, Halogen-C₁₋₄-alkylthio, C₁₋₄-Alkylsulfonyl, C₁₋₄-Alkylamino, C₁₋₄-Dialkylamino, steht
gegebenenfalls in Gegenwart eines Katalysators und gegebenenfalls in Gegenwart von Verdünnungsmitteln umsetzt.

5. Endoparasitizide Mittel, **gekennzeichnet durch** einen Gehalt an mindestens einem 6-substituierten 1,2,4a,5a,8a,8b-Hexahydro- und 1,2,3,4,4a,5a,8a,8b-Oktahydro-6H-pyrrolo[3',4':4,5]furo[3,2-b]pyrid-8(7H)-on Derivat der Formel (I) gemäß Anspruch 1.

6. Verfahren zur Herstellung von endoparasitiziden Mitteln, **dadurch gekennzeichnet, dass** man 6-substituierte 1,2,4a,5a,8a,8b-Hexahydro- und 1,2,3,4,-4a,5a,8a,8b-Oktahydro-6H-pyrrolo[3',4':4,5]furo[3,2-b]pyrid-8(7H)-on Derivate der Formel (I) gemäß Anspruch 1 mit Streckmitteln und/oder oberflächenaktiven Mittel vermischt.

7. Verwendung von 6-substituierten 1,2,4a,5a,8a,8b-Hexahydro- und 1,2,3,4,-4a,5a,8a,8b-Oktahydro-6H-pyrrolo[3',4':4,5]furo[3,2-b]pyrid-8(7H)-on Derivaten der Formel (I) gemäß Anspruch 1 zur Herstellung von endoparasitiziden Mitteln.

8. Verwendung von 6-substituierten 1,2,4a,5a,8a,8b-Hexahydro- und 1,2,3,4,-4a,5a,8a,8b-Oktahydro-6H-pyrrolo[3',4':4,5]furo[3,2-b]pyrid-8(7H)-on Derivaten zur Herstellung von Mitteln zur Bekämpfung von Cestoden, Trematoden, Nematoden, Acantocephalen bei Mensch und Tier.

## Claims

1. 6-Substituted 1,2,4a,5a,8a,8b-hexahydro- and 1,2,3,4,4a,5a,8a,8b-octahydro-6H-pyrrolo[3',4':4,5]furo[3,2-b]pyrid-8(7H)-one derivatives of the general formula (I) and their salts 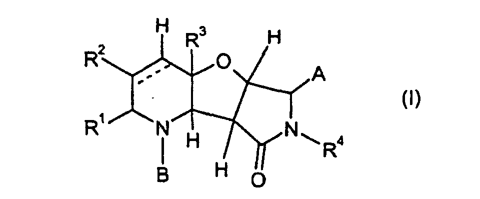 in which
R¹ represents hydrogen, straight-chain or branched C₁₋₄-alkyl or C₃₋₆-cycloalkyl,
R² represents hydrogen, straight-chain or branched C₁₋₄-alkyl, halo-C₁₋₄-alkyl, hydroxy-C₁₋₄-alkyl, C₁₋₄-alkanoyloxy-C₁₋₄-alkyl, C₁₋₂-alkoxyalkyl, C₁₋₂-alkylthioalkyl, C₁₋₂-alkylsulphinylalkyl, C₁₋₂-alkylsulphonylalkyl, amino-C₁₋₂-alkyl, C₁₋₆-alkylaminoalkyl, C₁₋₆-dialkylaminoalkyl, C₃₋₆-cycloalkylaminoalkyl, C₃₋₆-cycloalkyl, C₁₋₄-alkoxycarbonyl,
R³ represents, hydrogen, straight-chain or branched C₁₋₄-alkyl,
R⁴ represents straight-chain or branched C₁₋₆-alkyl, halo-C₁₋₆-alkyl, C₁₋₄-alkoxycarbonyl-C₁₋₄-alkyl, C₂₋₆-alkenyl, C₃₋₆-cycloalkyl, C₃₋₆-cycloalkyl-C₁₋₂-alkyl, aryl, aryl-C₁₋₂-alkyl, pyridyl, thiazolyl, N-morpholinyl, 2-chloropyrid-5-ylmethyl and chlorothiazol-5-ylmethyl which may optionally be substituted by radicals from the group consisting of halogen, halo-C₁₋₄-alkyl, amino, hydroxyl, nitro, cyano, C₁₋₄-alkoxy, C₁₋₂-alkylenedioxy, halo-C₁₋₄-alkoxy, C₁₋₄-alkylthio, halo-C₁₋₄-alkylthio, C₁₋₄-alkylsulphonyl, C₁₋₄-alkylamino, C₁₋₄-dialkylamino, C₁₋₄-alkylcarbonyl, C₁₋₄-alkoxycarbonyl,
A represents hydroxyl, C₁₋₆-alkyl, C₂₋₆-alkenyl, C₂₋₆-alkynyl, C₃₋₆-cycloalkyl, C₃₋₆-cycloalkyl-C₁₋₂-alkyl, C₁₋₆-alkoxy, C₂₋₆-alkenyloxy, C₂₋₆-alkynyloxy, C₁₋₄-alkoxycarbonyl, mercapto, C₁₋₆-alkylthio, cyano, carbamoyl, thiocarbamoyl, aryl-C₁₋₂-alkyloxy, furyl, which may optionally be substituted by radicals from the group consisting of halogen, C₁₋₄-alkyl, halo-C₁₋₄-alkyl, amino, hydroxyl, nitro, cyano, C₁₋₄-alkoxy, C₁₋₂-alkylenedioxy, halo-C₁₋₄-alkoxy, C₁₋₄-alkylthio, halo-C₁₋₄-alkylthio, C₁₋₄-alkylsulphonyl, C₁₋₄-alkylamino, C₁₋₄-dialkylamino, or optionally represents a radical from the group consisting of A¹, A² and A³ 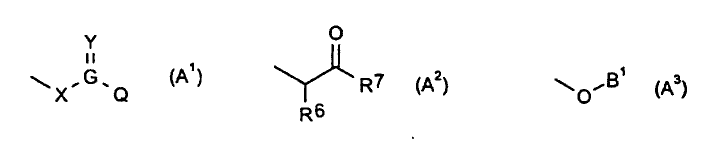 in which
X represents oxygen, 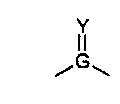 represents carboxyl or sulphonyl,
Q represents straight-chain or branched C₁₋₆-alkyl, C₂₋₆-alkenyl, C₂₋₆-alkynyl, C₃₋₆-cycloalkyl, C₃₋₆-cycloalkoxy, C₁₋₆-alkoxy, C₂₋₆-alkenyloxy, C₂₋₆-alkynyloxy, C₁₋₄-alkylamino, C₁₋₄-dialkylamino, a cyclic amino group attached via nitrogen, aryl, aryl-C₁₋₂-alkyl, aryl-C₁₋₂-alkyloxy, which are optionally substituted,
R⁶ represents hydrogen or methyl,
R⁷ represents straight-chain or branched C₁₋₆-alkyl, C₂₋₆-alkenyl, C₃₋₆-cycloalkyl, aryl, which are optionally substituted,
R⁶ and R⁷ together with the atoms to which they are attached represent a 5-, 6- or 7-membered carbocyclic ring which may optionally be substituted by C₁₋₄-alkyl,
B represents hydrogen, straight-chain or branched C₁₋₆-alkyl, C₁₋₄-alkanoyloxyalkyl, C₁₋₂-alkoxyalkyl, amino-C₁₋₆-alkyl, C₁₋₆-alkylamino-C₁₋₆-alkyl, C₁₋₆-dialkylamino-C₁₋₆-alkyl, C₂₋₆-alkenyl, C₂₋₆-alkynyl, carbamoyl-C₁₋₄-alkyl, carboxyl-C₁₋₄-alkyl, C₁₋₄-alkoxydicarbonyl, aryl, aryl-C₁₋₂-alkyl, pyridyl, pyrimidyl, pyrrolidyl, imidazolyl, thiazolyl, N-morpholinyl, pyridylmethyl and thiazolylmethyl which may optionally be substituted by radicals from the group consisting of halogen, C₁₋₄-alkyl, halo-C₁₋₄-alkyl, amino, hydroxyl, nitro, cyano, C₁₋₄-alkoxy, C₁₋₂-alkylenedioxy, halo-C₁₋₄-alkoxy, C₁₋₄-alkylthio, halo-C₁₋₄-alkylthio, C₁₋₄-alkylsulphonyl, C₁₋₄-alkylamino, C₁₋₄-dialkylamino, C₁₋₄-alkylcarbonyl, C₁₋₄-alkoxycarbonyl, or optionally represents a radical from the group consisting of B¹, B², B³ and B⁴ 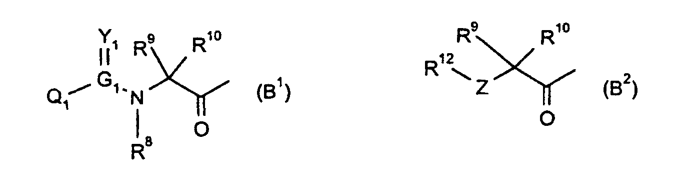 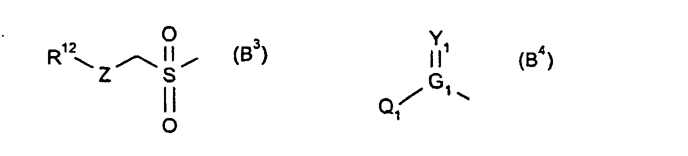 in which
R⁸ represents hydrogen, straight-chain or branched C₁₋₆-alkyl, C₃₋₆-cycloalkyl, or
R⁸ and R⁹ together with the atoms to which they are attached represent a 5- or 6-membered ring which may optionally be interrupted by sulphur and which is optionally substituted by hydroxyl, tert-butoxy, benzyloxy,
R⁹ represents hydrogen, straight-chain or branched C₁₋₆-alkyl,
R¹⁰ represents hydrogen or methyl, 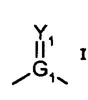
represents carbonyl, -C=CH-NO₂, -C=CH-CN, -C=N-R¹¹, sulphonyl,
R¹¹ represents halo-C₁₋₄-alkylcarbonyl, C₁₋₄-alkylsulphonyl, nitro or cyano, and
Q¹ represents a radical from the group consisting of G¹ and G² 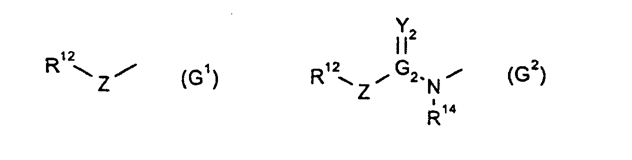
in which 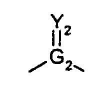 may represent carbonyl or sulphonyl,
Z represents oxygen or -NR¹³,
R¹², if Z represents nitrogen, represents a cyclic amino group which is attached via a nitrogen atom and which may optionally be substittued by radicals from the group consisting of halogen, C₁₋₄-alkyl, hydroxy-C₁₋₄-alkyl, amino-C₁₋₄-alkyl, C₁₋₄-alkylamino-C₁₋₄-alkyl, C₁₋₄-dialkylamino-C₁₋₄-alkyl, amino, hydroxyl, C₁₋₄-alkoxy, C₁₋₄-alkylcarbonyl, C₁₋₄-alkoxycarbonyl,
R¹² and R¹³ independently of one another represent hydrogen, straight-chain or branched C₁₋₆-alkyl, C₂₋₄-alkenyl, C₂₋₄-alkynyl, C₃₋₆-cycloalkyl, pyridylmethyl and thiazolylmethyl which may optionally be substituted by radicals consisting of halogen, C₁₋₄-alkyl, hydroxy-C₁₋₄-alkyl, amino-C₁₋₄-alkyl, C₁₋₄-alkylamino-C₁₋₄-alkyl, C₁₋₄-dialkylamino-C₁₋₄-alkyl, amino, hyroxyl, C₁₋₄-alkoxy, C₁₋₄-alkylcarbonyl, C₁₋₄-alkoxycarbonyl, or
R¹² and R¹³ together with the adjacent N atom represent a carbocyclic 5-, 6- or 7-membered ring system or represent a 7- to 10-membered bicyclic ring system which may optionally also be interrupted by oxygen, sulphur, sulphoxyl, sulphonyl, carbonyl, -N-O, -N=, -NR¹⁵- or by quarternized nitrogen and which is optionally substituted by C₁₋₄-alkyl, hydroxy-C₁₋₄-alkyl, amino-C₁₋₄-akyl, C₁₋₄-alkylamino-C₁₋₄-alkyl, C₁₋₄-dialkylamino-C₁₋₄-alkyl, amino, hydroxyl, C₁₋₄-alkoxy, C₁₋₄-alkylcarbonyl, C₁₋₄-alkoxycarbonyl, halogen,
R¹⁴ represents hydrogen or C₁₋₄-alkyl,
R¹⁵ represents hydrogen, straight-chain or branched C₁₋₆-alkyl, C₂₋₆-alkenyl, C₂₋₆-alkynyl, C₃₋₆-cycloalkyl, C₁₋₄-alkoxycarbonyl, C₁₋₄-alkylcarbonyl, C₃₋₆-cycloalkylcarbonyl, cyano, aryl, aryl-C₁₋₂-alkyl, pyridyl and thiazolyl, pyridylmethyl, thiazolylmethyl, which may optionally be substituted by radicals from the group consisting of halogen, C₁₋₄-alkyl, halo-C₁₋₄-alkyl, amino, hydroxyl, cyano, C₁₋₄-alkoxy, C₁₋₂-alkylenedioxy, halo-C₁₋₄-alkoxy, C₁₋₄-alkylthio, halo-C₁₋₄-alkylthio, C₁₋₄-alkylsulphonyl, C₁₋₄-alkylamino, C₁₋₄-dialkylamino, C₁₋₄-alkylcarbonyl, C₁₋₄-alkoxycarbonyl,
and their optical isomers and racemates,
where "aryl" in the definitions represents a mono-, di- or polyvalent aromatic radical selected from the group consisting of phenyl, naphthyl, tetrahydronaphthyl, indanyl and fluorenyl
and where, unless indicated otherwise, the optionally substituted radicals may carry one or more identical or different substituents selected from: alkyl having 1 to 4 carbon atoms, alkoxy having 1 to 4 carbon atoms, alkylthio, haloalkyl having 1 to 5 halogen atoms, where the halogen atoms are identical or different, halogen, cyano, nitro, amino, monoakyl- and dialkylamino having 1 to 4 carbon atoms per alkyl group, alkylcarbonyl radicals, alkoxycarbonyl having 2 to 4 carbon atoms, alkylsulphinyl having 1 to 4 carbon atoms, haloalkylsulphinyl having 1 to 4 carbon atoms and 1 to 5 halogen atoms, haloalkylsulphonyl having 1 to 4 carbon atoms and 1 to 5 halogen atoms, arylsulphonyl having 6 or 10 aryl carbon atoms, trialkylsilyl having 1 to 4 carbon atoms, acyl, aryl, aryloxy, which for their part may carry one of the abovementioned substituents, and also the form imino radical (-HC=N-O-alkyl).

2. Process for preparing the 6-hydroxy-1,2,4a,5a,8a,8b-hexahydro-6H-pyrrolo[3',4':4,5]furo[3,2-b]pyrid-8(7H)-one and/or 6-hydroxy-1,2,3,4,4a,-5a,8a,8b-octahydro-6H-pyrrolo[3',4':4,5]furo[3,2-b]pyrid-8(7H)-one derivatives of the general formulae (Ia) and (Ib) and their salts according to Claim 1 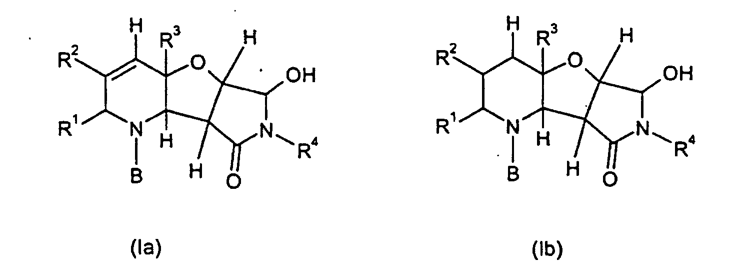 in which
R¹, R², R³, R⁴ and B are each as defined in Claim 1, **characterized in that** the 1,2,4a,5a,8a,8b-hexahydro-6H-pyrrolo[3',4':4,5]furo[3,2-b]pyridine-6,8-(7H)-dione
and/or 1,2,3,4,4a,5a,8a,8b-octahydro-6H-pyrrolo[3',4':4,5]furo[3,2-b]-pyridine-6,8(7H)-dione derivatives of the general formulae (IIa) and (IIb) and their salts 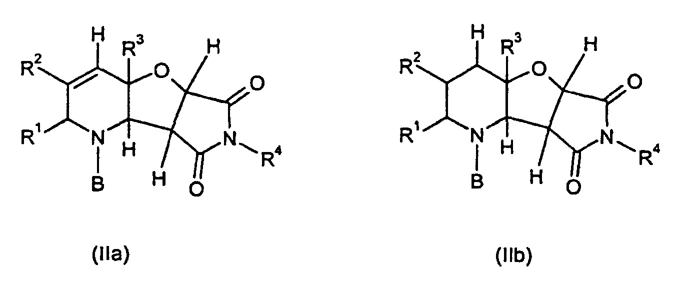 in which
R¹, R², R³, R⁴ and B are each as defined in Claim 1 are hydrogenated in the presence of a suitable diluent,
or that, in a first reaction step,
the 1,2,4a,5a,8a,8b-hexahydro-6H-pyrrolo[3',4':4,5]furo[3,2-b]pyridine-6,8-(7H)-dione and/or 1,2,3,4,4a,5a,8a,8b-octahydro-6H-pyrrolo[3',4':4,5]furo[3,2-b]pyridine-6,8(7H)-dione derivatives of the general formulae (IIc) and (IId) and their salts 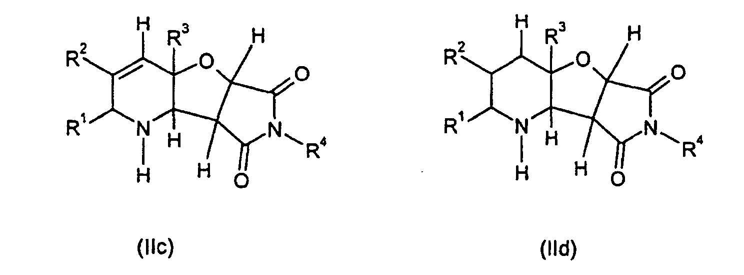 in which
R¹, R², R³ and R⁴ are each as defined in Claim 1
are hydrogenated in the presence of a suitable diluent to give 6-hydroxy-1,2,4a,5a,8a,8b-hexahydro-6H-pyrrolo[3',4':4,5]furo[3,2-b]pyrid-8(7H)-one and/or 6-hydroxy-1,2,3,4,4a,5a,8a,8b-octahydro-6H-pyrrolo[3',4':4,5]furo[3,2-b]pyrid-8(7H)-one derivatives of the general formulae (Ic) and (Id) 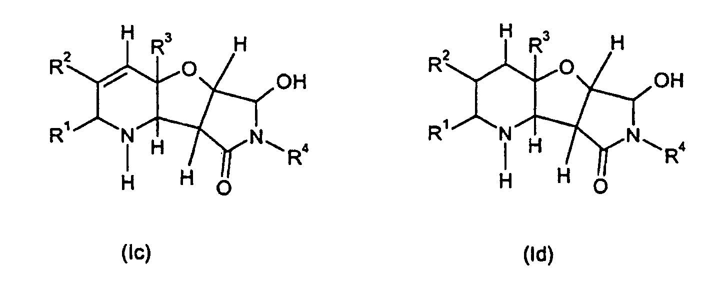 in which
R¹, R², R³ and R⁴ are each as defined in Claim 1,
or that, for the selective preparation of the novel derivatives of the general formulae (IIa) and their salts, in a first reaction step
the 4a,5a,8a,8b-tetrahydro-6H-pyrrolo[3',4':4,5]furo[3,2-b]pyridine-6,8(7H)-dione derivatives of the general formula (III) and their salts 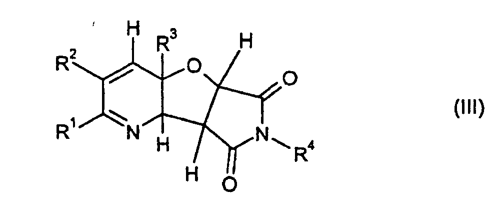 in which
R¹, R², R³ and R⁴ are each as defined in Claim 1
are hydrogenated in the presence of a suitable diluent to give 6-hydroxy-1,2,4a,5a,8a,8b-hexahydro-6H-pyrrolo[3',4':4,5]furo[3,2-b]pyrid-8(7H)-one derivatives of the general formula (Ic) 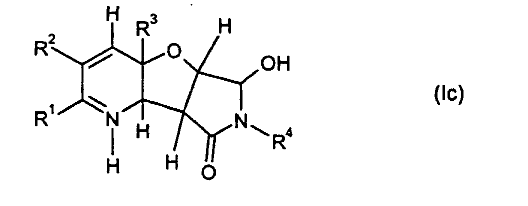 in which
R¹, R², R³ and R⁴ are each as defined in Claim 1,
and, subsequently in a second reaction step, the derivatives of the general formulae (Ic) and (Id) 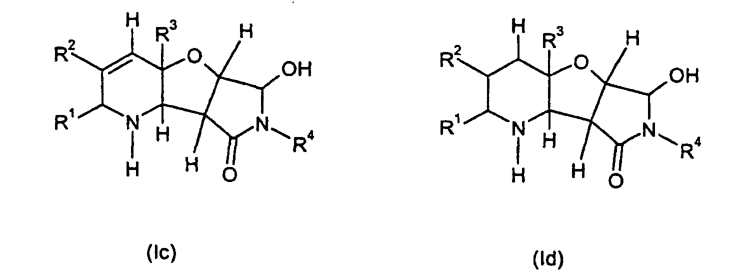 obtained in this manner
in which
R¹, R², R³ and R⁴ are each as defined in Claim 1
a) are reacted with compounds of the general formula (IV)
B - E (IV)
in which
B is as defined above, and
E represents an electron-withdrawing leaving group,
if appropriate in the presence of diluents and if appropriate in the presence of a basic reaction auxiliary, or
b) are reacted with compounds of the general formula (V) 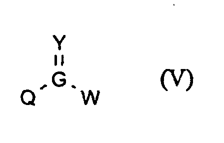 in which 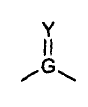 and Q are each as defined in Claim 1, and
W represents a suitable leaving group, such as, for example, halogen, alkoxy, alkylthio or aryloxy,
if appropriate in the presence of a catalyst, if appropriate in the presence of a basic reaction auxiliary and if appropriate in the presence of diluents, or that, to prepare the novel 6-hydroxy-1,2,4a,5a,8a,8b-hexahydro-6H-pyrrolo[3',4':4,5]furo[3,2-b]pyrid(7H)-one and/or 6-hydroxy-1,2,3,4,4a,5a,8a,8b-octahydro-6H-pyrrolo[3',4':4,5]furo[3,2-b]pyrid-(7H)-one derivatives of the general formulae (Ia) and (Ib) and their salts
in which the group 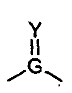 represents carbonyl, the compounds of the formula Ic or Id
c) are reacted with a carboxylic anhydride of the general formula (VI)
(Q-C=O)₂O (VI)
in which
Q is as defined under Claim 1,
if appropriate in the presence of a catalyst, if appropriate in the presence of diluents, or that the compounds of the formula (Ic) or (Id)
d) are reacted with amino acid derivatives of the general formula (VII) 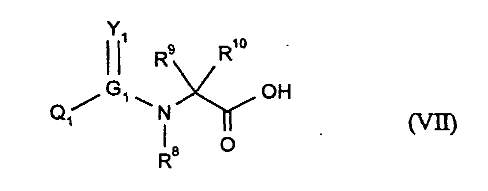 in which 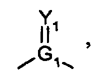 Q¹, R⁸, R⁹ and R¹⁰ are as defined in Claim 1,
if appropriate in the presence of coupling agents and if appropriate in the presence of a basic reaction auxiliary, if appropriate in the presence of diluents, or that the compounds of the formula (Ic) or (Id)
e) are reacted with compounds of the general formulae (VIII) or (IX)
R¹²―N=C=Y (VIII) 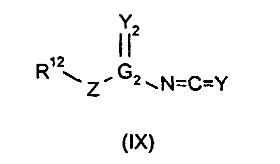
in which 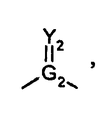 Z and R¹² are each as defined in Claim 1,
Y represents oxygen or sulphur,
if appropriate in the presence of a catalyst, if appropriate in the presence of diluents.

3. Process for preparing 6-substituted 1,2,4a,5a,8a,8b-hexahydro-6H-pyrrolo[3',4':4,5]furo[3,2-b]pyrid-8(7H)-one and/or 6-substituted 1,2,3,4,-4a,5a,8a,8b-octahydro-6H-pyrrolo[3',4':4,5]furo[3,2-b]pyrid-8(7H)-one derivatives of the general formulae (Ie) and (If) and their salts according to Claim 1 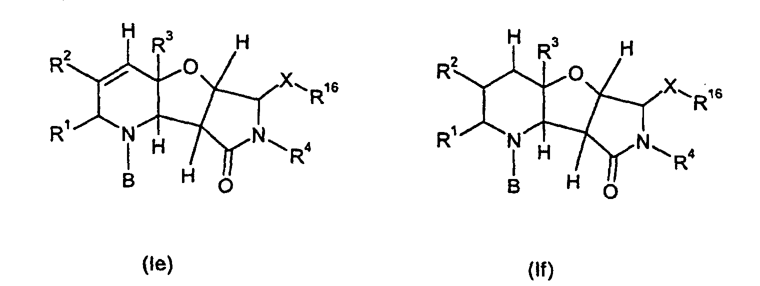 in which
R¹, R², R³, R⁴, X and B are each as defined in Claim 1,
R¹⁶ represents C₁₋₆-alkyl, C₂₋₆-alkenyl, C₂₋₆-alkynyl, aryl-C₁₋₂-alkyl, which are optionally substituted by radicals from the group consisting of halogen, C₁₋₄-alkyl, halo-C₁₋₄-alkyl, amino, hydroxyl, nitro, cyano, C₁₋₄-alkoxy, C₁₋₂-alkylenedioxy, halo-C₁₋₄-alkoxy, C₁₋₄-alkylthio, halo-C₁₋₄-akylthio, C₁₋₄-alkylsulphonyl, C₁₋₄-alkylamino, C₁₋₄-dialkylamino, or
optionally represents the group 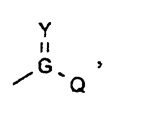 in which
G, Y and Q are each as defined in Claim 1,
and their optical isomers and racemates,
**characterized in that**
a) the derivatives of the general formulae (Ia) and (Ib) obtainable according to Claim 2 and their salts 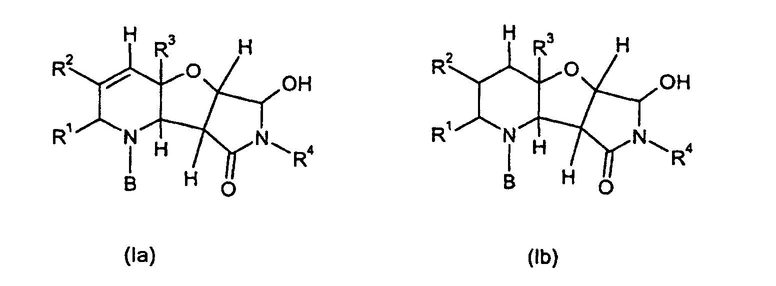 in which
R¹, R², R³, R⁴ and B are each as defined in Claim 1 are reacted with compounds of the general formula (X)
H-X-R¹⁶ (X)
in which
R¹⁶ and X are each as defined above,
if appropriate in the presence of an acid, if appropriate in the presence of diluents, or that
b) to prepare the 6-substituted 1,2,4a,5a,8a,8b-hexahydro-6H-pyrrolo[3',4':4,5]furo[3,2-b]pyrid-8(7H)-one and/or 6-substituted 1,2,3,4,4a,5a,8a,8b-octahydro-6H-pyrrolo[3',4':4,5]furo[3,2-b]pyrid-8(7H)-one derivatives of the general formulae (Ie) and (If) and their salts,
in which the radical R¹⁶ represents the group 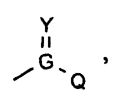 in which 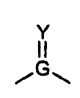 and Q are each as defined in Claim 1,
they are reacted with the compounds of the general formula (V) 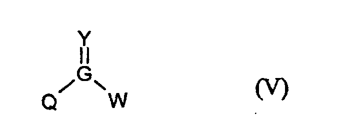 in which 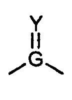 and Q are each as defined in Claim 1, and
W is a suitable leaving group,
if appropriate in the presence of a catalyst, if appropriate in the presence of a basic reaction auxiliary and if appropriate in the presence of diluents, or that
c) to prepare the derivatives of the general formulae (Ie) and (If) and their salts,
in which the group 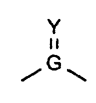 represents carbonyl,
they are reacted with a carboxylic anhydride of the general formula (VI)
(Q-C=O)₂O (VI)
in which
Q is as defined in Claim 1, if appropriate in the presence of a catalyst, if appropriate in the presence of diluents, or that
d) they are reacted with compounds of the general formulae (VIII) or (IX)
R¹²―N=C=Y (VIII) 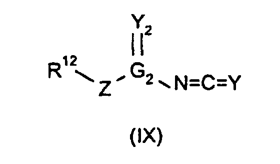
in which 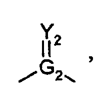 Z, Y and R¹² are each as defined under Claim 1, if appropriate in the presence of a catalyst, if appropriate in the presence of diluents, or that
e) to prepare the 6-substituted 1,2,4a,5a,8a,8b-hexahydro-6H-pyrrolo[3',4':4,5]furo[3,2-b]pyrid-8(7H)-one and/or 6-substituted 1,2,3,4,4a,5a,8a,8b-octahydro-6H-pyrrolo[3',4':4,5]furo[3,2-b]pyrid-8(7H)-one derivatives of the general formulae (Ie) and (If) and their salts 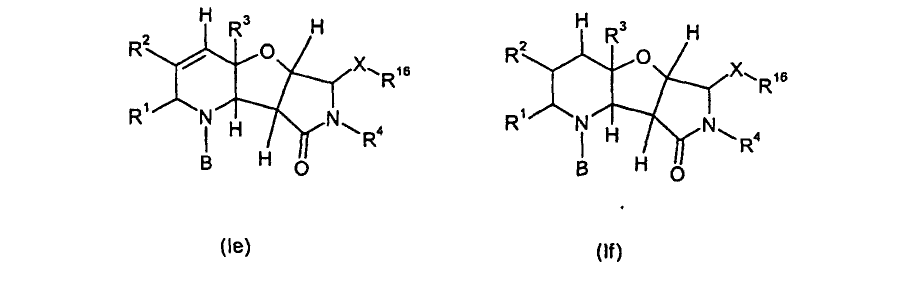 in which
R¹, R², R³, R⁴, X and B are as defined in Claim 1 and the radicals B and R¹⁶
represent the same group 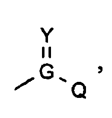 in which 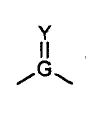 and Q are each as defined in Claim 1,
derivatives of the general formulae (Ic) and (Id) 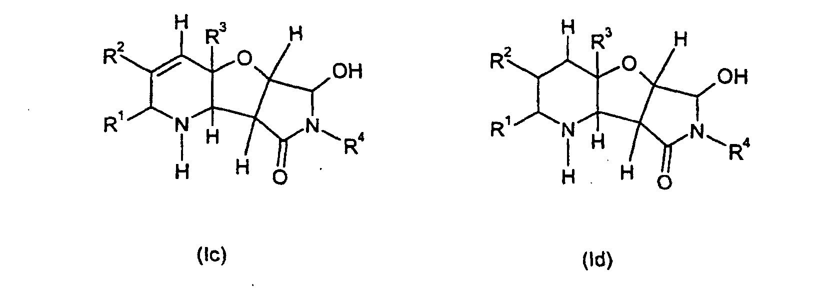 in which
R¹, R², R³, R⁴ are each as defined in Claim 1
are reacted with acylating agents of the formula (IV)
E-B (IV)
and/or
E-R¹⁶
either on the radical -NH- in position 1 or on the radical -OH in position 6 or on both radicals, where E-B and/or E-R¹⁶ are one of the compounds of the general formulae (V), (VI), (VIII) or (IX) below 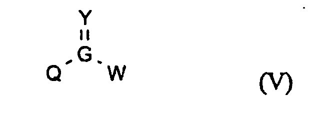
(Q-C=O)₂O (VI)
R¹²-N=C=Y (VIII) 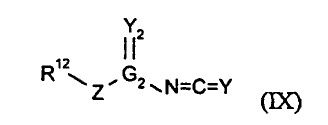
in which 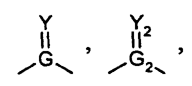 Q, Z, W and R¹² are each as defined in Claim 1;
the reaction being carried out, if appropriate, in the presence of a catalyst, if appropriate in the presence of a basic reaction auxiliary and if appropriate in the presence of diluents,
f) to prepare the 6-substituted 1,2,4a,5a,8a,8b-hexahydro-6H-pyrrolo[3',4':4,5]furo[3,2-b]pyrid-8(7H)-one and/or 6-substituted 1,2,3,4,4a,5a,8a,8b-octahydro-6H-pyrrolo[3',4':4,5]furo[3,2-b]pyrid-8(7H)-one derivatives of the general formulae (Ie) and (If) and their salts, 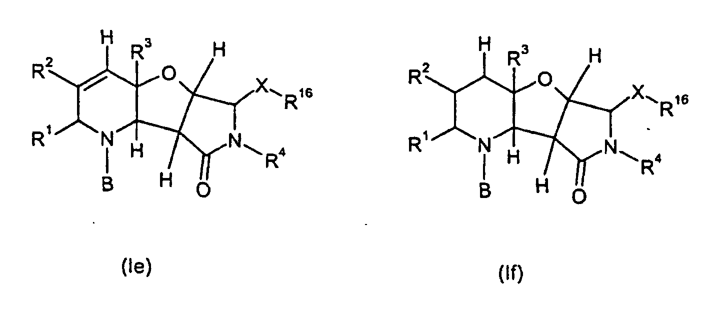 in which
R¹, R², R³, R⁴, X and B are each as defined in Claim 1 and the radical R¹⁶ represents
the group 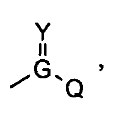 in which 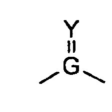 and Q are each as defined in Claim 1,
derivatives of the general formulae (Ic) and (Id) 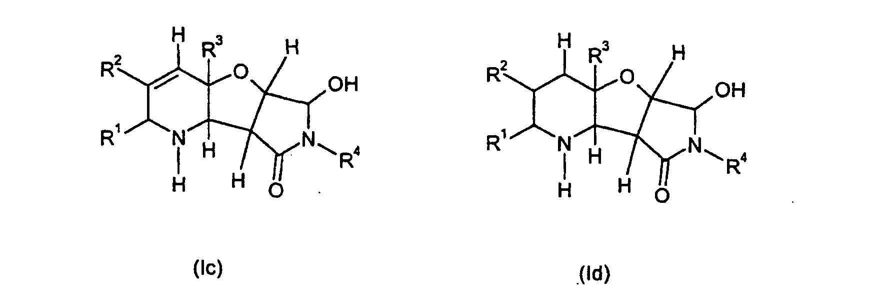 in which
R¹, R², R³, R⁴ are each as defined in Claim 1,
are reacted with an acylating agent of the formula (IV)
E-R¹⁶ (IV)
on the radical -OH in position 6, where E - R¹⁶ represents the compound of the general formula (VII) 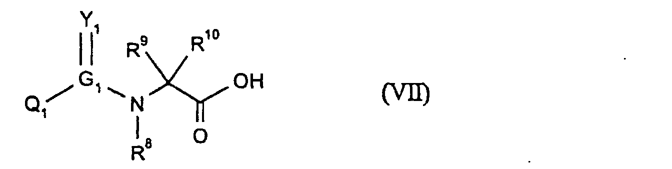 in which 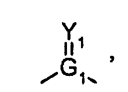 Q¹, R⁸, R⁹ and R¹⁰ are each as defined in Claim 1; and subsequently, in a second reaction step, the derivatives of the general formulae (Ig) and (Ih) 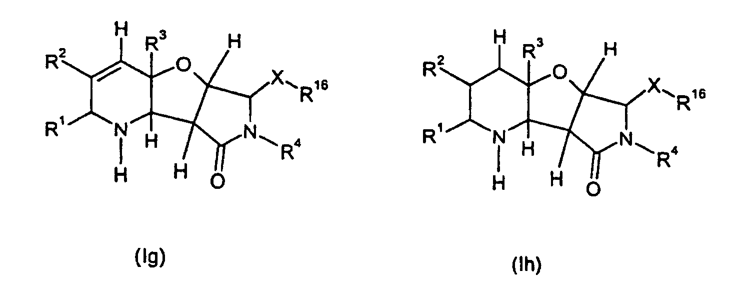 obtained in this manner
in which
R¹, R², R³, R⁴ and X are each as defined in Claim 1 and
the radical R¹⁶ represents the group 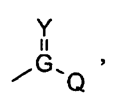 in which 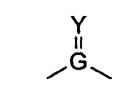 and Q are each as defined in Claim 1,
are reacted with an acylating agent of the formula (IV)
E - B (IV)
on the radical -NH- in position 1, where E-B is one of the compounds of the general formulae (V), (VI), (VII) or (IX) mentioned in Claim 1
in which 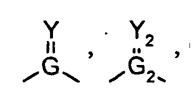 Q, Z, W, and R¹² are each as defined in Claims 1 and 2;
the reaction being carried out, if appropriate, in the presence of a catalyst, if appropriate in the presence of a basic reaction auxiliary and if appropriate in the presence of diluents.

4. Process for preparing the 6-substituted 1,2,4a,5a,8a,8b-hexahydro-6H-pyrrolo[3',4':4,5]furo[3,2-b]pyrid-8(7H)-one and/or 6-substituted 1,2,3,4,4a,5a,8a,8b-octahydro-6H-pyrrolo[3',4':4,5]furo[3,2-b]pyrid-8(7H)-one derivatives of the general formulae (Ii) and (Ij) and their salts according to Claim 1 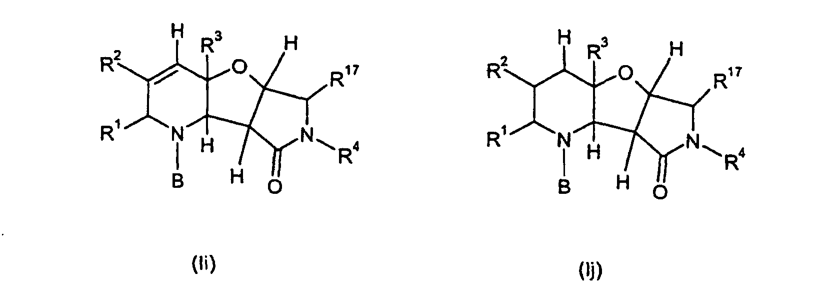 in which
R¹, R², R³, R⁴ and B are each as defined in Claim 1,
R¹⁷ represents C₁₋₆-alkyl, C₂₋₆-alkenyl, C₂₋₆-alkynyl, furyl, cyano, C₁₋₄-alkoxycarbonyl, carbamoyl, thiocarbamoyl, which are optionally substituted by radicals from the group consisting of halogen, C₁₋₄-alkyl, halo-C₁₋₄-alkyl, amino, hydroxyl, nitro, cyano, C₁₋₄-alkoxy, C₁₋₂-alkylenedioxy, halo-C₁₋₄-alkoxy, C₁₋₄-alkylthio, halo-C₁₋₄-alkylthio, C₁₋₄-alkylsulphonyl, C₁₋₄-alkylamino, C₁₋₄-dialkylamino, or optionally represents a radical from the group A² 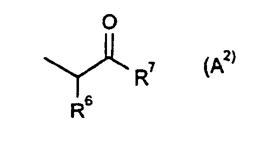 in which
R⁶ and R⁷ are each as defined above in Claim 1,
and their optical isomers and racemates,
**characterized in that**
either the derivatives of the general formulae (Ia) and (Ib) 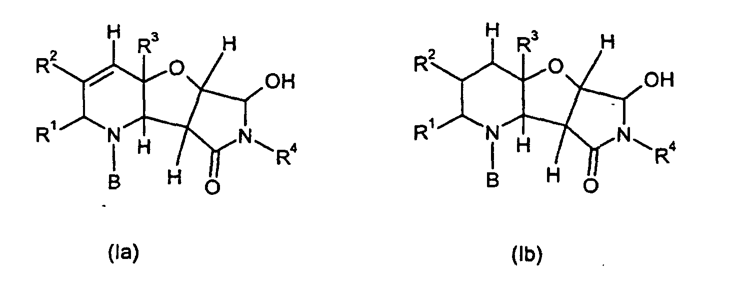 which are obtainable, for example, according to Claim 2
in which
R¹, R², R³, R⁴ and B are each as defined in Claim 1, or particularly preferably
the derivatives of the general formulae (Ie) and (If) 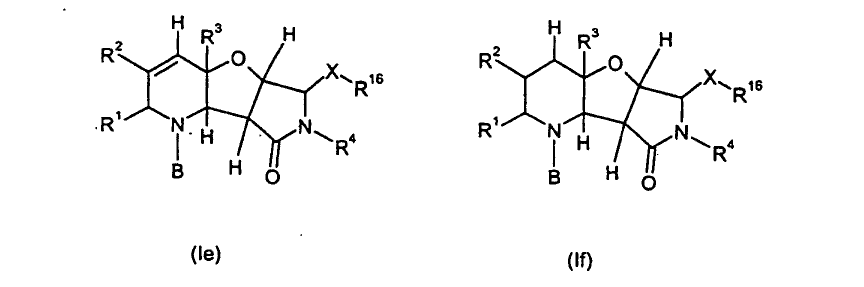 which are obtainable, for example, according to Claim 3
in which
R¹, R², R³, R⁴ and B are each as defined in Claim 3 and
R¹⁶ represents C₁₋₆-alkyl, aryl-C₁₋₂-alkyl, which are optionally substituted,
X represents oxygen, sulphur or sulphonyl,
a) are reacted with organometallic compounds of the general formula (XI)
(R¹⁸)₃M-R¹⁷ (XI)
in which
R¹⁸ represents C₁₋₄-alkyl,
M represents a metal atom, in particular silicon or tin,
R¹⁷ represents C₁₋₆-alkyl, C₂₋₆-alkenyl, C₂₋₆-alkynyl, which are optionally substituted by radicals from the group consisting of halogen, C₁₋₄-alkyl, halo-C₁₋₄-alkyl, amino, hydroxyl, nitro, cyano, C₁₋₄-alkoxy, C₁₋₂-alkylenedioxy, halo-C₁₋₄-alkoxy, C₁₋₄-alkylthio, halo-C₁₋₄-alkylthio, C₁₋₄-alkylsulphonyl, C₁₋₄-alkylamino, C₁₋₄-dialkylamino, or represents cyano,
if appropriate in the presence of diluents and if appropriate in the presence of a catalyst, or
b) are reacted with compounds of the general formula (XII) 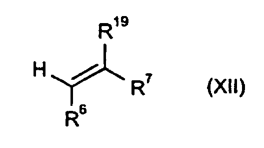 in which
R⁶ and R⁷ are each as defined above,
R¹⁹ represents hydrogen, -O-acyl, -O-Sn-O-SO₂-CF₃, -O-B(CH₂-CH₃)₂, or represents the radicals
-O-M(R¹⁸)₃
and 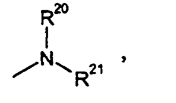
in which
M and R¹⁸ are each as defined above, and
R²⁰ and R²¹ independently of one another each represent hydrogen, straight-chain or branched alkyl, alkenyl, alkinyl, cycloalkyl, cycloalkylalkyl, arylalkyl, which are optionally substituted, or
R²⁰ and R²¹ together with the adjacent N atom represent a heterocyclic 5-, 6- or 7-membered ring system, which may optionally also be interrupted by oxygen, sulphur or nitrogen and which is optionally substituted,
if appropriate in the presence of a catalyst and if appropriate in the presence of diluents, or
c) are reacted with aromatics or heteroaromatics of the general formula (XIII)
H-R¹⁷ (XIII)
in which
R¹⁷ represents the furyl radical which is optionally substituted by radicals from the group consisting of halogen, C₁₋₄-alkyl, halo-C₁₋₄-alkyl, amino, hydroxyl, nitro, cyano, C₁₋₄-alkoxy, C₁₋₂-alkylenedioxy, halo-C₁₋₄-alkoxy, C₁₋₄-alkylthio, halo-C₁₋₄-alkylthio, C₁₋₄-alkylsulphonyl, C₁₋₄-alkylamino, C₁₋₄-dialkylamino,
if appropriate in the presence of a catalyst and if appropriate in the presence of diluents.

5. Endoparasiticidal compositions, **characterized in that** they comprise at least one 6-substituted 1,2,4a,5a,8a,8b-hexahydro- or 1,2,3,4,4a,5a,8a,8b-octahydro-6H-pyrrolo[3',4':4,5]furo[3,2-b]pyrid-8(7H)-one derivative of the formula (I) according to Claim 1.

6. Process for preparing endoparasiticidal compositions, **characterized in that** 6-substituted 1,2,4a,5a,8a,8b-hexahydro- and/or 1,2,3,4,4a,5a,8a,8b-octahydro-6H-pyrrolo[3',4':4,5]furo[3,2-b]pyrid-8(7H)-one derivatives of the formula (I) according to Claim 1 are mixed with extenders and/or surfactants.

7. Use of 6-substituted 1,2,4a,5a,8a,8b-hexahydro- and 1,2,3,4,4a,5a,8a,8b-octahydro-6H-pyrrolo[3',4':4,5]furo[3,2-b]pyrid-8(7H)-one derivatives of the formula (I) according to Claim 1 for preparing endoparasiticidal compositions.

8. Use of 6-substituted 1,2,4a,5a,8a,8b-hexahydro- and 1,2,3,4,4a,5a,8a,8b-octahydro-6H-pyrrolo[3',4':4,5]furo[3,2-b]pyrid-8(7H)-one derivatives for preparing compositions for controlling cestodes, trematodes, nematodes, acantocephalae in humans and animals.

## Revendications

1. Dérivés de 1,2,4a,5a,8a,8b-hexahydro- et de 1,2,3,4,4a,5a,8a,8b-octahydro-6H-pyrrolo[3',4':4,5]furo[3,2-b]pyrid-8(7H)-one substitués en 6, de la formule générale (I) et leurs sels : 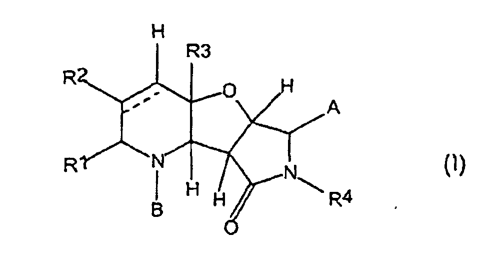 dans laquelle :
R¹ représente l'atome d'hydrogène, un radical alcoyle en C₁₋₄ linéaire ou ramifié ou cycloalcoyle en C₃₋₆ ;
R² représente l'atome d'hydrogène, un radical alcoyle en C₁₋₄ linéaire ou ramifié, halogénoalcoyle en C₁₋₄, hydroxyalcoyle en C₁₋₄, (alcanoyloxy en C₁₋₄) alcoyle en C₁₋₄, (alcoxy en C₁₋₂)alcoyle, (alcoyl en C₁₋₂)-thioalcoyle, (alcoylsulfinyl en C₁₋₂)alcoyle, (alcoylsulfonyl en C₁₋₂)alcoyle, aminoalcoyle en C₁₋₂, (alcoylamino en C₁₋₆)alcoyle, (dialcoylamino en C₁₋₆-alcoyle, (cycloalcoylamino en C₃₋₆)alcoyle, cycloalcoyle en C₃₋₆, (alcoxy en C₁₋₄)carbonyle;
R³ représente l'atome d'hydrogène, un radical alcoyle en C₁₋₄ linéaire ou ramifié ;
R⁶ représente un radical alcoyle en C₁₋₆ linéaire ou ramifié, halogénoalcoyle en C₁₋₆, (alcoxy en C₁₋₄)carbonylalcoyle en C₁₋₄, alcényle en C₂₋₆, cycloalcoyle en C₃₋₆, (cycloalcoyl en C₃₋₆)alcoyle en C₁₋₂, aryle, (aryl)alcoyle en C₁₋₂, pyridyle, thiazolyle, N-morpholinyle, 2-chloropyrid-5-ylméthyle et 2-chlorothiazol-5-ylméthyle, qui peuvent être le cas échéant, substitués par des restes de la série des atomes d'halogène, des radicaux halogénoalcoyle en C₁₋₄, amino, hydroxyle, nitro, cyano, alcoxy en C₁₋₄, alcoylènedioxy en C₁₋₂, halogénoalcoxy en C₁₋₄, alcoylthio en C₁₋₄, halogénoalcoylthio en C₁₋₄, alcoylsulfonyle en C₁₋₄, alcoylamino en C₁₋₄, di (alcoyl en C₁₋ ₄)amino, (alcoyl en C₁₋₄)carbonyle, (alcoxy en C₁₋ ₄)carbonyle ;
A représente un radical hydroxyle, alcoyle en C₁₋₆, alcényle en C₂₋₆, alcynyle en C₂₋₆, cycloalcoyle en C₃₋₆, (cycloalcoyl en C₃₋₆)alcoyle en C₁₋₂, alcoxy en C₁₋₆, alcényloxy en C₂₋₆, alcynyloxy en C₂₋₆, (alcoxy en C₁₋₄)carbonyle, mercapto, alcoylthio en C₁₋₆, cyano, carbamoyle, thiocarbamoyle, aryl(alcoyloxy en C₁₋₂), furyle, qui peuvent être le cas échéant, substitués par des restes de la série des atomes d'halogène, des radicaux alcoyle en C₁₋₄, halogénoalcoyle en C₁₋₄, amino, hydroxyle, nitro, cyano, alcoxy en C₁₋₄, alcoylènedioxy en C₁₋₂, halogénoalcoxy en C₁₋₄, alcoylthio en C₁₋₄, halogénoalcoylthio en C₁₋₄, alcoylsulfonyle en C₁₋₄, alcoylamino en C₁₋₄, di (alcoyl en C₁₋₄)amino, ou représente le cas échéant, un reste du groupe A¹, A² et A³ : 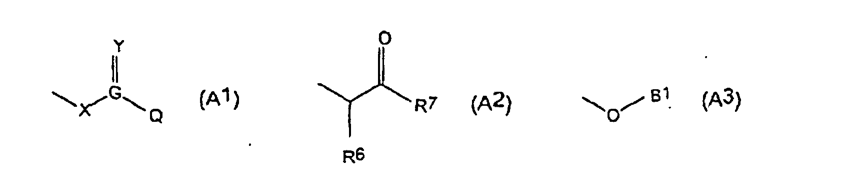
où
X représente l'atome d'oxygène ; 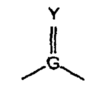 représente le radical carboxy ou sulfonyle ;
Q représente un radical alcoyle en C₁₋₆ linéaire ou ramifié, alcényle en C₂₋₆, alcynyle en C₂₋₆, cycloalcoyle en C₃₋₆, cycloalcoxy en C₃₋₆, alcoxy en C₁₋₆, alcényloxy en C₂₋₆, alcynyloxy en C₂₋₆, (alcoyl en C₁₋₄)-amino, di(alcoyl en C₁₋₄)amino, un radical aminocyclique relié par l'atome d'azote, un radical aryle, aryl(alcoyle en C₁₋₂), aryl (alcoxy en C₁₋₂), qui sont le cas échéant substitués ;
R⁶ représente l'atome d'hydrogène ou le radical méthyle ;
R⁷ représente un radical alcoyle en C₁₋₆ linéaire ou ramifié, alcényle en C₂₋₆, cycloalcoyle en C₃₋₆, aryle, qui sont le cas échéant substitués,
R⁶ et R⁷ représentent ensemble, avec les atomes d'azote sur lesquels ils sont liés, un carbocycle à 5, 6 ou 7 membres, qui peut être le cas échéant, substitué ;
B représente l'atome d'hydrogène, un radical alcoyle en C₁₋₆ linéaire ou ramifié, (alcanoyloxy en C₁₋₄)alcoyle, (alcoxy en C₁₋₂)alcoyle, aminoalcoyle en C₁₋₆, (alcoyl en C₁₋₆)aminoalcoyle en C₁₋₆, di(alcoyl en C₁₋₆)aminoalcoyle en C₁₋₆, alcényle en C₂₋₆, alcynyle en C₂₋₆, carbamoylalcoyle en C₁₋₄, carboxyalcoyle en C₁₋₄, (alcoxy en C₁₋₄)dicarbonyle, aryle, aryl (alcoyle en C₁₋₂), pyridyle, pyrimidyle, pyrrolidyle, imidazolyle, thiazolyle, N-morpholinyle, pyridylméthyle et thiazolylméthyle, qui peuvent être le cas échéant, substitués par des restes de la série des atomes d'halogène, des radicaux alcoyle en C₁₋₄, halogénoalcoyle en C₁₋₄, amino, hydroxyle, nitro, cyano, alcoxy en C₁₋₄, alcoylènedioxy en C₁₋₂, halogénoalcoxy en C₁₋₄, alcoylthio en C₁₋₄, halogénoalcoylthio en C₁₋₄, alcoylsulfonyle en C₁₋₄, alcoylamino en C₁₋₄, di (alcoyl en C₁₋₄)amino, (alcoyl en C₁₋₄)carbonyle, (alcoxy en C₁₋₄)carbonyle, ou représente le cas échéant, un reste du groupe B¹, B², B³ et B⁴ : 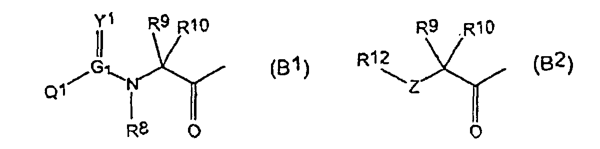 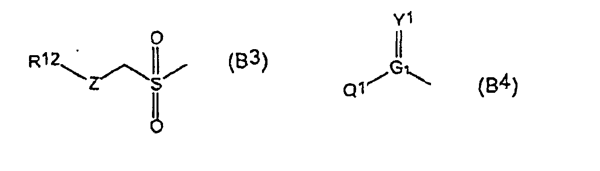
où
R⁶ représente l'atome d'hydrogène, un radical alcoyle en C₁₋₆ linéaire ou ramifié, cycloalcoyle en C₃₋₆, ou
R⁸ et R⁹ représentent avec les atomes sur lesquels ils sont liés, un cycle à 5 ou 6 membres, qui peut être le cas échéant, interrompu par l'atome de soufre et qui est le cas échéant substitué par le radical hydroxyle, t-butoxy, benzyloxy ;
R⁹ représente l'atome d'hydrogène, un radical alcoyle en C₁₋₆ linéaire ou ramifié ;
R¹⁰ représente l'atome d'hydrogène ou le radical méthyle ; 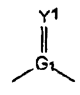 représente le radical carbonyle, -C=CH-NO₂, -C=CH-CN, -C=N-R¹¹, sulfonyle,
R¹¹ représente un radical halogéno(alcoyl en C₁₋₄)carbonyle, (alcoyl en C₁₋₄)sulfonyle, nitro ou cyano, et
Q¹ représente un reste du groupe G¹ et G² : 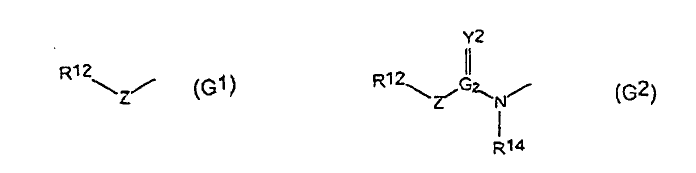 où 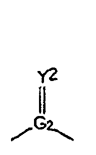 peut représenter carbonyle ou sulfonyle ;
Z représente l'atome d'oxygène ou -NR¹³ ;
R¹², dans le cas où Z représente l'atome d'azote, représente un radical amino cyclique lié par l'atome d'azote, qui peut être le cas échéant, substitué par des restes de la série des atomes d'halogène, des radicaux alcoyle en C₁₋₄, hydroxyalcoyle en C₁₋₄, aminoalcoyle en C₁₋₄, (alcoyl en C₁₋₄)-aminoalcoyle en C₁₋₄, di (alcoyl en C₁₋₄)aminoalcoyle en C₁₋₄, amino, hydroxyle, alcoxy en C₁₋₄, (alcoyl en C₁₋₄)-carbonyle, (alcoxy en C₁₋₄)carbonyle, ou
R¹² et R¹³ représentent indépendamment l'un de l'autre, l'atome d'hydrogène, un radical alcoyle en C₁₋₆ linéaire ou ramifié, alcényle en C₂₋₆, alcynyle en C₂₋₆, cycloalcoyle en C₃₋₆, pyridylméthyle et thiazolylméthyle, qui peuvent être le cas échéant, substitués par des restes de la série des atomes d'halogène, des radicaux alcoyle en C₁₋₄, hhydroxyalcoyle en C₁₋₄, aminoalcoyle en C₁₋₄, (alcoyl en C₁₋₄)aminoalcoyle en C₁₋₄, di(alcoyl en C₁₋₄)aminoalcoyle en C₁₋₄, amino, hydroxyle, alcoxy en C₁₋₄, (alcoyl en C₁₋₄)carbonyle, (alcoxy en C₁₋₄)carbonyle, ou
R¹² et R¹³ représentent ensemble, avec l'atome d'azote voisin, un système cyclique à 5, 6 ou 7 membres ou un système bicyclique de 7 à 10 membres, qui peut être le cas échéant, interrompu par l'atome d'oxygène, de soufre, le radical sulfoxyle, sulfonyle, carbonyle, -N-O-, -N=, -NR¹⁵- ou par un atome d'azote quaternaire, et qui peut être le cas échéant, substitué par des radicaux alcoyle en C₁₋₄, hydroxyalcoyle en C₁₋₄, aminoalcoyle en C₁₋₄, (alcoyl en C₁₋₄)aminoalcoyle en C₁₋₄, di (alcoyl en C₁₋₄)aminoalcoyle en C₁₋₄, amino, hydroxyle, alcoxy en C₁₋₄, (alcoyl en C₁₋₄)carbonyle, (alcoxy en C₁₋₄)carbonyle, l'atome d'halogène ;
R¹⁴ représente l'atome d'hydrogène ou un radical alcoyle en C₁₋₄ ;
R¹⁵ représente l'atome d'hydrogène, un radical alcoyle en C₁₋₆ linéaire ou ramifié, alcényle en C₂₋₆, alcynyle en C₂₋₆, cycloalcoyle en C₃₋₆, (alcoxy en C₁₋₄)carbonyle, (alcoyl en C₁₋₄)carbonyle, (cycloalcoyl en C₃₋₆)carbonyle, cyano, aryle, aryl (alcoyle en C₁₋₂), pyridyle et thiazolyle, pyridylméthyle, thiazolylméthyle, qui peuvent être le cas échéant substitués, par des restes de la série des atomes d'halogène, des radicaux alcoyle en C₁₋₄, halogénoalcoyle en C₁₋₄, amino, hydroxyle, cyano, alcoxy en C₁₋₄, alcoylènedioxy en C₁₋₂, halogénoalcoxy en C₁₋₄, alcoylthio en C₁₋₄, halogénoalcoylthio en C₁₋₄, alcoylsulfonyle en C₁₋₄, alcoylamino en C₁₋₄, di (alcoyl en C₁₋₄)amino, (alcoyl en C₁₋₄)carbonyle, (alcoxy en C₁₋₄)carbonyle ;
ainsi que leurs isomères optiques et racémates,
où « aryle » dans les définitions, représente un reste aromatique mono-, bi- ou polyvalent, choisi parmi phényle, naphtyle, tétrahydronaphtyle, indanyle et fluorényle, et
où, sauf indication contraire, les restes le cas échéant substitués, peuvent porter un ou plusieurs substituants identiques ou différents, choisis parmi : un radical alcoyle ayant 1 à 4 atomes de carbone, alcoxy ayant 1 à 4 atomes de carbone, alcoylthio, halogénoalcoyle ayant 1 à 5 atomes d'halogène, où les atomes d'halogène sont identiques ou différents, un atome d'halogène, le radical cyano, nitro, amino, des radicaux monoalcoyl- et dialcoylamino ayant 1 à 4 atomes de carbone par radical alcoyle, un reste alcoylcarbonyle, alcoxycarbonyle ayant 2 à 4 atomes de carbone, alcoylsulfinyle ayant 1 à 4 atomes de carbone, halogénoalcoylsulfinyle ayant 1 à 4 atomes de carbone et 1 à 5 atomes d'halogène, halogénoalcoylsulfonyle ayant 1 à 4 atomes de carbone et 1 à 5 atomes d'halogène, arylsulfonyle ayant 6 à 10 atomes de carbone, trialcoylsilyle ayant 1 à 4 atomes de carbone, acyle, aryle, aryloxy, qui peuvent porter pour leur part, un des substituants indiqués ci-dessus, ainsi que le reste formimino (-HC=N-O-alcoyle).

2. Procédé de préparation des dérivés de 6-hydroxy-1,2,4a,5a,8a,8b-hexahydro-6H-pyrrolo[3',4':4,5]furo[3,2-b]pyrid-8(7H)-one et de 6-hydroxy-1,2,3,4,4a,5a,8a,8b-octahydro-6H-pyrrolo[3',4':4,5]-furo[3,2-b]pyrid-8(7H)-one des formules générales (Ia) et (Ib), ainsi que de leurs sels selon la revendication 1 : 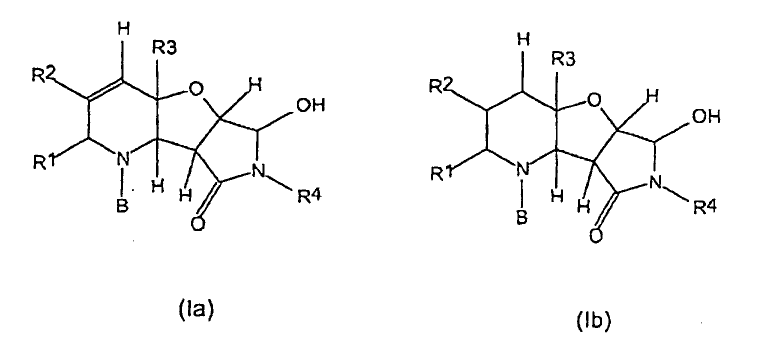 dans lesquelles :
R¹, R², R³, R⁴ et B possèdent la signification indiquée à la revendication 1, **caractérisé en ce que** l'on réalise l'hydrogénation des dérivés de 1,2,4a,5a,8a,8b-hexahydro-6H-pyrrolo[3',4':4,5]furo[3,2-b]pyridin-6,8(7H)-dione et/ou de 1,2,3,4,4a,5a,8a,8b-octahydro-6H-pyrrolo[3',4':4,5]furo[3,2-b]pyridin-6,8(7H)-dione des formules générales (IIa) et (IIb), ainsi que de leurs sels : 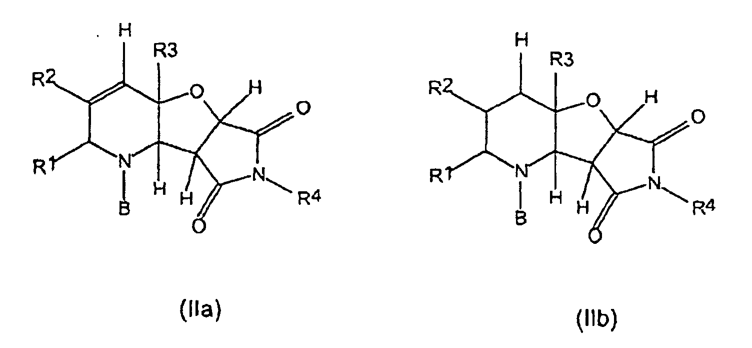
dans lesquelles :
R¹, R², R³, R⁴ et B possèdent la signification indiquée à la revendication 1,
en présence d'un agent de dilution approprié,
ou **en ce que** l'on réalise, dans une première étape de réaction, l'hydrogénation des dérivés de 1,2,4a,5a,8a,8b-hexahydro-6H-pyrrolo[3',4':4,5]furo[3,2-b]pyridin-6,8(7H)-dione et/ou de 1,2,3,4,4a,5a,8a,8b-octahydro-6H-pyrrolo[3',4':4,5]furo[3,2-b]pyridin-6,8(7H)-dione des formules générales (IIc) et (IId), ainsi que de leurs sels : 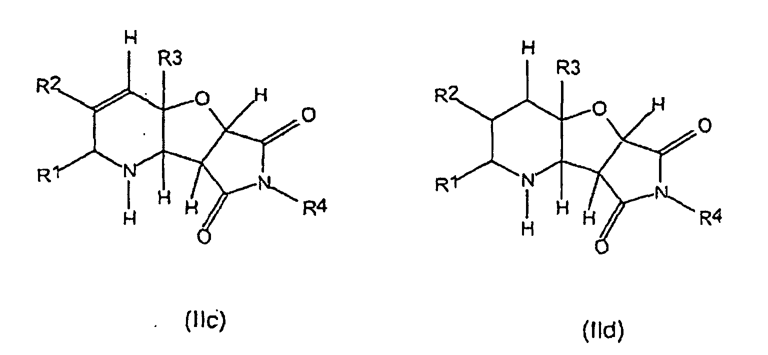 dans lesquelles :
R¹, R², R³ et R⁶ possèdent la signification indiquée à la revendication 1,
en présence d'un agent de dilution approprié, en les dérivés de 6-hydroxy-1,2,4a,5a,8a,8b-hexahydro-6H-pyrrolo[3',4':4,5]furo[3,2-b]pyrid-8(7H) -one et/ou de 6-hydroxy-1,2,3,4,4a,5a,8a,8b-octahydro-6H-pyrrolo[3',4':4,5]furo[3,2-b]pyrid-8(7H)-one des formules générales (Ic) et (Id) : 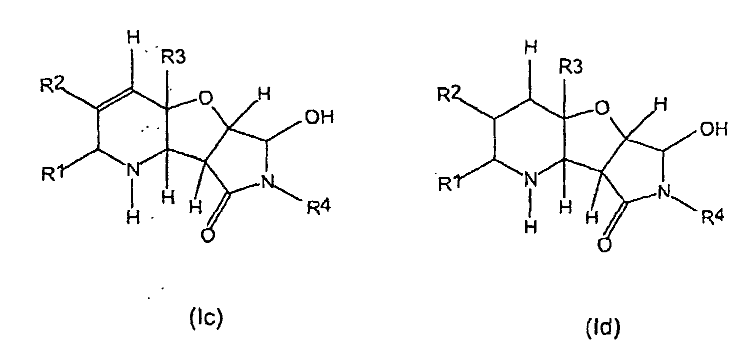 dans lesquelles :
R¹, R², R³ et R⁴ possèdent la signification indiquée à la revendication 1,
ou **en ce que**, pour la préparation sélective de nouveaux dérivés de la formule générale (IIa) et de leurs sels, dans une première étape de réaction, on réalise l'hydrogénation du dérivé de 4a,5a,8a,8b-tétrahydro-6H-pyrrolo[3',4':4,5]furo[3,2-b]pyrid-6,8(7H)-dione de la formule générale (III) et de ses sels : 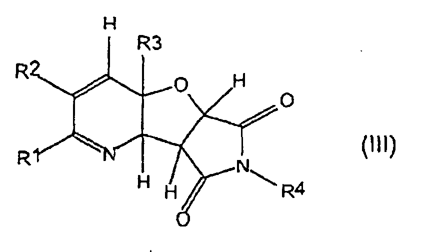 dans laquelle :
R¹, R², R³ et R⁴ possèdent la signification indiquée à la revendication 1,
en présence d'un agent de dilution approprié, en le dérivé de 6-hydroxy-1,2,4a,5a,8a,8b-hexahydro-6H-pyrrolo[3',4':4,5]furo[3,2-b]pyrid-8 (7H)-one de la formule générale (Ic) : 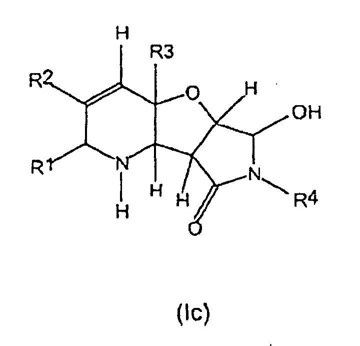 dans laquelle :
R¹, R², R³ et R⁴ possèdent la signification indiquée à la revendication 1,
et ensuite, dans une deuxième étape de réaction, on fait réagir les dérivés obtenus de cette manière, des formules générales (Ic) et (Id) : 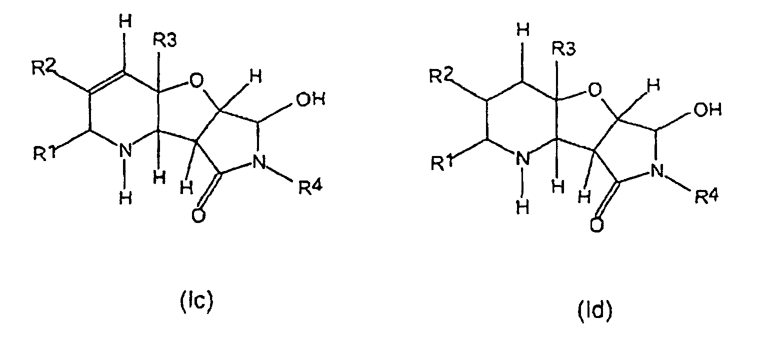 dans lesquelles :
R¹, R², R³ et R⁴ possèdent la signification indiquée à la revendication 1,
a) avec des composés de la formule générale (IV) :
B - E (IV)
dans laquelle:
B a la signification indiquée ci-dessus, et
E représente un groupe partant électrocapteur,
le cas échéant en présence d'agents de dilution et le cas échéant, en présence d'un auxiliaire de réaction basique, ou
b) avec des composés de la formule générale (V) : 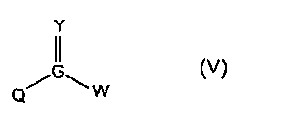 dans laquelle : 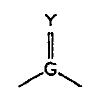
et Q ont les significations indiquées à la revendication 1, et
W représente un groupe partant approprié, comme par exemple un atome d'halogène, un radical alcoxy, alcoylthio ou aryloxy,
le cas échéant en présence d'un catalyseur, le cas échéant en présence d'un auxiliaire de réaction basique et le cas échéant, en présence d'agents de dilution, ou **en ce que** pour la préparation des nouveaux dérivés de 6-hydroxy-1,2,4a,5a,8a,8b-hexahydro-6H-pyrrolo[3',4':4,5]furo[3,2-b]pyrid-(7H)-one et/ou de 6-hydroxy-1,2,3,4,4a,5a,8a,8b-octahydro-6H-pyrrolo[3',4':4,5]furo[3,2-b]pyrid-(7H)-one des formules générales (Ia) et (Ib) et de leurs sels
dans lesquelles le radical 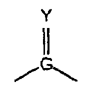 représente carbonyle, on fait réagir les composés de la formule Ic ou Id
c) avec un anhydride d'acide carboxylique de la formule générale (VI) :
(Q-C=O)₂O (VI)
dans laquelle :
Q a la signification indiquée à la revendication 1,
le cas échéant en présence d'un catalyseur, le cas échéant en présence d'agents de dilution, ou **en ce que** l'on fait réagir les composés des formules (Ic) ou (Id)
d) avec des dérivés d'acide aminé de la formule générale (VII) : 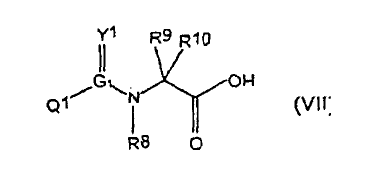 dans laquelle : 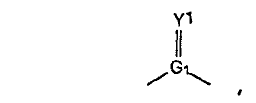 Q¹, R⁸, R⁹ et R¹⁰ ont les significations indiquées la revendication 1,
le cas échéant en présence d'un réactif de couplage et en présence d'un auxiliaire de réaction basique, le cas échéant, en présence d'agents de dilution, ou **en ce que** l'on fait réagir les composés des formules (Ic) ou (Id)
e) avec des composés des formules générales (VIII) ou (IX) :
R¹²―N=C=Y (VIII) 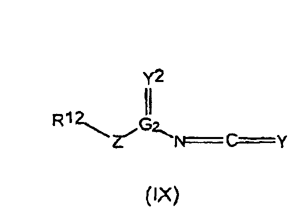
où le: 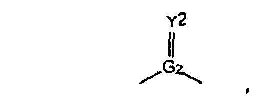 Z et R¹² ont les significations indiquées dans la revendication 1,
Y représente l'atome d'oxygène ou de soufre,
le cas échéant en présence d'un catalyseur, le cas échéant, en présence d'agents de dilution.

3. Procédé de préparation des dérivés de 1,2,4a,5a,8a,8b-hexahydro-6H-pyrrolo[3',4':4,5]furo[3,2-b]pyrid-8(7H)-one substitués en 6 et/ou de 1,2,3,4,4a,5a,5a,8a,8b-octahydro-6H-pyrrolo[3',4':4,5]-furo[3,2-b]pyrid-8(7H)-one substitués en 6 des formules générales (Ie) et (If), ainsi que de leurs sels selon la revendication 1 : 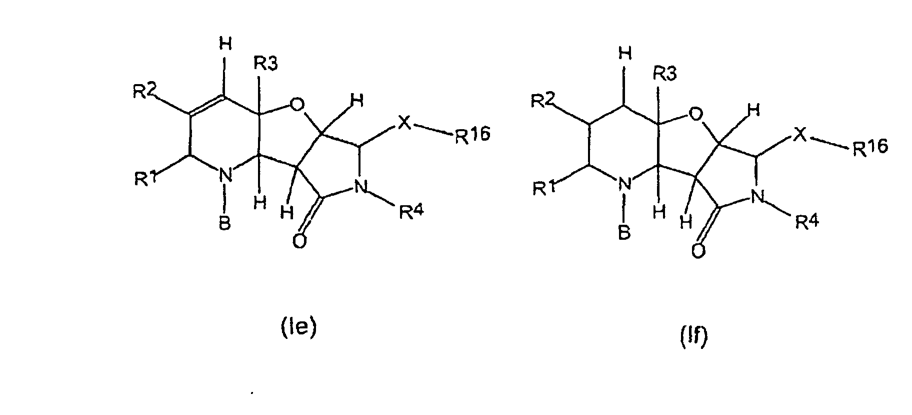 dans lesquelles :
R¹, R², R³, R⁴ et B possèdent la signification indiquée à la revendication 1,
R¹⁶ représente un radical alcoyle en C₁₋₆, alcényle en C₂₋₆, alcynyle en C₂₋₆, aryl(alcoyle en C₁₋₂), qui peuvent être le cas échéant substitués par des restes de la série des atomes d'halogène, des radicaux alcoyle en C₁₋₄, halogénoalcoyle en C₁₋₄, amino, hydroxyle, nitro, cyano, alcoxy en C₁₋₄, alcoylènedioxy en C₁₋₂, halogénoalcoxy en C₁₋₄, alcoylthio en C₁₋₄, halogénoalcoylthio en C₁₋₄, alcoylsulfonyle en C₁₋₄, alcoylamino en C₁₋₄, di (alcoyl en C₁₋₄)amino, ou représente le cas échéant, le radical 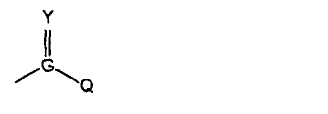
dans lequel
G, Y et Q ont la signification indiquée à la revendication 1,
ainsi que leurs isomères optiques et racémates,
**caractérisé en ce que** l'on fait réagir
a) les dérivés obtenus selon la revendication 2 des formules générales (Ia) et (Ib), ainsi que leurs sels : 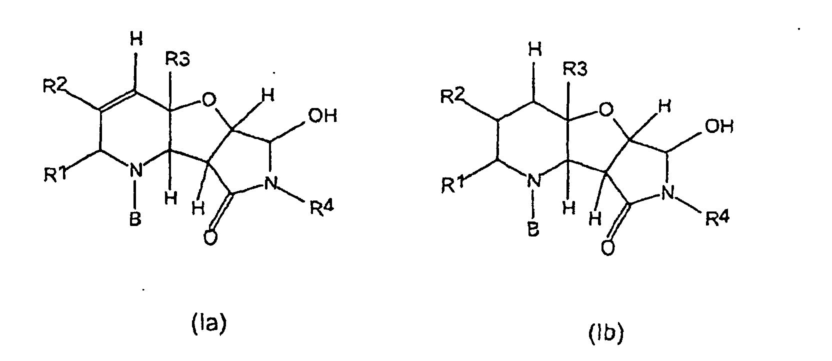 dans lesquelles :
R¹, R², R³, R⁴ et B possèdent la signification indiquée à la revendication 1,
avec des composés de la formule générale (X) :
H - X - R¹⁶ (X)
dans laquelle :
R¹⁶ et X possèdent la signification indiquée ci-dessus,
le cas échéant en présence d'un acide, le cas échéant en présence d'agents de dilution, ou **en ce que**,
b) pour la préparation des dérivés de 1,2,4a,5a,8a,8b-hexahydro-6H-pyrrolo[3',4':4,5]furo[3,2-b]pyrid-8(7H)-one substitués en 6 et/ou de 1,2,3,4,4a,5a,8a,8b-octahydro-6H-pyrrolo[3',4':4,5]-furo[3,2-b]pyrid-8(7H)-one substitués en 6 des formules générales (Ie) et (If), ainsi que de leurs sels,
dans lesquelles le reste R¹⁶ représente le radical 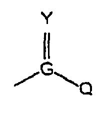 dans lequel 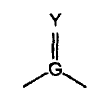 et Q ont la signification indiquée à la revendication 1,
avec des composés de la formule générale (V) : 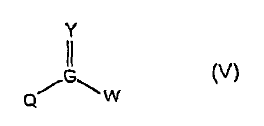 dans laquelle 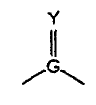 et Q ont la signification indiquée à la revendication 1, et
W représente un groupe partant approprié,
le cas échéant en présence d'un catalyseur, le cas échéant en présence d'un auxiliaire de réaction basique et le cas échéant en présence d'agents de dilution, ou **en ce que**,
c) pour la préparation des dérivés des formules générales (Ie) et (If), ainsi que de leurs sels,
dans lesquelles le radical 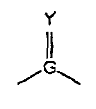 représente carbonyle,
on fait réagir avec un anhydride d'acide carboxylique de la formule générale (VI)
(Q-C=O)₂O (VI)
dans laquelle :
Q a la signification indiquée à la revendication 1,
le cas échéant en présence d'un catalyseur, le cas échéant en présence d'agents de dilution, ou **en ce que** l'on fait réagir
d) avec des composés des formules générales (VIII) ou (IX) :
R¹²―N=C=Y (VIII) 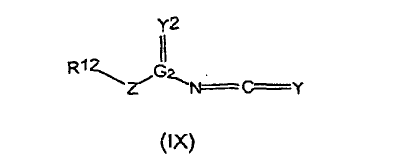
dans lesquelles 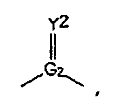 Z, Y et R¹² ont la signification indiquée à la revendication 1,
le cas échéant en présence d'un catalyseur, le cas échéant en présence d'agents de dilution, ou **en ce que**,
e) pour la préparation des dérivés de 1,2,4a,5a,8a,8b-hexahydro-6H-pyrrolo[3',4':4,5]furo[3,2-b]pyrid-8(7H)-one substitués en 6 et/ou de 1,2,3,4,4a,5a,8a,8b-octahydro-6H-pyrrolo[3',4':4,5]-furo[3,2-b]pyrid-8(7H)-one substitués en 6 des formules générales (Ie) et (If), ainsi que de leurs sels, 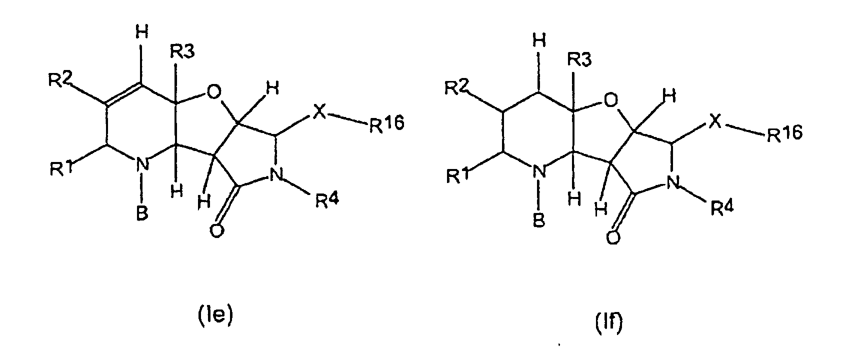 dans lesquelles
R¹, R², R³, R⁴, X et B possèdent la signification indiquée à la revendication 1, et les restes B et R¹⁶ représentent le même radical 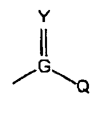 dans lequel 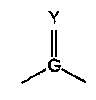 et Q ont la signification indiquée à la revendication 1,
on fait réagir des dérivés des formules générales (Ic) et (Id) : 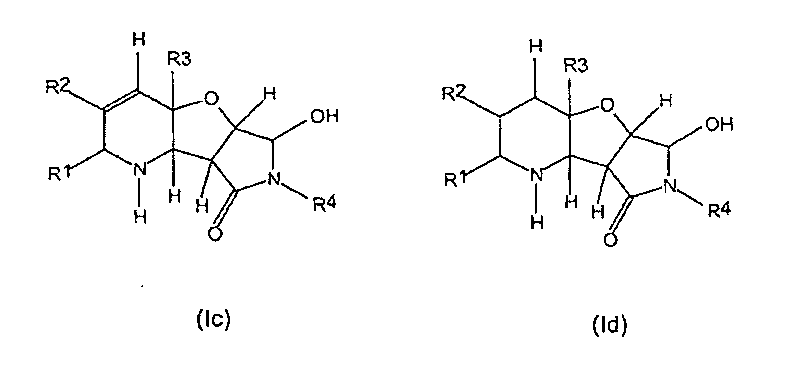 dans lesquelles
R¹, R², R³, R⁴ possèdent la signification indiquée à la revendication 1,
avec des agents d'acylation de la formule (IV) :
E - B (IV)
ou
E - R¹⁶
soit sur le reste -NH- en position 1 ou sur le reste -OH en position 6 ou sur les deux restes, où E-B ou E-R¹⁶ représentent l'un des composés suivants des formules générales (V), (VI), (VIII) ou (IX) : 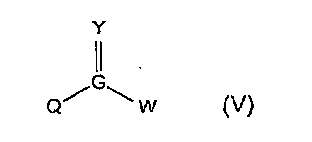
(Q―C=O)₂O (VI)
R¹²―N=C=Y (VIII) 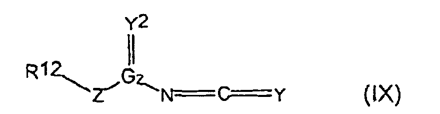
dans lesquelles 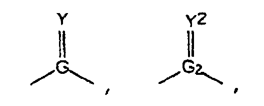 Q, Z, W et R¹² ont la signification indiquée à la revendication 1,
la réaction étant réalisée le cas échéant en présence d'un catalyseur, le cas échéant en présence d'un auxiliaire de réaction basique et le cas échéant en présence d'agents de dilution, ou **en ce que**,
f) pour la préparation des dérivés de 1,2,4a,5a,8a,8b-hexahydro-6H-pyrrolo[3',4':4,5]furo[3,2-b]pyrid-8(7H)-one substitués en 6 et/ou de 1,2,3,4,4a,5a,8a,8b-octahydro-6H-pyrrolo[3',4':4,5]-furo[3,2-b]pyrid-8(7H)-one substitués en 6 des formules générales (Ie) et (If), ainsi que de leurs sels, 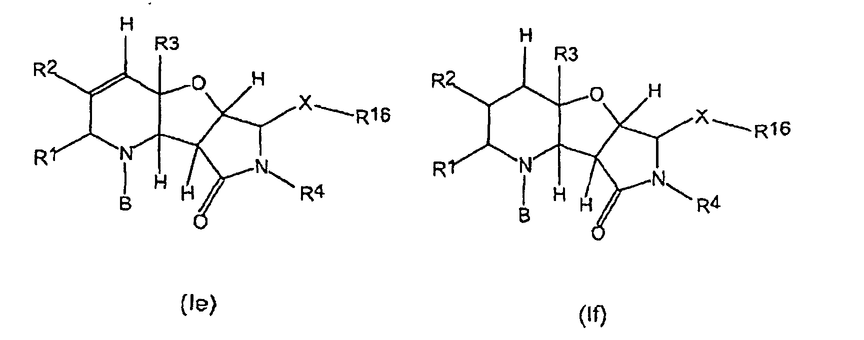 dans lesquelles
R¹, R², R³, R⁴, X et B possèdent la signification indiquée à la revendication 1, et le reste R¹⁶ représente le radical 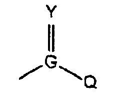 dans lequel 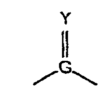 et Q ont la signification indiquée à la revendication 1,
on fait réagir des dérivés des formules générales (Ic) et (Id) : 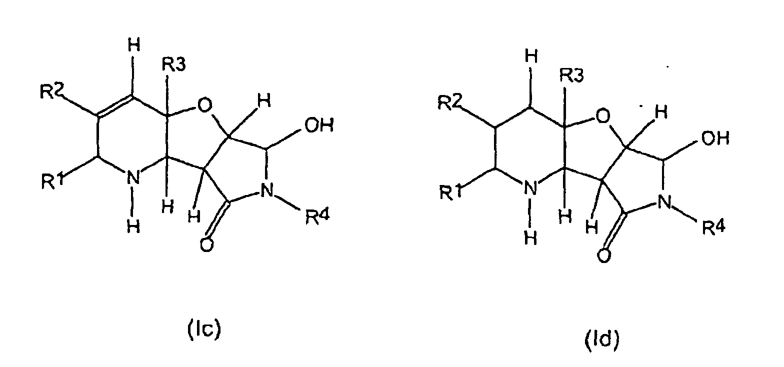 dans lesquelles
R¹, R², R³, R⁴ possèdent la signification indiquée à la revendication 1,
avec un agent d'acylation de la formule (IV) :
E - R¹⁶ (IV)
sur le reste -OH en position 6, où E - R¹⁶ représente le composé de la formule générale (VII) : 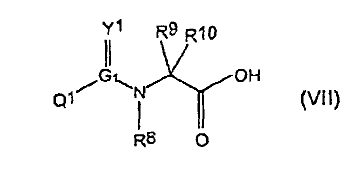 dans laquelle 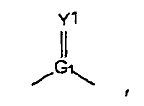 Q¹, R⁸, R⁹ et R¹⁰ ont la signification indiquée à la revendication 1,
et ensuite dans une deuxième étape de réaction, les dérivés obtenus de cette manière des formules générales (Ig) et (Ih) : 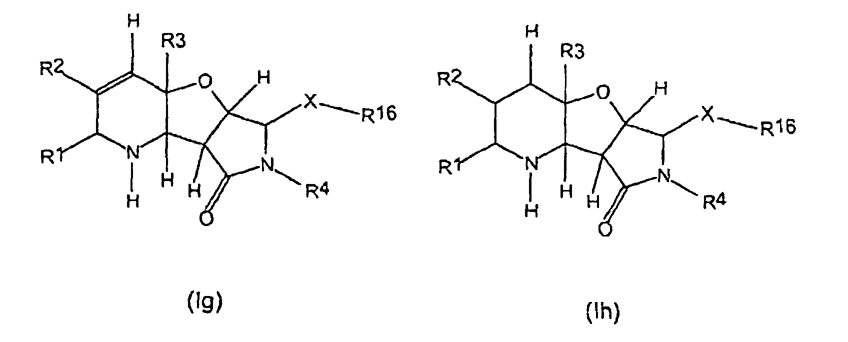 dans lesquelles
R¹, R², R³, R⁴ et X possèdent la signification indiquée à la revendication 1, et le reste R¹⁶ représente le radical 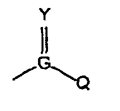 dans lequel 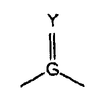 et Q ont la signification indiquée à la revendication 1,
sont mis à réagir avec un agent d'acylation de la formule (IV) :
E - B (IV)
sur le reste -NH en position 1, où E - B représente l'un des composés cités à la revendication 3e des formules générales (V), (VI), (VII) ou (IX),
dans lesquelles 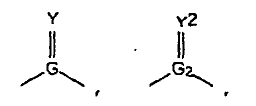 Q, Z, W et R¹² ont la signification indiquée aux revendications 1 et 2,
la réaction étant réalisée le cas échéant en présence d'un catalyseur, le cas échéant en présence d'un auxiliaire de réaction basique et le cas échéant en présence d'agents de dilution.

4. Procédé de préparation des dérivés de 1,2,4a,5a,8a,8b-hexahydro-6H-pyrrolo[3',4':4,5]furo[3,2-b]pyrid-8(7H)-one substitués en 6 et/ou de 1,2,3,4,4a,5a,8a,8b-octahydro-6H-pyrrolo[3',4':4,5]-furo[3,2-b]pyrid-8(7H)-one substitués en 6 des formules générales (Ii) et (Ij), ainsi que de leurs sels selon la revendication 1 : 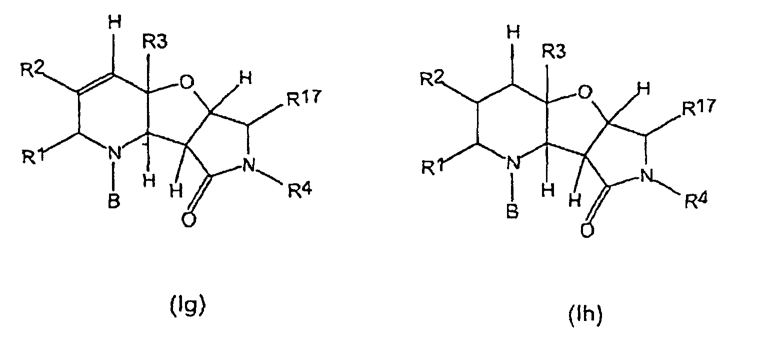 dans lesquelles :
R¹, R², R³, R⁴ et B possèdent la signification indiquée à la revendication 1,
R¹⁷ représente un radical alcoyle en C₁₋₆, alcényle en C₂₋₆, alcynyle en C₂₋₆, furyle, cyano, alcoxycarbonyle en C₁₋₄, carbamoyle, thiocarbamoyle, qui peuvent être le cas échéant substitués par des restes de la série des atomes d'halogène, des radicaux alcoyle en C₁₋₄, halogénoalcoyle en C₁₋₄, amino, hydroxyle, nitro, cyano, alcoxy en C₁₋₄, alcoylènedioxy en C₁₋₂, halogénoalcoxy en C₁₋₄, alcoylthio en C₁₋₄, halogénoalcoylthio en C₁₋₄, alcoylsulfonyle en C₁₋₄, alcoylamino en C₁₋₄, di (alcoyl en C₁₋₄)amino, ou le cas échéant, représente un reste parmi le groupe A² : 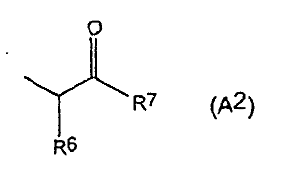
dans laquelle
R⁶ et R⁷ possèdent la signification indiquée ci-dessus à la revendication 1,
ainsi que de leurs isomères optiques et racémates,
**caractérisé en ce que** l'on fait réagir
les dérivés obtenus par exemple selon la revendication 2, des formules générales (Ia) et (Ib) : 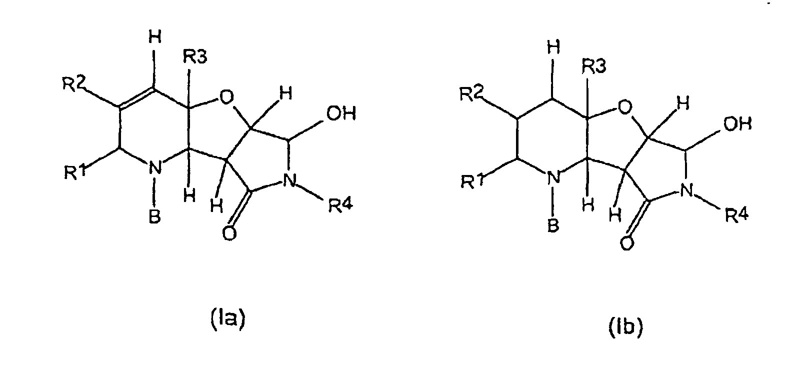 dans lesquelles :
R¹, R², R³, R⁴ et B possèdent la signification indiquée à la revendication 1, ou de manière particulièrement préférée,
les dérivés obtenus par exemple selon la revendication 3, des formules générales (Ie) et (If) : 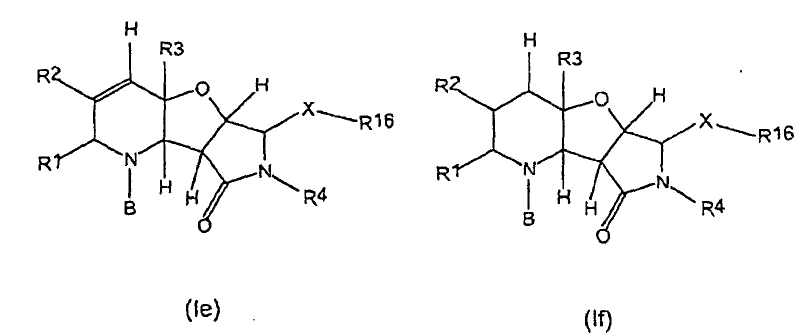 dans lesquelles :
R¹, R², R³, R⁴ et B possèdent la signification indiquée à la revendication 3, et
R¹⁶ représente un radical alcoyle en C₁₋₆, aryl (alcoyle en C₁₋₂), qui sont le cas échéant substitués,
X représentent l'atome d'oxygène, de soufre ou le radical sulfonyle,
a) avec des composés métallo-organique de la formule générale (XI) :
(R¹⁸)₃M - R¹⁷ (XI)
dans laquelle :
R¹⁹ représente un radical alcoyle en C₁₋₄,
M représente un atome métallique, en particulier l'atome de silicium ou d'étain,
R¹⁷ représente un radical alcoyle en C₁₋₆, alcényle en C₂₋₆, alcynyle en C₂₋₆, qui peuvent être le cas échéant substitués par des restes de la série des atomes d'halogène, des radicaux alcoyle en C₁₋₄, halogénoalcoyle en C₁₋₄, amino, hydroxyle, nitro, cyano, alcoxy en C₁₋₄, alcoylènedioxy en C₁₋₂, halogénoalcoxy en C₁₋₄, alcoylthio en C₁₋₄, halogénoalcoylthio en C₁₋₄, alcoylsulfonyle en C₁₋₄, alcoylamino en C₁₋₄, di (alcoyl en C₁₋₄)amino, ou représente le radical cyano,
le cas échéant en présence d'agents de dilution et le cas échéant, en présence d'un catalyseur, ou
b) avec des composés de la formule générale (XII) : 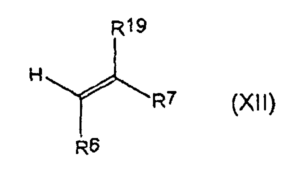 dans laquelle
R⁶ et R⁷ possèdent la signification indiquée ci-dessus,
R¹⁹ représente l'atome d'hydrogène, un radical -O-acyle, -O-Sn-O-SO₂-CF₃, -O-B(CH₂-CH₃)₂, ou représente le reste -O-M(R¹⁸)₃ et -NR²⁰R²¹,
où
M et R¹⁸ possèdent la signification indiquée, et
R²⁰ et R²¹ représentent indépendamment l'un de l'autre, l'atome d'hydrogène, un radical alcoyle, alcényle, alcynyle, cycloalcoyle, cycloalcoylalcoyle, arylalcoyle linéaire ou ramifié, qui sont le cas échéant substitués, ou
R²⁰ et R²¹ avec l'atome N voisin, représentent un système carbocyclique ayant 5, 6 ou 7 membres, qui peut être également, le cas échéant interrompu par l'atome d'oxygène, de soufre ou d'azote et qui est le cas échéant, substitué,
le cas échéant en présence d'un catalyseur et le cas échéant, en présence d'agents de dilution, ou
c) avec des aromatiques ou hétéroaromatiques de la formule générale (XIII) :
H - R¹⁷ (XIII)
où
R¹⁷ représente le radical furyle, qui peut être le cas échéant substitué par des restes de la série des atomes d'halogène, des radicaux alcoyle en C₁₋₄, halogénoalcoyle en C₁₋₄, amino, hydroxyle, nitro, cyano, alcoxy en C₁₋₄, alcoylènedioxy en C₁₋₂, halogénoalcoxy en C₁₋₄, alcoylthio en C₁₋₄, halogénoalcoylthio en C₁₋₄, alcoylsulfonyle en C₁₋₄, alcoylamino en C₁₋₄, di (alcoyl en C₁₋₄)amino,
le cas échéant, en présence d'un catalyseur et le cas échéant, en présence d'agents de dilution.

5. Agent endoparasiticide, **caractérisé par** une teneur en au moins un dérivé de 1,2,4a,5a,8a,8b-hexahydro- et de 1,2,3,4,4a,5a,8a,8b-octahydro-6H-pyrrolo[3',4':4,5]furo[3,2-b]pyrid-8(7H)-one substitué en 6, de la formule (I), selon la revendication 1.

6. Procédé de préparation d'agents endoparasiticides, **caractérisé en ce que** l'on mélange des dérivés de 1,2,4a,5a,8a,8b-hexahydro- et de 1,2,3,4,4a,5a,8a,8b-octahydro-6H-pyrrolo[3',4':4,5]-furo[3,2-b]pyrid-8(7H)-one substitués en 6, de la formule (I), selon la revendication 1 avec des diluants et/ou des agents tensioactifs.

7. Utilisation des dérivés de 1,2,4a,5a,8a,8b-hexahydro- et de 1,2,3,4,4a,5a,8a,8b-octahydro-6H-pyrrolo[3',4':4,5]furo[3,2-b]pyrid-8(7H)-one substitués en 6, de la formule (I), selon la revendication 1 pour la préparation d'agents endoparasiticides.

8. Utilisation des dérivés de 1,2,4a,5a,8a,8b-hexahydro- et de 1,2,3,4,4a,5a,8a,8b-octahydro-6H-pyrrolo[3',4':4,5]furo[3,2-b]pyrid-8(7H)-one pour la préparation d'agents pour lutter contre les Cestodes, les Trématodes, les Nématodes, les Acantocéphales chez l'homme et les animaux.
